# EUROPEAN PATENT APPLICATION

(11) **EP 1 947 194 A1**
(43) Date of publication of application: **23.07.2008**
(21) Application number: 06743492.8
(22) Date of filing: 08.05.2006
(51) Int. Cl.: C12Q 1/68

(54) **METHOD AND DEVICE FOR THE IN VITRO ANALYSIS OF MRNA OF GENES INVOLVED IN HAEMATOLOGICAL NEOPLASIAS**

(30) Priority: 27.10.2005 ES 200502618
(71) Applicant: Fundación para el Estudio de la Hematología y Hemoterapia de Aragón (FEHHA), 50008 Zaragoza (ES)
(72) Inventor: GIRALDO CASTELLANO, Pilar, E-50008 Zaragoza (ES); ÁLVAREZ CABEZA, Patricia, E-50008 Zaragoza (ES); SÁENZ JIMÉNEZ, Pilar, E-48160 Vizcaya (ES); MARTÍNEZ MARTÍNEZ, Antonio, E-48160 Vizcaya (ES); POCOVÍ MIERAS, Miguel, 50009 Zaragoza (ES); SIMÓN BUELA, Laureano, 50008-ZARAGOZA (ES)
(74) Representative: Illescas, Manuel
(86) International application number: PCT/ES2006/070054
(87) International publication number: WO 2007/048866

(57) **Abstract**

Method and device for the in vitro analysis of mRNA of genes involved in hematological neoplasias. The device, composed of probes which specifically hybridize with genes involved in hematological neoplasias, designed so that its behaviour in the hybridization is similar, permits the evaluation of the mRNA level in biological samples taken from subjects suspected to be suffering from hematological neoplasia and facilitating the comparison between the different samples and their grouping by similarity in the gene expression patterns, especially when the probes are disposed in the form of microarray. The application of the method of the invention to obtain and process data of gene expression differences from the device of the invention permits the identification of genes significant for distinguishing samples associated to hematological neoplasias, facilitates the diagnosis of neoplasias as CLL and permits making a prognosis of the evolution thereof.

LIST OF SEQUENCES

<110> FOUNDATION FOR THE STUDY OF HEMATOLOGY AND HEMOTHERAPY OF ARAGON
(FEHHA)
<120> METHOD AND DEVICE FOR THE IN VITRO ANALYSIS OF mRNA FROM GENES
INVOLVED IN HEMATOLOGICAL NEOPLASIAS

<130> EP-02097
<160> 607
<210> 1
<211> 50
<212> DNA
<213> Artificial sequence
<220>
<221> Probe
<223> SG1
<220>
<223> Designed to hybridize with cRNA of the human gene GABARAP

<400>
tccagtgatt gttttggttt ctgttccctt tctgactgcc caaggggctc 50

<210> 2
<211> 50
<212> DNA
<213> Artificial sequence
<220>
<221> Probe
<223> SG2
<220>
<223> Designed to hybridize with cRNA of the human gene IGLV6-57

<400>
ttatgatagc acctatcatg tgatattcgg cggagggacc aagctgaccg 50

<210> 3
<211> 55
<212> DNA
<213> Artificial sequence
<220>
<221> Probe
<223> SG3

<220>
<223> Designed to hybridize with cRNA of the human gene PCD

<400>
gccgtctctt gctccctaat tcatttccca ggaagctgtg atacagggtg aaata        55


<210> 4
<211> 53
<212> DNA
<213> Artificial sequence
<220>
<221> Probe
<223> SG4
<220>
<223> Designed to hybridize with cRNA of the human gene NCALD

<400>
ccactgaccc tgtctgtatt ttctcggagg ttgtttttct ccttctcctt ccc          53


<210> 5
<211> 55
<212> DNA
<213> Artificial sequence
<220>
<221> Probe
<223> SG5
<220>
<223> Designed to hybridize with cRNA of the human gene DLAD

<400>
gagtccacac caagccttca gaagtggagg attcatttgt acccagaatt ggcaa        55


<210> 6
<211> 55
<212> DNA
<213> Artificial sequence
<220>
<221> Probe
<223> SG6
<220>

<223> Designed to hybridize with cRNA of the human gene SOSTDC1

<400>
cgcttggaat ggaatgccag aaatgcatgg cagcagctaa taagtaaagc tgatt          55


<210> 7
<211> 55
<212> DNA
<213> Artificial sequence
<220>
<221> Probe
<223> SG7
<220>
<223> Designed to hybridize with cRNA of the human gene T1A-2

<400>
ggaaagaccg ttcaccagac ttggctcctc taaacatttg ctgttcaaac atgtt          55


<210> 8
<211> 55
<212> DNA
<213> Artificial sequence
<220>
<221> Probe
<223> SG8
<220>
<223> Designed to hybridize with cRNA of the human gene PYGB

<400>
ccctgaccac cactgtgccc ctcattgtta ctgccttgtg agataaaaac tgatt          55


<210> 9
<211> 51
<212> DNA
<213> Artificial sequence
<220>
<221> Probe
<223> SG9
<220>
<223> Designed to hybridize with cRNA of the human gene LIMS1

<400>
atgggcaagc agtggcatgt ggagcatttt gtttgtgcca agtgtgagaa a          51

<210> 10
<211> 51
<212> DNA
<213> Artificial sequence
<220>
<221> Probe
<223> SG10
<220>
<223> Designed to hybridize with cRNA of the human gene ABL1

<400>
atccaaatct gtcctcgtcc tccagctgtt atctggaaga agcccttcag c          51

<210> 11
<211> 50
<212> DNA
<213> Artificial sequence
<220>
<221> Probe
<223> SG11
<220>
<223> Designed to hybridize with cRNA of the human gene DRG1

<400>
attgtgaaga agtgaaacct ttcccttttc ccatctgccg gacgaaccac          50

<210> 12
<211> 50
<212> DNA
<213> Artificial sequence
<220>
<221> Probe
<223> SG12
<220>
<223> Designed to hybridize with cRNA of the human gene BRMS1

<400>
agaagacagc cgaagtcaaa gccacatcct cttgctgatg ttggatgcag          50

<210> 13
<211> 55
<212> DNA
<213> Artificial sequence
<220>
<221> Probe
<223> SG13
<220>
<223> Designed to hybridize with cRNA of the human gene ABHD1

<400>
gaggacagaa acagctgaca agagtaccat ttggggtctc agttcactct ttcct          55

<210> 14
<211> 55
<212> DNA
<213> Artificial sequence
<220>
<221> Probe
<223> SG14
<220>
<223> Designed to hybridize with cRNA of the human gene IFRD1

<400>
tgaattcctt cgaaatgtat ttgaacttgg acccccagtg atgcttgatg ctgca          55

<210> 15
<211> 55
<212> DNA
<213> Artificial sequence
<220>
<221> Probe
<223> SG15
<220>
<223> Designed to hybridize with cRNA of the human gene RPL36A

<400>
cgttgagccc aactgcagat ctaagagaat gctggctatt aaaagatgca agcat          55

<210> 16
<211> 50
<212> DNA
<213> Artificial sequence
<220>
<221> Probe
<223> SG16
<220>
<223> Designed to hybridize with cRNA of the human gene IGFBP2

<400>
agttctgaca cacgtattta tatttggaaa gagaccagca ccgagctcgg          50

<210> 17
<211> 50
<212> DNA
<213> Artificial sequence
<220>
<221> Probe
<223> SG17
<220>
<223> Designed to hybridize with cRNA of the human gene NDRG1

<400>
gactttcctt ccctctcctg cttcctcttt tcctgctccc taacctttcg          50

<210> 18
<211> 55
<212> DNA
<213> Artificial sequence
<220>
<221> Probe
<223> SG18
<220>
<223> Designed to hybridize with cRNA of the human gene FKBP9

<400>
cgtgactcac catgcccagc cacttagttt tttcttattc ccacctttct atccc     55

<210> 19
<211> 50
<212> DNA
<213> Artificial sequence
<220>
<221> Probe
<223> SG19
<220>
<223> Designed to hybridize with cRNA of the human gene NDP52

<400>
tttttttttt gaaacagtct ctctctgtca cccaggctgg cgtgcaatgg          50

<210> 20
<211> 55
<212> DNA
<213> Artificial sequence
<220>
<221> Probe
<223> SG20
<220>
<223> Designed to hybridize with cRNA of the human gene FABP5

<400>
ggtgcattgg ttcagcatca ggagtgggat gggaaggaaa gcacaataac aagaa          55

<210> 21
<211> 55
<212> DNA
<213> Artificial sequence
<220>
<221> Probe
<223> SG21
<220>
<223> Designed to hybridize with cRNA of the human gene FARP1

<400>
acggtttcca gtactaacaa agggaataaa aatacctcac gccacaatcc agcat          55

<210> 22
<211> 46

<212> DNA
<213> Artificial sequence
<220>
<221> Probe
<223> SG22
<220>
<223> Designed to hybridize with cRNA of the human gene PGF

<400>
ggaagaggag gagagagaag cagagaccca cagactgcca cctgtg                46

<210> 23
<211> 55
<212> DNA
<213> Artificial sequence
<220>
<221> Probe
<223> SG23
<220>
<223> Designed to hybridize with cRNA of the human gene GDI2

<400>
gcctgagaag gaaatcagac cagctttgga gctcttggaa ccaattgaac agaaa      55

<210> 24
<211> 55
<212> DNA
<213> Artificial sequence
<220>
<221> Probe
<223> SG24
<220>
<223> Designed to hybridize with cRNA of the human gene TAGLN2

<400>
gagggctgcc taccatggtc tggggcttga ggaagatgag tttgttgatt taaat      55

<210> 25
<211> 55

<212> DNA
<213> Artificial sequence
<220>
<221> Probe
<223> SG25
<220>
<223> Designed to hybridize with cRNA of the human gene BLVRB

<400>
ggacatgagg agcaaaggaa gggggcaata aatgttgagc caagagcttc aaatt          55

<210> 26
<211> 50
<212> DNA
<213> Artificial sequence
<220>
<221> Probe
<223> SG26
<220>
<223> Designed to hybridize with cRNA of the human gene PSMB4

<400>
ttacaaccgg tttcaaaccg ccactgtcac cgaaaaaggt gttgaaatag          50

<210> 27
<211> 50
<212> DNA
<213> Artificial sequence
<220>
<221> Probe
<223> SG27
<220>
<223> Designed to hybridize with cRNA of the human gene TACSTD2

<400>
gaccacatat gcttgtcact gggaaagaag cctgtttcag ctgcctgaac          50

<210> 28

<211> 50
<212> DNA
<213> Artificial sequence
<220>
<221> Probe
<223> SG28
<220>
<223> Designed to hybridize with cRNA of the human gene TCEB1

<400>
acaaggttcg ctacactaac agctccaccg agattcctga attcccaatt          50


<210> 29
<211> 55
<212> DNA
<213> Artificial sequence
<220>
<221> Probe
<223> SG29
<220>
<223> Designed to hybridize with cRNA of the human gene EEF1A1

<400>
gagaatgttt tgtggacacg ttggttttct tttttgcgtg tggcagtttt agtta     55


<210> 30
<211> 55
<212> DNA
<213> Artificial sequence
<220>
<221> Probe
<223> SG30
<220>
<223> Designed to hybridize with cRNA of the human gene H3F3A

<400>
ccgcatacgt ggagaacgtg cttaagaatc cactatgatg ggaaacattt cattc     55


<210> 31
<211> 55

<212> DNA
<213> Artificial sequence
<220>
<221> Probe
<223> SG31
<220>
<223> Designed to hybridize with cRNA of the human gene ODC1

<400>
gcatttgtag cttgtacaat ggcagaatgg gccaaaagct tagtgttgtg acctg          55


<210> 32
<211> 55
<212> DNA
<213> Artificial sequence
<220>
<221> Probe
<223> SG32
<220>
<223> Designed to hybridize with cRNA of the human gene XRCC5

<400>
cccagtcacc tctgtcttca gcaccctcat aagtcgtcac taatacacag ttttg          55

<210> 33
<211> 50
<212> DNA
<213> Artificial sequence
<220>
<221> Probe
<223> SG33
<220>
<223> Designed to hybridize with cRNA of the human gene IGFBP3

<400>
ttcacttctc tgtcttctgt ttctctccca cagtgtcgcc cttccaaagg               50


<210> 34
<211> 51
<212> DNA
<213> Artificial sequence

<220>
<221> Probe
<223> SG34
<220>
<223> Designed to hybridize with cRNA of the human gene NCL

<400>
aggaggagga ggtgaccaca agccacaagg aaagaagacg aagtttgaat a          51


<210> 35
<211> 55
<212> DNA
<213> Artificial sequence
<220>
<221> Probe
<223> SG35
<220>
<223> Designed to hybridize with cRNA of the human gene S100A2

<400>
ctgccatgga tctcttgggc ccaggactgt tgatgccttt gagttttgta ttcaa       55


<210> 36
<211> 53
<212> DNA
<213> Artificial sequence
<220>
<221> Probe
<223> SG36
<220>
<223> Designed to hybridize with cRNA of the human gene RPL24

<400>
tcagctcccc gagttggtgg aaaacgctaa actggcagat tagattttta aat         53


<210> 37
<211> 55
<212> DNA
<213> Artificial sequence
<220>

<221> Probe
<223> SG37
<220>
<223> Designed to hybridize with cRNA of the human gene GJA1

<400>
cacccctaag aatggttctg tgtatgtgaa tgagcgggtg gtaattgtgg ctaaa          55


<210> 38
<211> 50
<212> DNA
<213> Artificial sequence
<220>
<221> Probe
<223> SG38
<220>
<223> Designed to hybridize with cRNA of the human gene GUSB

<400>
aatgtttgga aaacagcccg tttacttgag caagactgat accacctgcg                50


<210> 39
<211> 55
<212> DNA
<213> Artificial sequence
<220>
<221> Probe
<223> SG39
<220>
<223> Designed to hybridize with cRNA of the human gene HSD17B1

<400>
gttggagacc agccagagca acacagtgag acccccatct ctacaaaaat aaaga          55


<210> 40
<211> 50
<212> DNA
<213> Artificial sequence
<220>
<221> Probe

<223> SG40
<220>
<223> Designed to hybridize with cRNA of the human gene SFTPB

<400>
ttttcaaaaa atcagcaatt ccccagcgta gtcaagggtg gacactgcac          50


<210> 41
<211> 50
<212> DNA
<213> Artificial sequence
<220>
<221> Probe
<223> SG41
<220>
<223> Designed to hybridize with cRNA of the human gene RPL23A

<400>
attcggcctg atggagagaa gaaggcatat gttcgactgg ctcctgatta          50


<210> 42
<211> 55
<212> DNA
<213> Artificial sequence
<220>
<221> Probe
<223> SG42
<220>
<223> Designed to hybridize with cRNA of the human gene CSRP2

<400>
ggctaactcg taggaagaga gcactgtatg gtatcctttt gctttattca ccagc     55

<210> 43
<211> 55
<212> DNA
<213> Artificial sequence
<220>
<221> Probe
<223> SG43
<220>

<223> Designed to hybridize with cRNA of the human gene CXADR

<400>
tgctgttgtg tgatcaaaca tgtctctgtg tagttccagc aaatcaagct gagct          55

<210> 44
<211> 50
<212> DNA
<213> Artificial sequence
<220>
<221> Probe
<223> SG44
<220>
<223> Designed to hybridize with cRNA of the human gene EEF1G

<400>
tttggaacca acaatagcag ctccatttct ggagtctggg tcttccgagg          50

<210> 45
<211> 55
<212> DNA
<213> Artificial sequence
<220>
<221> Probe
<223> SG45
<220>
<223> Designed to hybridize with cRNA of the human gene CKS2

<400>
tgcatgagct gtattcttca cagcaacaga gctcagttaa atgcaactgg caagt          55

<210> 46
<211> 51
<212> DNA
<213> Artificial sequence
<220>
<221> Probe
<223> SG46
<220>
<223> Designed to hybridize with cRNA of the human gene COL4A6

<400>
attaatggca tatggtccta gacaaaccat ctcctccttg ccggctcccc c          51

<210> 47
<211> 55
<212> DNA
<213> Artificial sequence
<220>
<221> Probe
<223> SG47
<220>
<223> Designed to hybridize with cRNA of the human gene CRYAB

<400>
gctgtcaccg cagcccccaa gaaatagatg ccctttcttg aattgcattt tttaa      55

<210> 48
<211> 55
<212> DNA
<213> Artificial sequence
<220>
<221> Probe
<223> SG48
<220>
<223> Designed to hybridize with cRNA of the human gene CYC1

<400>
gctctagctc tgggccctcc ttcagccccc atcatgggaa taaattaatt ttctc      55

<210> 49
<211> 50
<212> DNA
<213> Artificial sequence
<220>
<221> Probe
<223> SG49
<220>
<223> Designed to hybridize with cRNA of the human gene FNTB

<400>

tcactcctcc ctatcatgta ccgtgaaaac cccctctgat ggcctcaagg 50

<210> 50
<211> 50
<212> DNA
<213> Artificial sequence
<220>
<221> Probe
<223> SG50
<220>
<223> Designed to hybridize with cRNA of the human gene GOT1

<400>
aaagatcaag ttgtctgaag gagccaaagt gtgaatgtgg gtgtcggctg 50

<210> 51
<211> 46
<212> DNA
<213> Artificial sequence
<220>
<221> Probe
<223> SG51
<220>
<223> Designed to hybridize with cRNA of the human gene LGALS7

<400>
cttggttcct cccaatgcca gcaggttcca tgtaaacctg ctgtgc 46

<210> 52
<211> 50
<212> DNA
<213> Artificial sequence
<220>
<221> Probe
<223> SG52
<220>
<223> Designed to hybridize with cRNA of the human gene SERPINB5

<400>
ttgatagctg tcccatctgg tcatgtggtt ggcactagac tggtggcagg 50

<210> 53
<211> 50
<212> DNA
<213> Artificial sequence
<220>
<221> Probe
<223> SG53
<220>
<223> Designed to hybridize with cRNA of the human gene PSMC5

<400>
agaaaaattg ctgagctcat gccaggagca tcaggggctg aagtgaaggg          50

<210> 54
<211> 55
<212> DNA
<213> Artificial sequence
<220>
<221> Probe
<223> SG54
<220>
<223> Designed to hybridize with cRNA of the human gene PTN

<400>
ccctgtggca aactgaccaa gcccaaacct caagcagaat ctaagaagaa gaaaa     55

<210> 55
<211> 55
<212> DNA
<213> Artificial sequence
<220>
<221> Probe
<223> SG55
<220>
<223> Designed to hybridize with cRNA of the human gene RBBP8

<400>
ggcaaggagc agaagacata gacgttgaaa cagaaacaga aggatgaagg acagt     55

<210> 56
<211> 55
<212> DNA
<213> Artificial sequence
<220>
<221> Probe
<223> SG56
<220>
<223> Designed to hybridize with cRNA of the human gene RGS1

<400>
gcccctcaga actgggaagg ccaggtaact ctagttacac agaaactgtg actaa        55

<210> 57
<211> 50
<212> DNA
<213> Artificial sequence
<220>
<221> Probe
<223> SG57
<220>
<223> Designed to hybridize with cRNA of the human gene RXRA

<400>
tttcgagtaa tttttaaagc cttgctctgt tgtgtcctgt tgccggctct              50

<210> 58
<211> 54
<212> DNA
<213> Artificial sequence
<220>
<221> Probe
<223> SG58
<220>
<223> Designed to hybridize with cRNA of the human gene SDHD

<400>
gcttggagtg cttctgaata tacagaagtt ccatttaagg gcaagtttcc ccgt         54

<210> 59
<211> 50
<212> DNA
<213> Artificial sequence
<220>
<221> Probe
<223> SG59
<220>
<223> Designed to hybridize with cRNA of the human gene SEPW1

<400>
tctcaagggg aagccaagag aggcatcagg atgggtgggt ttctgattgt          50

<210> 60
<211> 55
<212> DNA
<213> Artificial sequence
<220>
<221> Probe
<223> SG60
<220>
<223> Designed to hybridize with cRNA of the human gene SSBP1

<400>
gccaggcctc agagacgtgg catatcaata tgtgaaaaag gggtctcgaa tttat          55

<210> 61
<211> 55
<212> DNA
<213> Artificial sequence
<220>
<221> Probe
<223> SG61
<220>
<223> Designed to hybridize with cRNA of the human gene TEGT

<400>
acgaaatcat ttgtttctaa gttgtgttta ttcctggagt gacatgccac cccga          55

<210> 62
<211> 50
<212> DNA
<213> Artificial sequence
<220>
<221> Probe
<223> SG62
<220>
<223> Designed to hybridize with cRNA of the human gene HIST1H2BN

<400>
tctcgtccaa ggccatgggc atcatgaact ccttcgtcaa tgacatcttc          50

<210> 63
<211> 55
<212> DNA
<213> Artificial sequence
<220>
<221> Probe
<223> SG63
<220>
<223> Designed to hybridize with cRNA of the human gene BECN1

<400>
ccagccctgg tcagttttga ttcttaaccc catggactcc tttccctttc ttctc     55

<210> 64
<211> 55
<212> DNA
<213> Artificial sequence
<220>
<221> Probe
<223> SG64
<220>
<223> Designed to hybridize with cRNA of the human gene ANXA7

<400>
gtcaaggacc gtatcagggt aatgtgcttg gtttgcacat gttgttattg cctta     55

<210> 65

<211> 55
<212> DNA
<213> Artificial sequence
<220>
<221> Probe
<223> SG65
<220>
<223> Designed to hybridize with cRNA of the human gene NDUFA1

<400>
gcgcatctct ggagttgatc gttactatgt gtcaaagggt ttggagaaca ttgat        55

<210> 66
<211> 50
<212> DNA
<213> Artificial sequence
<220>
<221> Probe
<223> SG66
<220>
<223> Designed to hybridize with cRNA of the human gene ARHGEF2

<400>
ctcaacctct tactgctgtt ctccctttct ccgtccttca tggaagccct              50

<210> 67
<211> 55
<212> DNA
<213> Artificial sequence
<220>
<221> Probe
<223> SG67
<220>
<223> Designed to hybridize with cRNA of the human gene C5orf13

<400>
ccattggcct gagtttcttg tgcattactc ctctccctcc ttcgttagaa taggt        55

<210> 68
<211> 52
<212> DNA
<213> Artificial sequence
<220>
<221> Probe
<223> SG68
<220>
<223> Designed to hybridize with cRNA of the human gene XBP1

<400>
gcatcctggc ttgcctccag ttttaggtcc tttagtttgc ttctgtaagc aa          52

<210> 69
<211> 50
<212> DNA
<213> Artificial sequence
<220>
<221> Probe
<223> SG69
<220>
<223> Designed to hybridize with cRNA of the human gene G1P2

<400>
agcatccgag caggatcaag ggccggaaat aaaggctgtt gtaagagaat          50

<210> 70
<211> 53
<212> DNA
<213> Artificial sequence
<220>
<221> Probe
<223> SG70
<220>
<223> Designed to hybridize with cRNA of the human gene GLUD1

<400>
gcccctactc acctgacttt gtatcctctc cttttagagg ctttgcattc tgc          53

<210> 71
<211> 55
<212> DNA
<213> Artificial sequence
<220>
<221> Probe
<223> SG71
<220>
<223> Designed to hybridize with cRNA of the human gene S100A2

<400>
ctgccatgga tctcttgggc ccaggactgt tgatgccttt gagttttgta ttcaa        55

<210> 72
<211> 50
<212> DNA
<213> Artificial sequence
<220>
<221> Probe
<223> SG72
<220>
<223> Designed to hybridize with cRNA of the human gene LASP1

<400>
tactaagaat aacaagaagc ccagtggtga ggaaagtgcg ttctcccagc        50

<210> 73
<211> 50
<212> DNA
<213> Artificial sequence
<220>
<221> Probe
<223> SG73
<220>
<223> Designed to hybridize with cRNA of the human gene TIA-2

<400>
tgtgaaaaat aacagtccac cccaagtcat acactggacc cagtgcctgc        50

<210> 74
<211> 55

<212> DNA
<213> Artificial sequence
<220>
<221> Probe
<223> SG74
<220>
<223> Designed to hybridize with cRNA of the human gene PGRMC1

<400>
cctgaaccac gaggaaacag tacagtcgct agtcaagtgg tttttaaagt aaagt        55


<210> 75
<211> 50
<212> DNA
<213> Artificial sequence
<220>
<221> Probe
<223> SG75
<220>
<223> Designed to hybridize with cRNA of the human gene ZFPL1

<400>
caagcctaag acactaagac cccagaccca aagccaagtc caccagagtg        50


<210> 76
<211> 50
<212> DNA
<213> Artificial sequence
<220>
<221> Probe
<223> SG76
<220>
<223> Designed to hybridize with cRNA of the human gene MAP4K1

<400>
atatggtgat ggtgttgatg gatggctctg tgaagctggt gaccccggag        50


<210> 77
<211> 55

<212> DNA
<213> Artificial sequence
<220>
<221> Probe
<223> SG77
<220>
<223> Designed to hybridize with cRNA of the human gene STAT1

<400>
gtggcactgc atacaatctg aggcctcctc tctcagtttt tatatagatg gcgag          55


<210> 78
<211> 54
<212> DNA
<213> Artificial sequence
<220>
<221> Probe
<223> SG78
<220>
<223> Designed to hybridize with cRNA of the human gene HNRPH3

<400>
gctgaattct agccctagat tttggagtaa aaccccttca gcacttgacc gaaa          54


<210> 79
<211> 53
<212> DNA
<213> Artificial sequence
<220>
<221> Probe
<223> SG79
<220>
<223> Designed to hybridize with cRNA of the human gene TMEM4

<400>
tcaagtttgc gtgtgagagc attgtggagg aatacgagga tgaactcatt gaa          53


<210> 80
<211> 51
<212> DNA

<213> Artificial sequence
<220>
<221> Probe
<223> SG80
<220>
<223> Designed to hybridize with cRNA of the human gene KIAA0247

<400>
gtttggtctg aatggtgtag ttgctggttc cctagagagg aaaaggtggc a          51


<210> 81
<211> 55
<212> DNA
<213> Artificial sequence
<220>
<221> Probe
<223> SG81
<220>
<223> Designed to hybridize with cRNA of the human gene RIS1

<400>
gtggcctggt gggcgtttct tcttgtactt atgtggtttt ttggctttta ataca          55


<210> 82
<211> 53
<212> DNA
<213> Artificial sequence
<220>
<221> Probe
<223> SG82
<220>
<223> Designed to hybridize with cRNA of the human gene ARS2

<400>
tcttcaacaa gcatgcagag aaaattgagg aagtgaaaaa ggaagtcgcg ttt          53


<210> 83
<211> 50
<212> DNA

<213> Artificial sequence
<220>
<221> Probe
<223> SG83
<220>
<223> Designed to hybridize with cRNA of the human gene CA12

<400>
catccactgg ctgaatgatc agggaggcat agcagtgaga gccataggtc          50



<210> 84
<211> 55
<212> DNA
<213> Artificial sequence
<220>
<221> Probe
<223> SG84
<220>
<223> Designed to hybridize with cRNA of the human gene EEF1B2

<400>
actgcttttg aggactatgt gcagtccatg gatgtggctg ctttcaacaa gatct     55



<210> 85
<211> 55
<212> DNA
<213> Artificial sequence
<220>
<221> Probe
<223> SG85
<220>
<223> Designed to hybridize with cRNA of the human gene TRIB2

<400>
gcctgtatat gttttgtgaa atggtcctgt ttttgggtag gtgacacgtg gactc     55



<210> 86
<211> 55
<212> DNA
<213> Artificial sequence

<220>
<221> Probe
<223> SG86
<220>
<223> Designed to hybridize with cRNA of the human gene FLJ22169

<400>
agcgtccagc tcccctcaac gctatatttt gacactaaaa aagaaggttt ctaaa          55


<210> 87
<211> 50
<212> DNA
<213> Artificial sequence
<220>
<221> Probe
<223> SG87
<220>
<223> Designed to hybridize with cRNA of the human gene ASNS

<400>
aaagaaggat attactaccg tcaagtcttt gaacgccatt acccaggccg               50


<210> 88
<211> 55
<212> DNA
<213> Artificial sequence
<220>
<221> Probe
<223> SG88
<220>
<223> Designed to hybridize with cRNA of the human gene WBSCR20C

<400>
ggtgagcaaa agtgttgcct gcagaaataa aatgcagaac gtactctacg ataaa          55


<210> 89
<211> 51
<212> DNA
<213> Artificial sequence

<220>
<221> Probe
<223> SG89
<220>
<223> Designed to hybridize with cRNA of the human gene AKR1A1

<400>
atcctctgta cccctttaat gacccgtact gagaccacag cttcttggcc t          51


<210> 90
<211> 55
<212> DNA
<213> Artificial sequence
<220>
<221> Probe
<223> SG90
<220>
<223> Designed to hybridize with cRNA of the human gene SPRR1A

<400>
cgggtgcatt tgaggatgga tttggggagg gatcaagtga accatcccta gtctt      55


<210> 91
<211> 50
<212> DNA
<213> Artificial sequence
<220>
<221> Probe
<223> SG91
<220>
<223> Designed to hybridize with cRNA of the human gene EFNB1

<400>
attaatgtct taattggctg ttgcctgggg aacaggagag ctgctgcagg            50


<210> 92
<211> 50
<212> DNA
<213> Artificial sequence
<220>

<221> Probe
<223> SG92
<220>
<223> Designed to hybridize with cRNA of the human gene RPS5

<400>
tcaatgctgc caagggctcc tcgaactcct atgccattaa gaagaaggac          50



<210> 93
<211> 51
<212> DNA
<213> Artificial sequence
<220>
<221> Probe
<223> SG93
<220>
<223> Designed to hybridize with cRNA of the human gene RPS9

<400>
tgctggattg tctcgttttc ctgccaaata aacaggatca gcgctttaaa a          51



<210> 94
<211> 55
<212> DNA
<213> Artificial sequence
<220>
<221> Probe
<223> SG94
<220>
<223> Designed to hybridize with cRNA of the human gene RPL17

<400>
acggaaaagg aacagattgt tcctaaacca gaagaggagg ttgcccagaa gaaaa          55



<210> 95
<211> 55
<212> DNA
<213> Artificial sequence
<220>

<221> Probe
<223> SG95
<220>
<223> Designed to hybridize with cRNA of the human gene ETS2

<400>
cgctgcctca atacccacaa aagaccattc ccagtataca taagcacagg atgtt        55



<210> 96
<211> 55
<212> DNA
<213> Artificial sequence
<220>
<221> Probe
<223> SG96
<220>
<223> Designed to hybridize with cRNA of the human gene EIF4B

<400>
tccttgcctg ctcctgatgc ttggacccct tttattgatc agagtgctct agaat        55



<210> 97
<211> 50
<212> DNA
<213> Artificial sequence
<220>
<221> Probe
<223> SG97
<220>
<223> Designed to hybridize with cRNA of the human gene ZYX

<400>
tttgatgtct agcccctccc atttccaacc cctccctagc atcccaggtg              50



<210> 98
<211> 55
<212> DNA
<213> Artificial sequence
<220>

<221> Probe
<223> SG98
<220>
<223> Designed to hybridize with cRNA of the human gene XPO6

<400>
cggacagtcc tttctccagc ccgacatcca cctttttaaa caaaatctct tctac        55



<210> 99
<211> 55
<212> DNA
<213> Artificial sequence
<220>
<221> Probe
<223> SG99
<220>
<223> Designed to hybridize with cRNA of the human gene LOC285148

<400>
gtcaatacgt agaggtacgc ttttttcctc aggcttaaac ctttgccact gatat        55



<210> 100
<211> 50
<212> DNA
<213> Artificial sequence
<220>
<221> Probe
<223> SG100
<220>
<223> Designed to hybridize with cRNA of the human gene C21orf33

<400>
aataaacgcc ttcccaaaat ggcaacttcc cacagccaca tttcagacct              50



<210> 101
<211> 50
<212> DNA
<213> Artificial sequence
<220>
<221> Probe

<223> SG101
<220>
<223> Designed to hybridize with cRNA of the human gene EEF1D

<400>
agtgtcgata tcgcagcttt caacaagatc tgaagcctga gtgtgtgtac          50

<210> 102
<211> 51
<212> DNA
<213> Artificial sequence
<220>
<221> Probe
<223> SG102
<220>
<223> Designed to hybridize with cRNA of the human gene PABPC1

<400>
tactacaagc ccaccaagct aaagaggctg cccagaaagc agttaacagt g          51

<210> 103
<211> 55
<212> DNA
<213> Artificial sequence
<220>
<221> Probe
<223> SG103
<220>
<223> Designed to hybridize with cRNA of the human gene RBBP6

<400>
gccagctcag cagaaagtca ggacagcaag aagaagaaga aaaagaagga aaaga          55

<210> 104
<211> 50
<212> DNA
<213> Artificial sequence
<220>
<221> Probe

<223> SG104
<220>
<223> Designed to hybridize with cRNA of the human gene HRAS

<400>
tttgaggaca tccaccagta cagggagcag atcaaacggg tgaaggactc          50


<210> 105
<211> 50
<212> DNA
<213> Artificial sequence
<220>
<221> Probe
<223> SG105
<220>
<223> Designed to hybridize with cRNA of the human gene GGA3

<400>
tactccaagc ctccgtcaca aaaaaaaaat caccagctgc catagacacg          50


<210> 106
<211> 54
<212> DNA
<213> Artificial sequence
<220>
<221> Probe
<223> SG106
<220>
<223> Designed to hybridize with cRNA of the human gene CFL1

<400>
cctgtatttt ttttaacaac atccccattc cccacctggt cctccccctt ccca          54


<210> 107
<211> 55
<212> DNA
<213> Artificial sequence
<220>
<221> Probe
<223> SG107

<220>
<223> Designed to hybridize with cRNA of the human gene EIF3S2

<400>
gtcattgcac tccatcctga gccacaagag caaaactccg tctcaaaaaa aaaaa          55


<210> 108
<211> 55
<212> DNA
<213> Artificial sequence
<220>
<221> Probe
<223> SG108
<220>
<223> Designed to hybridize with cRNA of the human gene KRT18

<400>
gcagggtacc ctttggggag caggaggcca ataaaaagtt cagagttcaa aaaaa          55


<210> 109
<211> 50
<212> DNA
<213> Artificial sequence
<220>
<221> Probe
<223> SG109
<220>
<223> Designed to hybridize with cRNA of the human gene IGFIR

<400>
gaaatggaga ataatccagt cctagcacct ccaagcctga gcaagatgat                50


<210> 110
<211> 50
<212> DNA
<213> Artificial sequence
<220>
<221> Probe
<223> SG110
<220>

<223> Designed to hybridize with cRNA of the human gene IL8

<400>
agtgattgag agtggaccac actgcgccaa cacagaaatt attgtaaagc          50



<210> 111
<211> 50
<212> DNA
<213> Artificial sequence
<220>
<221> Probe
<223> SG111
<220>
<223> Designed to hybridize with cRNA of the human gene IRF1

<400>
ccggacagca ccagtgatct gtacaacttc caggtgtcac ccatgccctc          50



<210> 112
<211> 50
<212> DNA
<213> Artificial sequence
<220>
<221> Probe
<223> SG112
<220>
<223> Designed to hybridize with cRNA of the human gene IRF2

<400>
tcatcaagaa aacatcggat atcacccagg cccgcgtcaa gagctgttaa          50





<210> 113
<211> 50
<212> DNA
<213> Artificial sequence
<220>
<221> Probe
<223> SG113

<220>
<223> Designed to hybridize with cRNA of the human gene LYN

<400>
tatgtgagag atccaacgtc caataaacag caaaggccag ttccagaatc          50


<210> 114
<211> 50
<212> DNA
<213> Artificial sequence
<220>
<221> Probe
<223> SG114
<220>
<223> Designed to hybridize with cRNA of the human gene LYZ

<400>
gttgtcaaaa cagagatgtc cgtcagtatg ttcaaggttg tggagtgtaa          50


<210> 115
<211> 50
<212> DNA
<213> Artificial sequence
<220>
<221> Probe
<223> SG115
<220>
<223> Designed to hybridize with cRNA of the human gene NFKB1A

<400>
gaggacgagc tgccctatga tgactgtgtg tttggaggcc agcgtctgac          50


<210> 116
<211> 50
<212> DNA
<213> Artificial sequence
<220>
<221> Probe
<223> SG116
<220>

<223> Designed to hybridize with cRNA of the human gene MAZ

<400>
ccagccctgg tcttgtcttt tcatccctct tccccacgac agaagaagtt          50


<210> 117
<211> 50
<212> DNA
<213> Artificial sequence
<220>
<221> Probe
<223> SG117
<220>
<223> Designed to hybridize with cRNA of the human gene CD79A

<400>
caccaagaac cgaatcatca cagccgaggg gatcatcctc ctgttctgcg          50



<210> 118
<211> 50
<212> DNA
<213> Artificial sequence
<220>
<221> Probe
<223> SG118
<220>
<223> Designed to hybridize with cRNA of the human gene MCM3

<400>
gaggaaatgc ctcaagtaca cactccaaag acggcagact cacaggagac          50


<210> 119
<211> 50
<212> DNA
<213> Artificial sequence
<220>
<221> Probe
<223> SG119
<220>

<223> Designed to hybridize with cRNA of the human gene MEIS1

<400>
gtttcgtaat ggacggtcag caacatatgg gaattagagc accaggacct          50



<210> 120
<211> 50
<212> DNA
<213> Artificial sequence
<220>
<221> Probe
<223> SG120
<220>
<223> Designed to hybridize with cRNA of the human gene PRTN3

<400>
cttcgtgatc tggggatgtg ccacccgcct tttccctgac ttcttcacgc          50



<210> 121
<211> 50
<212> DNA
<213> Artificial sequence
<220>
<221> Probe
<223> SG121
<220>
<223> Designed to hybridize with cRNA of the human gene MMP2

<400>
gaaccaaagt ctgaagagcg tgaagtttgg aagcatcaaa tccgactggc          50



<210> 122
<211> 50
<212> DNA
<213> Artificial sequence
<220>
<221> Probe
<223> SG122
<220>
<223> Designed to hybridize with cRNA of the human gene MMP7

<400>
gtagcagtct agggattaac ttcctgtatg ctgcaactca tgaacttggc                50

<210> 123
<211> 50
<212> DNA
<213> Artificial sequence
<220>
<221> Probe
<223> SG123
<220>
<223> Designed to hybridize with cRNA of the human gene MMP8

<400>
ttaccagagt tgcaagaggc aataaatggc ttaactgtag atatggctga                50

<210> 124
<211> 50
<212> DNA
<213> Artificial sequence
<220>
<221> Probe
<223> SG124
<220>
<223> Designed to hybridize with cRNA of the human gene DOK1

<400>
cctggtactg caactggcag tggcatcaaa agccacaact cagccctgta                50

<210> 125
<211> 50
<212> DNA
<213> Artificial sequence
<220>
<221> Probe
<223> SG125
<220>
<223> Designed to hybridize with cRNA of the human gene PDGFA

<400>
ccgctgtctg caagaccagg acggtcattt acgagattcc tcggagtcag          50

<210> 126
<211> 50
<212> DNA
<213> Artificial sequence
<220>
<221> Probe
<223> SG126
<220>
<223> Designed to hybridize with cRNA of the human gene PRL

<400>
attatctcaa gctcctgaag tgccgaatca tccacaacaa caactgctaa          50

<210> 127
<211> 50
<212> DNA
<213> Artificial sequence
<220>
<221> Probe
<223> SG127
<220>
<223> Designed to hybridize with cRNA of the human gene RAF1

<400>
ctattggtga tagtggagtc ccagcactac cttctttgac tatgcgtcgt          50

<210> 128
<211> 50
<212> DNA
<213> Artificial sequence
<220>
<221> Probe
<223> SG128
<220>
<223> Designed to hybridize with cRNA of the human gene RARB

<400>
tcagtggatt gacccaaacc gaatggcagc atcggcacac tgctcaatca          50

<210> 129
<211> 50
<212> DNA
<213> Artificial sequence
<220>
<221> Probe
<223> SG129
<220>
<223> Designed to hybridize with cRNA of the human gene OPRD1

<400>
acggtgacca agatctgcgt gttcctcttc gccttcgtgg tgcccatcct          50

<210> 130
<211> 50
<212> DNA
<213> Artificial sequence
<220>
<221> Probe
<223> SG130
<220>
<223> Designed to hybridize with cRNA of the human gene RET

<400>
caggcaggtg tgagtggagg caaggagatg gcaaagggat caccaggaac          50

<210> 131
<211> 50
<212> DNA
<213> Artificial sequence
<220>
<221> Probe
<223> SG131
<220>
<223> Designed to hybridize with cRNA of the human gene SREBF1

<400>

ttggtgcttc tctttgtcta cggtgagcca gtcacacggc cccactcagg          50

<210> 132
<211> 50
<212> DNA
<213> Artificial sequence
<220>
<221> Probe
<223> SG132
<220>
<223> Designed to hybridize with cRNA of the human gene TNF

<400>
agccctctgg cccaggcagt cagatcatct tctcgaaccc cgagtgacaa          50

<210> 133
<211> 50
<212> DNA
<213> Artificial sequence
<220>
<221> Probe
<223> SG133
<220>
<223> Designed to hybridize with cRNA of the human gene TERF1

<400>
cctgggtgac aggatcatac gtaccccaaa cctcaacatc acacagtata          50

<210> 134
<211> 50
<212> DNA
<213> Artificial sequence
<220>
<221> Probe
<223> SG134
<220>
<223> Designed to hybridize with cRNA of the human gene VCAM1

<400>

tctcccctgg accccggatt gctgctcaga ttggagactc agtcatgttg          50

<210> 135
<211> 50
<212> DNA
<213> Artificial sequence
<220>
<221> Probe
<223> SG135
<220>
<223> Designed to hybridize with cRNA of the human gene ATP5O

<400>
ccaagtattg aaattggagg ctaagactga tccgtcaatc ttgggtggaa          50

<210> 136
<211> 50
<212> DNA
<213> Artificial sequence
<220>
<221> Probe
<223> SG136
<220>
<223> Designed to hybridize with cRNA of the human gene TNFSF5

<400>
caggtgcttc ggtgtttgtc aatgtgactg atccaagcca agtgagccat          50

<210> 137
<211> 50
<212> DNA
<213> Artificial sequence
<220>
<221> Probe
<223> SG137
<220>
<223> Designed to hybridize with cRNA of the human gene POLR2C

<400>
gaagaaactg agtgatttac aaactcaatt aagccacgag atccagagtg          50

<210> 138
<211> 50
<212> DNA
<213> Artificial sequence
<220>
<221> Probe
<223> SG138
<220>
<223> Designed to hybridize with cRNA of the human gene PLA2G6

<400>
agctcagccc ccaccgatca gcctaaacaa cctagaacta caggatctca          50

<210> 139
<211> 50
<212> DNA
<213> Artificial sequence
<220>
<221> Probe
<223> SG139
<220>
<223> Designed to hybridize with cRNA of the human gene CCT6A

<400>
tcttggttga tgagatcatg cgagctggaa tgtcttctct gaaaggttga          50

<210> 140
<211> 50
<212> DNA
<213> Artificial sequence
<220>
<221> Probe
<223> SG140
<220>
<223> Designed to hybridize with cRNA of the human gene PDHA1

<400>
ccacagacca tctcatcaca gcctaccggg ctcacggctt tactttcacc          50

<210> 141
<211> 50
<212> DNA
<213> Artificial sequence
<220>
<221> Probe
<223> SG141
<220>
<223> Designed to hybridize with cRNA of the human gene LADH

<400>
cggaataaag gatgatgtct tccttagtgt tccttgcatt ttgggacaga          50

<210> 142
<211> 50
<212> DNA
<213> Artificial sequence
<220>
<221> Probe
<223> SG142
<220>
<223> Designed to hybridize with cRNA of the human gene SNRPB

<400>
gaactccaaa caagcagaaa gggaagagaa gcgagtcctc ggtctggtgc          50

<210> 143
<211> 50
<212> DNA
<213> Artificial sequence
<220>
<221> Probe
<223> SG143
<220>
<223> Designed to hybridize with cRNA of the human gene SNRPB

<400>
tacaaacccg aggaccctct tagggaagaa gccctacatt tgcagtgatt          50

<210> 144
<211> 50
<212> DNA
<213> Artificial sequence
<220>
<221> Probe
<223> SG144
<220>
<223> Designed to hybridize with cRNA of the human gene H2AFX

<400>
aggcgccctc gggcggcaag aaggccaccc aggcctccca ggagtactaa          50

<210> 145
<211> 50
<212> DNA
<213> Artificial sequence
<220>
<221> Probe
<223> SG145
<220>
<223> Designed to hybridize with cRNA of the human gene MRPL37

<400>
ggctatcctt acccctatcc ccataccctg tacttactgg acaaagccaa          50

<210> 146
<211> 50
<212> DNA
<213> Artificial sequence
<220>
<221> Probe
<223> SG146
<220>
<223> Designed to hybridize with cRNA of the human gene UBE2C

<400>
cctgctatca ccccaacgtg gacacccagg gtaacatatg cctggacatc          50

<210> 147
<211> 50
<212> DNA
<213> Artificial sequence
<220>
<221> Probe
<223> SG147
<220>
<223> Designed to hybridize with cRNA of the human gene RPS3

<400>
aagaaagcat tcatgggacc actgaagaaa gaccgaattg caaaggaaga          50

<210> 148
<211> 50
<212> DNA
<213> Artificial sequence
<220>
<221> Probe
<223> SG148
<220>
<223> Designed to hybridize with cRNA of the human gene ALDH1A1

<400>
actgggagag tacggtttcc atgaatatac agaggtcaaa acagtcacag          50

<210> 149
<211> 50
<212> DNA
<213> Artificial sequence
<220>
<221> Probe
<223> SG149
<220>
<223> Designed to hybridize with cRNA of the human gene AF1q

<400>

gcagaccagg agaaaaaccc tgaaggtgat ggcctccttg agtacagcac          50

<210> 150
<211> 50
<212> DNA
<213> Artificial sequence
<220>
<221> Probe
<223> SG150
<220>
<223> Designed to hybridize with cRNA of the human gene RUNX1

<400>
ctccctgaac cactccactg cctttaaccc tcagcctcag agtcagatgc          50

<210> 151
<211> 50
<212> DNA
<213> Artificial sequence
<220>
<221> Probe
<223> SG151
<220>
<223> Designed to hybridize with cRNA of the human gene MYBL1

<400>
atgggaagac agaagaccaa cttattatga ctgaacaagc aagaagatat          50

<210> 152
<211> 50
<212> DNA
<213> Artificial sequence
<220>
<221> Probe
<223> SG152
<220>
<223> Designed to hybridize with cRNA of the human gene ANXA6

<400>
tgagaaatat gacaagtctc tccaccaagc cattgagggt gacacctccg          50

<210> 153
<211> 50
<212> DNA
<213> Artificial sequence
<220>
<221> Probe
<223> SG153
<220>
<223> Designed to hybridize with cRNA of the human gene ALK

<400>
gtcattacga ggataccatt ctgaaaagca agaatagcat gaaccagcct          50

<210> 154
<211> 50
<212> DNA
<213> Artificial sequence
<220>
<221> Probe
<223> SG154
<220>
<223> Designed to hybridize with cRNA of the human gene MNDA

<400>
gtgtgagaaa ggagataaac ttcgactctt ctgccttcaa ctgagaacag          50

<210> 155
<211> 50
<212> DNA
<213> Artificial sequence
<220>
<221> Probe
<223> SG155
<220>
<223> Designed to hybridize with cRNA of the human gene APAF1

<400>
ggtggaacgt tgtcactggg gaatcctcac agaccttcta cacaaatgga          50

<210> 156
<211> 50
<212> DNA
<213> Artificial sequence
<220>
<221> Probe
<223> SG156
<220>
<223> Designed to hybridize with cRNA of the human gene BIRC3

<400>
aggcgacact ttcctaattg cttctttgtt ttgggccgga atcttaatat          50

<210> 157
<211> 50
<212> DNA
<213> Artificial sequence
<220>
<221> Probe
<223> SG157
<220>
<223> Designed to hybridize with cRNA of the human gene ABCC6

<400>
tgtccttcaa gatccacgca ggagagaagg tgggcatcgt tggcaggacc          50

<210> 158
<211> 50
<212> DNA
<213> Artificial sequence
<220>
<221> Probe
<223> SG158
<220>
<223> Designed to hybridize with cRNA of the human gene ATIC

<400>

gaactgggaa tcatcctcgc tcatacgaac cttcggctct tccaccactg          50

<210> 159
<211> 50
<212> DNA
<213> Artificial sequence
<220>
<221> Probe
<223> SG159
<220>
<223> Designed to hybridize with cRNA of the human gene ATM

<400>
tgaggaatta gatgaacgtg agcgaaactc tcgtccttct agtaaaaatc          50

<210> 160
<211> 50
<212> DNA
<213> Artificial sequence
<220>
<221> Probe
<223> SG160
<220>
<223> Designed to hybridize with cRNA of the human gene BAX

<400>
ttgtcgccct tttctacttt gccagcaaac tggtgctcaa ggccctgtgc          50

<210> 161
<211> 50
<212> DNA
<213> Artificial sequence
<220>
<221> Probe
<223> SG161
<220>
<223> Designed to hybridize with cRNA of the human gene CCND1

<400>
cttcctgtcc tactaccgcc tcacacgctt cctctccaga gtgatcaagt          50

<210> 162
<211> 50
<212> DNA
<213> Artificial sequence
<220>
<221> Probe
<223> SG162
<220>
<223> Designed to hybridize with cRNA of the human gene BCL2

<400>
ttgctgggac tgtacccaag aattaaacca agacaaagga gcttggcctg          50

<210> 163
<211> 50
<212> DNA
<213> Artificial sequence
<220>
<221> Probe
<223> SG163
<220>
<223> Designed to hybridize with cRNA of the human gene BCL3

<400>
tttcctctgg tgaacctgcc tacaccccta taccccatga tgtgccccat          50

<210> 164
<211> 50
<212> DNA
<213> Artificial sequence
<220>
<221> Probe
<223> SG164
<220>
<223> Designed to hybridize with cRNA of the human gene BCL6

<400>
tcaataacat cgttaacagg tccatgacgg gctctccccg cagcagcagc          50

<210> 165
<211> 50
<212> DNA
<213> Artificial sequence
<220>
<221> Probe
<223> SG165
<220>
<223> Designed to hybridize with cRNA of the human gene BCL7A

<400>
gttgtcgggg gatctggaag gagtgccacc ctctaaaaag atgaaactgg          50

<210> 166
<211> 50
<212> DNA
<213> Artificial sequence
<220>
<221> Probe
<223> SG166
<220>
<223> Designed to hybridize with cRNA of the human gene BCL7b

<400>
caaggaagaa ccagttccac tagagacaca ggtcgttgag gaagaggaag          50

<210> 167
<211> 50
<212> DNA
<213> Artificial sequence
<220>
<221> Probe
<223> SG167
<220>
<223> Designed to hybridize with cRNA of the human gene BCL10

<400>
gcacgacgcc ctttttttct actaattctt ctctgaattt gcctgttcta          50

<210> 168
<211> 50
<212> DNA
<213> Artificial sequence
<220>
<221> Probe
<223> SG168
<220>
<223> Designed to hybridize with cRNA of the human gene BCL2L1

<400>
gtagacaagg agatgcaggt attggtgagt cggatcgcag cttggatggc          50

<210> 169
<211> 50
<212> DNA
<213> Artificial sequence
<220>
<221> Probe
<223> SG169
<220>
<223> Designed to hybridize with cRNA of the human gene BCR

<400>
gcagggagtt cagcttgaag aggatgccgt cccgaaaaca gacaggggtc          50

<210> 170
<211> 50
<212> DNA
<213> Artificial sequence
<220>
<221> Probe
<223> SG170
<220>
<223> Designed to hybridize with cRNA of the human gene BCR

<400>
aatgtctgag gaagcatcgg agactgggtc ccgccatgcc tgtgtcatct          50

<210> 171
<211> 50
<212> DNA
<213> Artificial sequence
<220>
<221> Probe
<223> SG171
<220>
<223> Designed to hybridize with cRNA of the human gene OLIG2

<400>
caactacatc ctcatgctca ccaactcgct ggaggagatg aagcgactgg          50

<210> 172
<211> 50
<212> DNA
<213> Artificial sequence
<220>
<221> Probe
<223> SG172
<220>
<223> Designed to hybridize with cRNA of the human gene BIK

<400>
caccacactt aaggagaaca taatgaggtt ctggagatcc ccgaaccccg          50

<210> 173
<211> 50
<212> DNA
<213> Artificial sequence
<220>
<221> Probe
<223> SG173
<220>
<223> Designed to hybridize with cRNA of the human gene BCL2LAA

<400>
cagagccaca aggtaatcct gaaggcaatc acggaggtga aggggacagc          50

<210> 174
<211> 50
<212> DNA
<213> Artificial sequence
<220>
<221> Probe
<223> SG174
<220>
<223> Designed to hybridize with cRNA of the human gene BMI1

<400>
caatagacct cgaaaatcat cagtaaatgg gtcatcagca acttcttctg          50

<210> 175
<211> 50
<212> DNA
<213> Artificial sequence
<220>
<221> Probe
<223> SG175
<220>
<223> Designed to hybridize with cRNA of the human gene BLMH

<400>
ctaggcccca gcacaagtac aacaaacttt acacagtgga atacttaagc          50

<210> 176
<211> 50
<212> DNA
<213> Artificial sequence
<220>
<221> Probe
<223> SG176
<220>
<223> Designed to hybridize with cRNA of the human gene BLR1

<400>
ttctgggaac tggacagatt ggacaactat aacgacacct ccctggtgga          50

<210> 177
<211> 50
<212> DNA
<213> Artificial sequence
<220>
<221> Probe
<223> SG177
<220>
<223> Designed to hybridize with cRNA of the human gene IBSP

<400>
cgtcaatgaa tacgacaatg gatatgaaat ctatgaaagt gagaacgggg          50

<210> 178
<211> 50
<212> DNA
<213> Artificial sequence
<220>
<221> Probe
<223> SG178
<220>
<223> Designed to hybridize with cRNA of the human gene BTG1

<400>
gtagcactca aaacagcacc aacgtgcaaa tggtagacag ccgaatcagc          50

<210> 179
<211> 50
<212> DNA
<213> Artificial sequence
<220>
<221> Probe
<223> SG179
<220>
<223> Designed to hybridize with cRNA of the human gene BUB1

<400>
gaaatacaat caacggagaa agcatgagca atgggtaaat gaagacagac          50

<210> 180
<211> 50
<212> DNA
<213> Artificial sequence
<220>
<221> Probe
<223> SG180
<220>
<223> Designed to hybridize with cRNA of the human gene ABL1

<400>
gcaagctcct cagttcggtg aaggaaatca gtgacatagt gcagaggtag          50



<210> 181
<211> 50
<212> DNA
<213> Artificial sequence
<220>
<221> Probe
<223> SG181
<220>
<223> Designed to hybridize with cRNA of the human gene CASP1

<400>
ggactctcag cagatcaaac atctggaaat taccttaata tgcaagactc          50



<210> 182
<211> 50
<212> DNA
<213> Artificial sequence
<220>
<221> Probe
<223> SG182
<220>
<223> Designed to hybridize with cRNA of the human gene CASP3

<400>
cctggttatt attcttggcg aaattcaaag gatggctcct ggttcatcca          50

<210> 183
<211> 50
<212> DNA
<213> Artificial sequence
<220>
<221> Probe
<223> SG183
<220>
<223> Designed to hybridize with cRNA of the human gene CASP4

<400>
aaatatcccc caataaaaaa gctcatccga atatggaggc tggaccacct          50

<210> 184
<211> 50
<212> DNA
<213> Artificial sequence
<220>
<221> Probe
<223> SG184
<220>
<223> Designed to hybridize with cRNA of the human gene CASP5

<400>
ctggacaaac atctatccag accctagtac ctaatacgga tcaaaagtcg          50

<210> 185
<211> 50
<212> DNA
<213> Artificial sequence
<220>
<221> Probe
<223> SG185
<220>
<223> Designed to hybridize with cRNA of the human gene CASP6

<400>
ggttccctgt tttgcctcaa tgctaactaa aaagctgcat ttctttccaa          50

<210> 186

<211> 50
<212> DNA
<213> Artificial sequence
<220>
<221> Probe
<223> SG186
<220>
<223> Designed to hybridize with cRNA of the human gene CASP7

<400>
tcccctgtgt ggtctccatg ctcaccaagg aactctactt cagtcaatag          50

<210> 187
<211> 50
<212> DNA
<213> Artificial sequence
<220>
<221> Probe
<223> SG187
<220>
<223> Designed to hybridize with cRNA of the human gene CASP8

<400>
gcagcagcct tgaaggaagt cctgatgaat tttcaaatga ctttggacaa          50

<210> 188
<211> 50
<212> DNA
<213> Artificial sequence
<220>
<221> Probe
<223> SG188
<220>
<223> Designed to hybridize with cRNA of the human gene CASP9

<400>
gtccctcctg cttagggtcg ctaatgctgt ttcggtgaaa gggatttata          50

<210> 189

<211> 50
<212> DNA
<213> Artificial sequence
<220>
<221> Probe
<223> SG189
<220>
<223> Designed to hybridize with cRNA of the human gene CBFB

<400>
gctcccatga ttctgaatgg agtctgtgtt atctggaaag gctggattga          50


<210> 190
<211> 50
<212> DNA
<213> Artificial sequence
<220>
<221> Probe
<223> SG190
<220>
<223> Designed to hybridize with cRNA of the human gene CALD1

<400>
caaaagaggg aggagatgcg actcgaagca gaaagaatcg cctaccagag          50


<210> 191
<211> 50
<212> DNA
<213> Artificial sequence
<220>
<221> Probe
<223> SG191
<220>
<223> Designed to hybridize with cRNA of the human gene CAST

<400>
agcaggcatc agatacagga agtaacgatg ctcacaataa aaaagcagtt          50


<210> 192
<211> 50

<212> DNA
<213> Artificial sequence
<220>
<221> Probe
<223> SG192
<220>
<223> Designed to hybridize with cRNA of the human gene CDC25A

<400>
caggggagaa gagcaagagg gagatgtaca gtcgtctgaa gaagctctga          50

<210> 193
<211> 50
<212> DNA
<213> Artificial sequence
<220>
<221> Probe
<223> SG193
<220>
<223> Designed to hybridize with cRNA of the human gene CDC25B

<400>
ttccctcagc acccgaactt ctgtgaaccc caggactacc ggcccatgaa          50

<210> 194
<211> 50
<212> DNA
<213> Artificial sequence
<220>
<221> Probe
<223> SG194
<220>
<223> Designed to hybridize with cRNA of the human gene CD2

<400>
aaattccagc ttcaacccct cagaatccag caacttccca acatcctcct          50

<210> 195
<211> 50

<212> DNA
<213> Artificial sequence
<220>
<221> Probe
<223> SG195
<220>
<223> Designed to hybridize with cRNA of the human gene CD3E

<400>
aagatgcgaa cttttatctc tacctgaggg caagagtgtg tgagaactgc          50


<210> 196
<211> 50
<212> DNA
<213> Artificial sequence
<220>
<221> Probe
<223> SG196
<220>
<223> Designed to hybridize with cRNA of the human gene CD4

<400>
ctccaaccag ataaagattc tgggaaatca gggctccttc ttaactaaag          50


<210> 197
<211> 50
<212> DNA
<213> Artificial sequence
<220>
<221> Probe
<223> SG197
<220>
<223> Designed to hybridize with cRNA of the human gene CD5

<400>
ggaaaatatg ccctccccgt aatggtgaac caccagcacc tacccaccac          50


<210> 198
<211> 50

<212> DNA
<213> Artificial sequence
<220>
<221> Probe
<223> SG198
<220>
<223> Designed to hybridize with cRNA of the human gene CD6

<400>
tcatctaaag acaccttcct ttccactggc tgtcaagcca cagggcacca          50


<210> 199
<211> 50
<212> DNA
<213> Artificial sequence
<220>
<221> Probe
<223> SG199
<220>
<223> Designed to hybridize with cRNA of the human gene CD7

<400>
gtgctcgtgg cgggataaga attcggcggc atgtgtggtg tacgaggaca          50


<210> 200
<211> 50
<212> DNA
<213> Artificial sequence
<220>
<221> Probe
<223> SG200
<220>
<223> Designed to hybridize with cRNA of the human gene CD8

<400>
ttcattctca atctcacaag cgtgaagccg gaagacagtg gcatctactt          50


<210> 201
<211> 50
<212> DNA

<213> Artificial sequence
<220>
<221> Probe
<223> SG201
<220>
<223> Designed to hybridize with cRNA of the human gene CD9

<400>
gtgaagtcct gtcctgatgc catcaaagag gtcttcgaca ataaattcca          50

<210> 202
<211> 50
<212> DNA
<213> Artificial sequence
<220>
<221> Probe
<223> SG202
<220>
<223> Designed to hybridize with cRNA of the human gene MME

<400>
tcagaagcct ttcactgccg caagaattca tacatgaatc cagaaaagaa          50

<210> 203
<211> 50
<212> DNA
<213> Artificial sequence
<220>
<221> Probe
<223> SG203
<220>
<223> Designed to hybridize with cRNA of the human gene ITGAL

<400>
tgcctgaagc ccctccatga gaaggactct gagagtggtg gtggcaagga          50

<210> 204
<211> 50
<212> DNA

<213> Artificial sequence
<220>
<221> Probe
<223> SG204
<220>
<223> Designed to hybridize with cRNA of the human gene ITGAM

<400>
tttgtgaggt cccagacgga gaccaaagtg gagccgttcg aggtccccaa          50


<210> 205
<211> 50
<212> DNA
<213> Artificial sequence
<220>
<221> Probe
<223> SG205
<220>
<223> Designed to hybridize with cRNA of the human gene ITGAX

<400>
aggaggcaaa tggacaaatt gccccagaaa acgggacaca gacccccagc          50


<210> 206
<211> 50
<212> DNA
<213> Artificial sequence
<220>
<221> Probe
<223> SG206
<220>
<223> Designed to hybridize with cRNA of the human gene ANPEP

<400>
agccctggag aagacgaaag ccaacatcaa gtgggtgaag gagaacaagg          50


<210> 207
<211> 50
<212> DNA
<213> Artificial sequence

<220>
<221> Probe
<223> SG207
<220>
<223> Designed to hybridize with cRNA of the human gene CD14

<400>
tggataacct gacactggac gggaatccct tcctggtccc tggaactgcc          50

<210> 208
<211> 50
<212> DNA
<213> Artificial sequence
<220>
<221> Probe
<223> SG208
<220>
<223> Designed to hybridize with cRNA of the human gene SELE

<400>
aattgggctc ctgcacacag tggcccgcta caagttctac ctggctttcg          50

<210> 209
<211> 50
<212> DNA
<213> Artificial sequence
<220>
<221> Probe
<223> SG209
<220>
<223> Designed to hybridize with cRNA of the human gene FCGR3A

<400>
gtgaagacaa acattcgaag ctcaacaaga gactggaagg accataaatt          50

<210> 210
<211> 50
<212> DNA
<213> Artificial sequence

<220>
<221> Probe
<223> SG210
<220>
<223> Designed to hybridize with cRNA of the human gene FCGR3B

<400>
gcttggtgat ggtactcctt tttgcagtgg acacaggact atatttctct          50


<210> 211
<211> 50
<212> DNA
<213> Artificial sequence
<220>
<221> Probe
<223> SG211
<220>
<223> Designed to hybridize with cRNA of the human gene ITGB2

<400>
gataatcccc ttttcaagag cgccaccacg acggtcatga accccaagtt          50


<210> 212
<211> 50
<212> DNA
<213> Artificial sequence
<220>
<221> Probe
<223> SG212
<220>
<223> Designed to hybridize with cRNA of the human gene CD19

<400>
actccattcg gggccagcct ggacccaatc atgaggaaga tgcagactct          50


<210> 213
<211> 50
<212> DNA
<213> Artificial sequence

<220>
<221> Probe
<223> SG213
<220>
<223> Designed to hybridize with cRNA of the human gene MS4A1

<400>
ctccccaaga tcaggaatcc tcaccaatag aaaatgacag ctctccttaa          50


<210> 214
<211> 50
<212> DNA
<213> Artificial sequence
<220>
<221> Probe
<223> SG214
<220>
<223> Designed to hybridize with cRNA of the human gene CR2

<400>
catttagaag cacgagaagt atattctgtt gatccataca acccagccag          50


<210> 215
<211> 50
<212> DNA
<213> Artificial sequence
<220>
<221> Probe
<223> SG215
<220>
<223> Designed to hybridize with cRNA of the human gene CD22

<400>
acgtcattcc agattttcca gaagatgagg ggattcatta ctcagagctg          50


<210> 216
<211> 50
<212> DNA
<213> Artificial sequence
<220>

<221> Probe
<223> SG216
<220>
<223> Designed to hybridize with cRNA of the human gene FCER2

<400>
actccgggag gaggtgacaa agctaaggat ggagttgcag gtgtccagcg          50


<210> 217
<211> 50
<212> DNA
<213> Artificial sequence
<220>
<221> Probe
<223> SG217
<220>
<223> Designed to hybridize with cRNA of the human gene CD24

<400>
cagccagtct cttcgtggtc tcactctctc ttctgcatct ctactcttaa          50


<210> 218
<211> 50
<212> DNA
<213> Artificial sequence
<220>
<221> Probe
<223> SG218
<220>
<223> Designed to hybridize with cRNA of the human gene IL2RA

<400>
ctgctgagag cgtctgcaaa atgacccacg ggaagacaag gtggacccag          50


<210> 219
<211> 50
<212> DNA
<213> Artificial sequence
<220>

<221> Probe
<223> SG219
<220>
<223> Designed to hybridize with cRNA of the human gene DPP4

<400>
gacaaccttg accattacag aaattcaaca gtcatgagca gagctgaaaa          50


<210> 220
<211> 50
<212> DNA
<213> Artificial sequence
<220>
<221> Probe
<223> SG220
<220>
<223> Designed to hybridize with cRNA of the human gene ITGB1

<400>
gagaaaatga atgccaaatg ggacacgcaa gaaaatccga tttacaagag          50


<210> 221
<211> 50
<212> DNA
<213> Artificial sequence
<220>
<221> Probe
<223> SG221
<220>
<223> Designed to hybridize with cRNA of the human gene TNFRSF8

<400>
agacggtcac caagccccag gatatggctg agaaggacac cacctttgag          50


<210> 222
<211> 50
<212> DNA
<213> Artificial sequence
<220>
<221> Probe

<223> SG222
<220>
<223> Designed to hybridize with cRNA of the human gene CD33

<400>
ttacatggcc ccactgaaac ctcaagctgt tcaggtgccg cccctactgt          50



<210> 223
<211> 50
<212> DNA
<213> Artificial sequence
<220>
<221> Probe
<223> SG223
<220>
<223> Designed to hybridize with cRNA of the human gene CD34

<400>
cctgctggct gtcttgggca tcactggcta tttcctgatg aatcgccgca          50



<210> 224
<211> 50
<212> DNA
<213> Artificial sequence
<220>
<221> Probe
<223> SG224
<220>
<223> Designed to hybridize with cRNA of the human gene CD36

<400>
ggcctttgcc tctccagttg aaaacccaga caactattgt ttctgcacag          50



<210> 225
<211> 50
<212> DNA
<213> Artificial sequence
<220>
<221> Probe

<223> SG225
<220>
<223> Designed to hybridize with cRNA of the human gene CD38

<400>
agtgtggaag tccataattt gcaaccagag aaggttcaga cactagaggc          50


<210> 226
<211> 50
<212> DNA
<213> Artificial sequence
<220>
<221> Probe
<223> SG226
<220>
<223> Designed to hybridize with cRNA of the human gene TNFRSF5

<400>
acccaggagg atggcaaaga gagtcgcatc tcagtgcagg agagacagtg          50


<210> 227
<211> 50
<212> DNA
<213> Artificial sequence
<220>
<221> Probe
<223> SG227
<220>
<223> Designed to hybridize with cRNA of the human gene SPN

<400>
acccaggagg atggcaaaga gagtcgcatc tcagtgcagg agagacagtg          50


<210> 228
<211> 50
<212> DNA
<213> Artificial sequence
<220>
<221> Probe

<223> SG228
<220>
<223> Designed to hybridize with cRNA of the human gene CD44

<400>
gagctgtgga ggacagaaag ccaagtggac tcaacggaga ggccagcaag          50


<210> 229
<211> 50
<212> DNA
<213> Artificial sequence
<220>
<221> Probe
<223> SG229
<220>
<223> Designed to hybridize with cRNA of the human gene CD44v6

<400>
ctgggattgg ttttcatggt tgtttctacc atcagagtca aagaatcatc          50


<210> 230
<211> 50
<212> DNA
<213> Artificial sequence
<220>
<221> Probe
<223> SG230
<220>
<223> Designed to hybridize with cRNA of the human gene PTPRCCD

<400>
aacattctgt caatggtcct gcaagtccag ccttaaatca aggttcatag          50


<210> 231
<211> 50
<212> DNA
<213> Artificial sequence
<220>
<221> Probe
<223> SG231

<220>
<223> Designed to hybridize with cRNA of the human gene CD47

<400>
tcaggtgata gcctatatcc tcgctgtggt tggactgagt ctctgtattg 50

<210> 232
<211> 50
<212> DNA
<213> Artificial sequence
<220>
<221> Probe
<223> SG232
<220>
<223> Designed to hybridize with cRNA of the human gene ITGA2

<400>
agccgaagta ccaacaggag ttataatagg aagtataatt gctggaatcc 50

<210> 233
<211> 50
<212> DNA
<213> Artificial sequence
<220>
<221> Probe
<223> SG233
<220>
<223> Designed to hybridize with cRNA of the human gene ITGA3

<400>
ctaagaggca gaaggcggag atgaagagcc agccgtcaga gacagagagg 50

<210> 234
<211> 50
<212> DNA
<213> Artificial sequence
<220>
<221> Probe
<223> SG234

<220>
<223> Designed to hybridize with cRNA of the human gene ITGA4

<400>
cttggactta ttgtacttct gttgatctca tatgttatgt ggaaggctgg          50


<210> 235
<211> 50
<212> DNA
<213> Artificial sequence
<220>
<221> Probe
<223> SG235
<220>
<223> Designed to hybridize with cRNA of the human gene ITGA5

<400>
tcaactgcac caccaatcac cccattaacc caaagggcct ggagttggat          50


<210> 236
<211> 50
<212> DNA
<213> Artificial sequence
<220>
<221> Probe
<223> SG236
<220>
<223> Designed to hybridize with cRNA of the human gene ICAM3

<400>
ctatgcagcc gacagaagca atgggggaag aaccgtccag agctgagtga          50


<210> 237
<211> 50
<212> DNA
<213> Artificial sequence
<220>
<221> Probe
<223> SG237
<220>

<223> Designed to hybridize with cRNA of the human gene CDW52

<400>
ccctcagcat ccagcagcat gagcggaggc attttccttt tcttcgtggc          50

<210> 238
<211> 50
<212> DNA
<213> Artificial sequence
<220>
<221> Probe
<223> SG238
<220>
<223> Designed to hybridize with cRNA of the human gene ICAM1

<400>
gaacaaccgg aaggtgtatg aactgagcaa tgtgcaagaa gatagccaac          50

<210> 239
<211> 50
<212> DNA
<213> Artificial sequence
<220>
<221> Probe
<223> SG239
<220>
<223> Designed to hybridize with cRNA of the human gene NCAM1

<400>
gaggtgaccc cagactctga gaatgatttt gggaactaca actgtactgc          50

<210> 240
<211> 50
<212> DNA
<213> Artificial sequence
<220>
<221> Probe
<223> SG240
<220>

<223> Designed to hybridize with cRNA of the human gene CD58

<400>
catcaatcat tttgacaacc tgtatcccaa gcagcggtca ttcaagacac          50

<210> 241
<211> 50
<212> DNA
<213> Artificial sequence
<220>
<221> Probe
<223> SG241
<220>
<223> Designed to hybridize with cRNA of the human gene CD59

<400>
cgaacagctt gaaaatggtg ggacatcctt atcagagaaa acagttcttc          50

<210> 242
<211> 50
<212> DNA
<213> Artificial sequence
<220>
<221> Probe
<223> SG242
<220>
<223> Designed to hybridize with cRNA of the human gene SELL

<400>
taggaacata tggagttttt acaaacgctg catttgaccc gagtccttaa          50

<210> 243
<211> 50
<212> DNA
<213> Artificial sequence
<220>
<221> Probe
<223> SG243
<220>

<223> Designed to hybridize with cRNA of the human gene CD79B

<400>
gccacctatg aggacatagt gacgctgcgg acaggggaag tgaagtggtc          50

<210> 244
<211> 50
<212> DNA
<213> Artificial sequence
<220>
<221> Probe
<223> SG244
<220>
<223> Designed to hybridize with cRNA of the human gene CD81

<400>
cttcaaggag gactgccacc agaagatcga tgacctcttc tccgggaagc          50

<210> 245
<211> 50
<212> DNA
<213> Artificial sequence
<220>
<221> Probe
<223> SG245
<220>
<223> Designed to hybridize with cRNA of the human gene KAI1

<400>
cctgaggtca cctacccctg ttcctgcgaa gtcaaggggg aagaggacaa          50

<210> 246
<211> 50
<212> DNA
<213> Artificial sequence
<220>
<221> Probe
<223> SG246
<220>
<223> Designed to hybridize with cRNA of the human gene CD83

<400>
caaataagca tttagggcta gtgactcctc acaagacaga actggtatga          50

<210> 247
<211> 50
<212> DNA
<213> Artificial sequence
<220>
<221> Probe
<223> SG247
<220>
<223> Designed to hybridize with cRNA of the human gene CD86

<400>
caactcttat aaatgtggaa ccaacacaat ggagagggaa gagagtgaac          50

<210> 248
<211> 50
<212> DNA
<213> Artificial sequence
<220>
<221> Probe
<223> SG248
<220>
<223> Designed to hybridize with cRNA of the human gene TNFRSF6

<400>
cttccaaatg cagaagatgt agattgtgtg atgaaggaca tgatgtgaac          50

<210> 249
<211> 50
<212> DNA
<213> Artificial sequence
<220>
<221> Probe
<223> SG249
<220>
<223> Designed to hybridize with cRNA of the human gene TNFSF6

<400>
tgaggaactc taagtatccc caggatctgg tgatgatgga ggggaagatg          50

<210> 250
<211> 50
<212> DNA
<213> Artificial sequence
<220>
<221> Probe
<223> SG250
<220>
<223> Designed to hybridize with cRNA of the human gene SLC7A5

<400>
tgacatccca ggggctgtaa gtgccaacat gactgtgaag ggccagagtg          50

<210> 251
<211> 50
<212> DNA
<213> Artificial sequence
<220>
<221> Probe
<223> SG251
<220>
<223> Designed to hybridize with cRNA of the human gene SDC1

<400>
aaacaagcca acggcggggc ctaccagaag cccaccaaac aggaggaatt          50

<210> 252
<211> 50
<212> DNA
<213> Artificial sequence
<220>
<221> Probe
<223> SG252
<220>
<223> Designed to hybridize with cRNA of the human gene CDK4

<400>
ctggaaatgc tgacttttaa cccacacaag cgaatctctg cctttcgagc          50

<210> 253
<211> 50
<212> DNA
<213> Artificial sequence
<220>
<221> Probe
<223> SG253
<220>
<223> Designed to hybridize with cRNA of the human gene STAT3

<400>
agggcagttt gagtccctca cctttgacat ggagttgacc tcggagtgcg          50

<210> 254
<211> 50
<212> DNA
<213> Artificial sequence
<220>
<221> Probe
<223> SG254
<220>
<223> Designed to hybridize with cRNA of the human gene FGR

<400>
gactatgagg ctcgaactga ggatgacctc accttcacca agggcgagaa          50

<210> 255
<211> 50
<212> DNA
<213> Artificial sequence
<220>
<221> Probe
<223> SG255
<220>
<223> Designed to hybridize with cRNA of the human gene FOS

<400>
gagcgcagag cattggcagg aggggcaagg tggaacagtt atctccagaa          50

<210> 256
<211> 50
<212> DNA
<213> Artificial sequence
<220>
<221> Probe
<223> SG256
<220>
<223> Designed to hybridize with cRNA of the human gene CCNE1

<400>
tgtaccaagt ggagcaggtg gttgcgggca agcgttgtgc agagcccata          50

<210> 257
<211> 50
<212> DNA
<213> Artificial sequence
<220>
<221> Probe
<223> SG257
<220>
<223> Designed to hybridize with cRNA of the human gene CDA

<400>
ccgctatcca gaaggccgtc tcagaagggt acaaggattt cagggcaatt          50

<210> 258
<211> 50
<212> DNA
<213> Artificial sequence
<220>
<221> Probe
<223> SG258
<220>
<223> Designed to hybridize with cRNA of the human gene MAFB

<400>
gcaaggagga ggtgatccgg ctgaagcaga agaggcggac cctgaaaaac          50

<210> 259
<211> 50
<212> DNA
<213> Artificial sequence
<220>
<221> Probe
<223> SG259
<220>
<223> Designed to hybridize with cRNA of the human gene MPL

<400>
tgctgtacca cccacattgc caaccattcc tacctaccac taagctattg          50

<210> 260
<211> 50
<212> DNA
<213> Artificial sequence
<220>
<221> Probe
<223> SG260
<220>
<223> Designed to hybridize with cRNA of the human gene MYC

<400>
gttgcggaaa cgacgagaac agttgaaaca caaacttgaa cagctacgga          50

<210> 261
<211> 50
<212> DNA
<213> Artificial sequence
<220>
<221> Probe
<223> SG261
<220>
<223> Designed to hybridize with cRNA of the human gene MYB

<400>

agctaaaagg acagcaggtg ctaccagtaa gactgtcatc atgtgcttga          50

<210> 262
<211> 50
<212> DNA
<213> Artificial sequence
<220>
<221> Probe
<223> SG262
<220>
<223> Designed to hybridize with cRNA of the human gene CKMT1

<400>
gctggtgcag atggtggtgg acggagtgaa gctgctcatc gagatggaac          50

<210> 263
<211> 50
<212> DNA
<213> Artificial sequence
<220>
<221> Probe
<223> SG263
<220>
<223> Designed to hybridize with cRNA of the human gene CREBBP

<400>
cacgtctcac cccagactgg ttccccccac cccggactcg cagtcaccat          50

<210> 264
<211> 50
<212> DNA
<213> Artificial sequence
<220>
<221> Probe
<223> SG264
<220>
<223> Designed to hybridize with cRNA of the human gene CTGF

<400>

catcaagacc tgtgcctgcc attacaactg tcccggagac aatgacatct 50

<210> 265
<211> 50
<212> DNA
<213> Artificial sequence
<220>
<221> Probe
<223> SG265
<220>
<223> Designed to hybridize with cRNA of the human gene CXCR3

<400>
agctttgacc gctacctgaa catagttcat gccacccagc tctaccgccg 50

<210> 266
<211> 50
<212> DNA
<213> Artificial sequence
<220>
<221> Probe
<223> SG266
<220>
<223> Designed to hybridize with cRNA of the human gene CXCR4

<400>
attcatctgt ttccactgag tctgagtctt caagttttca ctccagctaa 50

<210> 267
<211> 50
<212> DNA
<213> Artificial sequence
<220>
<221> Probe
<223> SG267
<220>
<223> Designed to hybridize with cRNA of the human gene CCNA1

<400>
gcaatcatgt accctggatc ttttattggg ggctggggag aagagtatct 50

<210> 268
<211> 50
<212> DNA
<213> Artificial sequence
<220>
<221> Probe
<223> SG268
<220>
<223> Designed to hybridize with cRNA of the human gene CCNB1

<400>
ggacttacaa agcacatgac tgtcaagaac aagtatgcca catcgaagca          50


<210> 269
<211> 50
<212> DNA
<213> Artificial sequence
<220>
<221> Probe
<223> SG269
<220>
<223> Designed to hybridize with cRNA of the human gene CCMD2

<400>
aactggacca agccagcacc cctacagacg tgcgggatat cgacctgtga          50


<210> 270
<211> 50
<212> DNA
<213> Artificial sequence
<220>
<221> Probe
<223> SG270
<220>
<223> Designed to hybridize with cRNA of the human gene CYP1A1

<400>
accaagaact gcttagccta gtcaacctga ataataattt cggggaggtg          50

<210> 271
<211> 50
<212> DNA
<213> Artificial sequence
<220>
<221> Probe
<223> SG271
<220>
<223> Designed to hybridize with cRNA of the human gene CYP2A6

<400>
gcgcatgatg ctaggaatct tccagttcac gtcaacctcc acggggcagc          50

<210> 272
<211> 50
<212> DNA
<213> Artificial sequence
<220>
<221> Probe
<223> SG272
<220>
<223> Designed to hybridize with cRNA of the human gene DAD-1

<400>
cggtttgcct gagaatacag atcaacccac agaacaaagc ggatttccaa          50

<210> 273
<211> 50
<212> DNA
<213> Artificial sequence
<220>
<221> Probe
<223> SG273
<220>
<223> Designed to hybridize with cRNA of the human gene TNFRSF10C

<400>
tcatgcacca tcgtagggat catagttcta attgtgcttc tgattgtgtt          50

<210> 274
<211> 50
<212> DNA
<213> Artificial sequence
<220>
<221> Probe
<223> SG274
<220>
<223> Designed to hybridize with cRNA of the human gene DEK

<400>
acagaaagct acttctaaaa gtaaaaaatc tgtgaaaagt gccaatgtta          50

<210> 275
<211> 50
<212> DNA
<213> Artificial sequence
<220>
<221> Probe
<223> SG275
<220>
<223> Designed to hybridize with cRNA of the human gene DCK

<400>
gctggctcct gcataggaca ctgaaaacca acttcgatta tcttcaagag          50

<210> 276
<211> 50
<212> DNA
<213> Artificial sequence
<220>
<221> Probe
<223> SG276
<220>
<223> Designed to hybridize with cRNA of the human gene DHFR

<400>
ccaggtgttc tctctgatgt ccaggaggag aaaggcatta agtacaaatt          50

<210> 277
<211> 50
<212> DNA
<213> Artificial sequence
<220>
<221> Probe
<223> SG277
<220>
<223> Designed to hybridize with cRNA of the human gene TCF3

<400>
tcaggtgtgg ttggagaccc ccagatggtg ctttcagctc cccacccagg          50

<210> 278
<211> 50
<212> DNA
<213> Artificial sequence
<220>
<221> Probe
<223> SG278
<220>
<223> Designed to hybridize with cRNA of the human gene E2F1

<400>
tcagagacct cttcgactgt gactttgggg acctcacccc cctggatttc          50

<210> 279
<211> 50
<212> DNA
<213> Artificial sequence
<220>
<221> Probe
<223> SG279
<220>
<223> Designed to hybridize with cRNA of the human gene TCF3

<400>
tgccagatgc acctcaagtc ggacaaagcg cagaccaagc tgctcatcct          50

<210> 280
<211> 50
<212> DNA
<213> Artificial sequence
<220>
<221> Probe
<223> SG280
<220>
<223> Designed to hybridize with cRNA of the human gene EB-1

<400>
tactttgatg atattccccg atcaaaactg gagaggcaga tggctcagac          50

<210> 281
<211> 50
<212> DNA
<213> Artificial sequence
<220>
<221> Probe
<223> SG281
<220>
<223> Designed to hybridize with cRNA of the human gene CCR7

<400>
gccttcatcg gcgtcaagtt ccgcaacgat ctcttcaagc tcttcaagga          50

<210> 282
<211> 50
<212> DNA
<213> Artificial sequence
<220>
<221> Probe
<223> SG282
<220>
<223> Designed to hybridize with cRNA of the human gene EBI2

<400>
gtcagtgtat cgatttctag tgctgtgaag tcagcccctg aagaaaattc          50

<210> 283
<211> 50
<212> DNA
<213> Artificial sequence
<220>
<221> Probe
<223> SG283
<220>
<223> Designed to hybridize with cRNA of the human gene MUC-1

<400>
tacctcctct cacctcctcc aatcacagca cttctcccca gttgtctact          50

<210> 284
<211> 50
<212> DNA
<213> Artificial sequence
<220>
<221> Probe
<223> SG284
<220>
<223> Designed to hybridize with cRNA of the human gene EphA3

<400>
atgtgggcat tcagctccga cggcccttgt tccagaaggt aaccacggtg          50

<210> 285
<211> 50
<212> DNA
<213> Artificial sequence
<220>
<221> Probe
<223> SG285
<220>
<223> Designed to hybridize with cRNA of the human gene EPOR

<400>
tcagaagcat cctcctgctc atctgctttg gctaccttcc agcataacta          50

<210> 286

<211> 50
<212> DNA
<213> Artificial sequence
<220>
<221> Probe
<223> SG286
<220>
<223> Designed to hybridize with cRNA of the human gene ERCC1

<400>
aaacggacag tcagaccctc ctgaccacat ttggatctct ggaacagctc          50


<210> 287
<211> 50
<212> DNA
<213> Artificial sequence
<220>
<221> Probe
<223> SG287
<220>
<223> Designed to hybridize with cRNA of the human gene ERCC2

<400>
tcaggggcaa gacggactac ggcctcatgg tctttgccga caagcggttt          50



<210> 288
<211> 50
<212> DNA
<213> Artificial sequence
<220>
<221> Probe
<223> SG288
<220>
<223> Designed to hybridize with cRNA of the human gene ERCC3

<400>
cggcgctttg gcaccatgag ttctatgtct ggggccgacg acactgtgta          50


<210> 289

<211> 50
<212> DNA
<213> Artificial sequence
<220>
<221> Probe
<223> SG289
<220>
<223> Designed to hybridize with cRNA of the human gene ERCC5

<400>
tagtgatgac gatggaggga aagagaagat ggtcctcgtg accgccagat          50


<210> 290
<211> 50
<212> DNA
<213> Artificial sequence
<220>
<221> Probe
<223> SG290
<220>
<223> Designed to hybridize with cRNA of the human gene ERCC6

<400>
tgcactttcc atagaacttc tggtggtgaa ggaatttgga aactcaagcc          50


<210> 291
<211> 50
<212> DNA
<213> Artificial sequence
<220>
<221> Probe
<223> SG291
<220>
<223> Designed to hybridize with cRNA of the human gene CUZD1

<400>
cacagtgagg cattttgtaa atcaacgggc agactacaaa taccagaagc          50


<210> 292
<211> 50

<212> DNA
<213> Artificial sequence
<220>
<221> Probe
<223> SG292
<220>
<223> Designed to hybridize with cRNA of the human gene ETV6

<400>
aatcatgagt ggccgaacag accgtctgga gcacctagag tcccaggagc          50



<210> 293
<211> 50
<212> DNA
<213> Artificial sequence
<220>
<221> Probe
<223> SG293
<220>
<223> Designed to hybridize with cRNA of the human gene EIF4E

<400>
attatggaat acaaaactca caccgacagc atcaaaatgc caggcaggct          50



<210> 294
<211> 50
<212> DNA
<213> Artificial sequence
<220>
<221> Probe
<223> SG294
<220>
<223> Designed to hybridize with cRNA of the human gene EIF5A

<400>
ccttggcaag gagattgagc agaagtacga ctgtggagaa gagatcctga          50



<210> 295
<211> 50

<212> DNA
<213> Artificial sequence
<220>
<221> Probe
<223> SG295
<220>
<223> Designed to hybridize with cRNA of the human gene EZH2

<400>
tccacggcag ccttgtgaca gttcgtgccc ttgtgtgata gcacaaaatt          50


<210> 296
<211> 50
<212> DNA
<213> Artificial sequence
<220>
<221> Probe
<223> SG296
<220>
<223> Designed to hybridize with cRNA of the human gene FADD

<400>
atgtccccga tgtcatggaa ctcagacgca tctacctccg aagcgtcctg          50


<210> 297
<211> 50
<212> DNA
<213> Artificial sequence
<220>
<221> Probe
<223> SG297
<220>
<223> Designed to hybridize with cRNA of the human gene PTK2

<400>
gcacccaccg agagattgag atggcacaga agctattgaa ctctgacctg          50


<210> 298
<211> 50

<212> DNA
<213> Artificial sequence
<220>
<221> Probe
<223> SG298
<220>
<223> Designed to hybridize with cRNA of the human gene FAT

<400>
ttgtagagaa ccccatgccc cttacccgcc agggtatcaa agacacttcg          50


<210> 299
<211> 50
<212> DNA
<213> Artificial sequence
<220>
<221> Probe
<223> SG299
<220>
<223> Designed to hybridize with cRNA of the human gene FGFR1

<400>
ttcttctggt tcggccatca cggctctcct ccagtgggac tcccatgcta          50


<210> 300
<211> 50
<212> DNA
<213> Artificial sequence
<220>
<221> Probe
<223> SG300
<220>
<223> Designed to hybridize with cRNA of the human gene FGFR3

<400>
aggagctaga ggttctctcc ttgcacaacg tcacctttga ggacgccggg          50


<210> 301
<211> 50
<212> DNA

<213> Artificial sequence
<220>
<221> Probe
<223> SG301
<220>
<223> Designed to hybridize with cRNA of the human gene FHIT

<400>
tcctgcctct tggagatcag aggaggaaat ggcagcagaa gccgcagctc          50


<210> 302
<211> 50
<212> DNA
<213> Artificial sequence
<220>
<221> Probe
<223> SG302
<220>
<223> Designed to hybridize with cRNA of the human gene FLT3

<400>
tggagcgcgt gaacacggag atacactttg tcaccaaatg tgcctttcag          50


<210> 303
<211> 50
<212> DNA
<213> Artificial sequence
<220>
<221> Probe
<223> SG303
<220>
<223> Designed to hybridize with cRNA of the human gene GRIA3

<400>
gcgatacttg attgactgcg aagtcgaaag gattaacaca attttggaac          50


<210> 304
<211> 50
<212> DNA

<213> Artificial sequence
<220>
<221> Probe
<223> SG304
<220>
<223> Designed to hybridize with cRNA of the human gene FUS

<400>
gggacagccc atgattaatt tgtacacaga cagggaaact ggcaagctga          50


<210> 305
<211> 50
<212> DNA
<213> Artificial sequence
<220>
<221> Probe
<223> SG305
<220>
<223> Designed to hybridize with cRNA of the human gene EIF4E

<400>
cccacgcaga cacagctact aagagcggct ccaccactaa aaataggttt          50


<210> 306
<211> 50
<212> DNA
<213> Artificial sequence
<220>
<221> Probe
<223> SG306
<220>
<223> Designed to hybridize with cRNA of the human gene SIAT4A

<400>
agactttgag tctaacgtga cggccacctt ggcctccatc aataaaatcc          50


<210> 307
<211> 50
<212> DNA
<213> Artificial sequence

<220>
<221> Probe
<223> SG307
<220>
<223> Designed to hybridize with cRNA of the human gene CSF3

<400>
gcttcctgga ggtgtcgtac cgcgttctac gccaccttgc ccagccctga          50

<210> 308
<211> 50
<212> DNA
<213> Artificial sequence
<220>
<221> Probe
<223> SG308
<220>
<223> Designed to hybridize with cRNA of the human gene CSF2

<400>
ccctccaacc ccggaaactt cctgtgcaac ccagattatc acctttgaaa          50

<210> 309
<211> 50
<212> DNA
<213> Artificial sequence
<220>
<221> Probe
<223> SG309
<220>
<223> Designed to hybridize with cRNA of the human gene NR3C1

<400>
cagccagaac tggcagcggt tttatcaact gacaaaactc ttggattcta          50

<210> 310
<211> 50
<212> DNA
<213> Artificial sequence

<220>
<221> Probe
<223> SG310
<220>
<223> Designed to hybridize with cRNA of the human gene GSTT1

<400>
ataaggtgat gttccctgtg ttcctgggtg ggccagtatc tccccagaca          50


<210> 311
<211> 50
<212> DNA
<213> Artificial sequence
<220>
<221> Probe
<223> SG311
<220>
<223> Designed to hybridize with cRNA of the human gene GZMA

<400>
ggcctggtgt ctatattctt ctctcaaaga aacacctcaa ctggataatt          50


<210> 312
<211> 50
<212> DNA
<213> Artificial sequence
<220>
<221> Probe
<223> SG312
<220>
<223> Designed to hybridize with cRNA of the human gene DNAJA1

<400>
agatcttctg ccatctagac cggaagttcc taacataatt ggagaaacag          50


<210> 313
<211> 50
<212> DNA
<213> Artificial sequence

<220>
<221> Probe
<223> SG313
<220>
<223> Designed to hybridize with cRNA of the human gene HLF

<400>
gatcctgaca ccatccaggt cccagtgggt tatgagccag acccagcaga          50


<210> 314
<211> 50
<212> DNA
<213> Artificial sequence
<220>
<221> Probe
<223> SG314
<220>
<223> Designed to hybridize with cRNA of the human gene BST2

<400>
agaagagaaa accaggtctt aagcgtgaga atcgcggaca agaagtacta          50


<210> 315
<211> 50
<212> DNA
<213> Artificial sequence
<220>
<221> Probe
<223> SG315
<220>
<223> Designed to hybridize with cRNA of the human gene HOXA9

<400>
ggggtgactg tcccacgctt gacactcaca ctttgtccct gactgactat          50


<210> 316
<211> 50
<212> DNA
<213> Artificial sequence
<220>

<221> Probe
<223> SG316
<220>
<223> Designed to hybridize with cRNA of the human gene TLX1

<400>
gacaggttca caggtcaccc ctatcagaac cggacgcccc ccaagaagaa          50


<210> 317
<211> 50
<212> DNA
<213> Artificial sequence
<220>
<221> Probe
<223> SG317
<220>
<223> Designed to hybridize with cRNA of the human gene UHRF1

<400>
cgctacgatg gcatctacaa ggttgtgaaa tactggcccg agaaggggaa          50


<210> 318
<211> 50
<212> DNA
<213> Artificial sequence
<220>
<221> Probe
<223> SG318
<220>
<223> Designed to hybridize with cRNA of the human gene IER3

<400>
aaaaggcttc tctttctgct gctcaccatc gtcttctgcc agatcctgat          50


<210> 319
<211> 50
<212> DNA
<213> Artificial sequence
<220>

<221> Probe
<223> SG319
<220>
<223> Designed to hybridize with cRNA of the human gene IL1B

<400>
cagaccttcc aggagaatga cctgagcacc ttctttccct tcatctttga          50


<210> 320
<211> 50
<212> DNA
<213> Artificial sequence
<220>
<221> Probe
<223> SG320
<220>
<223> Designed to hybridize with cRNA of the human gene IL2

<400>
agcaaccatt gtagaatttc tgaacagatg gattaccttt tgtcaaagca          50


<210> 321
<211> 50
<212> DNA
<213> Artificial sequence
<220>
<221> Probe
<223> SG321
<220>
<223> Designed to hybridize with cRNA of the human gene IL3

<400>
gcacccacgc gacatccaat ccatatcaag gacggtgact ggaatgaatt          50


<210> 322
<211> 50
<212> DNA
<213> Artificial sequence
<220>
<221> Probe

<223> SG322
<220>
<223> Designed to hybridize with cRNA of the human gene IL6

<400>
gaacaagcca gagctgtcca gatgagtaca aaagtcctga tccagttcct          50

<210> 323
<211> 50
<212> DNA
<213> Artificial sequence
<220>
<221> Probe
<223> SG323
<220>
<223> Designed to hybridize with cRNA of the human gene IL6R

<400>
cacggagccc ttatgacatc agcaatacag actacttctt ccccagatag          50

<210> 324
<211> 50
<212> DNA
<213> Artificial sequence
<220>
<221> Probe
<223> SG324
<220>
<223> Designed to hybridize with cRNA of the human gene IL10

<400>
ccaagagaaa ggcatctaca aagccatgag tgagtttgac atcttcatca          50

<210> 325
<211> 50
<212> DNA
<213> Artificial sequence
<220>
<221> Probe

<223> SG325
<220>
<223> Designed to hybridize with cRNA of the human gene IL15

<400>
atacggaaag tgatgttcac cccagttgca aagtaacagc aatgaagtgc          50


<210> 326
<211> 50
<212> DNA
<213> Artificial sequence
<220>
<221> Probe
<223> SG326
<220>
<223> Designed to hybridize with cRNA of the human gene IRF4

<400>
atccagaaga ttaccacaga tctatccgcc attcctctat tcaagaatga          50


<210> 327
<211> 50
<212> DNA
<213> Artificial sequence
<220>
<221> Probe
<223> SG327
<220>
<223> Designed to hybridize with cRNA of the human gene JAK1

<400>
aacgcctgcc gtgcccacct aactgtccag atgaggttta tcagcttatg          50


<210> 328
<211> 50
<212> DNA
<213> Artificial sequence
<220>
<221> Probe

<223> SG328
<220>
<223> Designed to hybridize with cRNA of the human gene JAK2

<400>
aatcaacgcc cctcctttag ggatctagct cttcgagtgg atcaaataag          50


<210> 329
<211> 50
<212> DNA
<213> Artificial sequence
<220>
<221> Probe
<223> SG329
<220>
<223> Designed to hybridize with cRNA of the human gene MKI67

<400>
tgtgtcaaga aaataacaac cagaagtcat agggacagtg aagatatttg          50


<210> 330
<211> 50
<212> DNA
<213> Artificial sequence
<220>
<221> Probe
<223> SG330
<220>
<223> Designed to hybridize with cRNA of the human gene XRCC5

<400>
tgggaaattg ttgtccagga tggaattact ctgatcacca aagaggaagc          50


<210> 331
<211> 50
<212> DNA
<213> Artificial sequence
<220>
<221> Probe
<223> SG331

<220>
<223> Designed to hybridize with cRNA of the human gene LAG3

<400>
agcaagggat tcaccctcgc caggctcaga cgcaagatag aggagctgga          50


<210> 332
<211> 50
<212> DNA
<213> Artificial sequence
<220>
<221> Probe
<223> SG332
<220>
<223> Designed to hybridize with cRNA of the human gene LCK

<400>
cccttcaggc atcaagttga ccatcaacaa actcctggac atggcagccc          50


<210> 333
<211> 50
<212> DNA
<213> Artificial sequence
<220>
<221> Probe
<223> SG333
<220>
<223> Designed to hybridize with cRNA of the human gene LCP1

<400>
tttcaaggac ccgaagatta gtacaagtct gcctgttctg gacctcatcg          50


<210> 334
<211> 50
<212> DNA
<213> Artificial sequence
<220>
<221> Probe
<223> SG334

<220>
<223> Designed to hybridize with cRNA of the human gene LEPR

<400>
agaattgttc ctgggcacaa ggacttaatt ttcagaagag aacggacatt          50


<210> 335
<211> 50
<212> DNA
<213> Artificial sequence
<220>
<221> Probe
<223> SG335
<220>
<223> Designed to hybridize with cRNA of the human gene LMO2

<400>
tgtgcgaaca ggacatctac gagtggacta agatcaatgg gatgatatag          50


<210> 336
<211> 50
<212> DNA
<213> Artificial sequence
<220>
<221> Probe
<223> SG336
<220>
<223> Designed to hybridize with cRNA of the human gene LIF

<400>
cgtgacggac ttcccgccct tccacgccaa cggcacggag aaggccaagc          50


<210> 337
<211> 50
<212> DNA
<213> Artificial sequence
<220>
<221> Probe
<223> SG337
<220>

<223> Designed to hybridize with cRNA of the human gene LRP

<400>
ctggaccagt cagaagccga gaaagctcgc aaggaacttt tggagctgga          50

<210> 338
<211> 50
<212> DNA
<213> Artificial sequence
<220>
<221> Probe
<223> SG338
<220>
<223> Designed to hybridize with cRNA of the human gene LSP1

<400>
ggaccgagtc cctaaaccgc tccatagaga agagtaacag tgtgaagaaa          50

<210> 339
<211> 50
<212> DNA
<213> Artificial sequence
<220>
<221> Probe
<223> SG339
<220>
<223> Designed to hybridize with cRNA of the human gene LYL1

<400>
agtgtctaca ttgggccagc aggacctttt agcatcttcc ctagcagccg          50

<210> 340
<211> 50
<212> DNA
<213> Artificial sequence
<220>
<221> Probe
<223> SG340
<220>

<223> Designed to hybridize with cRNA of the human gene NFKB1

<400>
catccttccg caaactcagc tttaccgagt ctctgaccag tggtgcctca          50


<210> 341
<211> 50
<212> DNA
<213> Artificial sequence
<220>
<221> Probe
<223> SG341
<220>
<223> Designed to hybridize with cRNA of the human gene MAL

<400>
caccttgatc atcctgtaca taattggagc ccacggtgga gagacttcct          50


<210> 342
<211> 50
<212> DNA
<213> Artificial sequence
<220>
<221> Probe
<223> SG342
<220>
<223> Designed to hybridize with cRNA of the human gene MAGEA1

<400>
ctcctcccct cctctcctct cagctagtcc atgtttctcc aacacaagtt          50

<210> 343
<211> 50
<212> DNA
<213> Artificial sequence
<220>
<221> Probe
<223> SG343
<220>
<223> Designed to hybridize with cRNA of the human gene MAPK10

<400>
agactaaaaa tggtgtagta aaaggacagc cttctccttc agcacaggtg          50

<210> 344
<211> 50
<212> DNA
<213> Artificial sequence
<220>
<221> Probe
<223> SG344
<220>
<223> Designed to hybridize with cRNA of the human gene MBP1

<400>
ccatgcagag tctgtcctcc cagtcggtgc agcaaactca gggaagccat          50

<210> 345
<211> 50
<212> DNA
<213> Artificial sequence
<220>
<221> Probe
<223> SG345
<220>
<223> Designed to hybridize with cRNA of the human gene MCL1

<400>
ggctcccagt gactactttt tgacttctgt ttgtcttacg cttctctcag          50

<210> 346
<211> 50
<212> DNA
<213> Artificial sequence
<220>
<221> Probe
<223> SG346
<220>
<223> Designed to hybridize with cRNA of the human gene MDM2

<400>

tgaatctctc gactcagaag attatagcct tagtgaagaa ggacaagaac 50

<210> 347
<211> 50
<212> DNA
<213> Artificial sequence
<220>
<221> Probe
<223> SG347
<220>
<223> Designed to hybridize with cRNA of the human gene ABCB1

<400>
tagctcgtgc ccttgttaga cagcctcata ttttgctttt ggatgaagcc 50

<210> 348
<211> 50
<212> DNA
<213> Artificial sequence
<220>
<221> Probe
<223> SG348
<220>
<223> Designed to hybridize with cRNA of the human gene ABCB4

<400>
tagcgatttt tggaccaggc gatgatgcag tgaagcagca gaagtgcaac 50

<210> 349
<211> 50
<212> DNA
<213> Artificial sequence
<220>
<221> Probe
<223> SG349
<220>
<223> Designed to hybridize with cRNA of the human gene MEN1

<400>
attgcctcag ccaagaccta ctatcgggat gaacacatct acccctacat 50

<210> 350
<211> 50
<212> DNA
<213> Artificial sequence
<220>
<221> Probe
<223> SG350
<220>
<223> Designed to hybridize with cRNA of the human gene CXCL9

<400>
gaaggaactg attaaaaagt gggagaaaca ggtcagccaa aagaaaaagc          50


<210> 351
<211> 50
<212> DNA
<213> Artificial sequence
<220>
<221> Probe
<223> SG351
<220>
<223> Designed to hybridize with cRNA of the human gene MLF1

<400>
ggggacaaac tccacatcaa aggctcatct gtgaaaagca acaaaaaata          50


<210> 352
<211> 50
<212> DNA
<213> Artificial sequence
<220>
<221> Probe
<223> SG352
<220>
<223> Designed to hybridize with cRNA of the human gene MLL

<400>
gaaaacagga ccattgccca tagtccaaca tcttttacag aaagttcatc          50

<210> 353
<211> 50
<212> DNA
<213> Artificial sequence
<220>
<221> Probe
<223> SG353
<220>
<223> Designed to hybridize with cRNA of the human gene MMP9

<400>
agtgagttga accaggtgga ccaagtgggc tacgtgacct atgacatcct          50

<210> 354
<211> 50
<212> DNA
<213> Artificial sequence
<220>
<221> Probe
<223> SG354
<220>
<223> Designed to hybridize with cRNA of the human gene NDUFB

<400>
ccggaacaag agtatgttat ttaaaaggga attgcaaccc agtgaagaag          50

<210> 355
<211> 50
<212> DNA
<213> Artificial sequence
<220>
<221> Probe
<223> SG355
<220>
<223> Designed to hybridize with cRNA of the human gene MPO

<400>
atcaccaccg tgtctaagaa caacatcttc atgtccaact catatccccg          50

<210> 356
<211> 50
<212> DNA
<213> Artificial sequence
<220>
<221> Probe
<223> SG356
<220>
<223> Designed to hybridize with cRNA of the human gene ABCC3

<400>
ccaggtaaag caaatgaaat tgaaggactc gcgcatcaag ctgatgagtg 50

<210> 357
<211> 50
<212> DNA
<213> Artificial sequence
<220>
<221> Probe
<223> SG357
<220>
<223> Designed to hybridize with cRNA of the human gene ABCC5

<400>
catcggtcct tctgtccaac gacagttccc gattctatgc catgtttgct 50

<210> 358
<211> 50
<212> DNA
<213> Artificial sequence
<220>
<221> Probe
<223> SG358
<220>
<223> Designed to hybridize with cRNA of the human gene MTCP1

<400>
cagatacagc atcatttaat ggtcagggga gtacaggagc tgttgcttaa                50

<210> 359
<211> 50
<212> DNA
<213> Artificial sequence
<220>
<221> Probe
<223> SG359
<220>
<223> Designed to hybridize with cRNA of the human gene MTCP1

<400>
gtgttgtgct cagtatccca agggaagatc tgtcgtctgt tcaggatttg                50

<210> 360
<211> 50
<212> DNA
<213> Artificial sequence
<220>
<221> Probe
<223> SG360
<220>
<223> Designed to hybridize with cRNA of the human gene MYOD1

<400>
ttgccacaac ggacgacttc tatgacgacc cgtgtttcga ctccccggac                50

<210> 361
<211> 50
<212> DNA
<213> Artificial sequence
<220>
<221> Probe
<223> SG361
<220>
<223> Designed to hybridize with cRNA of the human gene MX1

<400>
cgtcctgtcg gagccctgtc tctctctctg taataaactc atttctagca          50

<210> 362
<211> 50
<212> DNA
<213> Artificial sequence
<220>
<221> Probe
<223> SG362
<220>
<223> Designed to hybridize with cRNA of the human gene NINJ1

<400>
agtacgacct taacaacccg gacaagcacg ccaagctgga cttcctcaac          50

<210> 363
<211> 50
<212> DNA
<213> Artificial sequence
<220>
<221> Probe
<223> SG363
<220>
<223> Designed to hybridize with cRNA of the human gene NPM1

<400>
tagagtgatg gattgatacc tagcagcctg tgaaaaggaa tcagtgaatt          50

<210> 364
<211> 50
<212> DNA
<213> Artificial sequence
<220>
<221> Probe
<223> SG364
<220>
<223> Designed to hybridize with cRNA of the human gene IL32

<400>
aggaggagct gacaccccag aagtgctctg aaccccaatc ctcaaaatga          50

<210> 365
<211> 50
<212> DNA
<213> Artificial sequence
<220>
<221> Probe
<223> SG365
<220>
<223> Designed to hybridize with cRNA of the human gene NUMA1

<400>
ccccaacact cgcagtggaa cccgccgttc tccgcgcatt gccaccacca          50


<210> 366
<211> 50
<212> DNA
<213> Artificial sequence
<220>
<221> Probe
<223> SG366
<220>
<223> Designed to hybridize with cRNA of the human gene POU2F2

<400>
ccaactggcc ccagtacaaa gatcaaggct gaagacccca gtggcgattc          50


<210> 367
<211> 50
<212> DNA
<213> Artificial sequence
<220>
<221> Probe
<223> SG367
<220>
<223> Designed to hybridize with cRNA of the human gene POU2F2

<400>
tcccaagctt ccagcagtct gagcacaaca gttactacct tatcctcagc          50

<210> 368
<211> 50
<212> DNA
<213> Artificial sequence
<220>
<221> Probe
<223> SG368
<220>
<223> Designed to hybridize with cRNA of the human gene OGGI

<400>
gaccccatcg aatgcctttt ctcttttatc tgttcctcca acaacaacat          50

<210> 369
<211> 50
<212> DNA
<213> Artificial sequence
<220>
<221> Probe
<223> SG369
<220>
<223> Designed to hybridize with cRNA of the human gene CDKN2B

<400>
gcggatccca acggagtcaa ccgtttcggg aggcgcgcga tccaggtcat          50

<210> 370
<211> 50
<212> DNA
<213> Artificial sequence
<220>
<221> Probe
<223> SG370
<220>
<223> Designed to hybridize with cRNA of the human gene CDKN2A

<400>
tgaggagcca gcgtctaggg cagcagccgc ttcctagaag accaggtcat          50

<210> 371

<211> 50
<212> DNA
<213> Artificial sequence
<220>
<221> Probe
<223> SG371
<220>
<223> Designed to hybridize with cRNA of the human gene CDKN2C

<400>
ttagcctgat gcaggcaaac ggggctgggg gagccacaaa tcttcaataa          50


<210> 372
<211> 50
<212> DNA
<213> Artificial sequence
<220>
<221> Probe
<223> SG372
<220>
<223> Designed to hybridize with cRNA of the human gene CDKN1A

<400>
gaggcccgtg agcgatggaa cttcgacttt gtcaccgaga caccactgga          50


<210> 373
<211> 50
<212> DNA
<213> Artificial sequence
<220>
<221> Probe
<223> SG373
<220>
<223> Designed to hybridize with cRNA of the human gene CDKN1B

<400>
cctgcaaccg acgattcttc tactcaaaac aaaagagcca acagaacaga          50


<210> 374
<211> 50

<212> DNA
<213> Artificial sequence
<220>
<221> Probe
<223> SG374
<220>
<223> Designed to hybridize with cRNA of the human gene CDK5R1

<400>
agaacctgaa gaagtcgctg tcgtgcgcca acctgtccac attcgcccag          50

<210> 375
<211> 50
<212> DNA
<213> Artificial sequence
<220>
<221> Probe
<223> SG375
<220>
<223> Designed to hybridize with cRNA of the human gene TOPORS

<400>
aaccagccca gtaacattgt gtctcttcaa actgagccat caaggcaatt          50

<210> 376
<211> 50
<212> DNA
<213> Artificial sequence
<220>
<221> Probe
<223> SG376
<220>
<223> Designed to hybridize with cRNA of the human gene P55CDC

<400>
ggatgcccat tcccaggtgt gctccatcct ctggtctccc cattacaagg          50

<210> 377

<211> 50
<212> DNA
<213> Artificial sequence
<220>
<221> Probe
<223> SG377
<220>
<223> Designed to hybridize with cRNA of the human gene CDKN1C

<400>
cgcctgagaa gtcgtcgggc gatgtccccg cgccgtgtcc ctctccaagc          50


<210> 378
<211> 50
<212> DNA
<213> Artificial sequence
<220>
<221> Probe
<223> SG378
<220>
<223> Designed to hybridize with cRNA of the human gene TP73

<400>
ctgcatcgag tatttcacct cccaagggtt acagagcatt taccacctgc          50


<210> 379
<211> 50
<212> DNA
<213> Artificial sequence
<220>
<221> Probe
<223> SG379
<220>
<223> Designed to hybridize with cRNA of the human gene PAX5

<400>
cccccccgct ggacagggca gctactcagc accgacgctg acagggatgg          50

<210> 380
<211> 50
<212> DNA
<213> Artificial sequence
<220>
<221> Probe
<223> SG380
<220>
<223> Designed to hybridize with cRNA of the human gene PBX1

<400>
acagtccgca gggcatcagt gctaatggag gttggcagga tgctactacc          50

<210> 381
<211> 50
<212> DNA
<213> Artificial sequence
<220>
<221> Probe
<223> SG381
<220>
<223> Designed to hybridize with cRNA of the human gene PBX3

<400>
ccaaattccg gttcttctgg ttcttttaac ctcccaaatt ctggggacat          50

<210> 382
<211> 50
<212> DNA
<213> Artificial sequence
<220>
<221> Probe
<223> SG382
<220>
<223> Designed to hybridize with cRNA of the human gene ENPP1

<400>
aaacatctat gctgtcaaga ccgccgtgtc agtcacccag gggggccaca          50

<210> 383

<211> 50
<212> DNA
<213> Artificial sequence
<220>
<221> Probe
<223> SG383
<220>
<223> Designed to hybridize with cRNA of the human gene PCA1

<400>
tcactggact cagcttctat caacaaagaa aagagccagt ttcagacatt          50


<210> 384
<211> 50
<212> DNA
<213> Artificial sequence
<220>
<221> Probe
<223> SG384
<220>
<223> Designed to hybridize with cRNA of the human gene PCNA

<400>
gctcctgaag ccgaaaccag ctagactttc ctccttcccg cctgcctgta          50

<210> 385
<211> 50
<212> DNA
<213> Artificial sequence
<220>
<221> Probe
<223> SG385
<220>
<223> Designed to hybridize with cRNA of the human gene PDGFRB

<400>
acgactatat catccccctg cctgacccca aacccgaggt tgctgacgag          50


<210> 386
<211> 50
<212> DNA

<213> Artificial sequence
<220>
<221> Probe
<223> SG386
<220>
<223> Designed to hybridize with cRNA of the human gene PRKCQ

<400>
ggctgtcatt tgccgacaga gcactgatca acagcatgga ccagaatatg          50


<210> 387
<211> 50
<212> DNA
<213> Artificial sequence
<220>
<221> Probe
<223> SG387
<220>
<223> Designed to hybridize with cRNA of the human gene PLK1

<400>
cctcagcaac ggcagcgtgc agatcaactt cttccaggat cacaccaagc          50


<210> 388
<211> 50
<212> DNA
<213> Artificial sequence
<220>
<221> Probe
<223> SG388
<220>
<223> Designed to hybridize with cRNA of the human gene PML

<400>
actcaagaac ctggcccaga cctacctggc gagaaacatg agcgagcgca          50


<210> 389
<211> 50
<212> DNA
<213> Artificial sequence

<220>
<221> Probe
<223> SG389
<220>
<223> Designed to hybridize with cRNA of the human gene PRAME

<400>
tttgatgagt gtgggatcac ggatgatcag ctccttgccc tcctgccttc          50

<210> 390
<211> 50
<212> DNA
<213> Artificial sequence
<220>
<221> Probe
<223> SG390
<220>
<223> Designed to hybridize with cRNA of the human gene PRKCI

<400>
tctcagttta ctaatgaacc tgtccagctc actccagatg acgatgacat          50


<210> 391
<211> 50
<212> DNA
<213> Artificial sequence
<220>
<221> Probe
<223> SG391
<220>
<223> Designed to hybridize with cRNA of the human gene PTEN

<400>
atattctgac accactgact ctgatccaga gaatgaacct tttgatgaag          50


<210> 392
<211> 50
<212> DNA
<213> Artificial sequence
<220>
<221> Probe

<223> SG392
<220>
<223> Designed to hybridize with cRNA of the human gene PTGS1

<400>
gtacagctac gagcagttct tgttcaacac ctccatgttg gtggactatg          50


<210> 393
<211> 50
<212> DNA
<213> Artificial sequence
<220>
<221> Probe
<223> SG393
<220>
<223> Designed to hybridize with cRNA of the human gene PTHLH

<400>
agtgactggg agtgggctag aaggggacca cctgtctgac acctccacaa          50


<210> 394
<211> 50
<212> DNA
<213> Artificial sequence
<220>
<221> Probe
<223> SG394
<220>
<223> Designed to hybridize with cRNA of the human gene SPI1

<400>
gatctatacc aacgccaaac gcacgagtat tacccctatc tcagcagtga          50

<210> 395
<211> 50
<212> DNA
<213> Artificial sequence
<220>
<221> Probe
<223> SG395
<220>

<223> Designed to hybridize with cRNA of the human gene PTK2B

<400>
ggagcgtgga tgatctcctg ccttccttgc cgtcatcttc acggacagag          50

<210> 396
<211> 50
<212> DNA
<213> Artificial sequence
<220>
<221> Probe
<223> SG396
<220>
<223> Designed to hybridize with cRNA of the human gene RAD51

<400>
ggttccttga gcacccggaa acggtcacga gacccagagg aagaattata          50

<210> 397
<211> 50
<212> DNA
<213> Artificial sequence
<220>
<221> Probe
<223> SG397
<220>
<223> Designed to hybridize with cRNA of the human gene RAG1

<400>
ggcaagctta ggggacccat taggcataga ggactctctg gaaagccaag          50

<210> 398
<211> 50
<212> DNA
<213> Artificial sequence
<220>
<221> Probe
<223> SG398
<220>

<223> Designed to hybridize with cRNA of the human gene RARA

<400>
cagcttccag ttagtggata tagcacacca tccccagcca ccattgagac          50

<210> 399
<211> 50
<212> DNA
<213> Artificial sequence
<220>
<221> Probe
<223> SG399
<220>
<223> Designed to hybridize with cRNA of the human gene KRAS2

<400>
gttgatgatg ccttctatac attagttcga gaaattcgaa aacataaaga          50
<210> 400
<211> 50
<212> DNA
<213> Artificial sequence
<220>
<221> Probe
<223> SG400
<220>
<223> Designed to hybridize with cRNA of the human gene RB1

<400>
aactggcaga aatgacttct actcgaacac gaatgcaaaa gcagaaaatg          50

<210> 401
<211> 50
<212> DNA
<213> Artificial sequence
<220>
<221> Probe
<223> SG401
<220>
<223> Designed to hybridize with cRNA of the human gene VEGF

<400>

aaatgttcct gcaaaaacac acactcgcgt tgcaaggcga ggcagcttga 50

<210> 402
<211> 50
<212> DNA
<213> Artificial sequence
<220>
<221> Probe
<223> SG402
<220>
<223> Designed to hybridize with cRNA of the human gene ZYX

<400>
gaagaagttc ggccctgtgg tggccccaaa gcccaaagtg aatcccttcc 50

<210> 403
<211> 50
<212> DNA
<213> Artificial sequence
<220>
<221> Probe
<223> SG403
<220>
<223> Designed to hybridize with cRNA of the human gene CD28

<400>
cccctatttc ccggaccttc taagcccttt tgggtgctgg tggtggttgg 50

<210> 404
<211> 50
<212> DNA
<213> Artificial sequence
<220>
<221> Probe
<223> SG404
<220>
<223> Designed to hybridize with cRNA of the human gene CD28

<400>
caaaattgaa gttatgtatc ctcctcctta cctagacaat gagaagagca 50

<210> 405
<211> 50
<212> DNA
<213> Artificial sequence
<220>
<221> Probe
<223> SG405
<220>
<223> Designed to hybridize with cRNA of the human gene RBBP4

<400>
ttctgtatca gaagacaata tcatgcaagt gtggcaaatg gcagagaaca          50

<210> 406
<211> 50
<212> DNA
<213> Artificial sequence
<220>
<221> Probe
<223> SG406
<220>
<223> Designed to hybridize with cRNA of the human gene DDX6

<400>
tgtatgtggc agaataccac agcgagcctg tagaagatga gaaaccttaa          50

<210> 407
<211> 50
<212> DNA
<213> Artificial sequence
<220>
<221> Probe
<223> SG407
<220>
<223> Designed to hybridize with cRNA of the human gene APEX

<400>
gcgccttgat tactttttgt tgtcccactc tctgttacct gcattgtgtg          50

<210> 408
<211> 50
<212> DNA
<213> Artificial sequence
<220>
<221> Probe
<223> SG408
<220>
<223> Designed to hybridize with cRNA of the human gene DPF2

<400>
cagggcatcc atcttgcctc caatttaccc ccgtgatgat ggcggcagtg          50

<210> 409
<211> 50
<212> DNA
<213> Artificial sequence
<220>
<221> Probe
<223> SG409
<220>
<223> Designed to hybridize with cRNA of the human gene RGS1

<400>
ctggcttgtg aagactataa gaaaacagag tctgatcttt tgccctgtaa          50

<210> 410
<211> 50
<212> DNA
<213> Artificial sequence
<220>
<221> Probe
<223> SG410
<220>
<223> Designed to hybridize with cRNA of the human gene HMMR

<400>
tttgccctga agaccccatt aaaagaaggc aatacaaact gttaccgagc          50

<210> 411
<211> 50

<212> DNA
<213> Artificial sequence
<220>
<221> Probe
<223> SG411
<220>
<223> Designed to hybridize with cRNA of the human gene STAT5B

<400>
tccttgacac cgatggggac ttcgatctgg aggacacaat ggacgtagcg          50


<210> 412
<211> 50
<212> DNA
<213> Artificial sequence
<220>
<221> Probe
<223> SG412
<220>
<223> Designed to hybridize with cRNA of the human gene BIRC5

<400>
cgctttcctt tctgtcaaga agcagtttga agaattaacc cttggtgaat          50


<210> 413
<211> 50
<212> DNA
<213> Artificial sequence
<220>
<221> Probe
<223> SG413
<220>
<223> Designed to hybridize with cRNA of the human gene TAL1

<400>
ttccccctat gagatggaga ttactgatgg tccccacacc aaagttgtgc          50


<210> 414
<211> 50
<212> DNA

<213> Artificial sequence
<220>
<221> Probe
<223> SG414
<220>
<223> Designed to hybridize with cRNA of the human gene NXF1

<400>
tatggtgaag gaaaccgtag gtctggaaga ggcggttctg gtattcggtc          50

<210> 415
<211> 50
<212> DNA
<213> Artificial sequence
<220>
<221> Probe
<223> SG415
<220>
<223> Designed to hybridize with cRNA of the human gene TCL1A

<400>
ttctggtgga gagaagtgag aataggcagc ccccaaataa aaaatattca          50


<210> 416
<211> 50
<212> DNA
<213> Artificial sequence
<220>
<221> Probe
<223> SG416
<220>
<223> Designed to hybridize with cRNA of the human gene TRA@

<400>
attattccag aagacacctt cttccccagc ccagaaagtt cctgtgatgt          50


<210> 417
<211> 50
<212> DNA
<213> Artificial sequence
<220>

<221> Probe
<223> SG417
<220>
<223> Designed to hybridize with cRNA of the human gene TCR beta

<400>
cagccgtgta tctccgtgcc agcagcttca aaggtgccca ggccggttag          50


<210> 418
<211> 50
<212> DNA
<213> Artificial sequence
<220>
<221> Probe
<223> SG418
<220>
<223> Designed to hybridize with cRNA of the human gene DNTT

<400>
ctgtatttgg agtggggctg aagacttctg agaagtggtt caggatgggt          50


<210> 419
<211> 50
<212> DNA
<213> Artificial sequence
<220>
<221> Probe
<223> SG419
<220>
<223> Designed to hybridize with cRNA of the human gene TERT

<400>
attcctgctc aagctgactc gacaccgtgt cacctacgtg ccactcctgg          50

<210> 420
<211> 50
<212> DNA
<213> Artificial sequence
<220>
<221> Probe
<223> SG420

<220>
<223> Designed to hybridize with cRNA of the human gene ERBB4

<400>
ttgacaaccc tgactactgg aaccacagcc tgccacctcg gagcaccctt 50

<210> 421
<211> 50
<212> DNA
<213> Artificial sequence
<220>
<221> Probe
<223> SG421
<220>
<223> Designed to hybridize with cRNA of the human gene ERBB2

<400>
cacccagcac cttcaaaggg acacctacgg cagagaaccc agagtacctg 50

<210> 422
<211> 50
<212> DNA
<213> Artificial sequence
<220>
<221> Probe
<223> SG422
<220>
<223> Designed to hybridize with cRNA of the human gene EGFR

<400>
cccagaaagg cagccaccaa attagcctgg acaaccctga ctaccagcag 50

<210> 423
<211> 50
<212> DNA
<213> Artificial sequence
<220>
<221> Probe
<223> SG423
<220>
<223> Designed to hybridize with cRNA of the human gene THPO

<400>
cttctaaaca catcctacac ccactcccag aatctgtctc aggaagggta          50

<210> 424
<211> 50
<212> DNA
<213> Artificial sequence
<220>
<221> Probe
<223> SG424
<220>
<223> Designed to hybridize with cRNA of the human gene TIAM1

<400>
aggaagtcat ttgggttagg cgtgaagact ttgccccctc caggaaactg          50

<210> 425
<211> 50
<212> DNA
<213> Artificial sequence
<220>
<221> Probe
<223> SG425
<220>
<223> Designed to hybridize with cRNA of the human gene TK1

<400>
ggaagctctt tgccccacag cagattctgc aatgcagccc tgccaactga          50

<210> 426
<211> 50
<212> DNA
<213> Artificial sequence
<220>
<221> Probe
<223> SG426
<220>
<223> Designed to hybridize with cRNA of the human gene TNFRSF1A

<400>
agaagtggga ggacagcgcc cacaagccac agagcctaga cactgatgac          50

<210> 427
<211> 50
<212> DNA
<213> Artificial sequence
<220>
<221> Probe
<223> SG427
<220>
<223> Designed to hybridize with cRNA of the human gene TOP2A

<400>
gcctgatcct gccaaaacca agaatcgccg caaaaggaag ccatccactt          50

<210> 428
<211> 50
<212> DNA
<213> Artificial sequence
<220>
<221> Probe
<223> SG428
<220>
<223> Designed to hybridize with cRNA of the human gene FAIM3

<400>
accagcctgc cgccatgatg gaggacagtg attcagatga ctacatcaat          50

<210> 429
<211> 50
<212> DNA
<213> Artificial sequence
<220>
<221> Probe
<223> SG429
<220>
<223> Designed to hybridize with cRNA of the human gene PLS3

<400>

gtggcaatct aacagaagat gacaagcaca ataatgccaa gtatgcagtg          50

<210> 430
<211> 50
<212> DNA
<213> Artificial sequence
<220>
<221> Probe
<223> SG430
<220>
<223> Designed to hybridize with cRNA of the human gene TRADD

<400>
cgccacctgc ccagactttt ctgttccagg gtcagcctgt agtgaatcgg          50

<210> 431
<211> 50
<212> DNA
<213> Artificial sequence
<220>
<221> Probe
<223> SG431
<220>
<223> Designed to hybridize with cRNA of the human gene TNFSF10

<400>
   gtaacaaatg agcacttgat agacatggac catgaagcca gttttttcgg          50

<210> 432
<211> 50
<212> DNA
<213> Artificial sequence
<220>
<221> Probe
<223> SG432
<220>
<223> Designed to hybridize with cRNA of the human gene TRAP 1

<400>
aagtttgagg acaggtcccc agccgccgag tgcctatcag agaaggagac          50

<210> 433
<211> 50
<212> DNA
<213> Artificial sequence
<220>
<221> Probe
<223> SG433
<220>
<223> Designed to hybridize with cRNA of the human gene THBS1

<400>
tagagtggtg atgtatgaag ggaagaaaat catggctgac tcaggaccca          50

<210> 434
<211> 50
<212> DNA
<213> Artificial sequence
<220>
<221> Probe
<223> SG434
<220>
<223> Designed to hybridize with cRNA of the human gene SUMO1

<400>
atgtgattga agtttatcag gaacaaacgg ggggtcattc aacagtttag          50

<210> 435
<211> 50
<212> DNA
<213> Artificial sequence
<220>
<221> Probe
<223> SG435
<220>
<223> Designed to hybridize with cRNA of the human gene UVRAG

<400>
gaagaaatca tcgggctgga agcacaggtt tcgcctcagg tgatcagcta          50

<210> 436
<211> 50
<212> DNA
<213> Artificial sequence
<220>
<221> Probe
<223> SG436
<220>
<223> Designed to hybridize with cRNA of the human gene VPREB1

<400>
ctgtcctgct catgctgttt gtctactgca caggttgtgg tcctcagccg          50


<210> 437
<211> 50
<212> DNA
<213> Artificial sequence
<220>
<221> Probe
<223> SG437
<220>
<223> Designed to hybridize with cRNA of the human gene WNT16

<400>
tggtgctgct atgtccgttg caggaggtgt gaaagcatga ctgatgtcca          50


<210> 438
<211> 50
<212> DNA
<213> Artificial sequence
<220>
<221> Probe
<223> SG438
<220>
<223> Designed to hybridize with cRNA of the human gene WT1

<400>
accacagcac agggtacgag agcgataacc acacaacgcc catcctctgc          50

<210> 439

<211> 50
<212> DNA
<213> Artificial sequence
<220>
<221> Probe
<223> SG439
<220>
<223> Designed to hybridize with cRNA of the human gene XRCC3

<400>
gacaccttgt tggagtgtgt gaataagaag gtccccgtac tgctgtctcg          50

<210> 440
<211> 50
<212> DNA
<213> Artificial sequence
<220>
<221> Probe
<223> SG440
<220>
<223> Designed to hybridize with cRNA of the human gene CCND3

<400>
ttataccttt gccatgtacc cgccatccat gatcgccacg ggcagcattg          50

<210> 441
<211> 50
<212> DNA
<213> Artificial sequence
<220>
<221> Probe
<223> SG441
<220>
<223> Designed to hybridize with cRNA of the human gene CDK2

<400>
accctttctt ccaggatgtg accaagccag taccccatct tcgactctga          50

<210> 442
<211> 50
<212> DNA

<213> Artificial sequence
<220>
<221> Probe
<223> SG442
<220>
<223> Designed to hybridize with cRNA of the human gene p14ARF

<400>
aataaaataa ggggaatagg ggagcgggga cgcgagcagc accagaatcc          50



<210> 443
<211> 50
<212> DNA
<213> Artificial sequence
<220>
<221> Probe
<223> SG443
<220>
<223> Designed to hybridize with cRNA of the human gene HELLS

<400>
gaccgagaaa gagctgttgt ggaagtgaat atccctgtag aatctgaagt          50



<210> 444
<211> 50
<212> DNA
<213> Artificial sequence
<220>
<221> Probe
<223> SG444
<220>
<223> Designed to hybridize with cRNA of the human gene GATA2

<400>
tttcggcttc ccacccacgc cacccaaaga agtgtctcct gaccctagca          50

<210> 445
<211> 50
<212> DNA
<213> Artificial sequence
<220>

<221> Probe
<223> SG445
<220>
<223> Designed to hybridize with cRNA of the human gene GATA1

<400>
atccggccca agaagcgcct gattgtcagt aaacgggcag gtactcagtg          50


<210> 446
<211> 50
<212> DNA
<213> Artificial sequence
<220>
<221> Probe
<223> SG446
<220>
<223> Designed to hybridize with cRNA of the human gene MLLT10

<400>
ttaaattctc aacagctcac accagtacac aggcaccccc acttcacaca          50


<210> 447
<211> 50
<212> DNA
<213> Artificial sequence
<220>
<221> Probe
<223> SG447
<220>
<223> Designed to hybridize with cRNA of the human gene PICALM

<400>
cggaaatgga accactaaga atgatgtaaa ttggagtcaa ccaggtgaaa          50


<210> 448
<211> 50
<212> DNA
<213> Artificial sequence
<220>
<221> Probe

<223> SG448
<220>
<223> Designed to hybridize with cRNA of the human gene KIT

<400>
atgtatgaca taatgaagac ttgctgggat gcagatcccc taaaaagacc          50


<210> 449
<211> 50
<212> DNA
<213> Artificial sequence
<220>
<221> Probe
<223> SG449
<220>
<223> Designed to hybridize with cRNA of the human gene IL3RA

<400>
gcctggagga gtgtctggtg actgaagtac aggtcgtgca gaaaacttga          50


<210> 450
<211> 50
<212> DNA
<213> Artificial sequence
<220>
<221> Probe
<223> SG450
<220>
<223> Designed to hybridize with cRNA of the human gene CEBPA

<400>
aacgtggaga cgcagcagaa ggtgctggag ctgaccagtg acaatgaccg          50


<210> 451
<211> 50
<212> DNA
<213> Artificial sequence
<220>
<221> Probe
<223> SG451

<220>
<223> Designed to hybridize with cRNA of the human gene ITGA6

<400>
agatccatgc tcagccatct gataaagaga ggcttacttc tgatgcatag          50


<210> 452
<211> 50
<212> DNA
<213> Artificial sequence
<220>
<221> Probe
<223> SG452
<220>
<223> Designed to hybridize with cRNA of the human gene CML66

<400>
caggttgcta agcagcaagt agcaagccta gaaaccaatg atcctatttt          50


<210> 453
<211> 50
<212> DNA
<213> Artificial sequence
<220>
<221> Probe
<223> SG453
<220>
<223> Designed to hybridize with cRNA of the human gene TRAF3

<400>
actggagaga tgaatatcgc ctctggctgc ccagtctttg tggcccaaac          50


<210> 454
<211> 50
<212> DNA
<213> Artificial sequence
<220>
<221> Probe
<223> SG454
<220>

<223> Designed to hybridize with cRNA of the human gene DAPK1

<400>
ccttttgctg aaggcatcct ctgtgttcaa aatcaacctg gatggcaatg          50

<210> 455
<211> 50
<212> DNA
<213> Artificial sequence
<220>
<221> Probe
<223> SG455
<220>
<223> Designed to hybridize with cRNA of the human gene MAP3K12

<400>
cttcccggcc tgcaaccccc aaaatggatt ctgcgaggat gacaatgttt          50

<210> 456
<211> 50
<212> DNA
<213> Artificial sequence
<220>
<221> Probe
<223> SG456
<220>
<223> Designed to hybridize with cRNA of the human gene PRKDC

<400>
ttaggattaa ttgagtggct tgaaaatact gttaccttga aggaccttct          50

<210> 457
<211> 50
<212> DNA
<213> Artificial sequence
<220>
<221> Probe
<223> SG457
<220>
<223> Designed to hybridize with cRNA of the human gene DNMT3B

<400>
ggcggaagcc catgcaacga tctctcaaat gtgaatccag ccaggaaagg                50

<210> 458
<211> 50
<212> DNA
<213> Artificial sequence
<220>
<221> Probe
<223> SG458
<220>
<223> Designed to hybridize with cRNA of the human gene GSTP1

<400>
cctcccctga gtacgtgaac ctccccatca atggcaacgg gaaacagtga                50

<210> 459
<211> 50
<212> DNA
<213> Artificial sequence
<220>
<221> Probe
<223> SG459
<220>
<223> Designed to hybridize with cRNA of the human gene HLA-DRA

<400>
agccctgtgg aactgagaga gcccaacgtc ctcatctgtt tcatcgacaa                50

<210> 460
<211> 50
<212> DNA
<213> Artificial sequence
<220>
<221> Probe
<223> SG460
<220>
<223> Designed to hybridize with cRNA of the human gene HOXA10

<400>
tcccttcgcc aaattatccc acaacaatgt catcgtcgga gagccccgcc                50

<210> 461
<211> 50
<212> DNA
<213> Artificial sequence
<220>
<221> Probe
<223> SG461
<220>
<223> Designed to hybridize with cRNA of the human gene IRF8

<400>
ggatatggcc cccttgcgct ccaaactcat tctcgtgcag attgagcagc          50

<210> 462
<211> 50
<212> DNA
<213> Artificial sequence
<220>
<221> Probe
<223> SG462
<220>
<223> Designed to hybridize with cRNA of the human gene ID2

<400>
aattcccttc tgagttaatg tcaaatgaca gcaaagcact gtgtggctga          50

<210> 463
<211> 50
<212> DNA
<213> Artificial sequence
<220>
<221> Probe
<223> SG463
<220>
<223> Designed to hybridize with cRNA of the human gene ACTB

<400>
tgatgatatc gccgcgctcg tcgtcgacaa cggctccggc atgtgcaagg          50

<210> 464
<211> 50
<212> DNA
<213> Artificial sequence
<220>
<221> Probe
<223> SG464
<220>
<223> Designed to hybridize with cRNA of the human gene ACTB

<400>
acgttgctat ccaggctgtg ctatccctgt acgcctctgg ccgtaccact          50

<210> 465
<211> 50
<212> DNA
<213> Artificial sequence
<220>
<221> Probe
<223> SG465
<220>
<223> Designed to hybridize with cRNA of the human gene GRK4

<400>
atggtgaaac cccgtctcta ctaaaaatac aaaaattagc cgggcgtggt          50


<210> 466
<211> 50
<212> DNA
<213> Artificial sequence
<220>
<221> Probe
<223> SG466
<220>
<223> Designed to hybridize with cRNA of the human gene GAPD

<400>
tgcttttaac tctggtaaag tggatattgt tgccatcaat gaccccttca          50


<210> 467

<211> 50
<212> DNA
<213> Artificial sequence
<220>
<221> Probe
<223> SG467
<220>
<223> Designed to hybridize with cRNA of the human gene GAPD

<400>
gtctcctctg acttcaacag cgacacccac tcctccacct ttgacgctgg          50


<210> 468
<211> 50
<212> DNA
<213> Artificial sequence
<220>
<221> Probe
<223> SG468
<220>
<223> Designed to hybridize with cRNA of the human gene STAT1

<400>
atccaaatat tgacaaagac catgcctttg gaaagtatta ctccaggcca          50


<210> 469
<211> 50
<212> DNA
<213> Artificial sequence
<220>
<221> Probe
<223> SG469
<220>
<223> Designed to hybridize with cRNA of the human gene 18S rRNA

<400>
ttgactatct agaggaagta aaagtcgtaa caaggtttcc gtaggtgaac          50

<210> 470
<211> 50
<212> DNA

<213> Artificial sequence
<220>
<221> Probe
<223> SG470
<220>
<223> Designed to hybridize with cRNA of the human gene TFRC

<400>
ccagctttac tggagaactt gaaactgcgt aaacaaaata acggtgcttt          50


<210> 471
<211> 50
<212> DNA
<213> Artificial sequence
<220>
<221> Probe
<223> SG471
<220>
<223> Designed to hybridize with cRNA of the human gene 28S rRNA

<400>
tctccccttc cttggtgcct tctcggctct tgacacttag ccgctgtctc          50


<210> 472
<211> 50
<212> DNA
<213> Artificial sequence
<220>
<221> Probe
<223> SG472
<220>
<223> Designed to hybridize with cRNA of the human gene RPL37A

<400>
tcacggtaaa gtccgccatc agaagactga aggagttgaa agaccagtag          50

<210> 473
<211> 50
<212> DNA
<213> Artificial sequence
<220>

<221> Probe
<223> SG473
<220>
<223> Designed to hybridize with cRNA of the human gene RPL41

<400>
tggaggaaga agcgaatgcg caggctgaag cgcaaaagaa gaaagatgag          50


<210> 474
<211> 50
<212> DNA
<213> Artificial sequence
<220>
<221> Probe
<223> SG474
<220>
<223> Designed to hybridize with cRNA of the human gene HNRPL

<400>
acccttacac tctgaagttg tgtttctcca ctgctcagca cgcctcctaa          50


<210> 475
<211> 50
<212> DNA
<213> Artificial sequence
<220>
<221> Probe
<223> SG475
<220>
<223> Designed to hybridize with cRNA of the human gene ILF2

<400>
ctatcttgct tctgaaatat ctacctggga tggagtgata gtaacacctt          50


<210> 476
<211> 50
<212> DNA
<213> Artificial sequence
<220>
<221> Probe

<223> SG476
<220>
<223> Designed to hybridize with cRNA of the human gene TAGLN2

<400>
gttccctaag aaatccaagg agaatcctcg gaacttctca gataaccagc          50


<210> 477
<211> 50
<212> DNA
<213> Artificial sequence
<220>
<221> Probe
<223> SG477
<220>
<223> Designed to hybridize with cRNA of the human gene CANP2

<400>
aaggcattag aagaagcagg tttcaagatg ccctgtcaac tccaccaagt          50


<210> 478
<211> 50
<212> DNA
<213> Artificial sequence
<220>
<221> Probe
<223> SG478
<220>
<223> Designed to hybridize with cRNA of the human gene PSMA5

<400>
accacaagtc tatgactttg aaagaagcca tcaagtcttc actcatcatc          50


<210> 479
<211> 50
<212> DNA
<213> Artificial sequence
<220>
<221> Probe
<223> SG479

<220>
<223> Designed to hybridize with cRNA of the human gene PMM1

<400>
atccacttct ttgggaacga gactagccct ggtgggaacg actttgagat          50

<210> 480
<211> 50
<212> DNA
<213> Artificial sequence
<220>
<221> Probe
<223> SG480
<220>
<223> Designed to hybridize with cRNA of the human gene MLF2

<400>
aatacgggtg atggtgcccc caaggtctac caagagacat cagagatgcg          50

<210> 481
<211> 50
<212> DNA
<213> Artificial sequence
<220>
<221> Probe
<223> SG481
<220>
<223> Designed to hybridize with cRNA of the human gene PPP1CC

<400>
gagacctgta acgcctccaa ggggtatgat cacaaagcaa gcaaagaaat          50

<210> 482
<211> 50
<212> DNA
<213> Artificial sequence
<220>
<221> Probe
<223> SG482
<220>
<223> Designed to hybridize with cRNA of the human gene CASC3

<400>
atcacaggta tatggaggag tgacctacta taaccccgcc cagcagcagg          50

<210> 483
<211> 50
<212> DNA
<213> Artificial sequence
<220>
<221> Probe
<223> SG483
<220>
<223> Designed to hybridize with cRNA of the human gene KIAA0864

<400>
tgccttacca tctctgccac ctgtggaatc gctgagagat tgccagaagc          50

<210> 484
<211> 50
<212> DNA
<213> Artificial sequence
<220>
<221> Probe
<223> SG484
<220>
<223> Designed to hybridize with cRNA of the human gene TXNRD1

<400>
gccattggaa tggacgattc cgtcaagaga taacaacaaa tgttatgcaa          50

<210> 485
<211> 50
<212> DNA
<213> Artificial sequence
<220>
<221> Probe
<223> SG485
<220>
<223> Designed to hybridize with cRNA of the human gene PSMD7

<400>

tgcacaacct catcaacaac aagattgcca accgggatgc agagaagaaa          50

<210> 486
<211> 50
<212> DNA
<213> Artificial sequence
<220>
<221> Probe
<223> SG486
<220>
<223> Designed to hybridize with cRNA of the human gene EIF2B2

<400>
caccttccta catctaccgc ctgatgagtg aactctacca tcctgatgat          50

<210> 487
<211> 50
<212> DNA
<213> Artificial sequence
<220>
<221> Probe
<223> SG487
<220>
<223> Designed to hybridize with cRNA of the human gene HLA-A

<400>
tagaaaagga gggagctact ctcaggctgc aagcagtgac agtgcccagg          50

<210> 488
<211> 50
<212> DNA
<213> Artificial sequence
<220>
<221> Probe
<223> SG488
<220>
<223> Designed to hybridize with cRNA of the human gene MCM7

<400>
ggcagcttca atcgccccag aaatatacgg gcatgaagat gtgaagaagg          50

<210> 489
<211> 50
<212> DNA
<213> Artificial sequence
<220>
<221> Probe
<223> SG489
<220>
<223> Designed to hybridize with cRNA of the human gene TNFRSF25

<400>
ctgacctaca cataccgcca ctgctggcct cacaagcccc tggttactgc          50

<210> 490
<211> 50
<212> DNA
<213> Artificial sequence
<220>
<221> Probe
<223> SG490
<220>
<223> Designed to hybridize with cRNA of the human gene LGALS3

<400>
agtacaatca tcgggttaaa aaactcaatg aaatcagcaa actgggaatt          50

<210> 491
<211> 50
<212> DNA
<213> Artificial sequence
<220>
<221> Probe
<223> SG491
<220>
<223> Designed to hybridize with cRNA of the human gene HLA-DPA1

<400>
gaacagatta cagcttccac aagttccatt acctgacctt tgtgccctca          50

<210> 492
<211> 50
<212> DNA
<213> Artificial sequence
<220>
<221> Probe
<223> SG492
<220>
<223> Designed to hybridize with cRNA of the human gene PLAU

<400>
ctcgtctgtt ccctccaagg ccgcatgact ttgactggaa ttgtgagctg          50

<210> 493
<211> 50
<212> DNA
<213> Artificial sequence
<220>
<221> Probe
<223> SG493
<220>
<223> Designed to hybridize with cRNA of the human gene COL3A1

<400>
tcgaggattc cctggtaatc caggtgcccc aggttctcca ggccctgctg          50

<210> 494
<211> 50
<212> DNA
<213> Artificial sequence
<220>
<221> Probe
<223> SG494
<220>
<223> Designed to hybridize with cRNA of the human gene ANK2

<400>
ctcagttcct gaggacatct ttgacacaag gcccatttgg gatgagtcta          50

<210> 495
<211> 50

<212> DNA
<213> Artificial sequence
<220>
<221> Probe
<223> SG495
<220>
<223> Designed to hybridize with cRNA of the human gene SERPINA9

<400>
gcacctctat tgcagaatta caataacaca ttcaataaaa ctaaaatatg          50


<210> 496
<211> 50
<212> DNA
<213> Artificial sequence
<220>
<221> Probe
<223> SG496
<220>
<223> Designed to hybridize with cRNA of the human gene NPM3

<400>
ggcaccagat tgttacgatg agcaatgatg tttctgagga ggagagcgag          50


<210> 497
<211> 50
<212> DNA
<213> Artificial sequence
<220>
<221> Probe
<223> SG497
<220>
<223> Designed to hybridize with cRNA of the human gene CCR6

<400>
gcagaccagt gagaccgcag ataacgacaa tgcgtcgtcc ttcactatgt          50


<210> 498
<211> 50
<212> DNA

<213> Artificial sequence
<220>
<221> Probe
<223> SG498
<220>
<223> Designed to hybridize with cRNA of the human gene HCK

<400>
ccgagctgcc aacatcttgg tctctgcatc cctggtgtgt aagattgctg          50

<210> 499
<211> 50
<212> DNA
<213> Artificial sequence
<220>
<221> Probe
<223> SG499
<220>
<223> Designed to hybridize with cRNA of the human gene GNL3

<400>
aggacataca tgaagaattg ccaaaacgga aagaaaggaa gcaggaggag          50

<210> 500
<211> 50
<212> DNA
<213> Artificial sequence
<220>
<221> Probe
<223> SG500
<220>
<223> Designed to hybridize with cRNA of the human gene GRB2

<400>
ccggcatgtt tccccgcaat tatgtcaccc ccgtgaaccg gaacgtctaa          50

<210> 501
<211> 50
<212> DNA
<213> Artificial sequence
<220>

<221> Probe
<223> SG501
<220>
<223> Designed to hybridize with cRNA of the human gene BCL2A1

<400>
ggagttcata atgaataaca caggagaatg gataaggcaa aacggaggct          50


<210> 502
<211> 50
<212> DNA
<213> Artificial sequence
<220>
<221> Probe
<223> SG502
<220>
<223> Designed to hybridize with cRNA of the human gene ELF1

<400>
gtttagaggt ctaatgctat gttttcatat tacagagtga atttgtattt          50


<210> 503
<211> 50
<212> DNA
<213> Artificial sequence
<220>
<221> Probe
<223> SG503
<220>
<223> Designed to hybridize with cRNA of the human gene CTSB

<400>
tgctctacaa gtcaggagtg taccaacacg tcaccggaga gatgatgggt          50

<210> 504
<211> 50
<212> DNA
<213> Artificial sequence
<220>
<221> Probe
<223> SG504

<220>
<223> Designed to hybridize with cRNA of the human gene GCET2

<400>
atcctggttc tctctcttcc tccatctgtg actttgaaca tgctacttgg          50


<210> 505
<211> 50
<212> DNA
<213> Artificial sequence
<220>
<221> Probe
<223> SG505
<220>
<223> Designed to hybridize with cRNA of the human gene FN1

<400>
tcgtgctttg acccctacac agtttcccat tatgccgttg gagatgagtg          50


<210> 506
<211> 50
<212> DNA
<213> Artificial sequence
<220>
<221> Probe
<223> SG506
<220>
<223> Designed to hybridize with cRNA of the human gene PDCD1

<400>
ttcctagcgg aatgggcacc tcatcccccg cccgcagggg ctcagccgac          50


<210> 507
<211> 50
<212> DNA
<213> Artificial sequence
<220>
<221> Probe
<223> SG507
<220>

<223> Designed to hybridize with cRNA of the human gene HLA-DRB3

<400>
gttcatctac ttcaggaatc agaaaggaca ctctggactt cagccaacag          50


<210> 508
<211> 50
<212> DNA
<213> Artificial sequence
<220>
<221> Probe
<223> SG508
<220>
<223> Designed to hybridize with cRNA of the human gene HLA-DQA1

<400>
gcagtcagtc acagaagatg tttctgagac cagcttcctc tccaagagtg          50


<210> 509
<211> 50
<212> DNA
<213> Artificial sequence
<220>
<221> Probe
<223> SG509
<220>
<223> Designed to hybridize with cRNA of the human gene GCET2

<400>
ggtgaaggct ccacaaacct gtgacacatg caagctcaag ccattgcatt          50


<210> 510
<211> 50
<212> DNA
<213> Artificial sequence
<220>
<221> Probe
<223> SG510
<220>
<223> Designed to hybridize with cRNA of the human gene SERPNINA9

<400>
ggagccccaa ggaatgaaat atggtaaccc agcaacatcc catgaagcta          50

<210> 511
<211> 50
<212> DNA
<213> Artificial sequence
<220>
<221> Probe
<223> SG511
<220>
<223> Designed to hybridize with cRNA of the human gene CDKN3

<400>
ctgcacatct atcatcaaga gattcacaat caagatctgt atcaagataa          50

<210> 512
<211> 50
<212> DNA
<213> Artificial sequence
<220>
<221> Probe
<223> SG512
<220>
<223> Designed to hybridize with cRNA of the human gene ELF1

<400>
cttctctcaa gcttcttggc cttcaagtat tctttctcta tcgtcttttg          50

<210> 513
<211> 50
<212> DNA
<213> Artificial sequence
<220>
<221> Probe
<223> SG513
<220>
<223> Designed to hybridize with cRNA of the human gene CATSD

<400>
gtgccgctga ttcagggcga gtacatgatc ccctgtgaga aggtgtccac          50

<210> 514
<211> 50
<212> DNA
<213> Artificial sequence
<220>
<221> Probe
<223> SG514
<220>
<223> Designed to hybridize with cRNA of the human gene HSPB1

<400>
atggctacat ctcccggtgc ttcacgcgga aatacacgct gcccccggt          50
<210> 515
<211> 50
<212> DNA
<213> Artificial sequence
<220>
<221> Probe
<223> SG515
<220>
<223> Designed to hybridize with cRNA of the human gene ACTN1

<400>
cccctacaca accatcacgc ctcaggagat caatggcaaa tgggaccacg          50

<210> 516
<211> 50
<212> DNA
<213> Artificial sequence
<220>
<221> Probe
<223> SG516
<220>
<223> Designed to hybridize with cRNA of the human gene BMP6

<400>
gaaccccgag tatgtcccca aaccgtgctg tgcgccaact aagctaaatg          50

<210> 517
<211> 50
<212> DNA
<213> Artificial sequence
<220>
<221> Probe
<223> SG517
<220>
<223> Designed to hybridize with cRNA of the human gene FAM38A

<400>
gtctgtggtc atccccaatc tcttccccaa gtacatccgt gcccccaacg          50

<210> 518
<211> 50
<212> DNA
<213> Artificial sequence
<220>
<221> Probe
<223> SG518
<220>
<223> Designed to hybridize with cRNA of the human gene CD48

<400>
ttatacttgc caagtcagca attctgtgag cagcaagaat ggcaccgtct          50

<210> 519
<211> 50
<212> DNA
<213> Artificial sequence
<220>
<221> Probe
<223> SG519
<220>
<223> Designed to hybridize with cRNA of the human gene IL4R

<400>
tcacagtggg agaagcggtc ccgaggccag gaaccagcca agtgcccaca          50

<210> 520
<211> 50
<212> DNA
<213> Artificial sequence
<220>
<221> Probe
<223> SG520
<220>
<223> Designed to hybridize with cRNA of the human gene DRP2

<400>
ccagtgtgcg aagttctgat gtgactgcca acaccctgct ggcctcttga          50

<210> 521
<211> 50
<212> DNA
<213> Artificial sequence
<220>
<221> Probe
<223> SG521
<220>
<223> Designed to hybridize with cRNA of the human gene JUNB

<400>
ggaggacaag gtgaagacgc tcaaggccga gaacgcgggg ctgtcgagta          50

<210> 522
<211> 50
<212> DNA
<213> Artificial sequence
<220>
<221> Probe
<223> SG522
<220>
<223> Designed to hybridize with cRNA of the human gene S100A8

<400>
aattgctaga gaccgagtgt cctcagtata tcaggaaaaa gggtgcagac          50

<210> 523

<211> 50
<212> DNA
<213> Artificial sequence
<220>
<221> Probe
<223> SG523
<220>
<223> Designed to hybridize with cRNA of the human gene ZNFN1A1

<400>
agttggaggc attcgacttc ctaacggaaa actaaagtgt gatatctgtg          50


<210> 524
<211> 50
<212> DNA
<213> Artificial sequence
<220>
<221> Probe
<223> SG524
<220>
<223> Designed to hybridize with cRNA of the human gene MERTK

<400>
tcattgcccg atgaactttt gtttgctgac gactcctcag aaggctcaga          50


<210> 525
<211> 50
<212> DNA
<213> Artificial sequence
<220>
<221> Probe
<223> SG525
<220>
<223> Designed to hybridize with cRNA of the human gene KLF13

<400>
tgcctagtgc tgaggcctct ggggctggaa agcctcagca gaaaggaggc          50


<210> 526
<211> 50

<212> DNA
<213> Artificial sequence
<220>
<221> Probe
<223> SG526
<220>
<223> Designed to hybridize with cRNA of the human gene CBFB

<400>
aatttcccct gagggaatcg ctttttaagt gatccttaca gtggtgtttt          50


<210> 527
<211> 50
<212> DNA
<213> Artificial sequence
<220>
<221> Probe
<223> SG527
<220>
<223> Designed to hybridize with cRNA of the human gene CEBPB

<400>
tcctctccga cctcttctcc gacgactacg ggggcaagaa ctgcaagaag          50


<210> 528
<211> 50
<212> DNA
<213> Artificial sequence
<220>
<221> Probe
<223> SG528
<220>
<223> Designed to hybridize with cRNA of the human gene CEBPD

<400>
cggggcggga gagactcagc aacgacccat acctcagacc cgacggcccg          50


<210> 529
<211> 50
<212> DNA

<213> Artificial sequence
<220>
<221> Probe
<223> SG529
<220>
<223> Designed to hybridize with cRNA of the human gene TFCP2

<400>
ctgcagttga gggagcagca acaacagcag cagcaacagc agcagaagca          50

<210> 530
<211> 50
<212> DNA
<213> Artificial sequence
<220>
<221> Probe
<223> SG530
<220>
<223> Designed to hybridize with cRNA of the human gene CREB1

<400>
cgcctgtaat cccagcactt tgggaggcca aggtgggtgg atcacctgtg          50


<210> 531
<211> 50
<212> DNA
<213> Artificial sequence
<220>
<221> Probe
<223> SG531
<220>
<223> Designed to hybridize with cRNA of the human gene NFIC

<400>
gcggcgatta ctacacttcg cccagctcgc ccacgagtag cagccgcaac          50


<210> 532
<211> 50
<212> DNA
<213> Artificial sequence
<220>

<221> Probe
<223> SG532
<220>
<223> Designed to hybridize with cRNA of the human gene GABPB2

<400>
tgctccattg tccaattctt cagaaactcc agtagtggcc acagaagaag          50


<210> 533
<211> 50
<212> DNA
<213> Artificial sequence
<220>
<221> Probe
<223> SG533
<220>
<223> Designed to hybridize with cRNA of the human gene EGR1

<400>
gatggcttga catgtgcaat tgtgagggac atgctcacct ctagccttaa          50


<210> 534
<211> 50
<212> DNA
<213> Artificial sequence
<220>
<221> Probe
<223> SG534
<220>
<223> Designed to hybridize with cRNA of the human gene KLF1

<400>
gccactaccg gaaacacacg gggcagcgcc ccttccgctg ccagctctgc          50

<210> 535
<211> 50
<212> DNA
<213> Artificial sequence
<220>
<221> Probe
<223> SG535

<220>
<223> Designed to hybridize with cRNA of the human gene ELF1

<400>
tatggctgac cctgtttcag aagcaggata gtataaaagc atcagcctaa          50


<210> 536
<211> 50
<212> DNA
<213> Artificial sequence
<220>
<221> Probe
<223> SG536
<220>
<223> Designed to hybridize with cRNA of the human gene ETS1

<400>
ctcgagctgg ccccagactt tgttggggac atcttatggg aacatctaga          50


<210> 537
<211> 50
<212> DNA
<213> Artificial sequence
<220>
<221> Probe
<223> SG537
<220>
<223> Designed to hybridize with cRNA of the human gene ETS2

<400>
tatttgaaat aagaattcag acatctgagg ttttatttca tttttcaata          50


<210> 538
<211> 50
<212> DNA
<213> Artificial sequence
<220>
<221> Probe
<223> SG538
<220>

<223> Designed to hybridize with cRNA of the human gene FLI1

<400>
attcgcaagt gctgtgcgct tgtcagacca tcagaccagg gccaaccaat          50

<210> 539
<211> 50
<212> DNA
<213> Artificial sequence
<220>
<221> Probe
<223> SG539
<220>
<223> Designed to hybridize with cRNA of the human gene GATA3

<400>
gagccctgct cgatgctcac agggccccca gcgagagtcc ctgcagtccc          50

<210> 540
<211> 50
<212> DNA
<213> Artificial sequence
<220>
<221> Probe
<223> SG540
<220>
<223> Designed to hybridize with cRNA of the human gene CBFA2T1

<400>
tgttggacaa atgtgaagat gcattgtagt ttaaccatat gcccacattt          50

<210> 541
<211> 50
<212> DNA
<213> Artificial sequence
<220>
<221> Probe
<223> SG541
<220>
<223> Designed to hybridize with cRNA of the human gene HIF1A

<400>
atgcattctt agcaaaattg cctagtatgt taatttgctc aaaatacaat          50

<210> 542
<211> 50
<212> DNA
<213> Artificial sequence
<220>
<221> Probe
<223> SG542
<220>
<223> Designed to hybridize with cRNA of the human gene TCF1

<400>
cccgagcagc tgagcagggc cggggaactg gccaagctga ggtgcccagg          50

<210> 543
<211> 50
<212> DNA
<213> Artificial sequence
<220>
<221> Probe
<223> SG543
<220>
<223> Designed to hybridize with cRNA of the human gene HOXD8

<400>
caaatttccc gtttcccggc aggaggtgaa ggacggggaa acgaaaaagg          50

<210> 544
<211> 50
<212> DNA
<213> Artificial sequence
<220>
<221> Probe
<223> SG544
<220>
<223> Designed to hybridize with cRNA of the human gene HOXD9

<400>

cctcagcttg cagcgaccac ccgatcccag gctgttcgct gaaggaggag          50

<210> 545
<211> 50
<212> DNA
<213> Artificial sequence
<220>
<221> Probe
<223> SG545
<220>
<223> Designed to hybridize with cRNA of the human gene MAFB

<400>
agcgctccta gggtgagagg cttagccatc cctgaccctg gcagtgcact          50

<210> 546
<211> 50
<212> DNA
<213> Artificial sequence
<220>
<221> Probe
<223> SG546
<220>
<223> Designed to hybridize with cRNA of the human gene MAFK

<400>
gcggcgggca ggcgggtggg ggcacacccc tcgtacctgt cactgggatg          50

<210> 547
<211> 50
<212> DNA
<213> Artificial sequence
<220>
<221> Probe
<223> SG547
<220>
<223> Designed to hybridize with cRNA of the human gene EDF1

<400>
ttggccgcca gttccgttct cctcacgggc cgaacggaac aaggggtcca          50

<210> 548
<211> 50
<212> DNA
<213> Artificial sequence
<220>
<221> Probe
<223> SG548
<220>
<223> Designed to hybridize with cRNA of the human gene ELF4

<400>
ctagatgggg tccaccccca ttcctgctca agcatgggca cctaccacat          50

<210> 549
<211> 50
<212> DNA
<213> Artificial sequence
<220>
<221> Probe
<223> SG549
<220>
<223> Designed to hybridize with cRNA of the human gene ZNF42

<400>
tccgcgtaca cacgggcgag aaaccctttg cctgccccga gtgtggccag          50

<210> 550
<211> 50
<212> DNA
<213> Artificial sequence
<220>
<221> Probe
<223> SG550
<220>
<223> Designed to hybridize with cRNA of the human gene NF1

<400>
ggaattgatg aagaaaccag tgaagaatcc ctcctgactc ccacatctcc          50

<210> 551

<211> 50
<212> DNA
<213> Artificial sequence
<220>
<221> Probe
<223> SG551
<220>
<223> Designed to hybridize with cRNA of the human gene NFATC1

<400>
ttctcgtctc acgcagttcg aggaggaccc tagaaagcca ggagctgtga          50


<210> 552
<211> 50
<212> DNA
<213> Artificial sequence
<220>
<221> Probe
<223> SG552
<220>
<223> Designed to hybridize with cRNA of the human gene PAX6

<400>
ggtgtgtcag ttccagttca agttcccgga agtgaacctg atatgtctca          50


<210> 553
<211> 50
<212> DNA
<213> Artificial sequence
<220>
<221> Probe
<223> SG553
<220>
<223> Designed to hybridize with cRNA of the human gene PAX8

<400>
gcaccacccc aggtcctcct gcagtgcggc atccccttgg cagctgccgt          50


<210> 554
<211> 50

<212> DNA
<213> Artificial sequence
<220>
<221> Probe
<223> SG554
<220>
<223> Designed to hybridize with cRNA of the human gene PLP

<400>
gccagtgatg gtgggtgggg gctgggcctt ccccgccacc tccacccctg          50

<210> 555
<211> 50
<212> DNA
<213> Artificial sequence
<220>
<221> Probe
<223> SG555
<220>
<223> Designed to hybridize with cRNA of the human gene PLZF

<400>
gtgctccctc tcactggtgg ggcgtggctt cctcacagag agggacgaca          50

<210> 556
<211> 50
<212> DNA
<213> Artificial sequence
<220>
<221> Probe
<223> SG556
<220>
<223> Designed to hybridize with cRNA of the human gene RBP4

<400>
gtacaggctg atcgtccaca acggttactg cgatggcaga tcagaaagaa          50

<210> 557
<211> 50
<212> DNA
<213> Artificial sequence

<220>
<221> Probe
<223> SG557
<220>
<223> Designed to hybridize with cRNA of the human gene RORA

<400>
cagacctgga gcgccacaca ctgcacatct tttggtgatc ggggtcaggc          50


<210> 558
<211> 50
<212> DNA
<213> Artificial sequence
<220>
<221> Probe
<223> SG558
<220>
<223> Designed to hybridize with cRNA of the human gene SP1

<400>
cctgcttccc cttcctaagt ctgtcatcct ctggaaggga tgggtggtgc          50


<210> 559
<211> 50
<212> DNA
<213> Artificial sequence
<220>
<221> Probe
<223> SG559
<220>
<223> Designed to hybridize with cRNA of the human gene STAT1

<400>
atatcaatag aaggatgtac atttccaaat tcacaagttg tgtttgatat          50

<210> 560
<211> 50
<212> DNA
<213> Artificial sequence
<220>
<221> Probe

<223> SG560
<220>
<223> Designed to hybridize with cRNA of the human gene TBP

<400>
tgcagggtgt ggcaccaggt gatgcccttc tgtaagtgcc caccgcggga          50


<210> 561
<211> 50
<212> DNA
<213> Artificial sequence
<220>
<221> Probe
<223> SG561
<220>
<223> Designed to hybridize with cRNA of the human gene TCF7

<400>
aagggctgcc gggccaggcg cggtggctca cgcctgtaat cccagcactt          50


<210> 562
<211> 50
<212> DNA
<213> Artificial sequence
<220>
<221> Probe
<223> SG562
<220>
<223> Designed to hybridize with cRNA of the human gene ETV7

<400>
cagccattgc ttgggaaggc tgggaggcct cccatccagg acactggggg          50


<210> 563
<211> 50
<212> DNA
<213> Artificial sequence
<220>
<221> Probe
<223> SG563

<220>
<223> Designed to hybridize with cRNA of the human gene ZAP70

<400>
cgcggaagga gcagggcaca tacgccctgt ccctcatcta tgggaagacg          50


<210> 564
<211> 55
<212> DNA
<213> Artificial sequence
<220>
<221> Probe
<223> SC1
<220>
<223> Negative control

<400>
ggggaaagaa agatcgatga gaaagaggaa cggggattga caggaatgaa gaagg     55

<210> 565
<211> 50
<212> DNA
<213> Artificial sequence
<220>
<221> Probe
<223> SC2
<220>
<223> Negative control

<400>
cgtctcggtg aactcaagtg aatcaaggga agagaattcc taactgcacg          50


<210> 566
<211> 55
<212> DNA
<213> Artificial sequence
<220>
<221> Probe
<223> SC3
<220>

<223> Negative control

<400>
tcagcttcct ttctcccttt cctccttcct tccttctcac catcctgtcc ttttt          55

<210> 567
<211> 55
<212> DNA
<213> Artificial sequence
<220>
<221> Probe
<223> SC4
<220>
<223> Negative control

<400>
gaggaaacag gaaagaagag gaaggaagga agacagggaa gaaaagaggg aggga          55

<210> 568
<211> 54
<212> DNA
<213> Artificial sequence
<220>
<221> Probe
<223> SC5
<220>
<223> Negative control

<400>
tctgtgggga ggggttgttt tggggttgtt tttgtttttt cttgccaggt agat          54

<210> 569
<211> 55
<212> DNA
<213> Artificial sequence
<220>
<221> Probe
<223> SC6
<220>
<223> Negative control

<400>
ctctctctct ctctctctct ctctctgtct ctccccacct ctctcacatc ccttg          55

<210> 570
<211> 50
<212> DNA
<213> Artificial sequence
<220>
<221> Probe
<223> SC7
<220>
<223> Negative control

<400>
tgtgtgtgtg tgtgtgtgtg tgtgtgtgtg tggtgttgtg tgtgtttgtg          50

<210> 571
<211> 25
<212> DNA
<213> Artificial sequence
<220>
<221> Probe
<223> SCN1
<220>
<223> Negative control

<400>
gtcgtcaaga tgctaccgtt cagga          25

<210> 572
<211> 50
<212> DNA
<213> Artificial sequence
<220>
<221> Probe
<223> SCN2
<220>
<223> Positive hybridization control

<400>

aggccgtgaa gatatattca tcagctacca tgcgaaagac gatgtccaaa 50

<210> 573
<211> 50
<212> DNA
<213> Artificial sequence
<220>
<221> Probe
<223> SCN3
<220>
<223> Positive hybridization control

<400>
cgagccaggc aaagcggagt aactcagttc gaagtaaggg gaaaatgtcg 50

<210> 574
<211> 50
<212> DNA
<213> Artificial sequence
<220>
<221> Probe
<223> SCN4
<220>
<223> Gives rise to non-specific hybridization

<400>
gcttttcttc gtttatcatc accatcacca tcatcatcat catcatcatc 50

<210> 575
<211> 50
<212> DNA
<213> Artificial sequence
<220>
<221> Probe
<223> SCN5
<220>
<223> Negative control

<400>
gccaggatct caaacccacc aaaccgttcc agaatcatgg accgctttcg 50

<210> 576
<211> 50
<212> DNA
<213> Artificial sequence
<220>
<221> Probe
<223> SCN6
<220>
<223> Positive hybridization control

<400>
gtccaagcga gaagatactt aacaatatca acattaccat taccagcaga          50

<210> 577
<211> 50
<212> DNA
<213> Artificial sequence
<220>
<221> Probe
<223> SCN7
<220>
<223> Negative control

<400>
atcccgatgc aaaaggcgga cgacaaggac tacggtctag aagctctgga          50

<210> 578
<211> 50
<212> DNA
<213> Artificial sequence
<220>
<221> Probe
<223> SCN8
<220>
<223> Positive hybridization control

<400>
ccacatcacc ttcaccattt ttcttcttcg ttgattctct gtttgtgttt          50

<210> 579
<211> 50
<212> DNA
<213> Artificial sequence
<220>
<221> Probe
<223> SCN9
<220>
<223> Gives rise to non-specific hybridization

<400>
tcctctgttt tgctcttgcc ttatccgctg cttctctctc cctctctttc          50

<210> 580
<211> 50
<212> DNA
<213> Artificial sequence
<220>
<221> Probe
<223> SCN10
<220>
<223> Negative control

<400>
tgtgacacga cctacaacaa tggctgcaac ggtggtctca tggactatgc          50

<210> 581
<211> 50
<212> DNA
<213> Artificial sequence
<220>
<221> Probe
<223> SCN11
<220>
<223> Positive hybridization control

<400>
agaataataa atgaagtgat gatgattgat gattgatgat tgatgattat          50

<210> 582
<211> 50
<212> DNA

<213> Artificial sequence
<220>
<221> Probe
<223> SCN12
<220>
<223> Positive hybridization control

<400>
agtgacaagt ttcgctgatt atagtggtta atgctggtct tatcttcgtc          50


<210> 583
<211> 50
<212> DNA
<213> Artificial sequence
<220>
<221> Probe
<223> SCN13
<220>
<223> Positive hybridization control

<400>
atgggcggtg cggggttgat gttgacgacg gtgtggctgc ggttgggtat          50


<210> 584
<211> 50
<212> DNA
<213> Artificial sequence
<220>
<221> Probe
<223> SSPC1
<220>
<223> Acts as positive internal process control: spiked control

<400>
cctcaacgcc tacaaaagcc agtttatccc gtcaaaggat tccgtttcta          50

<210> 585
<211> 50
<212> DNA
<213> Artificial sequence
<220>

<221> Probe
<223> SSPC2
<220>
<223> Acts as positive internal process control: spiked control

<400>
cgttatacgg aagatgatga accgcttcat gccactgaat acgttgaaaa          50


<210> 586
<211> 50
<212> DNA
<213> Artificial sequence
<220>
<221> Probe
<223> SSPC3
<220>
<223> Acts as positive internal process control: spiked control

<400>
ctaccaagtg gctaaacgga ccgcagattt gtacggaaaa gaaatgcctc          50


<210> 587
<211> 50
<212> DNA
<213> Artificial sequence
<220>
<221> Probe
<223> SSPC4
<220>
<223> Acts as positive internal process control: spiked control

<400>
aacacaagcg gtttccacca tgtccacaag aaaggggagg taacaccagg          50


<210> 588
<211> 50
<212> DNA
<213> Artificial sequence
<220>
<221> Probe

<223> SSPC5
<220>
<223> Acts as positive internal process control: spiked control

<400>
gccagcaaca actcaaccag caacaaaacc agtttcacag gtgtcaggac          50


<210> 589
<211> 50
<212> DNA
<213> Artificial sequence
<220>
<221> Probe
<223> SSPC6
<220>
<223> Acts as positive internal process control: spiked control

<400>
aagcagattc agtagcagat gccgttcaaa aggtcgattt aagtagaagt          50


<210> 590
<211> 50
<212> DNA
<213> Artificial sequence
<220>
<221> Probe
<223> SSPC7
<220>
<223> Acts as positive internal process control: spiked control

<400>
gacttcctga tattgttgat acatgcggaa caggggagaa cggtatttcc          50


<210> 591
<211> 70
<212> DNA
<213> Artificial sequence
<220>
<221> Antisense oligonucleotide
<223> C2

<220>
<223> Complementary to SCN2
<220>
<223> Biotin residue at 5′

<400>
acgctataaa catccactac cgacattttc cccttacttt gaactgagtt actccgcttt    60
gcctggctcg                                                            70

<210> 592
<211> 70
<212> DNA
<213> Artificial sequence
<220>
<221> Antisense oligonucleotide
<223> C3
<220>
<223> Complementary to SCN3
<220>
<223> Biotin residue at 5′

<400>
gagcgaaccg gcgtaagttg tttggacatc gtctttcgca tggtagctga tgaatatatc    60
ttcacggcct                                                            70

<210> 593
<211> 70
<212> DNA
<213> Artificial sequence
<220>
<221> Antisense oligonucleotide
<223> C6
<220>
<223> Complementary to SCN6
<220>
<223> Biotin residue at n 5′

<400>
atcatacgcc acttcatgtt tctgctggta atggtaatgt tgatattgtt aagtatcttc    60
tcgcttggac                                                            70

<210> 594
<211> 70
<212> DNA
<213> Artificial sequence
<220>
<221> Antisense oligonucleotide
<223> C8
<220>
<223> Complementary to SCN8
<220>
<223> Residue of biotin at 5'

<400>
actgaaaaca gaacaaacac aaacacaaac agagaatcaa cgaagaagaa aaatggtgaa   60
ggtgatgtgg                                                          70

<210> 595
<211> 70
<212> DNA
<213> Artificial sequence
<220>
<221> Antisense oligonucleotide
<223> C11
<220>
<223> Complementary to SCN11
<220>
<223> Residue of biotin at 5'

<400>
aacattcaag catgatgatg ataatcatca atcatcaatc atcaatcatc atcacttcat   60
ttattattct                                                          70

<210> 596
<211> 70
<212> DNA
<213> Artificial sequence
<220>
<221> Antisense oligonucleotide
<223> C12
<220>
<223> Complementary to SCN12

<220>
<223> Residue of biotin at 5′

<400>
ttaggattct aaatagtagc gacgaagata agaccagcat taaccactat aatcagcgaa    60
acttgtcact                                                            70

<210> 597
<211> 70
<212> DNA
<213> Artificial sequence
<220>
<221> Antisense oligonucleotide
<223> C13
<220>
<223> Complementary to SCN13
<220>
<223> Residue of biotin at 5′

<400>
tctgaaggct tgcttgatcc atacccaacc gcagccacac cgtcgtcaac atcaaccccg    60
caccgcccat                                                            70

<210> 598
<211> 24
<212> DNA
<213> Artificial sequence
<220>
<221> Primer
<223> PSMB4_F
<220>
<223> Gene PSMB4

<400>
ttctgggaga tggacacagc tata                                           24

<210> 599
<211> 21
<212> DNA
<213> Artificial sequence

<220>
<221> Primer
<223> PSMB4_R
<220>
<223> Gene PSMB4

<400>
ccacaaaggg ttcatcttcg a                                                21


<210> 600
<211> 21
<212> DNA
<213> Artificial sequence
<220>
<221> Primer
<223> CD23A_F
<220>
<223> Gene CD23A

<400>
tgccctgaaa agtggatcaa t                                                21


<210> 601
<211> 21
<212> DNA
<213> Artificial sequence
<220>
<221> Primer
<223> CD23A_R
<220>
<223> Gene CD23A

<400>
ccatgtcgtc acaggcatac c                                                21


<210> 602
<211> 21
<212> DNA
<213> Artificial sequence
<220>

<221> Primer
<223> LCP1_F
<220>
<223> Gene LCP1

<400>
ccaggtaccc ttctcgcttt t                                                21

<210> 603
<211> 20
<212> DNA
<213> Artificial sequence
<220>
<221> Primer
<223> LCP1_R
<220>
<223> Gene LCP1

<400>
ctcctggccc tcatcttgaa                                                  20

<210> 604
<211> 24
<212> DNA
<213> Artificial sequence
<220>
<221> Primer
<223> ABCC5_F
<220>
<223> Gene ABCC5

<400>
ccctcaaagt ctgcaacttt aagc                                             24

<210> 605
<211> 21
<212> DNA
<213> Artificial sequence
<220>
<221> Primer

<223> ABCC5_R
<220>
<223> Gene ABCC5

<400>
acacaccaaa ccacacagca a                                                21


<210> 606
<211> 21
<212> DNA
<213> Artificial sequence
<220>
<221> Primer
<223> POU2F2_F
<220>
<223> Gene POU2F2_F

<400>
gaggaccagc atcgagacaa a                                                21


<210> 607
<211> 20
<212> DNA
<213> Artificial sequence
<220>
<221> Primer
<223> POU2F2_R
<220>
<223> Gene POU2F2_F

<400>
aaccagacgc ggatcacttc                                                  20

## Description

### Field of the invention

The invention relates to the technical-industrial sector of the extracorporeal *in vitro* diagnosis of biological samples, by genetic engineering techniques, applied to the diagnosis of specific types of neoplasias from their gene expression patterns and/or to the prognosis of their evolution. More specifically, the invention relates to the identification of neoplasias originating from hematopoietic cells from the evaluation of the levels of messenger RNA of significant genes in biological samples such as peripheral blood samples, preferably by the use of microarrays. With this it is possible to identify samples corresponding to patients suffering from CLL, permitting the diagnosis thereof and, furthermore, it is possible to classify samples from patients suffering from CLL in samples which belong to patients wherein the CLL is going to remain stable or wherein it is going to progress, enabling the prognosis of the future evolution of these patients.

### Background of the invention

Each day, the human body produces billions of new white and red cells and platelets which replace the hematopoietic cells which are lost as a consequence of a normal process of renewal, disease or trauma. The organized production process of hematopoietic cells and homeostasis is known with the name of hematopoiesis (Weissman IL *et al.,* 2000; Leung AYH *et al.,* 2005.

In man, hematopoiesis is confined to the bone marrow (B.M.) of the greater part of the bones, and gradually, with age, this is replaced by fat, which in the adult, 70% of the bone marrow is located in the pelvis, vertebra and sternum (Bernard *et al.,* 1976).

All the mature blood cells are generated from a relatively low number of hematopoietic cells known hematopoietic stem cells. The hematopoietic stem cell has two characteristics which are the pluripotentiality or capacity to give rise to different hematopoietic cell strains and the self-renewal or property of self-perpetuation, generating cells the same as its self (Weissman IL *et al.,* 2000). This capacity is essential for the maintenance of hematopoiesis throughout the life which, without self renewal, would quickly exhaust the reserve of available stem cells. Hematopoietic stem cells are capable of generating different mature hematopoietic cell types through a series of intermediate progenitors and precursors. These progenitors and precursors suffer an ordered sequence of events which transform them into mature cells. This process is known with the name of differentiation (Lee MF *et al.,* 2005). The differentiation of the hematopoietic cells involves changes which affect, among others, the size and form of the cell, gene expression, proteins, response to signals and localization of the cells.

The terminally differentiated cells have lost their capacity for division and suffer apoptosis after a period of time which goes from hours for neutrophils to decades for some lymphocytes. This fact means the B.M. should constantly ensure cell exchange (Datta SR *et al.,* 1999).

The hematopoiesis process comprises a complex interaction between intrinsic genetic events of the hematopoietic cells and environment wherein they are found. This interaction is that which determines if the hematopoietic precursors and progenitors must stay quiescent, proliferate, be differentiated in one or another line or enter into apoptosis (Domen J *et al.,* 1999). All the genetic and environmental mechanisms which govern the production of blood cells operate by altering the relative balance of these fundamental cell processes.

Environmental and genetic factors are critical in hematopoiesis. Thus, for example, the gene expression belongings to the Rb families (Bergh *et al.,* 1999), cyclins (Della Ragione F et *al.,* 1997) or Hox (Magli MC *et al.,* 1997) regulate the proliferation of hematopoietic cells at early stages of differentiation. The genes of the bcl-2 family regulate apoptosis in hematopoietic cells (O'Gorman DM *et al.,* 2001). A great variety of genes among which are found C/EBP (Tenen DG *et al.,* 1997), Pax5 (Nutt *SL et al.,* 1999) and Ikaros (Nichogiannopoulou *A. et al.,* 1998) seem to be involved in hematopoietic differentiation and line compromise.

### Hematological neoplasias

Hematological neoplasias are malignant processes which affect any one of the cell types involved in the hematopoietic system. As a consequence of this transformation, the cell is blocked in a stage of differentiation and starts to accumulate due to uncontrolled proliferation, to a failure of the apoptotic mechanisms or a blocking of its differentiation process.

The malignant transformation of the hematopoietic cells during the different stages they pass through in their differentiation to mature cells originates a great number of different neoplasias (Guttmacher AE *et al.,* 2003). This type of neoplasias is therefore a very heterogeneous group of diseases which only has the hematopoietic origin of the cell type transformed in common.

### Classification of hematological neoplasias

Generically, it is possible to establish two groups: lymphoid neoplasias which affect the different cell type and degrees of maturity which form the lymphoid line, both B and T, and the other large group is constituted by the myeloid neoplasis which affect various cell types of the myeloid line. However, this simplistic classification is currently more developed, as detailed below.

From a clinical standpoint, classically, lymphoma leukemias have been differentiated in arbitrary form, indicating the leukemias as those neoplasias which affect the bone marrow and have peripheral expression, i.e. circulation of anomalous cells in blood, and lymphomas as those neoplasias which remain localized in the lymph nodes or other lymphoid tissues and which lack, at least initially, leukemic behaviour. In the case of leukemias, the acute processes of the chronics has initially been differentiated by the morpho-cytological characteristics of the proliferating cells (immature and atypical in the first case and differentiated in the second) and to the clinical manifestations of the disease. At present, the knowledge of the immunological markers and the genetic alterations which affect the hematopoietic cells help to differentiate the different processes more accurately.

Today, it is known that hematological neoplasias, as occurs in other types of cancer, have a multigenic origin. The great technological revolution produced in recent years has made it possible to know the molecular basis of several neoplasias. The use of these techniques makes it possible to identify relevant genes in human cancer, confirm the results obtained in basic research in animal models, establish patters of susceptibility, more accurately classify the neoplasias, improve the diagnosis of the disease, identify new therapeutic targets and improve the therapeutic selection for each patient.

Also, the diversity which exists between individuals is important and has its clinical repercussion, based on the genetic differences: if we are capable of recognising these genetic differences, we will also be capable of advancing in discovering toxicity and differences in response to treatment. (Westbrook CA *et al.,* 2005).

In 1995, the World Health Organization (WHO) in collaboration with the European Hematology Association and pathologists, clinicians and scientists throughout the world, started a project in order to obtain an agreed classification of the hematopoietic tissue and lymphoid organs. This project led to the development of a system for the definition, classification and establishment of agreed diagnostic criteria for myeloid, lymphoid and histiocytic neoplasias (Jaffe ES *et al.,* 2001). The classification criteria of the WHO are the same used in the REAL (Revised European American Lymphoma) classification published by the International Lymphoma Study Group in 1994 (Harris NL *et al.,* 1994). The REAL classification system, unlike other previous classification systems is based on the definition of "real" entities and not morphological subtypes. All available information is used to establish these "real" entities, i.e. morphological, immunophenotypical and biological data are combined with the genetic and clinical characteristics (Harris NL *et al.,* 1999a).

The WHO classification, which was presented in 1997, stratifies the entities in accordance with the cell line affected: myeloid, lymphoid, histiocytic/dentritic and mastocytic. Within each category, the disease is defined in accordance with the morphology, immunophenotype, genetic and clinical data (Harris NL *et al.,* 1999b). In many neoplasias, the stage wherein the accumulated tumour cell is found does not coincide with the stage in which the initial transformer event has occurred. Thus, many hematological neoplasias originate in the initial precursors and the specific genetic alteration may determine which cell continues advancing in its differentiating until stopping and accumulating in more advanced stages of differentiation (Shaffer AL *et al.,* 2002). In contrast, other neoplasias can develop in the more advanced stages of differentiation, as occurs in the cells from the follicular centres wherein the genetic translocations and rearranging produce activation of genes which contribute to tumour development. The classification for each entity reflects the best stimulation for its cell line and stage of differentiation, recognising that the knowledge available at present is imperfect and that changes may occur in the assignment to a cell line and in classification as the available knowledge improves.

The current criteria of diagnosis and classification of these neoplasias are based on a combination of (Braziel RM *et al,* 2003):
- Morphological evaluation of the cell: Observation under the microscope of the cells involved. Information is obtained on the type of cell and degree of its maturity.
- Study of the immunophenotype: Recognition of antigens expressed on the surface of the neoplastic cell. These antigens are expressed differently and to different degrees in accordance with the line and of the stage the cell is at. The expression of surface antigens characteristic of the line and stage of differentiation of the cell is known, for example, the expression of CD19 and CD20 is typical of line B cells, whilst the expression of CD3 is typical of line T. The study of CD23 is key when differentiating NHLCM from CLL (Gong JZ *et al.,* 2001).
   An attempt has always been made to relate the different types of neoplasias with their corresponding normal cell population through their morphological and immunophenotypical characteristics. Many neoplasias therefore seem "trapped" in determined stages of development as they have morphological and immunophenotypical characteristics similar to those of the hematopoietic cell at that stage of differentiation (Shaffer AL, *et al.,* 2002).
- Clinical characteristics: Signs and symptoms of the patient at the time of diagnosis.
- Determination of molecular markers: Measurement of some molecules which are associated to concrete entities such as the presence of PML/RARA in promyelocytic leukemia or which give a better or worse prognosis, such as, for example, the expression of CD38 in CLL cells marker of bad prognosis (Durig J *et al.,* 2002)
- Cytogenetic studies based on the search for genetic alterations in the DNA of tumour cells. In many cases, specific rearranging occur which are characteristic of types of tumour or stages (Mitelman F*, et al.,* 1997). In accordance with the chromosome translocations, it is possible to establish different groups with clinical significance, for example, in LLA-B, where the presence of fusion oncoproteins is frequent, the presence of t(2;21)/TEL1-AML1 and t(1;19)/E2A-PBX1 is associated with a response to the treatment whilst the prognosis for patients with t(9;22)/BCR-ABL and t(4;11)/MLL-AF4 is much worse (Arico M *et al.,* 2000). Searches are also usually made for specific mutations, deletions or insertions in a gene which have been related to more favourable prognosis such as, for example, the myelodysplastic syndromes associated to 5q- (Boultwood J *et al.,* 1994).

As has previously been commented, the WHO establishes four large groups of hematological neoplasias in accordance with the strain involved (myeloid, lymphoid, histiocytic/dentritic and mastocytic lines). Below the neoplasias belonging to the myeloid line and the lymphoid line are described in more detail as they are those which arise with greatest frequency. Those corresponding to the histiocytic/dentritic and mastocytic lines for the moment are very isolated entities.

### 1. Myeloid neoplasias

They group together all the neoplasias originated in the myeloid line of differentiation, the WHO distinguishes four large groups (Vardiman JW *et al.,* 2002).

### 1.1 Myeloproliferative syndromes (MPS)

Myeloproliferative syndromes (MPS) are clonal alterations of the hematopoietic stem cell characterized by effective hematopoiesis which leads to an increase in the blood levels of one or more hematopoietic and hepatosplenomegaly lines. They constitute a group of entities wherein there exists an increase in precursors of the myeloid series or fibrosis of the bone marrow (myelofibrosis); this group also includes systemic mastocytosis. The following can be highlighted:
- Chronic myeloproliferative syndromes (CMPS). Clonal alteration of the hematopoeietic stem cell. Characterized by an effective hematopoiesis which produces increase in peripheral blood of one or more cell lines and frequently hepatosplenomegaly, medullary hypercellularity with maturity but without dysplasia.
- Chronic myeloid leukemia (CML). It is a clonal process secondary to an acquired genetic alteration of the pluripotent cell. The disease is characterized by the superproduction of neutrophils and of their precursors. It has three phases: the first called chronic phase of undefined duration, followed by the acceleration phase and finally the blastic crisis which is really secondary acute leukemia.
   CML has a low incidence of approximately one case per 100,000 inhabitants/year and appears most frequently in the sixth and seventh decades of life. It can be considered a rare disease.
   It is the characteristic leukemia par excellence as the term leukaemia was applied to this entity for the first time. 95% of the cases have a genetic marker, the Philadelphia chromosome, originated by the translocation of a fragment of chromosome 22 which adheres to chromosome 9 or t(9;22) (q34;q11). This translocation causes the fusion gene bcr-abl. The protein coded by this chimeric gene, BCR-ABL, has an increased thyrosine-kinase activity compared with the normal abl protein activity as oncogenic growth factor (Pane F *et al.,* 2002), although really the mechanisms which produce the superproduction of myeloid cells are not totally clarified. It is possible that other proto-oncogenes such as p-53 intervene in the process and in the transformation of chronic phase to blastic crisis. The few cases in which the Philadelphia chromosome is detected represent atypical myeloproliferative symptoms and correspond to the variant of MDS known as chronic myelomonocytic leukemia (CMML).
   The diagnosis is based on the high cell counts for the blank series, appearance of morphologically normal myeloid cells and in all the stages of differentiation, but with a high number of myelocytes and neutrophils, there are generally basophilia and thrombocytosis. In the acceleration phase an increase in immature cells occurs in the peripheral blood and in the blastic crisis the predominant cell is the myeloblast (65%) or the lymphoblast (35%).
- Vaquez's disease (VD). It is the myeloproliferative syndrome characterized by the increase in mass of the red series. Vazquez's disease is a benign haematological disease, whose suffering does not influence shortening of survival. However, it is a clonal disease which may evolve in 15% of patients to myelofibrosis or acute leukemia (5%).
- Essential Thrombocythemia (ET). Myeloproliferative syndrome characterized by platelet production 15 times greater than normal. It may be associated to thrombotic or hemorrhagic complications secondary to platelet dysfunction. It appears at around 60 years of age, with equal incidence in both sexes.
- Myelofibrosis (MF). It is a neoplastic clonal disorder of the pluripotent stem cell. It is characterized by a great production of abnormal megakaryocytes. These cells release molecules (growth factor derived from platelets, platelet factor 4) which stimulate the proliferation of fibroblasts and build collagen fibres in the bone marrow. The bone marrow is incapable of functioning normally and the hematopoietic precursor cells translate to the liver and spleen, giving rise to extramedullary hematopoiesis. Characterized by fibrosis of B.M and splenomegaly. It appears in people over 50 years of age and has no preference of sex.
- Mastocytosis. Group of entities characterized by the proliferation of mastocytic cells in different parts of the body. Systemic mastocytosis (SM), is a rare disease which typically affects adults and has bone alterations in 70% of patients (Chen CC *et al.,* 1994).

### 1.2. Myelodysplastic/myeloproliferative syndromes (MDS/MPS)

The WHO has established a somewhat different classification, separating MDS/MPS as entities differentiated from the other MDS, since they share characteristics with the CMPS that make them different. This group includes three entities: chronic myelomonocytic leukaemia, chronic atypical myeloid, leukeumia, juvenile myelomonocytic leukaemia and non-classifiable MDS/MPS.
Myelodysplastic syndromes (MDS) are clonal proliferations of the hematopoeietic stem cell which share at the time of diagnosis, clinical, morphological and analytical data which are superimposed between AML and CMPS. They are characterized by the hypercellularity of bone marrow due to the proliferation of one or more myeloid lines (Heaney ML, 1999). The presence of dysplasia in at least one line (myeloid, erythroid or megakaryocytic-platelet) is a characteristic of MDS. The incidence is variable depending on the variety. An incidence of 3 cases x 100,000 inhabitants over 60/ year is estimated. The FAB classification establishes 4 diagnostic categories (Bennett JM *et al.,* 1984): simple refractory anemia (RA), refractory anemia with ring sideroblasts (ARS), refractory anemia excess blasts (RAEB) and refractory anemia with excess blasts in transformation (RAEB-T) and chronic myelomonocytic leukemia (CMML).

With regard to the MDS, the WHO establishes five differentiated categories (Harris NL, *et al.,* 1999): refractory anemia, refractory cytopenia with multiline dysplasia, refractory anemia with excess blasts, non-classifiable MDS and MDS associated to an isolated defect in chromosome 5 (of the 5q) or syndrome 5q-.

### 1.3. Acute myeloblastic leukemia (AML)

Clonal proliferation of immature cells of the myeloid line. They may appear de novo or secondary in patients with myelodysplastic syndrome (MDS). The classification prepared by the French-American-British group (FAB) considers eight varieties (M0-M7) based on morphological criteria and on the immunophenotype of the neoplastic cells (Bennett JM, *et al.,* 1976). Despite the fact that this classification has been accepted for many years, the discovery that many genetic alterations have a predictive characteristic and the incorporation of the cytogenetic analysis to the diagnosis of acute leukemias (Bene MC *et al.,* 2001) has made it possible to subclassify the disease and establish the evaluation of the prognosis, as occurs with translocation t(15;17) which characterized promyelocytic variety leukemia which is characterized by the expression of a retinoic acid receptor (RAR), characteristic which makes this type of leukaemia sensitive to treatment with transretinoic acid (TRA) in most cases.

The WHO classifies AML by incorporating morphological, immunophenotypical, genetic and clinical data to be able to define biological homogeneous entities and with clinical relevance. Thus, AML is classified into four large categories: 1.- AML with recurrent genetic anomalies. 2.- AML with multiline dysplasia. 3.- AML related to treatment and 4.- non-classifiable AML (ref WHO). The three first categories recognise the importance of biological factors which predict the evolution of the process. The cytogenic analysis represents the most powerful prognosis factor (Roumier C, *et al.,* 2003). It is used to identify subgroups of AML with different prognosis: low risk with favourable response to treatment (t(8;21), t(15;17) or inv(16)), intermediate risk (normal karyotype or t(9;11) or high risk (inv(3), -5del(5q) or - 7del(7q), or more than three alterations). There is molecular heterogeneity within the risk group. In some cases of patients with normal karyotype, the presence of mutations has been found in gene FLT3 (Kottaridis PD, *et al.,* 2001.) and MLL (Dohner K *et al.,* 2002).

The medullary image in the microscopic examination of aspirate is generally that of invasion by cells similar to one another, of immature morphological characteristics which distort the normal cell distribution constituting authentic cell sheets. Medullary hyperproduction conditions which areas of inactive bone marrow come to again present a new focus of hematopoiesis in the adult age, in this case of abnormal cells.

Approximately 80-90% of young patients with AML, achieve complete remission of the disease after chemotherapy. However, the majority relapses, and a cure occurs in 30%. The oncogenic transplant of bone marrow has managed to increase the cure rate to 50%, but it is limited by the availability of identical donor HLA. It is therefore a group of neoplasias with diverse genetic abnormalities and variable response to treatment (Giles FJ *et al.,* 2002)

### 2. Lymphoid neoplasias

The WHO's classification is a refinement of the REAL classification (Harris NL *et al.* 1994). Three large groups of lymphoid neoplasias: 1.- Lymphoid neoplasias derived from B cells. 2.- Lymphoid neoplasias derived from T and NK cells. 3.-Hodgkin's lymphoma. This classification includes solid neoplasias and lymphoid leukemias, as in many of them their occurs a transformation from one phase to another and the distinction between them, may be artificial. Thus, chronic lymphatic leukemia B and the lymphocytic NHL are originated by the same cell and represent different manifestations of the same neoplasia, the same occurs with lymphoblastic lymphoma and lymphoblastic leukaemia

### 2.1. Neoplasias derived from B, T and NK cells

The WHO's classification divides these neoplasias in accordance with the stage of maturity of the cells in neoplasias of precursor cells and neoplasias of mature cells (WHO Classification Tumours of Haematopoietic and lymphoid tissues. In Pathology and genetics of tumours of Haematopoietic and lymphoid tissues. ES Jaffe, NL Harris, H Stein, JW Vardiman. IARC Press. Lyon, 2001). Due to the high number of entities described, the following are highlighted:
- Acute lymphoblastic leukemia (ALL): Clonal proliferation of lymphoid precursors. In approximately 80% of the cases, the precursors belong to the lymphoid B line. The molecular analysis of the genetic alterations of the leukemic cells have significantly contributed to the understanding of the pathogenesis and prognosis of ALL (Ferrando AA *et al.,* 2005). Despite the fact that the frequency of genetic subtypes differs in children and in adults, the general mechanisms which lead to ALL are a consequence of the abnormal expression of proto-oncogenes due to chromosome translocations which create fusion genes or a hyperploidy. This initial oncogenic event is probably insufficient to produce leukemia and it is believed that other alterations which cooperate with this first one are necessary to definitively alter the proliferation and survival of the transformed cell. All these alterations contribute to the leukemic transformation of the hematopoietic stem cells or of their progenitors as they affect key regulating processes, maintaining or increasing their capacity for self-renewal, escape from the normal proliferation controls, blocking of differentiation and promoting resistance to apoptotic signals (Hanahan D, *et al.,* 2000).
   The overall appearance of the bone marrow is similar to that described for myeloid leukemia. The research of the minimal residual disease is important, a factor which condiciona with su presence the probable relapse of the disease. The FAB classification defines 3 stages in accordance with the morphology (L1-L3).
   It is the most frequent leukemia in the childhood, and in the clinical course and the response to treatment depends on the type of genetic alteration, for example, patients with hyperdiploidy have a favourable prognosis when it is treated with treatment schemes which include antimetabolites but, in general terms, children are cured with standard chemotherapy and prophylaxis of the CNS and in adults only 20% have prolonged survival with chemotherapy, the allogenic autologous transplant is useful for cases considered high risk.
- Chronic lymphatic leukemia (CLL). CLL is characterized by clonal proliferation and accumulation of lymphocytes with mature appearance and resistant to apoptosis in B.M, blood and lymphoid organs (Galton DA, 1966). When the lymphodenopathy is dominant, the clinical symptoms are called Lymphocytic lymphoma. The lymphocytes affected are line B in 95% of the cases and 5% of the cases involve T lymphocytes.
   It is the most frequent leukemia in the Western world. The average age of patients diagnosed is 65 years old, only 10-15% of the cases arise under 50 years (Jemal A *et al.,* 2003). It is the most common cause of leukaemia in adults of the counties of the Western world and involves around 25% of all leukemias. The incidence is 3 cases per each 100,000 inhabitants and year, with a predominance in males, with a male/female proportion of 1.7:1. In recent years, it has increasingly been diagnosed in younger patients. The proportion of cases diagnosed at early stages of the disease (Rai KR, *et al.,* 1975) has increased from 10 to 50%, probably due to an early diagnosis thanks to routine lymphocyte counts. The disease affects more men than women.
   The prognosis and clinical course of the disease is extremely variable. Some patients have a rapidly progressive evolution and die in the 2-3 years after the diagnosis, whilst in others, the course is indolent and they live for 10-20 years without problems related to the CLL. Intermediate cases occur in half of patients.
   Approximately, 20% of patients are asymptomatic at the time of diagnosis, performing this as a consequence of a routine blood analysis. When symptoms exist, they are not specific and include fatigue, weakness and discomfort.
   The Binet classification (Binet JL *et al.,* 1981) defines 3 stages of disease in accordance with the concentration of haemoglobin, number of platelets, number of lymph nodes involved and the presence of visceromegalies. The Rai classification (Rai KR *et al.,* 1975) uses the same indicators but classifies patients in five groups.
   This neoplasia is not characterized by a unique and recurrent genomic alteration. There are some markers which give a more unfavourable prognosis such as the presence of deletions in chromosomes 17 and 11 and those patients with absence of mutations in IgVh genes (40% of the cases) and high proportion of cells expressing CD38 is characterized by a more agressive clinical course and a worse response to treatment (Hamblin TJ *et al.,* 1999; Durig J *et al.,* 2002). Another recently described marker is ZAP-70, independent prognosis marker whose expression is indirectly related to the mutational state of the gene of the heavy chains of immunoglobulins (Crespo M *et al.,* 2003).
- Multiple myeloma: MM). MM is a malignant disease wherein a clone of plasma cells (terminal cells of the B lymphoid line) of the bone marrow suffers uncontrolled proliferation. It involves 10-15% of all the malignant diseases and is characteristic of advanced ages, only 2% of the cases are diagnosed before 40 years of age. For unknown reasons, the incidence of the disease is increasing.
   These cells produce and secrete monoclonal immunoglobulin or fragments of immunoglobulins, composed by a heavy and light chain class (kappa or lambda). Occasionally, the myeloma cannot be secreted or the protein is not detectable in serum or urine. The neoplastic plasma cell produces other molecules such as IL6, tumour necrosis factor or osteoclast activator factor which contributes to producing osteolysis, hypercalcemia and renal insufficiency, characteristics alterations of the disease.
   The diagnosis can be casual on performing an analysis in patients without symptomology or limited disease (20% of cases). The disease in these patients can remain stable for years and early treatment in the asymptomatic phase does not provide any advantages.
   Patients with monoclonal component but which do not meet the MM diagnosis are considered carriers of monoclonal gammapathy of indeterminate meaning (MGIM). Among 10 and 20% of these patients develop MM in 10 years (Kyle RA, 1997; Zhan F *et al.,* 2002). The monoclonal component can also be associated to other diseases such as lymphoma, non-hematological neoplasias and diseases of the connective tissue.
- Lymphoplasmocytoid lymphoma and Waldenstrom's macroglobulinemia. It is the clinical expression of a low-degree lymphoproliterative disease, characterized by the infiltration of anomalous lymphoplasmocytic cells in bone marrow, lymph node and spleen, accompanied by monoclonal production of immunoglobin M, which conditions an increase in blood viscosity and the appearance of haemorrhagic vascular manifestations and by difficulty in circulation in the small vessels.
- Non-Hodgkin's lymphoma (NHL). NHL are solid tumours of the lymphoid tissue which are much more heterogeneous than Hodgkin's disease. The complexity and diversity of the NHL as regards morphology, genetics, phenotype and clinical behaviour has given rise to the existence of multiple classifications, none of them completely satisfactory.

It is the most frequent hematological disease and, in terms of years of life lost, it is the fourth most important neoplasia of the Western world and it seems that its incidence is increasing.

It may appear at all ages, but the average appearance is 50 years of age. The cause of the disease is not clear. Specific chromosome translocations have been described associated to certain types of lymphomas, for which reason they are of great use in diagnosis (Montoto S *et al.,* 2003). Most of the Burkitt-type lymphomas present translocation t(8;14), wherein the c-MYC oncogene of chromosome 8 is transferred to the next region in chromosome 14 where the heavy immunoglobins chains are coded. 90% of ollicular lymphomas are characterized by translocation t(14;18), where the bcl-2 gene of the chromosome 18 is transferred to the region of the heavy immunoglobulin chains. It is well known that the overexpression of bcl-2 inhibits apoptosis (programmed cell death). It is easy that this chromosome rearranging requires other stimulation, such as, for example, the coexpression of a second proto-oncogene or an antigenic stimulation to develop the malignant proliferation. An example of combination of multiple combined causes constitute the lymphoma associated to AIDS. The appearance of aggressive extranodal lymphomas is the result of the combination of immunosuppression by HIV, deregulation of a proto-oncogene (c-MYC) and a secondary viral infection (Epstein-Barr's virus), the same occurs in patients subjected to organ transplant (Harris NL *et al.,* 2001).

The clinical presentation of the disease is more irregular than in Hodgkin's disease. It may behave indolently without requiring immediate treatment or, in contrast, behave aggressively which is quickly fatal.

The most frequent nodal condition is cervical. As regards extranodal condition, the signs and symptoms depend on the affected organ. The bone marrow appears infiltrated with greater frequency in the low degree NHL and may cause pancytopenia. The presence of malignant cells in peripheral blood is also frequent in low-degree NHL, but of very bad prognosis in those of high-degree.

The diagnosis is carried out by the histological study of the lymphatic tissue. The additional information is obtained by monoclonal antibodies directed against specific lymphocytic antigens (immunophenotype); this helps to identify the degree of maturity of the malignant cell and determine the T or B origin thereof. The presence of mutation in genes which code Ig in the NHL of strain B are usually used for the identification of some subtypes of NHL (Kuppers R *et al.,* 1999).

### 2.2. Hodgkin's Lymphoma (LH)

It is an infrequent disease and has predilection for the masculine sex in a proportion of 2/1. It is characterized by the presence of large cells, bi or multi-nucleus called Reed-Sternberg (RS) and other smaller and mononuclear cells which appear in a small quantity in the tumour; the rest of the cells are lymphocytes, granulocytes, fibroblasts and plasma cells. This inflammatory infiltrate probably reflects the immune response of the host with the malignant cells. The nature of the RS and Hodgkin's cells have been greatly studied but continues being disputed. They may be derived from an initial stage of the lymphoid cells.

In some cases, the existence of DNA for Epstein-Barr's virus has been detected in the tumour. One hypothesis is that the bimodal distribution of the disease is due to the infection in young subjects and the other peak would be caused by average environmental causes.

The diagnosis is obtained by biopsy of a lymph node. To plan the treatment, it is necessary to determine the extension of the disease. (Küppers R, 2002; Cossman J, 2001; Devilard E *et al.,* 2002).

### Problems in classification

The great quantity of hematopoietic cells and the many stages of differentiation through which they pass further complicates the classification of the neoplasis originating from this type of cells. Despite the efforts to establish a classification based on "real" entities, some of the categories are ambiguous and in many cases contain very heterogeneous groups as regards a response to therapy of clinical course. This heterogeneity is that responsible for, on the one hand, the incessant search for markers capable of differentiating some behaviours from others and, on the other hand, that the disputed classification of this type of neoplasis is subjected to continuous revisions.

An ideal classification system should be precise, reproducible, easy to use and should especially have biological and clinical significance (Chan WC, *et al.,* 2005). The current diagnosis systems and the classification of the hematological neoplasias are based on the recognition of histological and morphological, immunophenotypical and cytogenetic characteristics and study of a molecular marker with prognostic value. However, in some of the diagnostic categories defined in this way, the following is observed:
- A marked heterogeneous therapy response. Within the same disease there are patients who reach full remission, partial remission, do not respond, which relapse after a certain therapy. The capacity to predict a response is especially important in this type of neoplasis since the transplant of stem cells is an effective but toxic alternative response. The capacity to determine what patients would respond to a conventional therapy before giving it may be beneficial to be able to apply the most effective treatment to each patient.
- A variable clinical behaviour. Within this category there are patients whose disease is going to remain stable for long periods of time and which are not going to need therapy and those whose disease is going to progress rapidly requiring aggressive therapy.

These variations point to the existence of molecular heterogeneity within the diagnostic categories, differences which the conventional methods of diagnosis are not capable of determining and hence, the search for new forms of analysis which provide a greater resolution in the characterization of this type of neoplasias.

In this line, the use of expression arrays have demonstrated being effective not only in deciphering the biological and clinical diversity which is found in many tumours, but in understanding the biological and pathological processes which affect many symptoms and, in particular, the hematopoietic system. The expression arrays are ordered arrays of sequences associated to a solid support, complementary to mRNA or to its corresponding cDNA or cRNA, which allow the analysis of the differential expression of hundreds or thousands of genes simultaneously. One of the supports to which they are frequently bound is to rectangular fragments of glass similar to slides, a format which is frequently alluded to by the terms microarray, biochip or, simply, chip. Their use is becoming increasingly frequent for the diagnosis of various diseases or for the evolution of the evaluation of the susceptibility of suffering from them.

### First works of arrays and hematological neoplasias

In 1999, the Golub group published one of the first articles referring to the role of arrays in the classification of hematological neoplasias (Golub TR *et al.,* 1999). An array with 6817 genes represented was used for the study of expression profiles in AML and ALL. A group of 50 genes was selected with the capacity of predicting the type of leukemia (class predictor) and they were used to classify a group of unknown samples in the correct categories. The study of the expression of these 50 genes is sufficient for the classification of a sample of acute leukemia in AML or ALL. Despite the fact that the distinction between AML and ALL is well established with the current diagnostic methods, the study revealed the existence of specific expression patterns associated with each type of acute leukemia and proved the use of expression profiles in cancer classification.

In 2000, the Alizadeh group published an article in which a specialized array is used, the lymphochip which contains genes expressed preferentially in lymphoid cells or if which an immunological or oncological importance is known with 17,856 sequences (Alizadeh AA *et al.,* 1999). This group used the "lymphochip" for the study of gene expression patterns associated to differences in clinical behaviour in a Diffuse Large B-Cell Lymphoma (DLCL) (Alizadeh AA, *et al.* 2000). The DLCL is a NHL with a very heterogeneous behaviour and impossible to distinguish using conventional diagnostic methods: 40% of patients respond well to therapy and have prolonged survival whilst 60% die due to the disease. The authors found that the gene expression could be related to the clinical behaviour of the tumours. This was one of the first articles to speak of arrays for the "subclassification" of hematological neoplasias, i.e. the use of expression profiles for the identification of two different groups of DLCL from the transcriptional standpoint, DLCL subtypes with clinical behaviour impossible to predict with conventional diagnostic criteria.

At present there are multiple publications wherein, directly or indirectly appear the arrays applied not only to classification and subclassification, but also to the study, diagnosis, prognosis, identification of new markers in haematological diseases (Greiner TC, 2004; Alizadeh AA *et al,* 2000; Bea S *et al.,* 2005; Dave SS *et al.,* 2004), as well as patent applications which disclose the use of this type of device for the differentiation between different types of hematological neoplasias. Thus, for example, patent application W02003/008552 discloses the use with diagnostic purposes of differences in the expression pattern of genes to differentiate between mixed line leukemia (MLL), acute lymphoblastic leukemia (ALL) and acute leukemia myelogenous leukemia (AML), defending the possibility of making this differential diagnosis with the data obtained after the diagnosis of samples from patients afflicted by each one of these types of leukemia by the use of commercial chips from Affymetrix. Although genes are indicated with variations in the expression between the three types of leukemias which would permit the differentiation between them, no specific sequences are mentioned other than those present in the Affymetrix chip which could have been used to detect these genes by devices different from those of said company, nor does it consider the design of devices or methods which would permit the diagnosis of other types of leukemias or, in general, neoplasias derived from hematopoietic cells.

Patent application W02005/024043, for its part, also relates to the field of gene expression analysis to go into greater detail in the knowledge of differences existing at a molecular level between the different neoplasias derived from hematopoietic cells, specifically centering on the case of lymphomas, to extract data which help in its diagnosis or in the prognosis of its evolution. In particular, it discloses a method to obtain useful functions to predict the evolution of individuals affected by different types of lymphomas evaluating in lymph node biopsies to what extent patterns or genetic prints contribute in each one of them, groups of genes which are expressed in a coordinated manner and which are related to the cell origin of the neoplasia, the different types of non-malignant cells present in the biopsy and the oncogenic mechanisms responsible for cancer. The different patterns or genetic prints are also deduced in this case from the data obtained with commercial chips from Affymetrix. Furthermore, application W02005/024043 states it provides an alternative microarray, composed of a fewer number of sequences than the Affymetrix microarrays, which would also permit the analysis of differences in gene expression between lymphomas and their application for deducing functions of prediction of survival and for the differentiation between different types of lymphomas. Although it indicates the genes whose analysis would be made possible by that microarray, the specification of application W02005/024043 does not indicate the sequence of the probes which would compose the microarray, only mentioning that they would be cDNA type and leaving doubts over whether that cDNA would appear complete or the analysis of the corresponding gene expression would be carried out using as probe only one fragment of said cDNA, which would remain tp be determined.

It would be interesting to have compositions and methods which would permit ifferentiation between neoplasias of hematopoietic origin based on their molecular level difference, specifically designed for this group of neoplasias, wherein it would evaluate the expression of a more reduced number of genes than in the commercial microarrays used in the studies described in the aforementioned patient applications and which enabled both the diagnosis of certain neoplasias and the prediction of their future evolution, thus helping in the prescription of a suitable treatment for each patient, a particularly interesting characteristic in those neoplasias, as is the case of CLL, wherein the prognosis of the future evolution of the patient is difficult with the knowledge and tests available to date. Furthermore, it would be particularly convenient that the probes used to evaluate the expression of the expressed genes had been designed specifically so that, in addition to being specific and with a perfectly defined sequence, all had a similar behaviour, which would make them suitable, in general, to use in combination in a same test and, in particular, to form part of the same ordered array associated to a solid support, such as chips or microarrays. The compositions and methods of this invention meet this need.

Instead of commercial microarrays to detect genes significant for distinguishing between neoplasias or creating functions which predict the survival of the individual suffering from it, the invention provides new oligonucleotides, of perfectly defined sequence, capable of specifically detecting genes which have been selected as they are known to be significant for the biology of blood cells or for the pathology of different neoplasias, oligonucleotides which also have the feature of having being designed so that they share common characteristics which have a similar behaviour to those used as probes in hybridization, which makes them suitable to be used in compositions which comprise combinations thereof Said compositions and in particular those wherein these nucleotides are arranged in ordered form on an easy to handle solid support such as glass similar to slides, are suitable for carrying out tests to detect statistically significant genes or differentiate samples taken from individuals suffering from certain types of neoplasias originating from hematopoietic cells of samples taken from individuals not suffering from said neoplasias, as they are compositions which contain a number of nucleotides less than those commercial microarrays designed with a more general purpose, being specifically designed for the analysis of samples from individuals suffering from neoplasias and composed of a known sequence of probes, perfectly reproducible, which are designed to be used together in the same test as they are of similar behaviour. The additional inclusion in the microarrays of the invention of oligonucleotides of low homology with human genes, but chosen so that the rest of their characteristics are similar to those of the oligonucleotides of the invention designed to act as probes capable of recognizing human genes with high specificity, permits the use of said microarrays for the identification of statistically significant genes in the identification of samples associated to certain neoplasias of hematopoietic origin by the use of tests wherein it is feasible to establish controls in all their phases. As shown in the examples which appear further on, in the present specification the use of these microarrays in combination with various statistical techniques permits the correct classification of different biological samples by a method which is precise, reproducible, easy to use and with biological and clinical significance, as they are based on differences of gene expression with significance for the biological processes which are being analysed. In particular, the use of a microarray of the invention in combination with the method of the invention permits the identification of blood samples in patients suffering from chronic lymphatic leukemia (alteration not considered in applications W02003/008552 and W02005/024043 and whose diagnosis has not been described by the use of commercial microarrays), distinguishing those of both samples obtained from healthy individuals and samples related to other types of leukemias, and those corresponding to Jurkat or U937 cells, facilitating the diagnosis of CLL through the analysis of expression levels of statistically significant genes to do this and even permitting the obtainment of functions which enable the mathematical calculation of the probability of a sample belonging to individuals afflicted with stable chronic lymphatic leukemia from samples belonging to individuals afflicted with progressive chronic lymphatic leukemia, a distinction which is now difficult to carry out a priori by the available techniques, which means it is a useful and novel tool for the prognosis of the future evolution of individuals afflicted with this disease, individuals whose diagnosis may also have been carried out by compositions and method of the invention or may have been known thanks to the application of a different method, but for which, on having a tool which makes it possible to make a prognosis on how the CLL they are suffering from is going to later evolve, it would be easier to decide if it is suitable to subject them to an immediate aggressive treatment or simply keep them under observation to check that their gene expression data continue indicating that the disease is going to remain stable for a long period of time.

### Summary of the invention

The invention provides compositions which include at least one oligonucleotide from the group composed of:
SG1, SG2, SG3, SG4, SG5, SG6, SG7, SG8, SG9, SG10, SG11, SG12, SG13, SG14, SG15, SG16, SG17, SG18, SG19, SG20, SG21, SG22, SG23, SG24, SG25, SG26, SG27, SG28, SG29, SG30, SG31, SG32, SG33, SG34, SG35, SG36, SG37, SG38, SG39, SG40, SG41, SG42, SG43, SG44, SG45, SG46, SG47, SG48, SG49, SG50, SG51, SG52, SG53, SG54, SG55, SG56, SG57, SG58, SG59, SG60, SG61, SG62, SG63, SG64, SG65, SG66, SG67, SG68, SG69, SG70, SG71, SG72, SG73, SG74, SG75, SG76, SG77, SG78, SG79, SG80, SG81, SG82, SG83, SG84, SG85, SG86, SG87, SG88, SG89, SG90, SG91, SG92, SG93, SG94, SG95, SG96, SG97, SG98, SG99, SG100, SG101, SG102, SG103, SG104, SG105, SG106, SG107, SG108, SG109, SG110, SG111, SG112, SG113, SG114, SG115, SG116, SG117, SG118, SG119, SG120, SG121, SG122, SG123, SG124, SG125, SG126, SG127, SG128, SG129, SG130, SG131, SG132, SG133, SG134, SG135, SG136, SG137, SG138, SG139, SG140, SG141, SG142, SG143, SG144, SG145, SG146, SG147, SG148, SG149, SG150, SG151, SG152, SG153, SG154, SG155, SG156, SG157, SG158, SG159, SG160, SG161, SG162, SG163, SG164, SG165, SG166, SG167, SG168, SG169, SG170, SG171, SG172, SG173, SG174, SG175, SG176, SG177, SG178, SG179, SG180, SG181, SG182, SG183, SG184, SG185, SG186, SG187, SG188, SG189, SG190, SG191, SG192, SG193, SG194, SG195, SG196, SG197, SG198, SG199, SG200, SG201, SG202, SG203, SG204, SG205, SG206, SG207, SG208, SG209, SG210, SG211, SG212, SG213, SG214, SG215, SG216, SG217, SG218, SG219, SG220, SG221, SG222, SG223, SG224, SG225, SG226, SG227, SG228, SG229, SG230, SG231, SG232, SG233, SG234, SG235, SG236, SG237, SG238, SG239, SG240, SG241, SG242, SG243, SG244, SG245, SG246, SG247, SG248, SG249, SG250, SG251, SG252, SG253, SG254, SG255, SG256, SG257, SG258, SG259, SG260, SG261, SG262, SG263, SG264, SG265, SG266, SG267, SG268, SG269, SG270, SG271, SG272, SG273, SG274, SG275, SG276, SG277, SG278, SG279, SG280, SG281, SG282, SG283, SG284, SG285, SG286, SG287, SG288, SG289, SG290, SG291, SG292, SG293, SG294, SG295, SG296, SG297, SG298, SG299, SG300, SG301, SG302, SG303, SG304, SG305, SG306, SG307, SG308, SG309, SG310, SG311, SG312, SG313, SG314, SG315, SG316, SG317, SG318, SG319, SG320, SG321, SG322, SG323, SG324, SG325, SG326, SG327, SG328, SG329, SG330, SG331, SG332, SG333, SG334, SG335, SG336, SG337, SG338, SG339, SG340, SG341, SG342, SG343, SG344, SG345, SG346, SG347, SG348, SG349, SG350, SG351, SG352, SG353, SG354, SG355, SG356, SG357, SG358, SG359, SG360, SG361, SG362, SG363, SG364, SG365, SG366, SG367, SG368, SG369, SG370, SG371, SG372, SG373, SG374, SG375, SG376, SG377, SG378, SG379, SG380, SG381, SG382, SG383, SG384, SG385, SG386, SG387, SG388, SG389, SG390, SG391, SG392, SG393, SG394, SG395, SG396, SG397, SG398, SG399, SG400, SG401, SG402, SG403, SG404, SG405, SG406, SG407, SG408, SG409, SG410, SG411, SG412, SG413, SG414, SG415, SG416, SG417, SG418, SG419, SG420, SG421, SG422, SG423, SG424, SG425, SG426, SG427, SG428, SG429, SG430, SG431, SG432, SG433, SG434, SG435, SG436, SG437, SG438, SG439, SG440, SG441, SG442, SG443, SG444, SG445, SG446, SG447, SG448, SG449, SG450, SG451, SG452, SG453, SG454, SG455, SG456, SG457, SG458, SG459, SG460, SG461, SG462, SG465, SG468, SG469, SG470, SG471, SG472, SG473, SG474, SG475, SG476, SG477, SG478, SG479, SG480, SG481, SG482, SG483, SG484, SG485, SG486, SG487, SG488, SG489, SG490, SG491, SG492, SG493, SG494, SG495, SG496, SG497, SG498, SG499, SG500, SG501, SG502, SG503, SG504, SG505, SG506, SG507, SG508, SG509, SG510, SG511, SG512, SG513, SG514, SG515, SG516, SG517, SG518, SG519, SG520, SG521, SG522, SG523, SG524, SG525, SG526, SG527, SG428, SG529, SG530, SG531, SG532, SG533, SG534, SG535, SG536, SG537, SG538, SG539, SG540, SG541, SG542, SG543, SG544, SG545, SG546, SG547, SG548, SG549, SG550, SG551, SG552, SG553, SG554, SG555, SG556, SG557, SG558, SG559, SG560, SG561, SG562, SG563, or combinations thereof.

Said oligonucleotides have been designed so that, in addition to being specific for the corresponding genes whose expression one wants to evaluate, they have a similar behaviour, as they are of similar lengths and all of them have GC in the range of 40% to 60%, in addition to corresponding to zones situated less than 3000 nucleotides from end 3' (poly(A)) of the mRNA which one wants to detect and evaluated and of being constituted by sequences which coincide in their sense with those of the corresponding mRNA. Therefore, they are suitable to be used in the same test or form part of a composition which comprises combinations thereof. A particular embodiment of the invention is constituted by the compositions which comprise mixtures of several of said oligonucleotides. Especially preferred are those compositions which comprise mixtures of oligonucleotides which correspond to genes significant for classifying a sample as associated to a certain neoplasia and/or to determine the future evolution thereof. Especially preferred embodiments of the invention are also those compositions which comprise the totality of the oligonucleotides from the group composed of: SG1, SG2, SG3, SG4, SG5, SG6, SG7, SG8, SG9, SG10, SG11, SG12, SG13, SG14, SG15, SG16, SG17, SG18, SG19, SG20, SG21, SG22, SG23, SG24, SG25, SG26, SG27, SG28, SG29, SG30, SG31, SG32, SG33, SG34, SG35, SG36, SG37, SG38, SG39, SG40, SG41, SG42, SG43, SG44, SG45, SG46, SG47, SG48, SG49, SG50, SG51, SG52, SG53, SG54, SG55, SG56, SG57, SG58, SG59, SG60, SG61, SG62, SG63, SG64, SG65, SG66, SG67, SG68, SG69, SG70, SG71, SG72, SG73, SG74, SG75, SG76, SG77, SG78, SG79, SG80, SG81, SG82, SG83, SG84, SG85, SG86, SG87, SG88, SG89, SG90, SG91, SG92, SG93, SG94, SG95, SG96, SG97, SG98, SG99, SG100, SG101, SG102, SG103, SG104, SG105, SG106, SG107, SG108, SG109, SG110, SG111, SG112, SG113, SG114, SG115, SG116, SG117, SG118, SG119, SG120, SG121, SG122, SG123, SG124, SG125, SG126, SG127, SG128, SG129, SG130, SG131, SG132, SG133, SG134, SG135, SG136, SG137, SG138, SG139, SG140, SG141, SG142, SG143, SG144, SG145, SG146, SG147, SG148, SG149, SG150, SG151, SG152, SG153, SG154, SG155, SG156, SG157, SG158, SG159, SG160, SG161, SG162, SG163, SG164, SG165, SG166, SG167, SG168, SG169, SG170, SG171, SG172, SG173, SG174, SG175, SG176, SG177, SG178, SG179, SG180, SG181, SG182, SG183, SG184, SG185, SG186, SG187, SG188, SG189, SG190, SG191, SG192, SG193, SG194, SG195, SG196, SG197, SG198, SG199, SG200, SG201, SG202, SG203, SG204, SG205, SG206, SG207, SG208, SG209, SG210, SG211, SG212, SG213, SG214, SG215, SG216, SG217, SG218, SG219, SG220, SG221, SG222, SG223, SG224, SG225, SG226, SG227, SG228, SG229, SG230, SG231, SG232, SG233, SG234, SG235, SG236, SG237, SG238, SG239, SG240, SG241, SG242, SG243, SG244, SG245, SG246, SG247, SG248, SG249, SG250, SG251, SG252, SG253, SG254, SG255, SG256, SG257, SG258, SG259, SG260, SG261, SG262, SG263, SG264, SG265, SG266, SG267, SG268, SG269, SG270, SG271, SG272, SG273, SG274, SG275, SG276, SG277, SG278, SG279, SG280, SG281, SG282, SG283, SG284, SG285, SG286, SG287, SG288, SG289, SG290, SG291, SG292, SG293, SG294, SG295, SG296, SG297, SG298, SG299, SG300, SG301, SG302, SG303, SG304, SG305, SG306, SG307, SG308, SG309, SG310, SG311, SG312, SG313, SG314, SG315, SG316, SG317, SG318, SG319, SG320, SG321, SG322, SG323, SG324, SG325, SG326, SG327, SG328, SG329, SG330, SG331, SG332, SG333, SG334, SG335, SG336, SG337, SG338, SG339, SG340, SG341, SG342, SG343, SG344, SG345, SG346, SG347, SG348, SG349, SG350, SG351, SG352, SG353, SG354, SG355, SG356, SG357, SG358, SG359, SG360, SG361, SG362, SG363, SG364, SG365, SG366, SG367, SG368, SG369, SG370, SG371, SG372, SG373, SG374, SG375, SG376, SG377, SG378, SG379, SG380, SG381, SG382, SG383, SG384, SG385, SG386, SG387, SG388, SG389, SG390, SG391, SG392, SG393, SG394, SG395, SG396, SG397, SG398, SG399, SG400, SG401, SG402, SG403, SG404, SG405, SG406, SG407, SG408, SG409, SG410, SG411, SG412, SG413, SG414, SG415, SG416, SG417, SG418, SG419, SG420, SG421, SG422, SG423, SG424, SG425, SG426, SG427, SG428, SG429, SG430, SG431, SG432, SG433, SG434, SG435, SG436, SG437, SG438, SG439, SG440, SG441, SG442, SG443, SG444, SG445, SG446, SG447, SG448, SG449, SG450, SG451, SG452, SG453, SG454, SG455, SG456, SG457, SG458, SG459, SG460, SG461, SG462, SG465, SG468, SG470, SG472, SG473, SG474, SG475, SG476, SG477, SG478, SG479, SG480, SG481, SG482, SG483, SG484, SG485, SG486, SG487, SG488, SG489, SG490, SG491, SG492, SG493, SG494, SG495, SG496, SG497, SG498, SG499, SG500, SG501, SG502, SG503, SG504, SG505, SG506, SG507, SG508, SG509, SG510, SG511, SG512, SG513, SG514, SG515, SG516, SG517, SG518, SG519, SG520, SG521, SG522, SG523, SG524, SG525, SG526, SG527, SG428, SG529, SG530, SG531, SG532, SG533, SG534, SG535, SG536, SG537, SG538, SG539, SG540, SG541, SG542, SG543, SG544, SG545, SG546, SG547, SG548, SG549, SG550, SG551, SG552, SG553, SG554, SG555, SG556, SG557, SG558, SG559, SG560, SG561, SG562, SG563.

Additionally, the invention provides oligonucleotides useful to be used as controls in the method of the invention. On the one hand as integrity controls, the pairs of oligonucleotides SG463 and SG464 (complementary, respectively at ends 5' and 3' of the β-actin gene) and SG466 and SG467 (complementary, respectively, to ends 5' and 3' of the GAPD gene) are provided. Additionally, oligonucleotides SSPC1, SSPC2, SSPC3, SSPC4, SSPC5, SSPC6 and SSPC7 are provided, which may be used as exogenous internal positive controls of the process quality after adding to the sample which contains the starting mRNA molecules of polyadenylated nucleic acids which contain fragments which correspond in their sequence to those of these oligonucleotides (such as the transcripts corresponding to the genes wherefrom said nucleotides are derived) and which are subjected to the same processing as the starting mRNA, as well as oligonucleotides SCN2, SCN3, SCN6, SCN8, SCN11, SCN12 and SCN13, designed to be used as positive hybridization controls and oligonucleotides SCN1, SCN5, SCN7, SCN10, SC1, SC2, SC3, SC4, SC5, SC6 and SC7, designed to be used as negative controls; they all comply with the conditions of having low homology with human genes, in addition to complying with the same conditions of the oligonucleotides complementary to human genes of being of similar lengths and all of them having GC contents in the range of 40% to 60%, correspond to zones situated at less than 3000 nucleotides from end 3' (poly(A)) of the non-human mRNA which would be capable of detecting and being constituted by sequences which coincide in their sense with those of the corresponding mRNA. Any composition which contains at least one of oligonucleotides SG463, SG464, SG466, SG467, SSPC1, SSPC2, SSPC3·, SSPC4, SSPC5, SSPC6, SSPC7, SCN2, SCN3, SCN6, SCN8, SCN11, SCN12, SCN13, SCN1, SCN5, SCN7, SCN10, SC1, SC2, SC3, SC4, SC5, SC6 and SC7, in combination with at least one of the oligonucleotides complementary to human genes of the invention mentioned above is also a composition included in the scope of the present invention.

It is especially preferred that the oligonucleotides which form part of a composition of the invention are bound to a solid support. In particular, those are preferred of said compositions wherein the distribution of the oligonucleotides on the solid support are of ordered form, whereby the solid support is a rectangular piece of glass similar to a microscope slide and that the oligonucleotides are bound to the glass by covalent bonds; the compositions which meet said characteristics are referred to in the rest of the specification with the words "microarray", "chip" or "microchip". Among these compositions in the form of microarray, there is a special preference for those which contain more than one copy of each one of the oligonucleotides which form part thereof, very especially preferring that the number of copies of each one of the nucleotides present is at least 12.

The scope of the invention also includes any diagnostic device which comprises a composition of the invention. The expression "diagnostic device" refers not only to that which serves to determine if the individual suffers from a disease or not but also those which serve to classify the disease an individual is suffering from as belonging to a subtype associated to a determined form of future evolution of said disease and, which therefore, also have a prognostic value of the future evolution of the disease.

The invention also provides a method for diagnosing a neoplasia originating from hematopoietic cells and/or making a prognosis of the evolution thereof which comprises *the in vitro* detection from a biological sample and the statistical analysis of the expression level of at least one significant gene for classifying the sample as associated or not to said neoplasia, a gene which is selected from the group composed of GABARAP, NPM3, ABCB1, ABCB4, ABCC3, ABCC5, ABCC6, ABHD1, ABL1, ACTN1, AF1q, AKR1A1, ALDH1A1, ALK, ANK2, ANPEP, ANXA6, ANXA7, APAF1, APEX, ARHGEF2, ARS2, ASNS, ATIC, ATM, ATP50, BAX, BCL10, BCL2, BCL2A1, BCL2L1, BCL2LAA, BCL3, BCL6, BCL7A, BCL7b, BCR, BECN1, BIK, BIRC3, BIRC5, BLMH, BLR1, BLVRB, BMI1, BMP6, BRMS1, BST2, BTG1, BUB1, C21orf33, C5orfl3, CA12, CALD1, CANP2, CASC3, CASP1, CASP3, CASP4, CASP5, CASP6, CASP7, CASP8, CASP9, CAST, CATSD, CBFA2T1, CBFB, CCNA1, CCNB1, CCND1, CCND2, CCND3, CCNE1, CCR6, CCR7, CCT6A, CD14, CD19, CD2, CD22, CD24, CD28, CD33, CD34, CD36, CD38, CD3E, CD4, CD44, CD47, CD48, CD5, CD58, CD59, CD6, CD7, CD79A, CD79B, CD8, CD81, CD83, CD86, CD9, CDA, CDC25A, CDC25B, CDK2, CDK4, CDK5R1, CDKN1A, CDKN1B, CDKN1C, CDKN2A, CDKN2B, CDKN2C, CDKN3, CDW52, CEBPA, CEBPB, CEBPD, CFL1, CKMT1, CKS2, CML66, COL3A1, COL4A6, CR2, CREB1, CREBBP, CRYAB, CSF2, CSF3, CSRP2, CTGF, CTSB, CUZD1, CXADR, CXCL9, CXCR3, CXCR4, CYC1, CYP1A1, CYP2A6, DAD-1, DAPK1, DCK, DDX6, DEK, DHFR, DLAD, DNAJA1, DNMT3B, DNTT, DOK1, DPF2, DPP4, DRG1, DRP2, E2F1, EB-1, EBI2, EDF1, EEF1A1, EEF1B2, EEF1D, EEF1G, EFNB1, EGFR, EGR1, EIF2B2, EIF3S2, EIF4B, EIF4E, EIF5A, ELF1, ELF4, ENPP1, EphA3, EPOR, ERBB2, ERBB4, ERCC1, ERCC2, ERCC3, ERCC5, ERCC6, ETS1, ETS2, ETV6, ETV7, EZH2, FABP5, FADD, FAIM3, FAM38A, FARP1, FAT, FCER2, FCGR3A, FCGR3B, FGFR1, FGFR3, FGR, FHIT, FKBP9, FLI1, FLJ22169, FLT3, FN1, FNTB, FOS, FUS, G1P2, GABPB2, GATA1, GATA2, GATA3, GCET2, GDI2, GGA3, GJA1, GLUD1, GNL3, GOT1, GRB2, GRIA3, GRK4, GSTP1, GSTT1, GUSB, GZMA, H2AFX, H3F3A, HCK, HELLS, HIF1A, HIST1H2BN, HLA-A, HLA-DPA1, HLA-DQA1, HLA-DRA, HLA-DRB3, HLF, HMMR, HNRPH3, HNRPL, HOXA10, HOXA9, HOXD8, HOXD9, HRAS, HSD17B1, HSPB1, IBSP, ICAM1, ICAM3, ID2, IER3, IFRD1, IGFBP2, IGFBP3, IGFIR, IGLV6-57, IL10, IL15, IL1B, IL2, IL2RA, IL3, IL32, IL3RA, IL4R, IL6, IL6R, IL8, ILF2, IRF1, IRF2, IRF4, IRF8, ITGA2, ITGA3, ITGA4, ITGA5, ITGA6, ITGAL, ITGAM, ITGAX, ITGB1, ITGB2, JAK1, JAK2, JUNB, KAI1, KIAA0247, KIAA0864, KIT, KLF1, KLF13, KRAS2, KRT18, LADH, LAG3, LASP1, LCK, LCP1, LEPR, LGALS3, LGALS7, LIF, LIMS1, LM02, LOC285148, LRP, LSP1, LYL1, LYN, LYZ, MAFB, MAFK, MAGEA1, MAL, MAP3K12, MAP4K1, MAPK10, MAZ, MBP1, MCL1, MCM3, MCM7, MDM2, MEIS1, MEN1, MERTK, MKI67, MLF1, MLF2, MLL, MLLT10, MME, MMP2, MMP7, MMP8, MMP9, MNDA, MPL, MPO, MRPL37, MS4A1, MTCP1, MUC-1, MX1, MYB, MYBL1, MYC, MYOD1, NCALD, NCAM1, NCL, NDP52, NDRG1, NDUFA1, NDUFB, NF1, NFATC1, NFIC, NFKB1, NFKB1A, NINJ1, NPM1, NR3C1, NUMA1, NXF1, ODC1, OGGI, OLIG2, OPRD1, p14ARF, P55CDC, PABPC1, PAX5, PAX6, PAX8, PBX1, PBX3, PCA1, PCD, PCNA, PDCD1, PDGFA, PDGFRB, PDHA1, PGF, PGRMC1, PICALM, PLA2G6, PLAU, PLK1, PLP, PLS3, PLZF, PML, PMM1, POLR2C, POU2F2, PPP1CC, PRAME, PRKCI, PRKCQ, PRKDC, PRL, PRTN3, PSMA5, PSMB4, PSMC5, PSMD7, PTEN, PTGS1, PTHLH, PTK2, PTK2B, PTN, PTPRCCD, PYGB, RAD51, RAF1, RAG1, RARA, RARB, RB1, RBBP4, RBBP6, RBBP8, RBP4, RET, RGS1, RGS1, RIS1, RORA, RPL17, RPL23A, RPL24, RPL36A, RPL37A, RPL41, RPS3, RPS5, RPS9, RUNX1, RXRA, S100A2, S100A8, SDC1, SDHD, SELE, SELL, SEPW1, SERPINA9, SERPINB5, SERPNINA9, SFTPB, SIAT4A, SLC7A5, SNRPB, SOSTDC1, SP1, SPI1, SPN, SPRR1A, SREBF1, SSBP1, STAT1, STAT3, STAT5B, SUM01, TACSTD2, TAGLN2, TAL1, TBP, TCEB1, TCF1, TCF3, TCF7, TCL1A, TCRbeta, TEGT, TERF1, TERT, TFCP2, TFRC, THBS1, THPO, TIA-2, TIAM1, TK1, TLX1, TMEM4, TNF, TNFRSF10C, TNFRSF1A, TNFRSF25, TNFRSF5, TNFRSF6, TNFRSF8, TNFSF10, TNFSF5, TNFSF6, TOP2A, TOPORS, TP73, TRA@, TRADD, TRAF3, TRAP1, TRIB2, TXNRD1, UBE2C, UHRF1, UVRAG, VCAM1, VEGF, VPREB1, WBSCR20C, WNT16, WT1, XBP1, XP06, XRCC3, XRCC5, ZAP70, ZFPL1, ZNF42, ZNFN1A1, ZYX, 18S rRNA, 28S rRNA, and whose expression level is determined by the evaluation of the concentration of its corresponding mRNA by the use of at least one probe which has a sequence complementary to a fragment of a strand of said gene, a probe which is selected from the group of oligonucleotides composed of: SG1, SG2, SG3, SG4, SG5, SG6, SG7, SG8, SG9, SG10, SG11, SG12, SG13, SG14, SG15, SG16, SG17, SG18, SG19, SG20, SG21, SG22, SG23, SG24, SG25, SG26, SG27, SG28, SG29, SG30, SG31, SG32, SG33, SG34, SG35, SG36, SG37, SG38, SG39, SG40, SG41, SG42, SG43, SG44, SG45, SG46, SG47, SG48, SG49, SG50, SG51, SG52, SG53, SG54, SG55, SG56, SG57, SG58, SG59, SG60, SG61, SG62, SG63, SG64, SG65, SG66, SG67, SG68, SG69, SG70, SG71, SG72, SG73, SG74, SG75, SG76, SG77, SG78, SG79, SG80, SG81, SG82, SG83, SG84, SG85, SG86, SG87, SG88, SG89, SG90, SG91, SG92, SG93, SG94, SG95, SG96, SG97, SG98, SG99, SG100, SG101, SG102, SG103, SG104, SG105, SG106, SG107, SG108, SG109, SG110, SG111, SG112, SG113, SG114, SG115, SG116, SG117, SG118, SG119, SG120, SG121, SG122, SG123, SG124, SG125, SG126, SG127, SG128, SG129, SG130, SG131, SG132, SG133, SG134, SG135, SG136, SG137, SG138, SG139, SG140, SG141, SG142, SG143, SG144, SG145, SG146, SG147, SG148, SG149, SG150, SG151, SG152, SG153, SG154, SG155, SG156, SG157, SG158, SG159, SG160, SG161, SG162, SG163, SG164, SG165, SG166, SG167, SG168, SG169, SG170, SG171, SG172, SG173, SG174, SG175, SG176, SG177, SG178, SG179, SG180, SG181, SG182, SG183, SG184, SG185, SG186, SG187, SG188, SG189, SG190, SG191, SG192, SG193, SG194, SG195, SG196, SG197, SG198, SG199, SG200, SG201, SG202, SG203, SG204, SG205, SG206, SG207, SG208, SG209, SG210, SG211, SG212, SG213, SG214, SG215, SG216, SG217, SG218, SG219, SG220, SG221, SG222, SG223, SG224, SG225, SG226, SG227, SG228, SG229, SG230, SG231, SG232, SG233, SG234, SG235, SG236, SG237, SG238, SG239, SG240, SG241, SG242, SG243, SG244, SG245, SG246, SG247, SG248, SG249, SG250, SG251, SG252, SG253, SG254, SG255, SG256, SG257, SG258, SG259, SG260, SG261, SG262, SG263, SG264, SG265, SG266, SG267, SG268, SG269, SG270, SG271, SG272, SG273, SG274, SG275, SG276, SG277, SG278, SG279, SG280, SG281, SG282, SG283, SG284, SG285, SG286, SG287, SG288, SG289, SG290, SG291, SG292, SG293, SG294, SG295, SG296, SG297, SG298, SG299, SG300, SG301, SG302, SG303, SG304, SG305, SG306, SG307, SG308, SG309, SG310, SG311, SG312, SG313, SG314, SG315, SG316, SG317, SG318, SG319, SG320, SG321, SG322, SG323, SG324, SG325, SG326, SG327, SG328, SG329, SG330, SG331, SG332, SG333, SG334, SG335, SG336, SG337, SG338, SG339, SG340, SG341, SG342, SG343, SG344, SG345, SG346, SG347, SG348, SG349, SG350, SG351, SG352, SG353, SG354, SG355, SG356, SG357, SG358, SG359, SG360, SG361, SG362, SG363, SG364, SG365, SG366, SG367, SG368, SG369, SG370, SG371, SG372, SG373, SG374, SG375, SG376, SG377, SG378, SG379, SG380, SG381, SG382, SG383, SG384, SG385, SG386, SG387, SG388, SG389, SG390, SG391, SG392, SG393, SG394, SG395, SG396, SG397, SG398, SG399, SG400, SG401, SG402, SG403, SG404, SG405, SG406, SG407, SG408, SG409, SG410, SG411, SG412, SG413, SG414, SG415, SG416, SG417, SG418, SG419, SG420, SG421, SG422, SG423, SG424, SG425, SG426, SG427, SG428, SG429, SG430, SG431, SG432, SG433, SG434, SG435, SG436, SG437, SG438, SG439, SG440, SG441, SG442, SG443, SG444, SG445, SG446, SG447, SG448, SG449, SG450, SG451, SG452, SG453, SG454, SG455, SG456, SG457, SG458, SG459, SG460, SG461, SG462, SG465, SG468, SG469, SG470, SG471, SG472, SG473, SG474, SG475, SG476, SG477, SG478, SG479, SG480, SG481, SG482, SG483, SG484, SG485, SG486, SG487, SG488, SG489, SG490, SG491, SG492, SG493, SG494, SG495, SG496, SG497, SG498, SG499, SG500, SG501, SG502, SG503, SG504, SG505, SG506, SG507, SG508, SG509, SG510, SG511, SG512, SG513, SG514, SG515, SG516, SG517, SG518, SG519, SG520, SG521, SG522, SG523, SG524, SG525, SG526, SG527, SG428, SG529, SG530, SG531, SG532, SG533, SG534, SG535, SG536, SG537, SG538, SG539, SG540, SG541, SG542, SG543, SG544, SG545, SG546, SG547, SG548, SG549, SG550, SG551, SG552, SG553, SG554, SG555, SG556, SG557, SG558, SG559, SG560, SG561, SG562, SG563.

The genes which form part of the aforementioned group are human genes. Therefore, whenever the words "subject" or "individual" are used hereinafter, they will make reference to a human being.

A particular case of this method is that which comprises an additional previous step of identification of genes significant for the classification of the biological sample analysed as associated or not to a specific type of neoplasia originating from hematopoietic cells, a classification which includes not only the diagnosis of the existence of said neoplasia in the individual from which the sample has been taken, but which may also consist, in additional or alternative form, of the discrimination between specific subtypes of said neoplasia which correspond to different future forms of evolution of said neoplasia this constituting the classification of one or another subtype of the evolution of the neoplasia considered in the future. In this particular case of the method of the invention which comprises a previous step of identification of genes significant for making the desired classification, said previous step comprises the steps of:
a) deciding the possible categories wherein the sample can be classified;
b) obtaining biological samples from individuals which have previously been assigned by a method different to that claimed to any of the possible classification categories, so that there are samples of each one of the possible categories;
c) obtaining the total mRNA of each one of the samples;
d) obtaining the corresponding total cRNA, labelled by a method which allows its subsequent detection, of at least one aliquot of each one of the samples of mRNA, an aliquot whereto is added before the obtainment of the cRNA at least one sequence of polyadenylated nucleotides of low homology with human genes for which it acts as internal positive control of the process;
e) adding to one of the aliquots of cRNA which are going to be used in step f) at least one oligonucleotide of low homology with human genes different from and not complementary to any possible sequence of nucleotides which have been added in step d), for which it acts as positive hybridization control;
f) hybridizing, in strict conditions, at least one aliquot of total cRNA of each one of the samples with at least one microarray which comprises at least two copies of each one of the oligonucleotides from the group composed of:
SG1, SG2, SG3, SG4, SG5, SG6, SG7, SG8, SG9, SG10, SG11, SG12, SG13, SG14, SG15, SG16, SG17, SG18, SG19, SG20, SG21, SG22, SG23, SG24, SG25, SG26, SG27, SG28, SG29, SG30, SG31, SG32, SG33, SG34, SG35, SG36, SG37, SG38, SG39, SG40, SG41, SG42, SG43, SG44, SG45, SG46, SG47, SG48, SG49, SG50, SG51, SG52, SG53, SG54, SG55, SG56, SG57, SG58, SG59, SG60, SG61, SG62, SG63, SG64, SG65, SG66, SG67, SG68, SG69, SG70, SG71, SG72, SG73, SG74, SG75, SG76, SG77, SG78, SG79, SG80, SG81, SG82, SG83, SG84, SG85, SG86, SG87, SG88, SG89, SG90, SG91, SG92, SG93, SG94, SG95, SG96, SG97, SG98, SG99, SG100, SG101, SG102, SG103, SG104, SG105, SG106, SG107, SG108, SG109, SG110, SG111, SG112, SG113, SG114, SG115, SG116, SG117, SG118, SG119, SG120, SG121, SG122, SG123, SG124, SG125, SG126, SG127, SG128, SG129, SG130, SG131, SG132, SG133, SG134, SG135, SG136, SG137, SG138, SG139, SG140, SG141, SG142, SG143, SG144, SG145, SG146, SG147, SG148, SG149, SG150, SG151, SG152, SG153, SG154, SG155, SG156, SG157, SG158, SG159, SG160, SG161, SG162, SG163, SG164, SG165, SG166, SG167, SG168, SG169, SG170, SG171, SG172, SG173, SG174, SG175, SG176, SG177, SG178, SG179, SG180, SG181, SG182, SG183, SG184, SG185, SG186, SG187, SG188, SG189, SG190, SG191, SG192, SG193, SG194, SG195, SG196, SG197, SG198, SG199, SG200, SG201, SG202, SG203, SG204, SG205, SG206, SG207, SG208, SG209, SG210, SG211, SG212, SG213, SG214, SG215, SG216, SG217, SG218, SG219, SG220, SG221, SG222, SG223, SG224, SG225, SG226, SG227, SG228, SG229, SG230, SG231, SG232, SG233, SG234, SG235, SG236, SG237, SG238, SG239, SG240, SG241, SG242, SG243, SG244, SG245, SG246, SG247, SG248, SG249, SG250, SG251, SG252, SG253, SG254, SG255, SG256, SG257, SG258, SG259, SG260, SG261, SG262, SG263, SG264, SG265, SG266, SG267, SG268, SG269, SG270, SG271, SG272, SG273, SG274, SG275, SG276, SG277, SG278, SG279, SG280, SG281, SG282, SG283, SG284, SG285, SG286, SG287, SG288, SG289, SG290, SG291, SG292, SG293, SG294, SG295, SG296, SG297, SG298, SG299, SG300, SG301, SG302, SG303, SG304, SG305, SG306, SG307, SG308, SG309, SG310, SG311, SG312, SG313, SG314, SG315, SG316, SG317, SG318, SG319, SG320, SG321, SG322, SG323, SG324, SG325, SG326, SG327, SG328, SG329, SG330, SG331, SG332, SG333, SG334, SG335, SG336, SG337, SG338, SG339, SG340, SG341, SG342, SG343, SG344, SG345, SG346, SG347, SG348, SG349, SG350, SG351, SG352, SG353, SG354, SG355, SG356, SG357, SG358, SG359, SG360, SG361, SG362, SG363, SG364, SG365, SG366, SG367, SG368, SG369, SG370, SG371, SG372, SG373, SG374, SG375, SG376, SG377, SG378, SG379, SG380, SG381, SG382, SG383, SG384, SG385, SG386, SG387, SG388, SG389, SG390, SG391, SG392, SG393, SG394, SG395, SG396, SG397, SG398, SG399, SG400, SG401, SG402, SG403, SG404, SG405, SG406, SG407, SG408, SG409, SG410, SG411, SG412, SG413, SG414, SG415, SG416, SG417, SG418, SG419, SG420, SG421, SG422, SG423, SG424, SG425, SG426, SG427, SG428, SG429, SG430, SG431, SG432, SG433, SG434, SG435, SG436, SG437, SG438, SG439, SG440, SG441, SG442, SG443, SG444, SG445, SG446, SG447, SG448, SG449, SG450, SG451, SG452, SG453, SG454, SG455, SG456, SG457, SG458, SG459, SG460, SG461, SG462, SG465, SG468, SG469, SG470, SG471, SG472, SG473, SG474, SG475, SG476, SG477, SG478, SG479, SG480, SG481, SG482, SG483, SG484, SG485, SG486, SG487, SG488, SG489, SG490, SG491, SG492, SG493, SG494, SG495, SG496, SG497, SG498, SG499, SG500, SG501, SG502, SG503, SG504, SG505, SG506, SG507, SG508, SG509, SG510, SG511, SG512, SG513, SG514, SG515, SG516, SG517, SG518, SG519, SG520, SG521, SG522, SG523, SG524, SG525, SG526, SG527, SG428, SG529, SG530, SG531, SG532, SG533, SG534, SG535, SG536, SG537, SG538, SG539, SG540, SG541, SG542, SG543, SG544, SG545, SG546, SG547, SG548, SG549, SG550, SG551, SG552, SG553, SG554, SG555, SG556, SG557, SG558, SG559, SG560, SG561, SG562, SG563, a microarray which additionally comprises:
a. at least two points which correspond to different aliquots of the solvent wherein nucleotides are found at the time of their deposit on the surface of the microarray, for which they serve as blank,
b. at least two copies of at least one oligonucleotide for each one of the polyadenylated sequences added in step d), an oligonucleotide whose sequence will correspond to a fragment, different from the polyadenylation zone, of the sequence of polyadenylated nucleotides whose evolution in the process has to be controlled;
c. for each one of the oligonucleotides added in step e), at least two copies of an oligonucleotide complementary thereto;
d. at least two copies of each member of at least one pair of oligonucleotides wherein the sequence of one of the members corresponds to a sequence of zone 5' and the sequence of the other corresponds to a sequence of zone 3' of the mRNA of a gene which is expressed in constitutive form in any cell of hematopoietic origin;
e. at least two copies of at least one oligonucleotide of low homology with human genes different from any of the oligonucleotides defined in section b. and different from any of the synthetic oligonucleotides added optionally in step e);
g) detecting and quantifying the signal of cRNA hybridized with each one of the copies of each one of the oligonucleotides present in the microarray, as well as the signal corresponding to the points of the solvent;
h) calculating the average level of intensity of hybridization of each one of the oligonucleotides of the microarray calculating the average of the intensities of the copies of each one of the oligonucleotides;
i) taking the hybridization as valid if the following conditions are complied with:
   a. the ratio between the average intensity and the average background of all the oligonucleotides of the microarray is greater than 10;
   b. the value of the average coefficient of variation of all the replicas of oligonucleotides should be less than 0.3;
   c. the average value of negative control should be less than 2.5 times the average value of the points corresponding to the solvent;
   d. there is a signal both in the hybridization controls and in the internal positive controls used as process control;
j) normalizing the data;
k) eliminating the oligonucleotides with values of average intensity minus average background noise less than approximately 2 times the average value obtained with the points corresponding to the solvent, as well as the oligonucleotides with an interquartile range of normalized intensity throughout the samples less than 0.3;
l) performing the statistical analysis to find the statistically significant oligonucleotides to differentiate between the different categories and be able to classify a sample which has not been previously assigned to any category, choosing said oligonucleotides among those which have not been eliminated in the previous steps, until obtaining "n" oligonucleotides which either have a value of p less than a limit which is chosen from the open range of 0 to 0.05, preferably using for it a method with capacity to reduce false positives, or that which best defines the category established;
m) checking that the grouping of the samples according to the differences in intensities between the different samples detected for the statistically significant oligonucleotides gives rise to the samples being classified in the same categories as those which had previously been assigned by a different method.

It is preferred that the average value calculated in section h) is the trimmed mean, for which reason it is preferable that the microarray comprises at least four copies of each one of the oligonucleotides present therein.

The normalization can be carried out with different methods. There is preference for the use of functions contained in access packages freely accessed over the Internet designed for the processing, calculation and graphic representation of data, such as the packages designed in R programming language, available to download from CRAN (http://cran.r-project.org/) or Bioconductor (http://www.bioconductor.org). The "variance stabilization normalization" method available in the "vsn" package in R.

The identification of the statistically significant oligonucleotides to differentiate between the different categories can be carried out using different methods, having preference for those wherein a value p is determined that determines the threshold of probability under which all the genes whose expression difference has a value less than p would be considered significant and, among these, those which have the capacity to carry out corrections on the value of p, such as, among others, Bonferroni's method or Welch's test. The value ofp will be chosen from the open range of 0 to 0.05, preferring, when possible, a value of p close to 0.001 and with correction, it being possible to increase said value at maximum to 0.05 (value which is not included among those possible) until which statistically significant oligonucleotides are found to differentiate between the categories among which one wants to classify the samples. A possibility for carrying out these calculations is, again, the use of functions contained in packages freely accessed over the Internet designed for the processing, calculation and graphic representation of data. In particular, the mt.maxT function of the multtest package in R can be used for the identification of the statistically significant oligonucleotides.

Another possibility for the identification of statistically significant oligonucleotides to be able to differentiate between the categories of established samples is the use of the "nearest shrunken centroids" method, a variation of the "nearest centroids" method (Tibshirani *et al.,* 2002), which identifies a group of genes which best characterizes a predefined class and uses this group of genes to predict the class which new samples belong to. To do this, again functions contained in packages freely accessed over the internet may be resorted to, such as the "pam^{a}" package in R, wherein it is possible to find functions to carry out the so-called "Prediction Analysis for Microarrays (PAM)", which makes use of the "nearest shrunken centroids" method.

After identifying the statistically significant genes to distinguish between categories of samples established from the corresponding oligonucleotides, they can be used for classifying new samples due to similarity between the expression profile of those genes in the sample analysed and those corresponding to each one of the classification categories. Alternatively, when there are only 2 possible classification categories (which will be normal when one wants to diagnose the presence or absence of a certain type of leukaemia in an individual), it is possible to obtain a mathematical function of classification of samples which determine the probability "pᵢ" of a sample "i" belonging to one category or another. To do this, a subunit of the samples is chosen which have been previously assigned to each one of the possible categories by a method different to that of the invention and the value of 0 is arbitrarily associated to each one of the samples of one of the categories "a" (typically, the category of "not" associated to the leukemia one wants to diagnose") of belonging to the other possible category, whilst each one of the samples of the subunit belonging to the other possible category "b" (typically, the category of "associated" to the leukemia one wants to diagnose") arbitrarily receives the value "1" for its probability of belonging to its own category. With this, logistical regression is used to calculate, with the aid of the probability values assigned to each one of the samples and the values of normalized trimmed mean intensity obtained for each one of the samples with each one of the "n" oligonucleotides which has been identified as a statistically significant oligonucleotide in the previous step, coefficients for each one of said oligonucleotides which make it possible to obtain a function of probability pᵢ of a sample "i" belonging to category "b", a function which will be of the type ${p}_{i} = 1 / \left(1+{e}^{xi}\right)$ and which results from the algebraic transformation of the expression which equals Neperian logarithm of the quotient between the probability of an event occurring and the probability that it does not occur at a function xᵢ which includes as variables each one of the factors which may influence the event, i.e. $ln ⁢ \frac{p}{1 - p} = {x}_{i}$ function xᵢ which, in the present case, will depend on the intensity values obtained for each one of the statistically significant oligonucleotides and which responds to an expression of the type: ${x}_{i} = constant + {\sum }_{m = 1}^{n} \left(coeff_{oliga}_{m}*{Imn}_{i_}⁢{olig}_{m}\right)$
where
coeff_oligₘ represents the coefficient calculated for a specific oligonucleotide "m"
Imnᵢ_oligₘ represents the average value of normalized intensity obtained in the hybridization of the sample i calculated for the oligonucleotide "m"
"m" varies from 1 to "n"
n is the total number of oligonucleotides considered significant.

The function pᵢ obtained after calculating by logistical regression the coefficient corresponding to each oligonucleotide permits classifying a sample "i" as belonging to one or another category, considering that the values of pᵢ over 0.5 (and which will be less than or equal to 0) indicate that the sample belongs to category "b", whilst the values of pᵢ less than 0.5 indicate that the sample belongs to category "a". Said function pᵢ will be considered valid if, on being applied to the samples wherefrom it has been deduced, it is capable of classifying them correctly and, furthermore, as it is applied to the subgroup of samples which have not been taken into account to deduce the function, but whose category is known as it has been previously assigned by a method other than that of the invention, it is also capable of classifying them correctly.

Alternatively, when the identification of the statistically significant genes has been performed using the "Prediction Analysis for Microarrays" method, the classifier can be obtained with the corresponding functions of the "pamr^{a}" package in R, which also starts from the assignment of the value of probability 0 to a subgroup of members of one of the categories and the value of probability 1 to a subgroup of the members of the other category. Again, the calculation of coefficients for statistically significant oligonucleotides permits the calculation of values of probability of belonging to one or another category, also considering that the values over 0.5 indicate belonging to the category whose members are arbitrarily assigned value 1 and the values less than 0.5 indicate belonging to the other category.

A particular case of the method of the invention is that wherein one wants to classify samples as associated or not to a type of leukemia. In that case, blood samples are preferred, especially those of peripheral blood, as biological samples to carry out *in vitro* the method of the invention.

Once the statistically significant genes have been identified to associate a determined type of neoplasia as originating from hematopoietic cells, the method of the invention can be used for classifying samples according to the expression level of said genes in said samples. The neoplasia can be, for example, a specific type of leukemia. A particular case of this embodiment of the method of the invention is constituted by the association of chronic lymphatic leukemia, thus allowing the diagnosis of this disease by the method of the invention. To do this, significant genes are considered to be those genes whose expression level is analysed on applying the method of the invention at least those of the group of CD79A, FAIM3, HLA-DRA, HLA-DRB3, HLA-DQA1 and the analysis is carried out on blood samples. The method can be additionally applied including the analysis of the expression level of at least genes IRF8 and COL3A1. Preferably, the analysis of the expression level of these genes is carried out by evaluating the level of their corresponding mRNA by hybridization of their corresponding cRNA with oligonucleotides SG117, SG428, SG459, SG507, SG508, SG461 and SG493, which are preferred to be associated to a solid support forming part of a microarray. When the evaluation of the hybridized cRNA with each one of those oligonucleotides is carried out thanks to the prior labelling of the cRNA with biotin, the staining of the microarray hybridized with streptavidin conjugated with a fluorophore and the detection of the signal emitted by said fluorophore, it is preferred that the fluorophore is Cy3, which permits diagnosing the presence of CLL in the subject from which the sample has been taken by the classification of sample "i" analysed as associated to CLL from the calculation of the probability that said sample is associated to CLL from the formula pᵢ = 1/(1 +e^{-xᵢ}), wherein xᵢ is calculated by the formula ${x}_{i} = - 719.241486 + ( 2.44756372 * {Imn}_{i} _CD ⁢ 79 ⁢ A ) + ( 7.38657611 * { Imn}_{i} _FAIM ⁢ 3 ) + ( 23.1465464 * { Imn}_{i} _HLA - DRA ) + ( 43.6287742 * { Imn}_{i} _IRF ⁢ 8 ) - ( 19.3978182 * { Imn}_{i} _COL ⁢ 3 ⁢ A ⁢ 1 ) - ( 2.8028646 * {Imn}_{i} _HLA - DRB ⁢ 3 ) + \left(49.5345672*{ Imn}_{i}_HLA-DQA⁢1\right)$ formula wherein each one of the values denominated with the abbreviation "Inmᵢ" followed by the abbreviation of a gene makes reference to the average value of normalized intensity obtained after detecting the hybridization signal corresponding to the oligonucleotide which is being used as probe to evaluate the expression of the said gene
and which permits classifying the subject as subject not suffering from CLL if the value of pᵢ is less than 0.5 and as subject suffering from CLL if the value of pᵢ is greater than 0.5.
Alternatively, significant genes can be considered as those whose expression level is analysed on applying the method of the invention for the diagnosis of CLL at least those of the group of CD79A, FAIM3, HLA-DRA, HLA-DRB3, HLA-DQA1, additionally including the analysis of the expression level of at least gene CDW52. Preferably, the analysis of the expression level of these genes is carried out by evaluating the level of its corresponding mRNA by hybridization of its corresponding cRNA with oligonucleotides SG117, SG428, SG459, SG507, SG508 and SG237, which it is preferred are associated to a solid support forming part of a microarray.

Another particular case of the application of the method of the invention for classifying samples as associated to a specific type of leukemia according to the expression level in said samples of statistically significant genes constitutes the classification of a sample as associated to a specific subtype of chronic lymphatic leukemia, "stable" CLL or "progressive" CLL, which makes it possible that the method of the invention serves to make a prognosis for the future evolution of subjects which have been diagnosed with CLL. When the samples analysed are of peripheral blood, the genes considered statistically significant to perform the classification of the samples are at least genes PSMB4, FCER2 and POU2F2, it being possible to additionally analyse the expression level of at least one gene selecting the group composed of ODC1, CD79A, CD2, CD3E, CD5, MS4A1, EIF4E, FHIT, NR3C1, LCP1, MAPK10, ABCC5, XRCC3, CML66, PLZF, RBP4 or the totality thereof.

An additional aspect of the invention is the use of devices to evaluate the expression level of at least one of the genes of the group composed of PSMB4, FCER2, POU2F2, ODC1, CD79A, CD2, CD3E, CD5, MS4A1, EIF4E, FHIT, NR3C1, LCP1, MAPK10, ABCC5, XRCC3, CML66, PLZF, RBP4, CD79A, FAIM3, HLA-DRA, HLA-DRB3, HLA-DQA1, IRF8 and COL3A1 with the aim of diagnosing the presence of CLL in an individual and/or making a prognosis of his/her evolution. A particular case of this aspect of the invention is the use of devices of evaluation of the expression level of at least one gene of the group composed of CD79A, FAIM3, HLA-DRA, HLA-DRB3, HLA-DQA1, IRF8 and COL3A1 for the diagnosis of the presence of CLL in an individual, wherein it is preferred that the device evaluates at least the expression level of genes CD79A, FAIM3, HLA-DRA, HLA-DRB3, HLA-DQA1, it being possible for the device to evaluate, additionally, the expression level of at least genes IRF8 and COL3A1 or at least gene CDW52. Another particular case of this aspect of the invention is the use of devices of evaluation of the expression level of at least one gene of the group composed of PSMB4, FCER2, POU2F2, ODC1, CD79A, CD2, CD3E, CD5, MS4A1, EIF4E, FHIT, NR3C1, LCP1, MAPK10, ABCC5, XRCC3, CML66, PLZF, RBP4, CD79A, FAIM3, HLA-DRA, HLA-DRB3, HLA-DQA1, IRF8 and COL3A1 to make a prognosis of the future evolution of CLL in an individual.

### Detailed description of the invention: Design of the microarray device

### Genes included in the microarray

A revision was performed of the scientific literature and genes were selected due to their special involvement in the biology of blood cells or in the pathology of the different neoplasias The genes selected can be included within these 4 large groups:
a) With an important role in the biology of the hematopoietic cells:
   - Genes whose protein is expressed or repressed in the different steps through which these cells pass in their differentiation to mature forms.
   - Genes whose protein is specifically expressed in accordance with the line whereto the cell belongs.
   - Genes which code adhesion molecules
b) Involved in different types of hematological neoplasias:
   - Genes whose expression (a level of MRNA or protein) is altered in different types of neoplasias, or associated to resistance to chemotherapy
c) Cancer-related:
   - Genes which code proteins associated with proliferation, metastasis or genes whose expression is increased in a large number of tumours.
d) Described in publications related to neoplasias:
   - Genes which, without having a special ratio with hematological neoplasias or blood cell biology, have appeared in the scientific literature as statistically associated to a type of neoplasia

The characteristics of the genes can be consulted, for example, in: www.ncbi.nlm.nih.gov/Genbank, selecting the "Gene" option in the drop-down menu which appears and entering the corresponding identification number (GenID) in the GenBank. The genes whose expression can be analysed with the microarray, their corresponding identification number in the GenBank, as well as the oligonucleotides present in the microarray to be used as probes to analyse the expression of said genes appear below in Table 1.

### Probes of oligonucleotides which represent each gene.

For each one of the 534 genes related to hematological neoplasias, as well as for the genes corresponding to β-actin, glyceraldehyde-3-phosphate dehydrogenase, 18S rRNA and 28S rRNA, the mrRNA sequence is sought in GenBank (www.ncbi.hlm.nih.gov/Genbank/). An oligonucleotide is designed (probe) from the GenBank sequence, specific for each one of the genes selected. In some genes several oligonucleotides were designed situated in zones 5' and 3' of the gene, in order to analyse the integrity of the mRNA.

To ensure specificity in the design of the probes, the following criteria were taken into consideration:
- Length of the probe to guarantee that all the probes are going to have a similar behaviour,
- GC content of the probe between 40 and 60%. This characteristic is also taken into consideration to ensure that all the probes are going to have a similar behaviour.
- Localization in the gene. Probes localized at least 3000 nucleotides from 3' (poly(A)) of the selected mRNA sequence were localized.
- Sense of the probe. A strand was chosen with "sense", i.e. the sequences of the oligonucleotides coincide with sequences of fragments of the corresponding mRNA, instead of being sequences complementary to said fragments. This decision involves that the labelled genetic material has to be antisense (complementary to sense).
- Specificity of the probe. To avoid non-specific hybridization, probes were selected which have a percentage of homology, calculated by the BLAST tool (available on the website http://www.ncbi.nlm.nih.gov/), less than 70%.

The data on the oligonucleotides used as probes, the identification number of its corresponding sequence in the attached list, as well as data (identification number in GenBank, usual abbreviation and name) of the genes for the detection of whose expression said oligonucleotides have been designed, are shown below in the Table 1. **Table 1.- Oligonucleotides used as probes to detect the expression of human genes**

| **Oligonucleotide** | **SEQ ID NO:** | **GenID** | **Usual abbreviation** | **Description** |
|---|---|---|---|---|
| SG1 | SEQ ID NO:1 | 11337 | GABARAP | Protein associated to the GABA receptor |
| SG2 | SEQ ID NO:2 | 28778 | IGLV6-57 | Variable lambda immunoglobulin 6-57 |
| SG3 | SEQ ID NO:3 | 5092 | PCD | 6-pyruvoyl-tetrahydropterine synthase/dimerization cofactor of the nuclear factor of 1 alpha hepatocytes (TCF1) |
| SG4 | SEQ ID NO:4 | 83988 | NCALD | delta neurocalcin |
| SG5 | SEQ ID NO:5 | 58511 | DLAD | deoxyribonuclease II beta |
| SG6 | SEQ ID NO:6 | 25928 | SOSTDC1 | which contains a sclerostin 1 domain |
| SG7 | SEQ ID NO:7 | 10630 | TIA-2 | glycoprotein associated to the lung cell membrane, type I |
| SG8 | SEQ ID NO:8 | 5834 | PYGB | phosphorylase, glycogen; brain |
| SG9 | SEQ ID NO:9 | 3987 | LIMS1 | with domains LIM and similar to the antigen of senescent cells 1 |
| SG10 | SEQ ID NO:10 | 25 | ABL1 | homologue to the viral oncogene of Abelson v-abl 1 murine leukemia |
| SG11 | SEQ ID NO:11 | 4733 | DRG1 | GTP-binding protein regulated by development 1 |
| SG12 | SEQ ID NO:12 | 25855 | BRMS1 | Metastasis suppressor of breast cancer 1 |
| SG13 | SEQ ID NO:13 | 84696 | ABHD1 | which contains an abhydrolase 1 domain |
| SG14 | SEQ ID NO:14 | 3475 | IFRD1 | Development regulator related to interferon 1 |
| SG15 | SEQ ID NO:15 | 6173 | RPL36A | Ribosomal protein L36a |
| SG16 | SEQ ID NO:16 | 3485 | IGFBP2 | Binding protein to the growth factor similar to insulin 2, 36kDa |
| SG17 | SEQ ID NO:17 | 10397 | NDRG1 | Gene regulated downstream by N-myc 1 |
| SG18 | SEQ ID NO:18 | 11328 | FKBP9 | FK506 9-binding protein, 63 kDa |
| SG19 | SEQ ID NO:19 | 10241 | NDP52 | protein of the nuclear domain 10 |
| SG20 | SEQ ID NO:20 | 2171 | FABP5 | protein which binds to fatty acids 5 (associated to psoriasis) |
| SG21 | SEQ ID NO:21 | 10160 | FARP1 | protein with FERM, RhoGEF (ARHGEF) and pleckstrin 1 domains (derived from chrondrocytes) |
| SG22 | SEQ ID NO:22 | 5228 | PGF | Placental growth factor, protein related to the endothelial growth factor |
| SG23 | SEQ ID NO:23 | 2665 | GDI2 | GDP 2 dissociation inhibitor |
| SG24 | SEQ ID NO:24 | 8407 | TAGLN2 | transgelin 2 |
| SG25 | SEQ ID NO:25 | 645 | BLVRB | biliverdin reductase B (flavin reductase (NADPH)) |
| SG26 | SEQ ID NO:26 | 5692 | PSMB4 | subunit of proteasome (prosome, macropain), beta-type, 4 |
| SG27 | SEQ ID NO:27 | 4070 | TACSTD2 | transducer of the calcium signal associated to tumours 2 |
| SG28 | SEQ ID NO:28 | 6921 | TCEB1 | Elongation factor of transcription B (SIII), polypeptide 1 (15kDa, elongin C) |
| SG29 | SEQ ID NO:29 | 1915 | EEF1A1 | elongation factor in the eukaryotic translation 1 alpha 1 |
| SG30 | SEQ ID NO:30 | 3020 | H3F3A | histone H3, family 3A |
| SG31 | SEQ ID NO:31 | 4953 | ODC1 | ornithine decarboxylase 1 |
| SG32 | SEQ ID NO:32 | 7520 | XRCC5 | Of repair of X rays which complement the defective repair in Chinese hamster cells 5 (reconnection of breakages in the double helix; autoantigen Ku, 80kDa) |
| SG33 | SEQ ID NO:33 | 3486 | IGFBP3 | binding protein to growth factor similar to insulin 3 |
| SG34 | SEQ ID NO:34 | 4691 | NCL | nucleolin |
| SG35 | SEQ ID NO:35 | 6273 | S100A2 | calcium S100 A2- binding protein |
| SG36 | SEQ ID NO:36 | 6152 | RPL24 | ribosomal protein L24 |
| SG37 | SEQ ID NO:37 | 2697 | GJA1 | Bone filling protein, alpha 1, 43kDa (connexin 43) |
| SG38 | SEQ ID NO:38 | 2990 | GUSB | glucuronidase, beta |
| SG39 | SEQ ID NO:39 | 3292 | HSD17B1 | hydroxysteroid (17-beta) dehydrogenase 1 |
| SG40 | SEQ ID NO:40 | 6439 | SFTPB | surfactant protein, associated to lung B |
| SG41 | SEQ ID NO:41 | 6147 | RPL23A | ribosomal protein L23a |
| SG42 | SEQ ID NO:42 | 1466 | CSRP2 | protein rich in cysteine and glycine 2 |
| SG43 | SEQ ID NO:43 | 1525 | CXADR | receptor of the coxsackie virus and adenovirus |
| SG44 | SEQ ID NO:44 | 1937 | EEF1G | elongation factor of eukartyotic 1 gamma elongation |
| SG45 | SEQ ID NO:45 | 1164 | CKS2 | subunit regulating the kinase CDC28 2 protein |
| SG46 | SEQ ID NO:46 | 1288 | COL4A6 | collagen, type IV, alpha 6 |
| SG47 | SEQ ID NO:47 | 1410 | CRYAB | crystalline, alpha B |
| SG48 | SEQ ID NO:48 | 1537 | CYC1 | cytochrome c-1 |
| SG49 | SEQ ID NO:49 | 2342 | FNTB | farnesyltransferase, CAAX box, beta |
| SG50 | SEQ ID NO:50 | 2805 | GOT1 | glutamic-oxaloacetic transaminase 1, soluble (aminotransferase 1 aspartate) |
| SG51 | SEQ ID NO:51 | 3963 | LGALS7 | Lectin, which binds to galactosides, soluble, 7 (galectin 7) |
| SG52 | SEQ ID NO:52 | 5268 | SERPINB5 | Serine (or cysteine) inhibitor proteinase, clade B (ovalbumin, member 5 |
| SG53 | SEQ ID NO:53 | 5705 | PSMC5 | Subunit of proteasome (prosome, macropain) 26S, ATPase, 5 |
| SG54 | SEQ ID NO:54 | 5764 | PTN | pleiotrophin (growth factor of bonding to heparin 8, growth promoter factor of neurites 1) |
| SG55 | SEQ ID NO:55 | 5932 | RBBP8 | Retinoblastoma 8-binding protein |
| SG56 | SEQ ID NO:56 | 5996 | RGS1 | Regulator of the signalling by proteins G 1 |
| SG57 | SEQ ID NO:57 | 6256 | RXRA | Retinoid X receptor, alpha |
| SG58 | SEQ ID NO:58 | 6392 | SDHD | succinate dehydrogenase complex, subunit D, integral membrane protein |
| SG59 | SEQ ID NO:59 | 6415 | SEPW1 | selenoprotein W, 1 |
| SG60 | SEQ ID NO:60 | 6742 | SSBP1 | binding protein to single-strand DNA 1 |
| SG61 | SEQ ID NO:61 | 7009 | TEGT | transcript of gene increased in the testicle (BAX 1 inhibitor) |
| SG62 | SEQ ID NO:62 | 8341 | HIST1H2BN | histone 1, H2bn |
| SG63 | SEQ ID NO:63 | 8678 | BECN1 | beclin 1 (protein similar to myosin which interacts with BCL2, of twisted helix) |
| SG64 | SEQ ID NO:64 | 310 | ANXA7 | annexin A7 |
| SG65 | SEQ ID NO:65 | 4694 | NDUFA1 | Alpha subcomplex of NADH dehydrogenase (ubiquinone) 1, 1, |
| SG66 | SEQ ID NO:66 | 9181 | ARHGEF2 | guanine rho/rac exchange factor (GEF) 2 |
| SG67 | SEQ ID NO:67 | 9315 | C5orfl3 | Open reading frame 13 of chromosome 5 |
| SG68 | SEQ ID NO:68 | 7494 | XBP1 | X 1 box-binding protein |
| SG69 | SEQ ID NO:69 | 9636 | G1P2 | protein inducible by the alpha interferon (clone IFI-15K) |
| SG70 | SEQ ID NO:70 | 2746 | GLUD1 | glutamate dehydrogenase 1 |
| SG71 | SEQ ID NO:71 | 6273 | S100A2 | calcium S100 A2-binding protein |
| SG72 | SEQ ID NO:72 | 3927 | LASP1 | LIM and SH3 1 protein |
| SG73 | SEQ ID NO:73 | 10630 | TIA-2 | glycoprotein associated to the lung cell membrane type I |
| SG74 | SEQ ID NO:74 | 10857 | PGRMC1 | Membrane component of the progesterone 1 receptor |
| SG75 | SEQ ID NO:75 | 7542 | ZFPL1 | protein similar to that of zinc finger 1 |
| SG76 | SEQ ID NO:76 | 11184 | MAP4K1 | Kinase protein activated by mitogens 1 |
| SG77 | SEQ ID NO:77 | 6772 | STAT1 | Signal transducer and transcription activator 1, 91kDa |
| SG78 | SEQ ID NO:78 | 3189 | HNRPH3 | Heterogeneous nuclear ribonucleoprotein H3 (2H9) |
| SG79 | SEQ ID NO:79 | 10330 | TMEM4 | Transmembrane protein 4 |
| SG80 | SEQ ID NO:80 | 9766 | KIAA0247 | KIAA0247 |
| SG81 | SEQ ID NO: 81 | 25907 | RIS1 | of senescence induced by Ras 1 |
| SG82 | SEQ ID NO:82 | 51593 | ARS2 | Arsenate-resistant protein ARS2 |
| SG83 | SEQ ID NO:83 | 771 | CA12 | Carbonic anhydrase XII |
| SG84 | SEQ ID NO:84 | 1933 | EEF1B2 | elongation factor of the eukaryotic 1 beta 2 translation |
| SG85 | SEQ ID NO:85 | 28951 | TRIB2 | homologue to tribbles 2 (Drosophila) |
| SG86 | SEQ ID NO:86 | 79065 | FLJ22169 | similar to that of autophagy 9 APG9 1 (S. cerevisiae) |
| SG87 | SEQ ID NO:87 | 440 | ASNS | asparagine synthetase |
| SG88 | SEQ ID NO:88 | 260294 | WBSCR20C | Chromosome region 20C of Williams Beuren syndrome |
| SG89 | SEQ ID NO:89 | 10327 | AKR1A1 | member A1 of the aldo-keto reductase 1 family (aldehyde reductase) |
| SG90 | SEQ ID NO:90 | 6698 | SPRR1A | Small proline lA-rich protein |
| SG91 | SEQ ID NO:91 | 1947 | EFNB1 | ephrin-B1 |
| SG92 | SEQ ID NO:92 | 6193 | RPS5 | ribosomal protein S5 |
| SG93 | SEQ ID NO:93 | 6203 | RPS9 | ribosomal protein S9 |
| SG94 | SEQ ID NO:94 | 6139 | RPL17 | ribosomal protein L17 |
| SG95 | SEQ ID NO:95 | 2114 | ETS2 | homologue to oncogene E26 of the erythroblastosis virus v-ets 2 (avian) |
| SG96 | SEQ ID NO:96 | 1975 | EIF4B | initiation factor of the eukaryotic translation 4B |
| SG97 | SEQ ID NO:97 | 7791 | ZYX | Zyxin |
| SG98 | SEQ ID NO:98 | 23214 | XPO6 | exportin 6 |
| SG99 | SEQ ID NO:99 | 285148 | LOC285148 | Hypothetical protein LOC285148 |
| SG100 | SEQ ID NO: 100 | 8209 | C21orf33 | open reading frame 33 of chromosome 21 |
| SG101 | SEQ ID NO:101 | 1936 | EEF1D | elongation factor of the eukaryotic translation 1 delta (guanine nucleotide exchange protein) |
| SG 102 | SEQIDNO:102 | 26986 | PABPC1 | poly(A)-binding protein, cytoplasmic1 |
| SG103 | SEQ ID NO: 103 | 5930 | RBBP6 | retinoblastoma 6-binding protein |
| SG104 | SEQ ID NO: 104 | 3265 | HRAS | homologue to the viral oncogene of the Harvey v-Ha-ras rat sarcoma |
| SG105 | SEQ ID NO:105 | 23163 | GGA3 | ARF-binding protein, which contains gamma-adaptin, associated to golgi |
| SG106 | SEQ ID NO: 106 | 1072 | CFL1 | cophilin 1 (non-muscular) |
| SG107 | SEQ ID NO: 107 | 8668 | EIF3S2 | initiation factor of the eukaryotic translation 3, subunit 2 beta, 36kDa |
| SG108 | SEQ ID NO:108 | 3875 | KRT18 | keratine 18 |
| SG109 | SEQ ID NO:109 | 3480 | IGFIR | growth factor receptor similar to insulin 1 |
| SG110 | SEQ ID NO:110 | 3576 | IL8 | Interleukin 8 |
| SG111 | SEQ ID NO:111 | 3659 | IRF1 | Interferon regulating factor 1 |
| SG112 | SEQ ID NO: 112 | 3660 | IRF2 | Interferon regulating factor 2 |
| SG113 | SEQ ID NO: 113 | 4067 | LYN | Homologue to the oncogene related to the viral sarcoma of Yamaguchi V-yes-1 |
| SG114 | SEQ ID NO: 114 | 4069 | LYZ | Lysozyme (renal amiloidosis) |
| SG115 | SEQ ID NO:115 | 4792 | NFKB1A | Nuclear factor of the gene enhancer of the kappa light polypeptide in B L-lymphocytes (p105) |
| SG116 | SEQ ID NO: 116 | 4150 | MAZ | Zinc finger protein associated to MYC (transcription factor of binding to purins) |
| SG117 | SEQ ID NO: 117 | 973 | CD79A | Antigen CD79A (associated to alpha immunoglobulins) |
| SG118 | SEQ ID NO:118 | 4172 | MCM3 | Deficient maintenance of minichromosomes (S. cerevisiae) 3 |
| SG119 | SEQ ID NO:119 | 421 | MEIS1 | Homologue to Meis1 (mouse) |
| SG120 | SEQ ID NO:120 | 5657 | PRTN3 | Proteinase 3 (serine proteinase, autoantigen of Wegener's granulomatosis of neutrophils) |
| SG121 | SEQ ID NO:121 | 4313 | MMP2 | Metalloproteinase of matrix 2 (gelatinase A, 72kD gelatinase of, 72kD collagenase type IV) |
| SG122 | SEQ ID NO:122 | 4316 | MMP7 | Metalloproteinase of matrix 7 (matrilysin, uterine) |
| SG123 | SEQ ID NO:123 | 4317 | MMP8 | Metalloproteinase of matrix 8 (neutrophil collagenase) |
| SG124 | SEQ ID NO:124 | 1796 | DOK1 | Adapter protein 1, 62kD (downstream respect to thyrosine kinase 1) |
| SG125 | SEQ ID NO:125 | 5154 | PDGFA | Alpha polypeptide of the platelet- derived growth factor |
| SG126 | SEQ ID NO:126 | 5617 | PRL | Prolactin |
| SG127 | SEQ ID NO:127 | 5894 | RAF1 | Homologue to the viral oncogene of murine leukemia V-raf-11 |
| SG128 | SEQ ID NO:128 | 5915 | RARB | Retinoic acid receptor, beta |
| SG129 | SEQ ID NO:129 | 4985 | OPRD1 | Opioid receptor, delta 1 |
| SG130 | SEQ ID NO:130 | 5979 | RET | proto-oncogene ret (multiple endocrine neoplasia and medullary thyroid carcinoma 1, Hirschsprung's disease) |
| SG131 | SEQ ID NO:131 | 6720 | SREBF1 | Transcription factor of binding to sterol regulatory elements 1 |
| SG132 | SEQ ID NO:132 | 7124 | TNF | Tumour necrosing factor (TNF superfamily, member 2) |
| SG133 | SEQ ID NO:133 | 7013 | TERF1 | Binding factor to telomeric repetitions (which interact with NIMA) 1 |
| SG134 | SEQ ID NO:134 | 7412 | VCAM1 | Molecule of adhesion to vascular cells 1 |
| SG135 | SEQ ID NO:135 | 539 | ATP5O | ATP synthase, H+ carrier, mitochondrial F1 complex, subunit O (protein which gives sensitivity to oligomycin) |
| SG136 | SEQ ID NO:136 | 959 | TNFSF5 | Tumour necrosing factor (ligand) superfamily, member 5 (hyper-IgM syndrome) |
| SG137 | SEQ ID NO:137 | 5432 | POLR2C | Polypeptide C (directed at DNA) of the RNA polymerase II (33kD) |
| SG138 | SEQ ID NO:138 | 8398 | PLA2G6 | Phospholipase A2, group VI (cytosolic, calcium-dependent) |
| SG139 | SEQ ID NO:139 | 908 | CCT6A | TCP 1 which contains chaperonin, subunit 6A (zeta 1) |
| SG140 | SEQ ID NO:140 | 5160 | PDHA1 | Pyruvate dehydrogenase (lipoamide) alpha 1 |
| SG141 | SEQ ID NO:141 | 3939 | LADH | Lactate dehydrogenase A |
| SG142 | SEQ ID NO:142 | 6628 | SNRPB | Polypeptides B and B1 of the small nuclear ribonucleoprotein |
| SG143 | SEQ ID NO:143 | 6628 | SNRPB | Polypeptides B and B1 of the small nuclear ribonucleoprotein |
| SG144 | SEQ ID NO:144 | 3014 | H2AFX | Histone family H2A, member X |
| SG145 | SEQ ID NO:145 | 51253 | MRPL37 | Mitochondrial ribosomal protein L37 |
| SG146 | SEQ ID NO:146 | 11065 | UBE2C | Enzyme which conjugates with ubiquin E2C |
| SG147 | SEQ ID NO:147 | 6188 | RPS3 | Ribosomal protein S3A |
| SG148 | SEQ ID NO:148 | 216 | ALDH1A1 | Aldehyde dehydrogenase 1 family, member A1 |
| SG149 | SEQ ID NO:149 | 10962 | AF1q | Lymphoid/myeloid or leukemia or of mixed line (homologue to trithorax, Drosophila); translocated to 11 |
| SG150 | SEQ ID NO:150 | 861 | RUNX1 | Runt 1-related transcription (acute myeloid leukemia 1; oncogene aml1) |
| SG151 | SEQ ID NO:151 | 4603 | MYBL1 | Similar to the homologue of the viral oncogene of avian myeloblastisis V-myb 1 |
| SG152 | SEQ ID NO:152 | 309 | ANXA6 | Annexin A6 |
| SG153 | SEQ ID NO:153 | 238 | ALK | Anaplastic lymphoma kinase (Ki-1) |
| SG154 | SEQ ID NO:154 | 4332 | MNDA | Antigen for nuclear differentiation myeloid cells |
| SG155 | SEQ ID NO:155 | 317 | APAF1 | Apoptotic protease activator factor |
| SG156 | SEQ ID NO: 156 | 330 | BIRC3 | which contains IAP repetitions of baculovirus 3 |
| SG157 | SEQ ID NO: 157 | 368 | ABCC6 | ATP-binding module, subfamily C (CFTR/MRP), member 6 |
| SG158 | SEQ ID NO: 158 | 471 | ATIC | 5-aminoimidazol-4-carboxamide ribonucleotide formyltransferase/IMP cyclohydrolase |
| SG159 | SEQ ID NO: 159 | 472 | ATM | Mutated ataxia-telangiectasia (includes complementary groups A, C and D) |
| SG160 | SEQ ID NO: 160 | 581 | BAX | protein X associated to BCL2 |
| SG161 | SEQ ID NO: 161 | 595 | CCND1 | Cyclin D 1 (PRAD 1: parathyroidal adenomatosis 1) |
| SG162 | SEQ ID NO: 162 | 596 | BCL2 | CLL/lymphoma of B 2 lymphocytes |
| SG163 | SEQ ID NO: 163 | 602 | BCL3 | CLL/lymphoma of B 3 lymphocytes |
| SG164 | SEQ ID NO:164 | 604 | BCL6 | CLL/lymphoma of B 6 lymphocytes (protein with zinc fingers 51) |
| SG165 | SEQ ID NO:165 | 605 | BCL7A | CLL/lymphoma of B 7A lymphocytes |
| SG166 | SEQ ID NO:166 | 9275 | BCL7b | CLL/lymphoma of B 7B lymphocytes |
| SG167 | SEQ ID NO:167 | 8915 | BCL10 | CLL/lymphoma of B10 lymphocytes |
| SG168 | SEQ ID NO:168 | 598 | BCL2L1 | similar to BCL2 1 |
| SG169 | SEQ ID NO:169 | 613 | BCR | Grouping breaking point region |
| SG170 | SEQ ID NO:170 | 613 | BCR | Grouping breaking point region |
| SG171 | SEQ ID NO:171 | 10215 | OLIG2 | Transcription factor of oligodendrocytes 2 line |
| SG172 | SEQ ID NO:172 | 638 | BIK | Mortal factor which interacts with BCL2 (apoptosis inducer) |
| SG173 | SEQ ID NO:173 | 10018 | BCL2LAA | Similar to BCL2 (which facilitates apoptosis) |
| SG174 | SEQ ID NO:174 | 648 | BMI1 | Homologue to the viral oncogene of murine leukemia (bmi-1) |
| SG175 | SEQ ID NO:175 | 642 | BLMH | bleomycin hydrolase |
| SG176 | SEQ ID NO:176 | 643 | BLR1 | Burkitt 1 lymphoma receptor, GTP-binding protein |
| SG177 | SEQ ID NO: 177 | 3381 | IBSP | Integrin-binding sialoprotein (bone sialoprotein bone sialoprotein II) |
| SG178 | SEQ ID NO:178 | 694 | BTG1 | Gene of translocation of B lymphocytes, anti-proliferative |
| SG179 | SEQ ID NO:179 | 699 | BUB1 | Disinhibited budding by benzimidazoles 1 (homologue of yeasts) |
| SG180 | SEQ ID NO:180 | 25 | ABL1 | homologue to the viral oncogene of that of Abelson's murine leukemia v-abl 1 |
| SG181 | SEQ ID NO:181 | 834 | CASP1 | caspase 1, apoptosis-related cysteine protease (interleukin 1, beta, convertase) |
| SG182 | SEQ ID NO:182 | 836 | CASP3 | caspase 3, apoptosis-related cysteine protease |
| SG183 | SEQ ID NO:183 | 837 | CASP4 | caspase 4, apoptosis-related cysteine protease |
| SG184 | SEQ ID NO: 184 | 838 | CASP5 | caspase 5, apoptosis-related cysteine protease |
| SG185 | SEQ ID NO: 185 | 839 | CASP6 | caspase 6, apoptosis-related cysteine protease |
| SG186 | SEQ ID NO: 186 | 840 | CASP7 | caspase 7, apoptosis-related cysteine protease |
| SG187 | SEQ ID NO: 187 | 841 | CASP8 | caspase 8, apoptosis-related cysteine protease |
| SG188 | SEQ ID NO:188 | 842 | CASP9 | caspase 9, apoptosis-related cysteine protease |
| SG189 | SEQ ID NO: 189 | 865 | CBFB | Nucleus binding factor, beta subunit |
| SG190 | SEQ ID NO: 190 | 800 | CALD1 | Caldesmon 1 |
| SG191 | SEQ ID NO: 191 | 831 | CAST | Calpastatin |
| SG192 | SEQ ID NO: 192 | 993 | CDC25A | Cell division cycle 25A |
| SG193 | SEQ ID NO: 193 | 994 | CDC25B | Cell division cycle 25B |
| SG194 | SEQ ID NO: 194 | 914 | CD2 | Antigen CD2 (p50), sheep red blood cell receptor |
| SG195 | SEQ ID NO: 195 | 916 | CD3E | Antigen CD3E, epilson polypeptide (complex TiT3) |
| SG196 | SEQ ID NO:196 | 920 | CD4 | Antigen CD4 (p55) |
| SG197 | SEQ ID NO: 197 | 921 | CD5 | Antigen CD5 (p56-62) |
| SG198 | SEQ ID NO: 198 | 923 | CD6 | Antigen CD6 |
| SG199 | SEQ ID NO: 199 | 924 | CD7 | Antigen CD7 (p41) |
| SG200 | SEQ ID NO:200 | 925 | CD8 | Antigen CD8, alpha polypeptide(p32) |
| SG201 | SEQ ID NO:201 | 928 | CD9 | Antigen CD9 (p24) |
| SG202 | SEQ ID NO:202 | 4311 | MME | Membrane metalloendopeptidase (neutral endopeptidase, encephalinase, CALLA, CD10) |
| SG203 | SEQ ID NO:203 | 3683 | ITGAL | Integrin, alpha L (antigen CD11A (p180), antigen associated to the function of lymphocytes 1; alpha polypeptide) |
| SG204 | SEQ ID NO:204 | 3684 | ITGAM | Integrin, alpha M (complement 3 component receptor, alpha; also known as CD11b (p170), polypeptide of the macrophage alpha antigen) |
| SG205 | SEQ ID NO:205 | 3687 | ITGAX | Integrin, alpha X (antigen CD11C (p150), alpha polypeptide) |
| SG206 | SEQ ID NO:206 | 90 | ANPEP | Alanyl-(membrane)aminopeptidase (aminopeptidase N, aminopeptidase M, microsomal aminopeptidase, CD13, p150) |
| SG207 | SEQ ID NO:207 | 929 | CD14 | Antigen CD 14 |
| SG208 | SEQ ID NO:208 | 6401 | SELE | Selectin E (endothelial adhesion molecule 1) |
| SG209 | SEQ ID NO:209 | 2214 | FCGR3A | Low-affinity receptor IIIa for the Fc fragment of IgG (CD16) |
| SG210 | SEQ ID NO:210 | 2215 | FCGR3B | Low-affinity receptor IIIb for the Fc fragment of IgG (CD 16) |
| SG211 | SEQ ID NO:211 | 3689 | ITGB2 | Integrin, beta 2 (antigen CD18 (p95), antigen associated to the function of the lymphocytes 1; beta subunit of the microphage 1 (mac-1) antigen) |
| SG212 | SEQ ID NO:212 | 930 | CD19 | Antigen CD 19 |
| SG213 | SEQ ID NO:213 | 931 | MS4A1 | Of 4 domains which are expanded by the membrane, subfamily A, member 1 |
| SG214 | SEQ ID NO:214 | 1380 | CR2 | Complement component receptor (3d/Epstein Barr's virus) 2 |
| SG215 | SEQ ID NO:215 | 933 | CD22 | Antigen CD22 |
| SG216 | SEQ ID NO:216 | 2208 | FCER2 | Low-affinity receptor II for the Fc fragment of IgE (CD23A) |
| SG217 | SEQ ID NO:217 | 934 | CD24 | Antigen CD24 (antigen of carcinoma of small lung cells of the grouping 4) |
| SG218 | SEQ ID NO:218 | 3559 | IL2RA | interleukin 2 receptor, alpha |
| SG219 | SEQ ID NO:219 | 1803 | DPP4 | Dipeptidyl peptidase IV (CD26, protein which forms complexes with adenosine deaminase 2) |
| SG220 | SEQ ID NO:220 | 3688 | ITGB1 | Integrin, beta 1 (fibronectin receptor, beta polypeptide, antigen CD29 includes MDF2, MSK12) |
| SG221 | SEQ ID NO:221 | 943 | TNFRSF8 | Tumour necrosing factor receptor superfamily, member 8 |
| SG222 | SEQ ID NO:222 | 945 | CD33 | Antigen CD33 (gp67) |
| SG223 | SEQ ID NO:223 | 947 | CD34 | Antigen CD34 |
| SG224 | SEQ ID NO:224 | 948 | CD36 | Antigen CD36 (collagen type I receptor, thrombospondin receptor) |
| SG225 | SEQ ID NO:225 | 952 | CD38 | Antigen CD38 (p45) |
| SG226 | SEQ ID NO:226 | 958 | TNFRSF5 | Tumour necrosing factor receptor superfamily, member 5 |
| SG227 | SEQ ID NO:227 | 6693 | SPN | Sarcospan (Gene associated to the Kras oncogene) |
| SG228 | SEQ ID NO:228 | 960 | CD44 | Antigen CD44 (homing function and Indian blood group function) |
| SG229 | SEQ ID NO:229 | 960 | CD44v6 | Antigen CD44 (homing function and Indian blood group function) |
| SG230 | SEQ ID NO:230 | 5788 | PTPRCCD | Protein thyrosine phosphatase, receptor type, C |
| SG231 | SEQ ID NO:231 | 961 | CD47 | Antigen CD47 (Antigen related to Rh, transducer of the signal associated to integrins) |
| SG232 | SEQ ID NO:232 | 3673 | ITGA2 | Integrin, alpha 2 (CD49B, alpha 2 subunit of receptor VLA-2) |
| SG233 | SEQ ID NO:233 | 3675 | ITGA3 | Integrin, alpha 3 (Antigen CD49C, alpha subunit 3 of receptor VLA-3) |
| SG234 | SEQ ID NO:234 | 3676 | ITGA4 | Integrin, alpha 4 (Antigen CD49D, alpha subunit 4 of receptor VLA-4) |
| SG235 | SEQ ID NO:235 | 3678 | ITGA5 | Integrin, alpha 5 (fibronectin receptor, alpha polypeptide) |
| SG236 | SEQ ID NO:236 | 3385 | ICAM3 | Intercellular adhesion molecule 3 |
| SG237 | SEQ ID NO:237 | 1043 | CDW52 | Antigen CDW52 (antigen CAMPATH-1) |
| SG238 | SEQ ID NO:238 | 3383 | ICAM1 | Intercellular adhesion molecule 1 (CD54), human rhinovirus receptor |
| SG239 | SEQ ID NO:239 | 4684 | NCAM1 | Neural cell adhesion molecule 1 |
| SG240 | SEQ ID NO:240 | 965 | CD58 | Antigen CD58 (antigen associated to the function of the lymphocytes 3) |
| SG241 | SEQ ID NO:241 | 966 | CD59 | Antigen CD59 p18-20 (antigen identified by the monoclonal antibodies 16.3A5, EJ16, EJ30, EL32 and G344) |
| SG242 | SEQ ID NO:242 | 6402 | SELL | Selectin L (lymphocyte adhesion molecule 1) |
| SG243 | SEQ ID NO:243 | 974 | CD79B | Antigen CD79B (associated to beta immunoglobulins) |
| SG244 | SEQ ID NO:244 | 975 | CD81 | Antigen CD81 (target of the antiproliferative antibody 1) |
| SG245 | SEQ ID NO:245 | 3732 | KAI1 | Kangai 1 (suppression of tumorigenicity 6, prostate; antigen CD82 (leukocytes R2 antigen, antigen detected by the monoclonal antibody IA4)) |
| SG246 | SEQ ID NO:246 | 9308 | CD83 | Antigen CD83 (activated B lymphocytes, immunoglobulins superfamily) |
| SG247 | SEQ ID NO:247 | 942 | CD86 | Antigen CD86 (ligand 2 of the antigen CD28, antigen B7-2) |
| SG248 | SEQ ID NO:248 | 355 | TNFRSF6 | Tumour necrosing factor receptor superfamily, member 6 |
| SG249 | SEQ ID NO:249 | 356 | TNFSF6 | Tumour necrosing factor receptor superfamily (ligand), member 6 |
| SG250 | SEQ ID NO:250 | 8140 | SLC7A5 | Solute-carrier family 7 (cationic amino acid carrier, y+ system), member 5 |
| SG251 | SEQ ID NO:251 | 6382 | SDC1 | Sindecan 1 |
| SG252 | SEQ ID NO:252 | 1019 | CDK4 | Cyclin-dependent kinase 4 |
| SG253 | SEQ ID NO:253 | 6774 | STAT3 | Signal transducer and transcription activator 3 (response factor in acute phase) |
| SG254 | SEQ ID NO:254 | 2268 | FGR | Homologue to Gardner-Rasheed's feline viral sarcoma oncogene(v-fgr) |
| SG255 | SEQ ID NO:255 | 2353 | FOS | Homologue to the murine viral osteosarcoma oncogene V-fos FBJ |
| SG256 | SEQ ID NO:256 | 898 | CCNE1 | Cyclin E1 |
| SG257 | SEQ ID NO:257 | 978 | CDA | Cytidine deaminase |
| SG258 | SEQ ID NO:258 | 9935 | MAFB | Homologue to the fibrosarcoma oncogene(avian) musculoaponeurotic V-maf |
| SG259 | SEQ ID NO:259 | 4352 | MPL | Oncogene of myeloproliferative leukemia virus |
| SG260 | SEQ ID NO:260 | 4609 | MYC | Homologue to the avian myelocyomatosis viral oncogene V-myc |
| SG261 | SEQ ID NO:261 | 4602 | MYB | Homologue to the avian myelocyomatosis viral oncogene V-myb |
| SG262 | SEQ ID NO:262 | 1159 | CKMT1 | Creatine kinase, mitochondrial 1 (ubicuous) |
| SG263 | SEQ ID NO:263 | 1387 | CREBBP | CREB binding protein (Rubinstein-Taybi's syndrome) |
| SG264 | SEQ ID NO:264 | 1490 | CTGF | Connective tissue growth factor |
| SG265 | SEQ ID NO:265 | 2833 | CXCR3 | Chemokene receptor 3 (motive C-X-C) |
| SG266 | SEQ ID NO:266 | 7852 | CXCR4 | Chemokene receptor 4 (motive C-X-C) (fusin) |
| SG267 | SEQ ID NO:267 | 8900 | CCNA1 | Cyclin A1 |
| SG268 | SEQ ID NO:268 | 891 | CCNB1 | Cyclin B 1 |
| SG269 | SEQ ID NO:269 | 894 | CCND2 | Cyclin D2 |
| SG270 | SEQ ID NO:270 | 1543 | CYP1A1 | Cytochrome P450, subfamily I (inducible by aromatic compounds), polypeptide 1 |
| SG271 | SEQ ID NO:271 | 1565 | CYP2A6 | Cytochrome P450, subfamily IID (of metabolization of debrisokine, spartin, etc.), polypeptide 6 |
| SG272 | SEQ ID NO:272 | 1603 | DAD-1 | Defender against cell death 1 |
| SG273 | SEQ ID NO:273 | 8794 | TNFRSF10C | Tumour necrosing factor receptor superfamily, member 10c, decoy without intracellular domain |
| SG274 | SEQ ID NO:274 | 7913 | DEK | Oncogene DEK (which binds to DNA) |
| SG275 | SEQ ID NO:275 | 1633 | DCK | Deoxycytidine kinase |
| SG276 | SEQ ID NO:276 | 1719 | DHFR | Dihydrofolate reductase |
| SG277 | SEQ ID NO:277 | 6929 | TCF3 | Transcription factor 3 (immunoglobulin enhancer binding factors E2A E12/E47) |
| SG278 | SEQ ID NO:278 | 1869 | E2F1 | Transcription factor E2F 1 |
| SG279 | SEQ ID NO:279 | 6929 | TCF3 | Transcription factor 3 (immunoglobulin enhancer binding factors E2A E12/E47) |
| SG280 | SEQ ID NO:280 | 56899 | EB-1 | Protein associated to E2a-Pbxl |
| SG281 | SEQ ID NO:281 | 1236 | CCR7 | Chemokene receptor 7 (motive C-C) |
| SG282 | SEQ ID NO:282 | 1880 | EBI2 | Gene induced by Epstein-Barr's 2 disease (receptor coupled to G proteins specific for lymphocytes) |
| SG283 | SEQ ID NO:283 | 4582 | MUC-1 | Mucin 1, transmembrane |
| SG284 | SEQ ID NO:284 | 2042 | EphA3 | EPHA3 |
| SG285 | SEQ ID NO:285 | 2057 | EPOR | Erythropoietin receptor |
| SG286 | SEQ ID NO:286 | 2067 | ERCC1 | Of repair of excision which intercomplements the deficiency in the repair of rodents, complementation group 1 (includes the antisense overlapping sequence) |
| SG287 | SEQ ID NO:287 | 2068 | ERCC2 | Of repair of excision which intercomplements the deficiency in the repair of rodents, complementation group 2 (xerodermia pigmentoso 2) |
| SG288 | SEQ ID NO:288 | 2071 | ERCC3 | Of repair of excision which intercomplements the deficiency in the repair of rodents, complementation group 3 (complements group B of xerodermia pigmentoso) |
| SG289 | SEQ ID NO:289 | 2073 | ERCC5 | Of repair of excision which intercomplements the deficiency in the repair of rodents, complementation group 5 (xerodermia pigmentoso, complementation group G (Cockayne's syndrome)) |
| SG290 | SEQ ID NO:290 | 2074 | ERCC6 | Of repair of excision which intercomplements the deficiency in the repair of rodents, complementation group 6 |
| SG291 | SEQ ID NO:291 | 50624 | CUZD1 | With CUB domains and similar to the zone pellucida 1 |
| SG292 | SEQ ID NO:292 | 2120 | ETV6 | Gene variant of ets 6 (TEL oncogene) |
| SG293 | SEQ ID NO:293 | 1977 | EIF4E | Initiation factor of eukaryotic translation 4E |
| SG294 | SEQ ID NO:294 | 1984 | EIF5A | Initiation factor of eukaryotic translation 5A |
| SG295 | SEQ ID NO:295 | 2146 | EZH2 | Zeste homologue enhancer (Drosophila) 2 |
| SG296 | SEQ ID NO:296 | 8772 | FADD | Associated to Fas (TNFRSF6) via apoptopic domain |
| SG297 | SEQ ID NO:297 | 5747 | PTK2 | Thyrosine kinase 2 protein |
| SG298 | SEQ ID NO:298 | 2195 | FAT | Homologue to the FAT tumour suppressor (Drosophila) |
| SG299 | SEQ ID NO:299 | 2260 | FGFR1 | Fibroblast growth factor receptor 1 (thyrosine kinase related to fms 2, Pfeiffer's syndrome) |
| SG300 | SEQ ID NO:300 | 2261 | FGFR3 | Fibroblast growth factor receptor 3 (achondroplasia, thanatophoric dwarfism) |
| SG301 | SEQ ID NO:301 | 2272 | FHIT | Fragile histidine triad gene |
| SG302 | SEQ ID NO:302 | 2322 | FLT3 | Thyrosine kinase related to Fms 3 |
| SG303 | SEQ ID NO:303 | 2892 | GRIA3 | Glutamate receptor, ionotrophic, AMPA 3 |
| SG304 | SEQ ID NO:304 | 2521 | FUS | Fusion, derived from the malignant liposarcoma t(12;16) |
| SG305 | SEQ ID NO:305 | 1977 | EIF4E | Initiation factor of eukaryotic translation 4E |
| SG306 | SEQ ID NO:306 | 6482 | SIAT4A | Sialyltransferase 4A (beta-galactosidase alpha-2.3- Sialyltransferase) |
| SG307 | SEQ ID NO:307 | 1440 | CSF3 | Colony stimulating factor 3 (granulocyte) |
| SG308 | SEQ ID NO:308 | 1437 | CSF2 | Colony stimulating factor 2 (granulocyte-microphage) |
| SG309 | SEQ ID NO:309 | 2908 | NR3C1 | Subfamily of nuclear receptors 3, group C, member 1 |
| SG310 | SEQ ID NO:310 | 2952 | GSTT1 | Glutathion S-transferase theta 1 |
| SG311 | SEQ ID NO:311 | 3001 | GZMA | Granzime A (granzime 1, serine stearase associated to cytotoxic T lymphocytes 3) |
| SG312 | SEQ ID NO:312 | 3301 | DNAJA1 | Homologue to DnaJ (Hsp40), subfamily A, member 1 |
| SG313 | SEQ ID NO:313 | 3131 | HLF | Hepatic leukemia factor |
| SG314 | SEQ ID NO:314 | 684 | BST2 | Antigen of bone marrow stroma cells 2 |
| SG315 | SEQ ID NO:315 | 3205 | HOXA9 | Homeotic box A9 |
| SG316 | SEQ ID NO:316 | 3195 | TLX1 | T lymphocyte leukemia, homeotic box 1 |
| SG317 | SEQ ID NO:317 | 29128 | UHRF1 | similar to ubiquitine, which contains PHD domains and RING fingers, 1 |
| SG318 | SEQ ID NO:318 | 8870 | IER3 | Immediate early response 3 |
| SG319 | SEQ ID NO:319 | 3553 | IL1B | Interleukin 1, beta |
| SG320 | SEQ ID NO:320 | 3558 | IL2 | Interleukin 2 |
| SG321 | SEQ ID NO:321 | 3562 | IL3 | Interleukin 3 (multiple colony stimulating factor) |
| SG322 | SEQ ID NO:322 | 3569 | IL6 | Interleukin 6 (interferon, beta 2) |
| SG323 | SEQ ID NO:323 | 3570 | IL6R | Interleukin receptor 6 |
| SG324 | SEQ ID NO:324 | 3586 | IL10 | Interleukin 10 |
| SG325 | SEQ ID NO:325 | 3600 | IL15 | Interleukin 15 |
| SG326 | SEQ ID NO:326 | 3662 | IRF4 | Interferon regulating factor 4 |
| SG327 | SEQ ID NO:327 | 3716 | JAK1 | Janus 2 kinase (a thyrosine kinase protein) |
| SG328 | SEQ ID NO:328 | 3717 | JAK2 | Janus 1 kinase (a thyrosine kinase protein) |
| SG329 | SEQ ID NO:329 | 4288 | MKI67 | Antigen identified by monoclonal antibody Ki-67 |
| SG330 | SEQ ID NO:330 | 7520 | XRCC5 | Of repair of X rays which complements the defective repair in Chinese hamster cells 5 (reconnection of breakages in the double helix; autoantigen Ku, 80kDa) |
| SG331 | SEQ ID NO:331 | 3902 | LAG3 | Lymphocyte activation gene 3 |
| SG332 | SEQ ID NO:332 | 3932 | LCK | Lymphocyte specific protein thyrosine kinase |
| SG333 | SEQ ID NO:333 | 3936 | LCP1 | Cytosolic protein of lymphocytes 1 (L-plastin) |
| SG334 | SEQ ID NO:334 | 3953 | LEPR | Leptin receptor |
| SG335 | SEQ ID NO:335 | 4005 | LMO2 | With LIM domains only 2 (similar to rombotin 1) |
| SG336 | SEQ ID NO:336 | 3976 | LIF | Leukemia inhibiting factor (cholinergic differentiation factor) |
| SG337 | SEQ ID NO:337 | 9961 | LRP | Main leap protein |
| SG338 | SEQ ID NO:338 | 4046 | LSP1 | Lymphocyte-specific protein 1 |
| SG339 | SEQ ID NO:339 | 4066 | LYL1 | Sequence derived from lymphoblastic leukemia 1 |
| SG340 | SEQ ID NO:340 | 4790 | NFKB1 | Nuclear factor of the enhancer of the gene of the light kappa polypeptide in B-1 lymphocytes(p105) |
| SG341 | SEQ ID NO:341 | 4118 | MAL | mal, T lymphocyte differentiation protein |
| SG342 | SEQ ID NO:342 | 4100 | MAGEA1 | Melanoma antigen, family A, 1 (directs the expression of antigen MZ2-E) |
| SG343 | SEQ ID NO:343 | 5602 | MAPK10 | Protein kinase activated by mitogens 10 |
| SG344 | SEQ ID NO:344 | 2023 | MBP1 | Enolase 1, (alpha) |
| SG345 | SEQ ID NO:345 | 4170 | MCL1 | Leukemia sequence of myeloid cells 1 (related to BCL2) |
| SG346 | SEQ ID NO:346 | 4193 | MDM2 | Human homologue of the double mouse diminuta 2; p53-binding protein |
| SG347 | SEQ ID NO:347 | 5243 | ABCB1 | ATP binding module, subfamily B (MDR/TAP), member 1 |
| SG348 | SEQ ID NO:348 | 5244 | ABCB4 | ATP binding module, subfamily B (MDR/TAP), member 4 |
| SG349 | SEQ ID NO:349 | 4221 | MEN1 | Multiple endocrine neoplasia I |
| SG350 | SEQ ID NO:350 | 4283 | CXCL9 | Chemokene ligand 9 (motive C-X-C) |
| SG351 | SEQ ID NO:351 | 4291 | MLF1 | Myeloid leukemia factor 1 |
| SG352 | SEQ ID NO:352 | 4297 | MLL | Myeloid/lymphoid leukemia or of mixed line (homologue to trithorax (Drosophila)) |
| SG353 | SEQ ID NO:353 | 4318 | MMP9 | Metalloproteinase of matrix 9 (gelatinase B, 92kD gelatinase, 92kD collagenase type IV) |
| SG354 | SEQ ID NO:354 | 4707 | NDUFB | NADH dehydrogenase (ubiquinone) 1 beta subcomplex, 1 (7kD, MNLL) |
| SG355 | SEQ ID NO:355 | 4353 | MPO | Myeloperoxidase |
| SG356 | SEQ ID NO:356 | 8714 | ABCC3 | ATP binding module, subfamily C (CFTR/MRP), member 3 |
| SG357 | SEQ ID NO:357 | 10057 | ABCC5 | ATP binding module, subfamily C (CFTR/MRP), member 5 |
| SG358 | SEQ ID NO:358 | 4515 | MTCP1 | Proliferation of mature T-lymphocytes 1 |
| SG359 | SEQ ID NO:359 | 4515 | MTCP1 | Proliferation of mature T-lymphocytes 1 |
| SG360 | SEQ ID NO:360 | 4654 | MYOD1 | Myogenic factor 3 |
| SG361 | SEQ ID NO:361 | 4599 | MX1 | Resistance to Myxovirus (flu) 1, homologue to the murine protein (protein inducible by interferon p78) |
| SG362 | SEQ ID NO:362 | 4814 | NINJ1 | Ninjurin 1 |
| SG363 | SEQ ID NO:363 | 4869 | NPM1 | Nucleophosmin (nucleolar phosphoprotein B23, numatrin) |
| SG364 | SEQ ID NO:364 | 9235 | IL32 | Interleukin 32 |
| SG365 | SEQ ID NO:365 | 4926 | NUMA1 | Nuclear protein of the myotic apparatus 1 |
| SG366 | SEQ ID NO:366 | 5452 | POU2F2 | transcription factor with POU domain, of class 2,2 |
| SG367 | SEQ ID NO:367 | 5452 | POU2F2 | transcription factor with POU domain, of class 2,2 |
| SG368 | SEQ ID NO:368 | 4968 | OGGI | 8-oxoguanine-DNA-glycosilase |
| SG369 | SEQ ID NO:369 | 1030 | CDKN2B | Cyclin-dependent kinase inhibitor 2B (p15, inhibits CDK4) |
| SG370 | SEQ ID NO:370 | 1029 | CDKN2A | Cyclin-dependent kinase inhibitor 2A (melanoma, p16, inhibits CDK4) |
| SG371 | SEQ ID NO:371 | 1031 | CDKN2C | Cyclin-dependent kinase inhibitor 2C (p18, inhibits CDK4) |
| SG372 | SEQ ID NO:372 | 1026 | CDKN1A | Cyclin-dependent kinase inhibitor 1A (p21, Cip1) |
| SG373 | SEQ ID NO:373 | 1027 | CDKN1B | Cyclin-dependent kinase inhibitor 1B (p27, Kip1) |
| SG374 | SEQ ID NO:374 | 8851 | CDK5R1 | Cyclin-dependent kinase 5, regulator subunit 1 (p35) |
| SG375 | SEQ ID NO:375 | 10210 | TOPORS | Of binding to topoisomerase I, rich inc arginine/serine |
| SG376 | SEQ ID NO:376 | 991 | P55CDC | CDC20 (cell division cycle 20, S. cerevisiae, homologue) |
| SG377 | SEQ ID NO:377 | 1028 | CDKN1C | Cyclin-dependent kinase inhibitor 1C (p57, Kip2) |
| SG378 | SEQ ID NO:378 | 7161 | TP73 | Tumour protein p73 |
| SG379 | SEQ ID NO:379 | 5079 | PAX5 | Paired box gene 5 (specific activating protein of the B lymphocytes line) |
| SG380 | SEQ ID NO:380 | 5087 | PBX1 | Transcription factor of B 1 prelymphocytes leukemia |
| SG381 | SEQ ID NO:381 | 5090 | PBX3 | Transcription factor of B prelymphocytes leukemia B 3 |
| SG382 | SEQ ID NO:382 | 5089 | ENPP1 | Ectonucleotide pyrophosphatase/phosphodiesterase 1 |
| SG383 | SEQ ID NO:383 | 5167 | PCA1 | Ectonucleotide pyrophosphatase/phosphodiesterase 1 |
| SG384 | SEQ ID NO:384 | 5111 | PCNA | Nuclear antigen of proliferating cells |
| SG385 | SEQ ID NO:385 | 5159 | PDGFRB | Platelet-derived growth factor receptor, beta polypeptide |
| SG386 | SEQ ID NO:386 | 5588 | PRKCQ | kinase C protein, theta |
| SG387 | SEQ ID NO:387 | 5347 | PLK1 | Plasminogen activator, urokinase |
| SG388 | SEQ ID NO:388 | 5371 | PML | Promielocytic leukemia |
| SG389 | SEQ ID NO:389 | 23532 | PRAME | Antigen expressed preferentially in melanoma |
| SG390 | SEQ ID NO:390 | 5584 | PRKCI | protein kinase C, iota |
| SG391 | SEQ ID NO:391 | 5728 | PTEN | Phosphatase and homologue to tensin (mutated in multiple advanced cancers 1) |
| SG392 | SEQ ID NO:392 | 5742 | PTGS 1 | prostaglandin-endoperoxide synthase 1 (prostaglandin G/H synthase and cyclooxigenase) |
| SG393 | SEQ ID NO:393 | 5744 | PTHLH | Hormone similar to the parathyroidal hormone |
| SG394 | SEQ ID NO:394 | 6688 | SPI1 | Oncogene of integration of provirus of focus forming virus in the spleen (SFFV) spil |
| SG395 | SEQ ID NO:395 | 2185 | PTK2B | Thyrosine kinase 2 beta protein |
| SG396 | SEQ ID NO:396 | 5889 | RAD51 | Homologue to RAD51 (S. cerevisiae) (homologue to RecA of E. coli) |
| SG397 | SEQ ID NO:397 | 5896 | RAG1 | Recombination activator gene 1 |
| SG398 | SEQ ID NO:398 | 5914 | RARA | Retinoic acid receptor, alpha |
| SG399 | SEQ ID NO:399 | 3845 | KRAS2 | Homologue to the viral oncogene of Kirsten 2 V-Ki-ras2 rat sarcoma |
| SG400 | SEQ ID NO:400 | 5925 | RB1 | Retinoblastoma 1 (including osteosarcoma) |
| SG401 | SEQ ID NO:401 | 7422 | VEGF | Vascular endothelial growth factor |
| SG402 | SEQ ID NO:402 | 7791 | ZYX | Zyxin |
| SG403 | SEQ ID NO:403 | 940 | CD28 | Antigen CD28 (Tp44) |
| SG404 | SEQ ID NO:404 | 940 | CD28 | Antigen CD28 (Tp44) |
| SG405 | SEQ ID NO:405 | 1656 | RBBP4 | retinoblastoma 4 binding protein |
| SG406 | SEQ ID NO:406 | 1656 | DDX6 | Polypeptide with DEAD/H box (Asp-Glu-Ala-Asp/His) 6 (RNA helicase, 54kD) |
| SG407 | SEQ ID NO:407 | 5928 | APEX | APEX nuclease (multifunctional DNA repair enzyme DNA) |
| SG408 | SEQ ID NO:408 | 5977 | DPF2 | D4, family 2 with zinc fingers and double PHD |
| SG409 | SEQ ID NO:409 | 5996 | RGS1 | G protein signalling regulator G 1 |
| SG410 | SEQ ID NO:4 10 | 3161 | HMMR | Motility receptor mediated by hyaluronane (RHAMM) |
| SG411 | SEQ ID NO:411 | 6777 | STAT5B | signal transducer and transcription activator 5B |
| SG412 | SEQ ID NO:412 | 332 | BIRC5 | Which contains IAP repetitions of baculovirus 5 (survivin) |
| SG413 | SEQ ID NO:413 | 6886 | TAL1 | Acute lymphocytic leukemia of T lymphocytes 1 |
| SG414 | SEQ ID NO:414 | 10482 | NXF1 | RNA exportation nuclear factor of RNA 1 |
| SG415 | SEQ ID NO:415 | 8115 | TCL1A | Leukemia/lymphoma of T 1A lymphocytes |
| SG416 | SEQ ID NO:416 | 6955 | TRA@ | T locus alpha lymphocyte receptor |
| SG417 | SEQ ID NO:417 | | TCR beta | mRNA of the beta chain of the T lymphocyte receptor (TCRB) of Homo sapiens |
| SG418 | SEQ ID NO:418 | 1791 | DNTT | Deoxynucleotidyl transferase, terminal |
| SG419 | SEQ ID NO:419 | 7015 | TERT | Inverse telomerase transcriptase |
| SG420 | SEQ ID NO:420 | 2066 | ERBB4 | Similar to the homologue to the viral oncogene of avian erythroblastic leukemia V-erb-a 4 |
| SG421 | SEQ ID NO:421 | 2064 | ERBB2 | Homologue to the to the viral oncogene of avian erythroblastic leukemia V-erb-b2 2 (homologue to the oncogene derived from neuro/glioblastoma) |
| SG422 | SEQ ID NO:422 | 1956 | EGFR | Epidermal growth factor receptor (homologue to the viral oncogene of avian erythroblastic leukemia (v-erb-b)) |
| SG423 | SEQ ID NO:423 | 7066 | THPO | Thrombopoietin (oncogene ligand of the myeloproliferative leukemia virus, growth factor and development of megakaryocytes) |
| SG424 | SEQ ID NO:424 | 7074 | TIAM1 | Invasion and metastasis of lymphoma of T lymphocytes 1 |
| SG425 | SEQ ID NO:425 | 7083 | TK1 | Thymidine kinase 1, soluble |
| SG426 | SEQ ID NO:426 | 7132 | TNFRSF1A | Tumour necrosing factor receptor superfamily, member 1A |
| SG427 | SEQ ID NO:427 | 7153 | TOP2A | Topoisomerase (DNA) II alpha (170kD) |
| SG428 | SEQ ID NO:428 | 1052 | CEBPD | binding protein to CCAAT/enhancer (C/EBP), delta |
| SG428 | SEQ ID NO:428 | 9214 | FAIM3 | Apoptosis inhibitor molecule mediated by Fas 3 |
| SG429 | SEQ ID NO:429 | 5358 | PLS3 | Plastin 3 (isoform T) |
| SG430 | SEQ ID NO:430 | 8717 | TRADD | associated to TNFRSF1A via the cell death domain |
| SG431 | SEQ ID NO:431 | 8743 | TNFSF10 | Tumour necrosing factor superfamily (ligand) member 10 |
| SG432 | SEQ ID NO:432 | 10131 | TRAP1 | Thermal shock protein 75 |
| SG433 | SEQ ID NO:433 | 7057 | THBS1 | Thrombospondin 1 |
| SG434 | SEQ ID NO:434 | 7341 | SUMO1 | Homologue to the supressor of mif two 3 SMT3 1 (yeast) |
| SG435 | SEQ ID NO:435 | 7405 | UVRAG | Gene associated to UV radiation resistance |
| SG436 | SEQ ID NO:436 | 7441 | VPREB1 | Gene of B 1 prelymphocytes |
| SG437 | SEQ ID NO:437 | 51384 | WNT16 | family of MMTV integration site, Wingless-type, member 16 |
| SG438 | SEQ ID NO:438 | 7490 | WT1 | Wilms tumour 1 |
| SG439 | SEQ ID NO:439 | 7517 | XRCC3 | Of repair of X rays which complement the defective repair in Chinese hamster cells 3 |
| SG440 | SEQ ID NO:440 | 896 | CCND3 | Cyclin D3 |
| SG441 | SEQ ID NO:441 | 1017 | CDK2 | Cyclin-dependent kinase 2 |
| SG442 | SEQ ID NO:442 | | p14ARF | Gene p14ARF from Homo sapiens, promoter region, complete sequence |
| SG443 | SEQ ID NO:443 | 3070 | HELLS | Helicase, specific for lymphoid cells |
| SG444 | SEQ ID NO:444 | 2624 | GATA2 | GATA 1-binding protein 2 |
| SG445 | SEQ ID NO:445 | 2623 | GATA1 | GATA 1-binding protein (globin transcription factor 1) |
| SG446 | SEQ ID NO:446 | 8028 | MLLT10 | Myeloid/lymphoid leukemia or of mixed line (homologue to trithorax (Drosophila)); translocated to 10 |
| SG447 | SEQ ID NO:447 | 8301 | PICALM | Clathrin assembly protein which binds to phosphatidylinositol |
| SG448 | SEQ ID NO:448 | 3815 | KIT | Homologue to the viral oncogene of Hardy-Zuckerman's feline sarcoma 4 V-kit |
| SG449 | SEQ ID NO:449 | 3563 | IL3RA | Interleukin 3 receptor, alpha (low affinity) |
| SG450 | SEQ ID NO:450 | 1050 | CEBPA | binding protein to CCAAT/enhancer (C/EBP), alpha |
| SG451 | SEQ ID NO:451 | 3655 | ITGA6 | Integrin, alpha 6 |
| SG452 | SEQ ID NO:452 | 84955 | CML66 | Tumour antigen of chronic myelogenous leukemia 66 |
| SG453 | SEQ ID NO:453 | 7187 | TRAF3 | Factor associated to the TNF receptor 3 |
| SG454 | SEQ ID NO:454 | 1612 | DAPK1 | Kinase protein associated to cell death 1 |
| SG455 | SEQ ID NO:455 | 8788 | MAP3K12 | Homologue similar to Delta (Drosophila) |
| SG456 | SEQ ID NO:456 | 5591 | PRKDC | Kinase protein, activated by DNA, catalytic polypeptide |
| SG457 | SEQ ID NO:457 | 1789 | DNMT3B | (cytosine-5-)-methyltransferase 3 beta of DNA |
| SG458 | SEQ ID NO:458 | 2950 | GSTP1 | Glutathion S-transferase pi |
| SG459 | SEQ ID NO:459 | 3122 | HLA-DRA | Complex greater than histocompatibility, class II, DR alpha |
| SG460 | SEQ ID NO:460 | 3206 | HOXA10 | Homeotic box A 10 |
| SG461 | SEQ ID NO:461 | 3394 | IRF8 | Binding protein to the agreed sequence of interferon 1 |
| SG462 | SEQ ID NO:462 | 3398 | ID2 | DNA binding inhibitor 2, negative dominant helix-loops-helix protein |
| SG463 | SEQ ID NO:463 | 60 | ACTB | Actin, beta |
| SG464 | SEQ ID NO:464 | 60 | ACTB | Actin, beta |
| SG465 | SEQ ID NO:465 | 2868 | GRK4 | Kinase of receptor coupled to a protein G 4 |
| SG466 | SEQ ID NO:466 | 2597 | GAPD | Glyceraldehyde-3-phosphate dehydrogenase |
| SG467 | SEQ ID NO:467 | 2597 | GAPD | Glyceraldehyde-3 -phosphate dehydrogenase |
| SG468 | SEQ ID NO:468 | 6772 | STAT1 | Signal transducer and transcription activator 1, 91kDa |
| SG469 | SEQ ID NO:469 | | 18S rRNA | Human rRNA gene 18S |
| SG470 | SEQ ID NO:470 | 7037 | TFRC | Transferrin receptor (p90, CD71) |
| SG471 | SEQ ID NO:471 | | 28S rRNA | Human ribosomal RNA gene 28S |
| SG472 | SEQ ID NO:472 | 6168 | RPL37A | Ribosomal protein L37a |
| SG473 | SEQ ID NO:473 | 6171 | RPL41 | Ribosomal protein L41 |
| SG474 | SEQ ID NO:474 | 3191 | HNRPL | Heterogeneous nucler ribonucleoprotein L |
| SG475 | SEQ ID NO:475 | 3608 | ILF2 | Binding factor to the interleukin-2 enhancer, 45kD |
| SG476 | SEQ ID NO:476 | 8407 | TAGLN2 | Transgelin 2 |
| SG477 | SEQ ID NO:477 | 824 | CAPN2 | Calpain 2, (m/II) major subunit |
| SG478 | SEQ ID NO:478 | 5686 | PSMA5 | Subunit of proteasome (prosome, macropain), type alpha, 5 |
| SG479 | SEQ ID NO:479 | 27254 | PMM1 | Phosphomannomutase 1 |
| SG480 | SEQ ID NO:480 | 8079 | MLF2 | Myeloid leukemia factor 2 |
| SG481 | SEQ ID NO:481 | 5501 | PPP1CC | Phosphatase protein 1, catalytic subunit, gamma isoform |
| SG482 | SEQ ID NO:482 | 22794 | CASC3 | Candidate for susceptibility to cancer 3 |
| SG483 | SEQ ID NO:483 | 23164 | KIAA0864 | Protein KIAA0864 |
| SG484 | SEQ ID NO:484 | 7296 | TXNRD1 | Thioredoxine reductase 1 |
| SG485 | SEQ ID NO:485 | 5713 | PSMD7 | Subunit of proteasome (prosome, macropain) 26S, no-ATPase, 7 (homologue to Mov34) |
| SG486 | SEQ ID NO:486 | 8892 | EIF2B2 | initiation factor of eukaryotic translation 2B, subunit 2 (beta, 39kD) |
| SG487 | SEQ ID NO:487 | 3105 | HLA-A | Complex greater than histocompatibility, class I, A |
| SG488 | SEQ ID NO:488 | 4176 | MCM7 | Minichromosome maintenance deficient (S. cerevisiae) 7 |
| SG489 | SEQ ID NO:489 | 8718 | TNFRSF25 | Tumour necrosing factor receptor superfamily, member 25 |
| SG490 | SEQ ID NO:490 | 3958 | LGALS3 | Lectin, which binds to galactosides, soluble, 3 (galectin 3) |
| SG491 | SEQ ID NO:491 | 311 | HLA-DPA1 | Complex greater than histocompatibility, class II, DP alpha 1 |
| SG492 | SEQ ID NO:492 | 5328 | PLAU | Plasminogen activator, urokinase |
| SG493 | SEQ ID NO:493 | 1281 | COL3A1 | Collagen, type III, alpha 1 (Ehlers-Danlos type IV syndrome, dominant autosomal) |
| SG494 | SEQ ID NO:494 | 287 | ANK2 | Ankyrin 2, neuronal |
| SG495 | SEQ ID NO:495 | 327657 | SERPINA9 | serine (or cysteine) proteinase inhibitor, clade A (alpha-1 antiproteinase, antitrypsin), member 9 |
| SG496 | SEQ ID NO:496 | 10360 | NPM3 | Nucleophosmin/nucleoplasmin 3 |
| SG497 | SEQ ID NO:497 | 1235 | CCR6 | Receptor 6 of chemokenes (motive C-C) |
| SG498 | SEQ ID NO:498 | 3055 | HCK | Hematopoietic cell kinase |
| SG499 | SEQ ID NO:499 | 26354 | GNL3 | similar to guanine 3 nucleotide binding protein (nucleolar) |
| SG500 | SEQ ID NO:500 | 2885 | GRB2 | Protein bound to growth factor receptor 2 |
| SG501 | SEQ ID NO:501 | 597 | BCL2A1 | protein related to BCL2 A1 |
| SG502 | SEQ ID NO:502 | 1997 | ELF1 | Factor similar to E741 (transcription factor with ets domain) |
| SG503 | SEQ ID NO:503 | 1508 | CTSB | Catepsin B |
| SG504 | SEQ ID NO:504 | 257144 | GCET2 | transcript expressed in the budding centre 2 |
| SG505 | SEQ ID NO:505 | 2335 | FN1 | Fibronectin 1 |
| SG506 | SEQ ID NO:506 | 5133 | PDCD1 | Programme cell death 1 |
| SG507 | SEQ ID NO:507 | 3125 | HLA-DRB3 | Complex greater than histocompatibility, class II, DR beta 3 |
| SG508 | SEQ ID NO:508 | 3117 | HLA-DQA1 | Complex greater than histocompatibility, class II, DQ alpha 1 |
| SG509 | SEQ ID NO:509 | 257144 | GCET2 | transcript expressed in the budding centre germinal 2 |
| SG510 | SEQ ID NO:510 | 327657 | SERPINA9 | serine (or cysteine) proteinase inhibitor, clade A (alpha-1 antiproteinase, antitrypsin), member 9 |
| SG511 | SEQ ID NO:511 | 1033 | CDKN3 | Cyclin dependent kinases 3 (phosphatase of dual specificity associated CDK2) |
| SG512 | SEQ ID NO:512 | 1997 | ELF1 | Factor similar to E74 1 (transcription factor with ets domain) |
| SG513 | SEQ ID NO:513 | 1509 | CATSD | Catepsin D (liposomal aspartylprotease) |
| SG514 | SEQ ID NO:514 | 3315 | HSPB1 | Thermal shock protein of 27kD 1 |
| SG515 | SEQ ID NO:515 | 87 | ACTN1 | Actinin, alpha 1 |
| SG516 | SEQ ID NO:516 | 654 | BMP6 | Morphogenetic bone protein 6 |
| SG517 | SEQ ID NO:517 | 9780 | FAM38A | family with similarity of sequence 38, member A |
| SG518 | SEQ ID NO:518 | 962 | CD48 | Antigen CD48 (membrane protein of B lymphocytes) |
| SG519 | SEQ ID NO:519 | 3566 | IL4R | Interleukin 4 receptor |
| SG520 | SEQ ID NO:520 | 1821 | DRP2 | Dystrophin related protein 2 |
| SG521 | SEQ ID NO:521 | 3726 | JUNB | Jun B Proto-oncogene |
| SG522 | SEQ ID NO:522 | 6279 | S100A8 | Calcium-binding protein S100 A8 (calgranuline A) |
| SG523 | SEQ ID NO:523 | 10320 | ZNFN1A1 | Protein with zinc fingers, subfamily 1A, (Ikaros) |
| SG524 | SEQ ID NO:524 | 10461 | MERTK | Thyrosine kinase of the C-mer proto-oncogene |
| SG525 | SEQ ID NO:525 | 51621 | KLF13 | Factor similar to that of Kruppel 13 |
| SG526 | SEQ ID NO:526 | 865 | CBFB | Nucleus-binding factor, beta subunit |
| SG527 | SEQ ID NO:527 | 1051 | CEBPB | binding protein to CCAAT/enhancer (C/EBP), beta |
| SG529 | SEQ ID NO:529 | 7024 | TFCP2 | Transcription factor CP2 |
| SG530 | SEQ ID NO:530 | 1385 | CREB1 | CAMP response element binding protein |
| SG531 | SEQ ID NO:531 | 4782 | NFIC | I/C nuclear factor (CCAAT binding factor transcription factor) |
| SG532 | SEQ ID NO:532 | 2553 | GABPB2 | Transcription factor of the protein which binds to GA, beta 2 subunit (47kD) |
| SG533 | SEQ ID NO:533 | 1958 | EGR1 | Early growth response 1 |
| SG534 | SEQ ID NO:534 | 10661 | KLF1 | Factor similar to that of Kruppel 1 (erythroid) |
| SG535 | SEQ ID NO:535 | 1997 | ELF1 | Factor similar to E74 1 (transcription factor with ets domain) |
| SG536 | SEQ ID NO:536 | 2113 | ETS1 | homologue to the E26 oncogene of erythroblastosis virus v-ets 1 (avian) |
| SG537 | SEQ ID NO:537 | 2114 | ETS2 | homologue to the E26 oncogene of erythroblastosis virus v-ets 2 (avian) |
| SG538 | SEQ ID NO:538 | 2313 | FLI1 | Integration of Friend 1 leukemia virus |
| SG539 | SEQ ID NO:539 | 2625 | GATA3 | GATA 3 binding protein |
| SG540 | SEQ ID NO:540 | 862 | CBFA2T1 | Nucleus binding factor, runt domain, alpha 2 subunit; translocated to 1; related to cyclin D |
| SG541 | SEQ ID NO:541 | 3091 | HIF1A | Factor inducible by hypoxia 1, alpha subunit (basic transcription factor of helix-loops-helix) |
| SG542 | SEQ ID NO:542 | 6927 | TCF1 | Transcription factor 1, hepatic; LF-B1, hepatic nuclear factor (HNF1), proximal factor of albumin |
| SG543 | SEQ ID NO:543 | 3234 | HOXD8 | Homeotic box D8 |
| SG544 | SEQ ID NO:544 | 3235 | HOXD9 | Homeotic box D9 |
| SG545 | SEQ ID NO:545 | 9935 | MAFB | Family of the oncogene of musculoaponeurotic fibrosarcoma (avian) V-maf |
| SG546 | SEQ ID NO:546 | 7975 | MAFK | Family of the oncogene of musculoaponeurotic fibrosarcoma (avian) V-maf, protein K |
| SG547 | SEQ ID NO:547 | 8721 | EDF1 | factor related to edothelialdifferentiation 1 |
| SG548 | SEQ ID NO:548 | 2000 | ELF4 | Factor similar to E74 4 (transcription factor with ets domain) |
| SG549 | SEQ ID NO:549 | 7593 | ZNF42 | Protein with zinc fingers 142 (clone pHZ-49) |
| SG550 | SEQ ID NO:550 | 4763 | NF1 | neurofibromin 1 |
| SG551 | SEQ ID NO:551 | 4772 | NFATC1 | Nuclear factor of activated T-lymphocytes, cytoplasmic, calcineurin 1-dependent |
| SG552 | SEQ ID NO:552 | 5080 | PAX6 | Paired box gene 6 (aniridia, keratitis) |
| SG553 | SEQ ID NO:553 | 7849 | PAX8 | Paired box gene 8 |
| SG554 | SEQ ID NO:554 | 57026 | PLP | Pyridoxal (pyridoxine, vitamin B6) phosphatase |
| SG555 | SEQ ID NO:555 | 2274 | PLZF | With four LIM domains and average 2 |
| SG556 | SEQ ID NO:556 | 5950 | RBP4 | Retinol 4-binding protein, plasma |
| SG557 | SEQ ID NO:557 | 6095 | RORA | Orphan receptor related to RAR A |
| SG558 | SEQ ID NO:558 | 6667 | SP1 | Transcription factor Sp1 |
| SG559 | SEQ ID NO:559 | 6772 | STAT1 | Signal transducer and transcription activator 1, 91kDa |
| SG560 | SEQ ID NO:560 | 6908 | TBP | TATA box-binding protein |
| SG561 | SEQ ID NO:561 | 6932 | TCF7 | Transcription factor 7 (T lymphocyte specific, box HMG) |
| SG562 | SEQ ID NO:562 | 51513 | ETV7 | Gene variant of ets 7 (oncogene TEL2) |
| SG563 | SEQ ID NO:563 | 7535 | ZAP70 | Zeta chain associated kinase protein (TCR) (70 kD) |

From among these genes, four of them (ACTB, GAPD, 18S rRNA and 28S rRNA), do not have a special relation with neoplasias and were initially included in the microarray because, for a long time, it was believed that their expression remained constant and they were used when normalizing the microarray data: they are the type of genes alluded to when we speak of "constitutive" genes at other points in the specification. At present, it is not thought that there is a gene whose expression remains constant in any circumstance, for which reason, in the present study, the genes ACTB, GAPD, 18S rRNA and 28S rRNA have received the same treatment as the other genes of the microarray, except for the fact that the first two of them have been used as integrity controls, as described further on.

In Table 1 it can be observed that there are genes which are represented by more than one oligonucleotide. This is the case because the existence of two or more probes per gene can be used to measure the integrity of the synthesized cRNA. The genes for which more than one oligonucleotide have been designed to act as probe, each one of which hybridizes with a different sequence, are indicated below in Table 2.

**Table 2.- Genes represented by more than one oligonucleotide as probe**

| **Usual abbreviation of the gene** | **Probe1** | **Probe2** | **Probe3** |
|---|---|---|---|
| ABL1 | SG10 | SG180 | |
| BCR | SG169 | SG170 | |
| CBFB | SG189 | SG526 | |
| CD28 | SG403 | SG404 | |
| EIF4E | SG293 | SG305 | |
| ELF1 | SG512 | SG535 | SG502 |
| ETS2 | SG95 | SG537 | |
| GCET2 | SG504 | SG509 | |
| MAFB | SG258 | SG545 | |
| MTCP1 | SG358 | SG359 | |
| POU2F2 | SG366 | SG367 | |
| RGS1 | SG56 | SG409 | |
| S100A2 | SG35 | SG71 | |
| SNRPB | SG142 | SG143 | |
| STAT1 | SG77 | SG559 | SG468 |
| TIA-2 | SG7 | SG73 | |
| TAGLN2 | SG24 | SG476 | |
| TCF3 | SG277 | SG279 | |
| XRCC5 | SG32 | SG330 | |
| ZYX | SG97 | SG402 | |
| CD44 | SG228 | SG229 | |
| ACTB | SG463 | SG464 | |
| GAPD | SG464 | SG467 | |

### Establishment of control probes

To decrease the variability, a large number of controls were included in each microarray. These controls suppose an objective measurement on the process quality, and therefore, of the quality of the data obtained. They are of several types and origins:

### a) Probes used as integrity controls

These probes were 2 pairs of oligonucleotides complementary to ends 5' and 3' of the β-actin genes (probes code SG463 and SG464) and glyceraldehyde-3-phosphate dehydrogenase (probes code SG466 and SG467). The ratio between the intensities of the probe located at end 3' and 5' makes it possible to check the quality of the starting RNA and the functioning of the labelling reaction. The details on these oligonucleotides appear in Table 3.

**Table 3.- Oligonucleotides used as integrity controls**

| **Oligonucleotide** | **SEQ ID NO:** | Source gene | **Gene Abbreviation** | **GenID No.** |
|---|---|---|---|---|
| SG463 | SEQ ID NO:463 | β-actin | ACTB | 60 |
| SG464 | SEQ ID NO:464 | β-actin | ACTB | 60 |
| SG466 | SEQ ID NO:466 | Glyceraldehyde-3-phosphate dehydrogenase | GAPD | 2597 |
| SG467 | SEQ ID NO:467 | Glyceraldehyde-3-phosphate dehydrogenase | GAPD | 2597 |

### b) Probes used as negative controls

These probes are largely formed by a group of oligonucleotides of 50 nucleotides (50-mer) which are not complementary to any known human sequence. For them, the BLAST tool was applied to these probes and it was observed that they did not hybridize with any human sequence. They are identified with codes SC1 (SEQ ID NO:564), SC2 (SEQ ID NO:565), SC3 (SEQ ID NO:566), SC4 (SEQ ID NO:567), SC5 (SEQ ID NO:568), SC6 (SEQ ID NO:569) and SC7 (SEQ ID NO:570) and oligonucleotides SCN1 (SEQ ID NO:571), SCN5 (SEQ ID NO:575), SCN7 (SEQ ID NO:577) and SCN10 (SEQ ID NO:580) are also used as negative controls. They are used to determine the optimum conditions of hybridization, washing and developing of the chips or microarrays. The appearance of a signal associated to them indicates the existence of non-specific hybridization.

### c) Exogenous probes used as internal positive controls: "Spiked controls"

"Spiked controls" are synthetic oligonucleotides whose sequence coincides with a fragment of a transcript of a non-human gene or of any other sequence of nucleotides of low homology with transcripts of human genes which is polyadenylated at 3', which is used as positive control, in the determination of the process quality, in the normalization of data and for the establishment of the linear range of the process (Benes V *et al.,* 2003). To do this, the transcripts or corresponding polyadenylated sequences are added to the total starting RNA before starting the labelling process, and therefore, they suffer the same reactions (labelling, hybridization and developing) as the total RNA of the samples.

7 "Spiked controls" are used. To ensure low homology with human genes 5 transcripts of *Bacillus subtilis* genes (dap, thr, trp, phe and lys) and 2 transcripts of genes of the Sharkav virus are used, frequently referred to as "Plum poxvirus" (Sppv), which is a plant virus. The details on these oligonucleotides are shown below in Table 4. The ATCC (*American Type Culture Collection*) numbers which appear after the name of the source genes refer to the identification number in the ATCC of *E*. *coli* strains containing recombinant plasmids which contain the sequence of the genes from which the transcripts added to the RNA are obtained and which were also used for the design of the sequences of the corresponding oligonucleotides bound to the microarray.

**Table 4.- Oligonucleotides used as "Spiked Controls"**

| **Oligonucleotides** | **SEQ ID NO:** | Source gene | **GeneBank code** | **Transcript size (nt)** | **Concentration (pM) in the "spiked controls"** solution |
|---|---|---|---|---|---|
| SSPC1 | SEQ ID NO:584 | Dap (ATCC no.87486) | L38424 | 1820 | 2000 |
| SSPC2 | SEQ ID NO:585 | Lys (ATCC no.87482) | X17013 | 1000 | 1250 |
| SSPC3 | SEQ ID NO:586 | Thr (ATCC no.87484) | X04603 | 1980 | 5 |
| SSPC4 | SEQ ID NO:587 | Plum pox virus, isolated PENN2 (Sppv1) | AF401296 | | 100 |
| SSPC5 | SEQ ID NO:588 | Plum pox potyvirus, mRNA coated protein (Sppv2) | X57975 | | 750 |
| SSPC6 | SEQ ID NO:589 | Phe (ATCC no.87483) | M24537 | 1320 | 1000 |
| SSPC7 | SEQ ID NO:590 | Trp (ATCC no.87485) | K01391 | 2500 | 500 |

### c. 1.: Preparation of the 5 "Spiked controls" of Bacillus subtilis

The *E. coli* bacteria with the recombinant plasmids were acquired from ATCC (Rockville, MD. USA) The plasmids (pBluescript II-KS) contained the cloned cDNA of *a Bacillus subtilis* gene, with cut-off sites for the NotI enzymes at end 5' and BamHI at end 3' and a poly extension (dA) prior to the cut-off site for BamHI.

After reconstituting and allowing the cells to grow during the night at 37°C in LB + Ampicillin medium, the plasmid was obtained with the Midipreps kit (Jetstar) following the manufacturer's recommendations. 10 µg of each one of the plasmids was linearized by digestion with 30U of NotI restriction enzyme, in the presence of 1XNE3 and 1XBSA buffer during 3 hours at 37°C. The linearized plasmids were subjected to extraction with phenol:chloroform:isoamilic alcohol (Ambion), precipitation with 0.1 vol of 3M sodium acetate (Sigma) and 2.5 vol of 100% Ethanol and elimination of salts with 80% Ethanol, following the aforementioned protocol. The DNA obtained was resuspended in 10 µl of RNase-free water.

Next, the transcripts with sense were synthesized with *an in vitro* transcription reaction (I.V.T) from 1 µg of plasmid linearized using the MegaScript T3 kit (Ambion) and following the manufacturer's recommendations. The plasmids obtained were purified with the RNeasy Total RNA Isolation Kit (QIAGEN), following the manufacturer's recommendations.

The quantification, determination of the purity, quality and size of the transcripts obtained were performed following the same methods which are described below for the total RNA.

### c.2. Preparation of the 2 "Spiked controls" which represent SPPV genes

The recombinant plasmids (Progenika Biopharma) contained the cloned cDNA of the two sppv1 and sspv2 genes inserted between two PvuII and PstI restriction sites. End 3' of each insert contains a polyadenylation extension.

JM109 cells were transformed with the plasmids which contained the transcripts. The cells were left to grow in plates with LB + Ampicillin medium at 37°C, the colonies with the transferred cells were selected and they were grown in LB + AMP liquid medium.

The recovery of the plasmids was performed with the Midipreps Plasmid Purification kit (Qiagen), following the manufacturer's recommendations. 10 µg of each plasmid was linearized with 30U of the PvuII restriction enzyme. The insert was extracted with phenol:chloroform:isoamilic alcohol (Ambion), precipitation with 0.1 volumes of 7.5 M sodium acetate and 2.5 volumes of 100% ethanol. The salts were eliminated by two washings with 80% ethanol. The DNA obtained was resuspended in 10 µl of Rnase-free water.

Next, the transcripts with sense were synthesized with 1 µg of plasmid linearized using the T7 MegaScript kit (Ambion) and following the manufacturer's recommendations. The product of the reaction was cleaned with the RNeasy Total RNA Isolation Kit (Qiagen).

The quantification, measuring of the purity of the transcripts obtained and verification of their size were then performed

A solution of "Spiked controls" was prepared from the transcripts obtained with different concentrations of each one of those "spiked" (see Table 3), so that they covered the whole range of intensities of the "scanner" reader system (values of intensity which go from 0 to 65,535 in arbitrary units). This solution was added in the same quantity to 5 µg of total starting RNA from each sample before starting the process.

### c.3. Design of probes representative of each one of the transcripts:

So that the behaviour of the probes was as similar as possible to the probes designed for the genes to be studied, with the Oligo 6.0 programme (M.B.I), those sequences were selected for each "Spiked control" which complied with the same requirements established for the probes of the genes represented (length, GC content, "sense" strand and distance to end 3') and which did not form stable loops (energy less than -7 Kcal/mol). The BLAST tool was applied to the sequences which complied with those requirements and that with less homology with human sequences was chosen.

After depositing and immobilizing the probes corresponding to the "Spiked controls" on the glass, it was verified: a) that the probes did not hybridrize in non-specific manner with the samples to analyse, b) that all the probes had similar hybridization characteristics, and c) that the signal of intensity obtained from each one of them can be related to the quantity of transcript added to the RNA.

### d) Hybridization controls

Snthetic oligonucleotides of DNA with 70 nucleotides (70-mer) were used As hybridization controls, modified at one end with a biotin molecule. These molecules are added in the same quantity to the sample just before hybridization, so that their value only depends on the processes of hybridization, developing and capture of images of the microarray. For each one of these 70-mer oligonucleotides, on the microarray there are several copies of an oligonucleotide with 50 nucleotides in length (50-mer), complementary to the corresponding 70-mer oligonucleotide with which it must hybridize. The 50-mer oligonucleotides which form part of the microarray and which are complementary to 70-mer oligonucleotides which are added to the cRNA before hybridizing are of codes SCN2, SCN3, SCN6, SCN8, SCN11, SCN12 and SCN13. To ensure low homology with human sequences, the sequences of these oligonucleotides were obtained from sequences of *Arabidopsis thaliana* and *Tripanosoma brucei.* Their characteristics appear in Table 5

**Table 5.- Oligonucleotides used in the microarray as positive hybridization controls**

| **50-mer oligonucleotide present in the microarray** | **SEQ ID NO:** | **Source gene** | **GenBank code** | **Complementary 70-mer oligonucleotide** |
|---|---|---|---|---|
| SCN2 | SEQ ID NO:572 | Alpha-1.4-fucosyltransferase (FT4-M) from *Arabidopsis thaliana* | AY026941 | C2 |
| SCN3 | SEQ ID NO:573 | mRNA of the thioredoxine of *Tripanosoma brucei* | AJ239128 | C3 |
| SCN6 | SEQ ID NO:576 | mRNA from a supposed expression protein of the RBP (complete CDS) from *Arabidopsis thaliana* | AY051079 | C6 |
| SCN8 | SEQ ID NO:578 | mRNA from a supposed transfer protein of lipids (Atlg48750) (complete CDS) from *Arabidopsis thaliana* | AY045879 | C8 |
| SCN11 | SEQ ID NO:581 | mRNA from a supposed transfer protein of lipids (Atlg48750) (complete CDS) from *Arabidopsis thaliana* | AY045879 | C11 |
| SCN12 | SEQ ID NO:582 | mRNA from a supposed transfer protein of lipids (Atlg48750) (complete CDS) from *Arabidopsis thaliana* | AY045879 | C12 |
| SCN13 | SEQ ID NO:583 | mRNA of the papain-type cysteine endopeptidase XCP2 (complete CDS) from *Arabidopsis thaliana* | AF191028 | C13 |

For the design of the 50-mer oligonucleotides it was verified, in a manner similar to that previously described for the "Spiked controls", that the oligonucleotides to be used did not hybridize in non-specific form with the samples to be analysed, that all the probes had similar hybridization characteristics and that the signal of intensity obtained from each one of them could be related to the quantity of the corresponding 70-mer oligonucleotide added to the cRNA. This made it possible to take as valid the oligonucleotides indicated in Table 5. The SCN4 (SEQ ID NO:574) and SCN9 (SEQ ID NO:579) oligonucleotides, designed in principle to act as hybridization controls, were seen to produce specific hybridization when human cRNA hybridized, for which reason they also appear in the microarray, as if they were probes which represent a human gene, but they are not taken into account as positive hybridization controls. For their part, oligonucleotides SCN1 (SEQ ID NO:571), SCN5 (SEQ ID NO:575), SCN7 (SEQ ID NO:577) and SCN10 (SEQ ID NO:580), which did not hybridize either in non-specific form with the samples, are also present in the microarray as negative hybridization controls, as no oligonucleotide complementary thereto were added to the cRNA.

For its part, the hybridization controls solution, which contained the 70-mer oligonucleotides complementary to the 50-mer oligonucleotides present in the microarray as positive hybridization controls, was prepared from the corresponding biotinylated 70-mer sequences using a different concentration for each one of them, as shown in Table 6:

**Table 6.- Composition of the positive hybridization controls solution**

| **70-mer Oligonucleotide added to the cRNA** | **SEQ ID NO:** | **Complementary 50-mer oligonucleotide** | **Concentration (pM) in the hybridization control solution** |
|---|---|---|---|
| C2 | SEQ ID NO:591 | SCN2 | 750 |
| C3 | SEQ ID NO:592 | SCN3 | 250 |
| C6 | SEQ ID NO:593 | SCN6 | 1500 |
| C8 | SEQ ID NO:594 | SCN8 | 1250 |
| C11 | SEQ ID NO:595 | SCN11 | 2000 |
| C12 | SEQ ID NO.596 | SCN12 | 4500 |
| C13 | SEQ ID NO:597 | SCN13 | 2500 |

### Blanks

Dimethyl sulfoxide (DMSO) without any probe was used, as this is the solvent wherein the oligonucleotides are found at the time of being deposited on the surface of the microarray.

### Description of the microarray device

Twelve replicas of each probe were deposited in different localizations on the surface of a solid support (glass in similar form to a microscope slide) using Microgrid II Spoter (Biorobotics). The 12 replicas of each probe were distributed on the support at random: 6 in the upper area and 6 in the lower area. Aminosylanized glass (Coming) was used as solid support. The moisture and the temperature were controlled throughout the printing process.

The covalent binding of the probes to the solid supports was carried out by cross-linking by ultraviolet radiation using the "Stratalinker" apparatus (Stratagene).

The quality control of the production process of the microarrays was the following: a) In each production run a microarray was stained with ethydium bromide which made it possible to analyze the size and form of the points printed. b) Another array of each run was hybridized with an already hybridized cRNA, analysing the hybridization signal, the background noise and the reproducibility of the replicas.

The characteristics of the array are shown below in Table 7:

**Table 7.- Characteristics of the microarray**

| | |
|---|---|
| Number of genes represented | 538 |
| Length of the oligonucleotides | 25-55 mer |
| Strand analysed | Sense |
| Number of oligonucleotides per gene | 1 (except 21 genes which are represented by 2 or 3 different oligonucleotides) |
| Number of replicas of each oligonucleotide | 12 |
| Blank | DMSO |
| Integrity controls | 4 |
| Spiked controls (internal positive controls) | 7 |
| Positive hybridization controls | 9 |
| Negative controls | 11 |
| Total number of points | 8192 (32 areas x 16 x16) |
| Size of the microarray | 25 x 75 mm |
| Area spotted | 16.38 x 17.82 mm |
| Distance between points | x- y- axis 360µm |

### Treatment of the samples

### Cell cultures

Cultures of Jurkat cells (cell line from Leukemia T) and U937 (cell line from promonocytic leukemia) were centrifuged for 10 minutes at 1200 rpm and, after decanting the supernatant, the precipitate was resuspended in RNAlater (Ambion Inc) and it was stored at -80°C at the time of extraction of the RNA. The RNA was extracted with TRIzol (Gibco-BRL Carlbad, CA, USA) following the manufacturer's recommendations.

### Blood Samples

The blood samples were directly collected in PAXgene Blood RNA Tubes - PreAnalytix (Qiagen) tubes. 2.5 ml of blood were extracted in each tube and two tubes per individual. The tubes were inverted several times to allow the blood to mix with the stabilizing liquid which the tube contains, and they were stored at -20°C until the night before RNA extraction.

### Extraction of the total RNA

The tubes with the sample were incubated at ambient temperature during the night previous to the RNA extraction. The PAXgene Blood RNA kit (Qiagen) was used for the extraction following the manufacturer's recommendations, including the intermediate step of treatment with DNase (RNase-Free DNase Set, Quiagen) in column. The RNA of each extraction tube was eluted in 80 µl of BR5 buffer. The RNA of the two tubes which correspond to each patient was gathered in a single tube.

### Purification of the total RNA

To ensure that the RNA obtained is free from free from contaminants that can interfere in later labelling reactions, it was purified in the following way: 16 µl (0.1 vol) of 7.5 M sodium acetate (Sigma) and 400 µl (2.5 vol) of 100% ethanol were added to 160 µl of total RNA solution. The solution was mixed in a "vortex" stirrer and it was incubated for 1 hour at -20°C. After 20 minutes of centrifugation at 12,000xg at 4°C, the precipitate was washed twice with 500 µl of 80% ethanol and it was resuspended in 35 µl of Rnase-free water. The RNAs obtained were stored at -80°C until their later use.

### Quantification of the total RNA

The quantification of the total RNA was carried out by the measurement of the absorbance at 260 nm in a spectrophotometer (DU 65, Beckman Coulter). 2 µl of the total RNA solution were diluted in 98 µl of 1 mM Tris-HCl pH 7.5 and the concentration was estimated (µg/ml) taking into account that 1 Unit of Optical Density at 260 nm corresponds to a RNA concentration of 44 µg/ml.

### Determination of the purity and quality of the RNA

The degree of purity was established from the absorbance ratio A260/A280 (nucleic acid/ proteins), considering that the RNA is suitable, of "good quality", when the A260/A280 ratio is between 1.9 and 2.1.

The quality of the total RNA was determined by viewing the RNA after electrophoresis. 500 ng of total RNA were subjected to electrophoresis in 1% agarose gel (FMC) in TAE 1x buffer with BrEt (0.5 mg/ml), under a potential differenceof 100V for 25 minutes in AC electrophoresis cuvettes (BioRad). As marker of molecular weights, phage φ 29 digested with the BamH I restriction enzyme was used. The gels were viewed in a Gel Doc (BioRad) ultraviolet light transiluminator.

### Sample labelling

The choice of the strand with sense as probe limited the labelling strategy at those approximations which yield an antisense labelled product (complementary to the probe immobilized on the solid support).

### cRNA Labelling

This type of labelling was performed during the course of an amplification process which consists of the use for the synthesis of single-strand cDNA, of an oligo(dT) primer which contains a promoter for the polymerase RNA enzyme of the T7 phage, an enzyme which will be used in the sample amplifications step.
a.- cDNA synthesis: step wherein DNA (cDNA) complementary to the starting mRNA was synthesized. 5 µg of total RNA was incubated with 2 µl of the "Spiked controls" solution and 100 pmol of T7-(dT)24 (Genset Corp) primer in final volume of 12 µl during 10 minutes at 70°C in a thermoblock, the mixture was cooled on ice and 4 µl of 5X First Strand Buffer (Gibco BRL Life Technologies), 0.1M 2 µl DTT (Gibco BRL Life Technologies), 1 µl dNTP mix 10mM (Gibco BRL Life Technologies) and 1 µl of SuperScript II RNase H RT (200 OR/µl) (Gibco BRL Life Technologies) were added. After 1 hour of incubation in a bath equipped with a thermostat (Selecta) at 42°C, the reaction was cooled on ice.
b.- Double chain DNA synthesis (dsDNA): a double chain of DNA was synthesized from the cDNA synthesized in the previous step. To 20 µl of previous reaction were added 91 µl of Rnase-free water, 30 µl of "Second Strand Reaction buffer" (Gibco BRL Life Technologies), 3 µl 10mM dNTPs (Gibco BRL Life Technologies), 10 U E. coli DNA Ligase (Gibco BRL Life Technologies), 40 O *E*. *coli* DNA polymerase I (Gibco BRL Life Technologies), 2 U *E*. *coli* RNase H (Gibco BRL Life Technologies) in a final volume of 150 µl. The reaction was incubated in a thermoblock at 16°C for 2 hours. Next, 10U of T4 DNA Polymerase (Gibco BRL Life Technologies) were added and the mixture was incubated at 16°C for 5 minutes. To stop the reaction, 10 µl of 0.5 M EDTA were added.
c.- Purification of the dsDNA: To eliminate possible remains of reaction products which may interfere in later labelling reactions, the DNA obtained through phenol/chloroform extraction and later precipitation was purified. To 162 µl of previous reaction 162 µl of phenol: chloroform: isoamilic alcohol solution (25:24:1) (Ambion) were added. It was centrifuged for 2 min at 12,000xg in a centrifuge at ambient temperature, the upper aqueous phase was collected. To this upper phase 0.5 volumes of 7.5M ammonium acetate (Sigma Chemical) and 2.5 volumes of 100% ethanol cooled to -20°C) were added. After stirring with "vortex" to mix well the components and centrifugation for 20 minutes at 12000xg at ambient temperature, the supernatant was eliminated and the precipitate was washed twice with 80% ethanol. The DNA obtained was resuspended in 10 µl of RNase-free water and it was concentrated in a "Speed-Vac" concentrator to a volume of 2 µl. This DNase was stored at -20°C until its later use.
d.- Synthesis and labelling of the cRNA: This reaction was carried out in a volume of 20 µl and using the T7 Megascript kit (Ambion), following the manufacturer's instructions and incorporating nucleotides modified with biotin, Bio-11-CTP and Bio-11 UTP (Perkin Elmer) in non-modified nucleotide/modified nucleotide ratio of 1:3. The reaction was incubated during 5 h and 15 minutes in a bath with thermostat (Selecta) at 37°C, stirring the reaction every 45 minutes. After this incubation, 1 µl of DNase was added and it was incubated for 30 min at 37°C.
e.-Purification of the biotinylated cRNA: The biotinylated cRNA was purified with the RNeasy Total RNA Isolation Kit (Qiagen) following the manufacturer's instructions. The biotinylated cRNAs obtained were eluted in a volume of 80 µl and they were stored at -80°C until its later use.

The quantity, purity and quality of the cRNA obtained were determined following the same methods described for the total RNA.

The cRNA was stored at -80°C until its later use.

### Fragmentation of the biotinylated cRNA

10 µg of biotinylated cRNA were fragmented in the presence of 5x (200 mM Tris-acetate, pH 8.1, 500 mM HOAC, 150mM MgOAc) fragmentation buffer during 35 minutes at 94°C in a thermoblock. It was verified that the fragmentation reaction had been carried out by viewing 1 µl of fragmentation solution in electrophoresis on 1% agarose gel.

### Hybridization of the cRNA labelled with the probes of the microarray

In this step the labelled genetic material were placed in contact with the probes immobilized on the solid support.

10 µl of the hybridization control solution were added to the biotinylated and fragmented cRNA solution and the mixture was incubated for 3 min at 95°C to denature the possible secondary structures. After incubation, the mixture was immediately taken to ice to prevent the possible renaturing of the sample.

The hybridization was carried out for 6 hours at 42°C in the Ventana Discovery automatic hybridization station (Ventana Medical Systems). The hybridization and washing buffers were supplied by Ventana Medical System. The microarrays were automatically stained in the hybridization station with streptavidin conjugated with Cy3 (Amersham Biosciences) using the manufacturer's recommendations.

### Capture of images and quantification of the microarrays

After the hybridization and developing, the images of the microarrays were identified and analysed by the ScanArray 4000 confocal fluorescent scanner (Perkin Elmer) equipped with a laser for the green (543 nm to excite the fluorophore Cy3). The "software" used was ScanArray 3.1. The use of the computer programme QuantArray 3.0 (Perkin Elmer) provided the absolute values of the intensity of hybridization and background noise in accordance with the light emitted by the Cy3 in each probe in an Excel format.

### Data analysis: Preliminary processing

In first place, the value of the background noise were subtracted from the values of absolute intensity of all the oligonucleotides. To do this, the values of absolute intensity and the values of background noise, which the programme used to convert the signals of the fluorophore returns, automatically, were used for each one of the microarray points: the corresponding intensity value is obtained from the zone which has been defined as point and the value of the background noise is obtained from the zone situated around the point.

Next, the average level of hybridization intensity of each one of the oligonucleotides of the microarray was calculated from the trimmed mean of the intensities of the 12 replicas of each one of the oligonucleotides. To do this, before calculating the average, the upper and lower values of the distribution points of hybridization signals obtained with each one of the replicas of the same oligonucleotide have to be eliminated. The calculation was performed using the Excel programme from Microsoft and, specifically, the TRIMMEAN function thereof, wherein the "percentage" parameter was set at 0.2, which supposes fixing the percentage of values eliminated in 20% of the upper values and 20% of the lower values; the function rounds up the number of data points excluded to the closest multiple of 2.

In last place, and to be able to determined the validity of the hybridization, it is necessary that a series of established criteria are met: 1) the ratio between the average intensity and the average background of all the oligonucleotides of the chip is greater than 10; 2) the value of the average coefficient of variation (standard deviation of the replicas compared with the average of the replicas) of all the replicas of oligonucleotides of the chip should be less than 0.3; 3) the average value of the negative control should be less than 2.5 times the value of the DMSO medium; 4) a signal should be obtained both in the hybridization controls and in the exogenous internal positive controls (Spiked controls).

The data analysis was performed in R, version 1.9.1. R is a programming language wherein both classical and modem statistical techniques can be applied (R Developmental Core Team, 2004; http://www.R-project.org), which has a series of functions stored in packages for the handling, calculation and graphic representation of data (Venables *et al.,* 2004). There are hundreds of packages written by different authors for R, with special statistical functions or which permit the access and handling of data and are available for downloading from the websites of CRAN (http://cran.r-project.org/) or Bioconductor (http://www.bioconductor.org). In some specific cases, the SPSS commercial statistical analysis software was used (Chicago, USA).

### Examples

### EXAMPLE 1.- RESULTS OBTAINED ON USING THE MICROARRAY DEVICE WITH SAMPLES OF U937 VS JURKAT CELLS

In order to know if the device permits differentiating two cells lines hybridized in 10 microchips: 5 samples of biotinylated cRNAs synthesized following the optimized working protocol, obtained from RNA of U937 cells (cell line from promonocytic leukemia) and 5 samples of biotinylated cRNAs obtained from RNA of Jurkat cells (cell line from T Leukemia).

The initial steps of preliminary processing of the data and validation of the hybridization mentioned previously in the "Data analysis: Preliminary processing" section were carried out and then the data was normalized and filtered:
- *Data normalization.* The "variance stabilization normalization" method was used, available in the "vsn" package in R. There are different packages available on the Internet for R, with special statistical functions or which permit the access and processing of data and are available for downloading from CRAN (http://cran.r-project.org/) or Bioconductor (http://www.bioconductor.org)
- *Data filtering.* Two filtering operations have been carried out with the "Filterfun" function of the of the "Genefilter" filter in R. The genes which did not pass any of the two filters were not used in the data analysis. The filters carried out were:
   - Filtering to exclude genes with an intensity value close to the DMSO. This filter made it possible to work with genes with an intensity value minus average background noise greater than 550 arbitrary units (approximately 2 times the value of the DMSO).
   - Filtering to exclude genes with minimum intensity variation throughout the samples. Genes were worked with an interquartile range of normalized intensity throughout samples greater than 0.3.

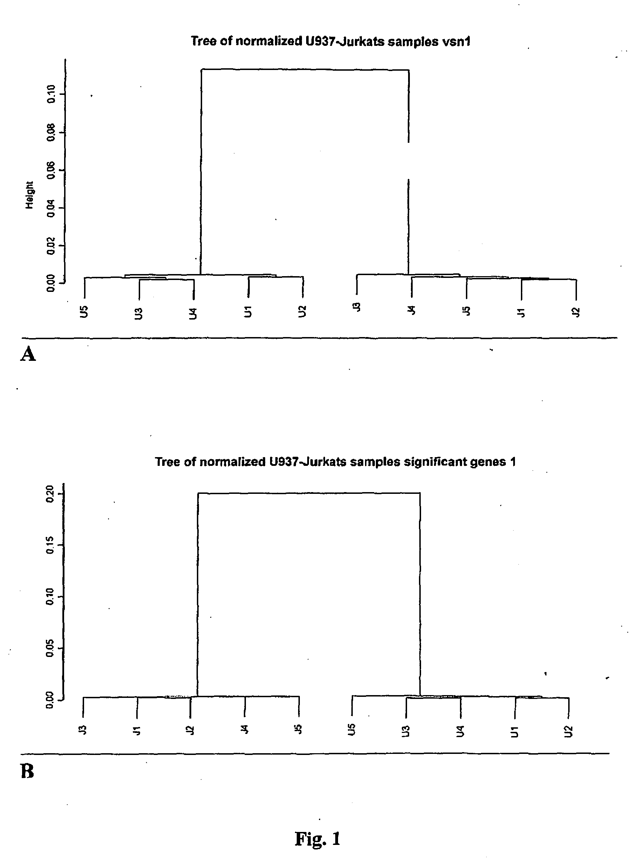

The data filtering left 83 probes which constituted the working list. With them a grouping was made of the non-supervised samples, which are those groupings wherein the structure of the data is not previously known, the system learning how the data are distributed among classes based on a distance function. A tree or hierarchical group was obtained with the grouping, wherein the samples are grouped in accordance with their similarity in the expression of certain genes, those corresponding to the oligonucleotides of the working list, so that the closest samples are those which have a similar expression profile. The grouping was performed with the *hclust* function of the *stats* package in R. The non-supervised analysis of the 10 samples produced their separation in two groups or main branches in accordance with the cell type whereto the samples belong: a group contains the 5 hybridizations carried out from U937 cells and the other group contains the 5 hybridizations carried out from Jurkat cells. The resulting tree of this non-supervised grouping is shown in part A of Figure 1.

Next, to find out if there were statistically significant differences between the two groups of samples, the "Step-down maxT multiple testing methods" method (maxT) was used, which is an application of the mt.maxT function of the multtest package of the software in R from Bioconductor, which applies a statistical test and carries out a strong control over the rate of false positives. To this function, the following should be provided:
a) Values on which one wants to apply the statistical tests, in this case, on the normalized values of the 83 oligonucleotides which passed the filters
b) Groups of which one wants to seek differences, in this case the 5 samples of Jurkat cells against the 5 samples of cells U937
c) Number of permutations one wants to perform. In this case, 100,000 permutations are carried out.
d) By default, Welch's test was chosen to specify the statistical tool to be used to test the hypothesis of non-association between the variables and the class labels.

The application of this analysis with a value of p<0.001 provided a list of 69 statistically significant probes between the two groups, which are the following:
SG12, SG20, SG23, SG24, SG38, SG39, SG45, SG49, SG53, SG59, SG60, SG62, SG76, SG78, SG89, SG92, SG94 , SG102, SG474, SG478, SG487, SG114, SG120, SG140, SG142, SG145, SG150, SG154, SG158, SG174, SG175, SG194, SG195, SG211, SG230, SG231, SG235 , SG260, SG264, SG266, SG268, SG270, SG272, SG282, SG294 , SG308, SG311, SG330, SG332, SG333, SG339, SG344, SG364, SG403, SG423, SG434, SG456, SG506, SG513, SG514, SG515, SG524, SG533, SG538, SG541, SG559

Once the statistically significant genes to distinguish between the two groups of samples are known (which would be the genes corresponding to the probes identified as statistically significant) the supervised grouping was carried out of the samples in accordance with the intensity of the signal of the 69 statistically significant probes obtained. The term "supervised", applied to a grouping, makes reference to the fact that the data structure is previously known, which makes it possible to use the prior information; with this, after a training process which allows the system to learn to distinguish between classes, it is possible to use the network to assign new members to the predefined classes. In this case, the supervised grouping of the samples in accordance with the intensity of the signal obtained with the 69 statistically significant probes obtained, is again a tree which is divided in two main branches in accordance with the cell type to which the samples belong. The tree obtained with the supervised grouping is shown in part B of Figure 1.

### EXAMPLE 2.- RESULTS OBTAINED ON USING THE "ARRAY" DEVICE WITH SAMPLES FROM HEALTHY SUBJECTS VS U937 AND JURKAT CELLS

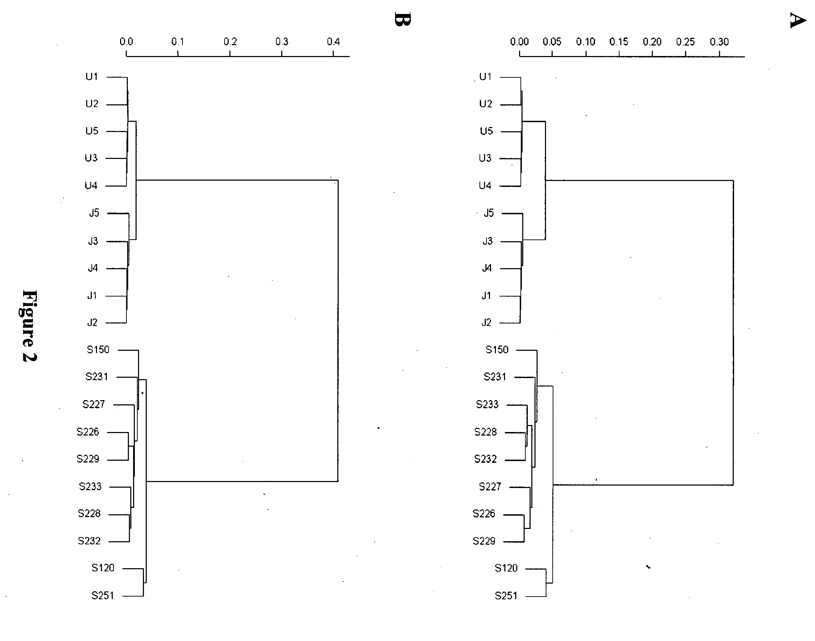

The expression of 5 samples of U937 cells and 5 samples of Jurkat cells was compared with the expression of 10 samples from total blood from healthy subjects. In a manner similar to that carried out in Example 1, the initial data processing steps, validation of the hybridizations, normalization and filtering were carried out. A total of 180 genes passed the filtering processes. The non-supervised grouping of the samples (carried out with the *hclust* function of the *stats* package of R applying Pearson's correlation) in accordance with the expression of the 180 genes, provided a tree with two main branches: one branch contains all the samples from cell cultures and the other branch contains all the samples from total blood from healthy subjects, which demonstrates that the tool is capable of finding expression differences. The tree obtained after making this non-supervised grouping is shown in part A of Figure 2.

The maxT test (p<0.001) to find genes with statistically significant differences between the samples from U937 and Jurkart cell cultures and the 10 samples from total blood of healthy subjects was performed. The statistical analysis provided a list of 131 probes with statistically significant differences between both groups of samples. They are the following:
SG1, SG4, SG7, SG8, SG10, SG13, SG15, SG16, SG17, SG18, SG19, SG20, SG26, SG29, SG30, SG34, SG36, SG39, SG42, SG44, SG49, SG51, SG52, SG58, SG64, SG65 , SG67, SG76, SG77, SG80, SG84, SG86, SG89, SG92, SG93, SG94, SG98, SG99, SG101, SG102, SG107, SG463, SG464 , SG474, SG475, SG485, SG487, SG466, SG467, SG471, SG472, SG473, SG120, SG129, SG138, SG141, SG144, SG145, SG147, SG158, SG163, SG164 , SG176, SG185, SG186, SG197, SG207, SG208, SG217, SG227, SG231, SG265, SG266, SG277, SG278, SG283, SG285, SG299, SG307, SG308, SG311,SG313, SG318, SG319, SG328, SG333, SG336, SG342, SG344, SG357, SG361, SG376, SG384, SG389, SG395, SG398, SG403, SG404, SG407, SG416, SG420, SG423, SG430, SG436, SG446, SG455, SG461, SG489, SG491, SG492, SG493, SG498, SG500, SG504, SG505, SG506, SG514, SG516, SG517, SG520, SG526, SG530, SG533, SG538, SG545, SG547, SG554, SG555, SG558.

The grouping of the 20 samples, in accordance with the expression of the statistically significant probes found, gave rise again to a tree with two main branches, one corresponding to the samples from cell cultures and another corresponding to the samples from healthy individuals. Said grouping appears in part B of figure 2.

### EXAMPLE 3.- RESULTS OBTAINED WITH SAMPLES FROM PATIENTS WITH CHRONIC LYMPHATIC LEUKEMIA (CLL) VS U937 AND JURKAT CELLS

The expression profiles were compared of samples from U937 and Jurkats cell cultures with 26 samples from total blood of subjects with CLL.

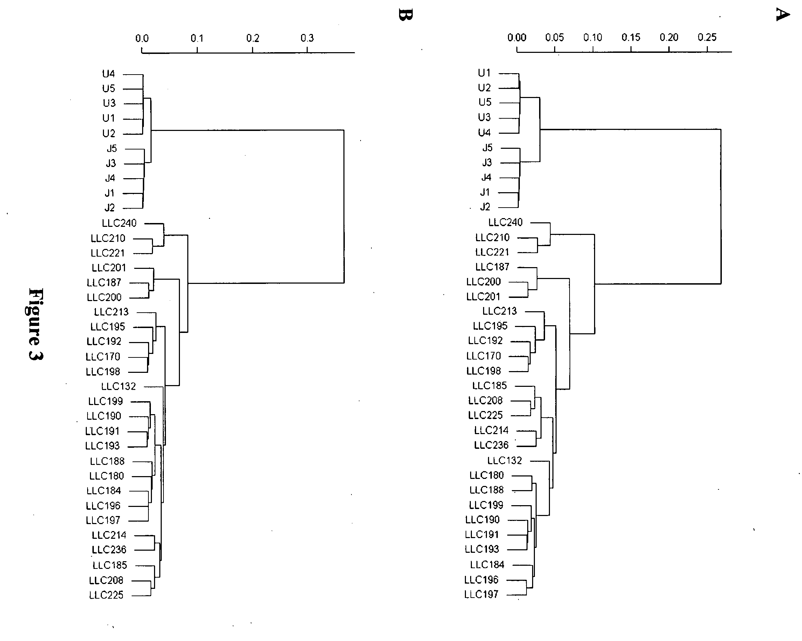

The samples underwent preliminary processing of the data, they were normalized and filtered in a manner analogous to those used in Examples 1 and 2 and a total of 236 probes passed through the filters. The non-supervised grouping of the samples in accordance with the expression of the probes which passed through the filters showed a tree with two main branches: one which contained the samples of cell cultures and the other the CLL samples. Said tree is shown in part A of figure 3.

The maxT test (p<0.001) to find genes with statistically significant differences between the two groups of samples was carried out. This analysis provided a list of 120 probes. They are the following:
SG2, SG4, SG8, SG10, SG13, SG15, SG16, SG19, SG20, SG23, SG26, SG28, SG31, SG34, SG36, SG39, SG48, SG58, SG60, SG65, SG76, SG77, SG84, SG89, SG94, SG9, SG97, SG99, SG102, SG106, SG107, SG463, SG464, SG474, SG475, SG481, SG465, SG485, SG487, SG466, SG467, SG471, SG473, SG115, SG116, SG117, SG120, SG129, SG134, SG135, SG138, SG139, SG141, SG145, SG158, SG161, SG163, SG176, SG178, SG185, SG207, SG208, SG210, SG217, SG227, SG231, SG237, SG264, SG272, SG277, SG281, SG283, SG286, SG294, SG298, SG299, SG307, SG308, SG319, SG328, SG330, SG333, SG336, SG342, SG344, SG345, SG347, SG361, SG384, SG389, SG395, SG404, SG407, SG416, SG423, SG428, SG430, SG432, SG434, SG444, SG446, SG453, SG458, SG459, SG491, SG498, SG507, SG508, SG511, SG517, SG518, SG522, SG526, SG530, SG533, SG538, SG541, SG554, SG558, SG561.

The grouping of the 30 samples in accordance with the expression of the 120 statistically significant probes found again gave rise to a tree with two main branches, one corresponding to the samples from cell cultures and another corresponding to the samples from healthy individuals. Said grouping appears in part B of figure 3.

### - EXAMPLE 4: RESULTS OBTAINED WITH SAMPLES FROM HEALTHY SUBJECTS VS PATIENTS WITH CHRONIC LYMPHATIC LEUKEMIA (CLL)

68 hybridizations which met the quality criteria from 68 samples of different healthy subjects and with clinical diagnosis of CLL were divided in 2 groups: Training Group used to obtain the functions of the classifier and Test Group, used to test the classifier obtained. The Training group was composed of 30 samples (10 from healthy subjects and 20 from CLL subjects) and the Test Group was composed of 38 samples (5 samples from healthy subjects and 33 samples from subjects with CLL).

To obtain the classification function, the results obtained from the hybridizations of the Training group were worked with. The steps carried out to obtain the classification function were:
- *Data normalization.* The "variance stabilization normalization" method, available in the "vsn" package in R, was used.
- *Data filtering.* Two filtering operations have been carried out with the "Filterfun" function of thee "Genefilter" package in R. The genes which did not pass any of the two filters were not used in the data analysis. From the 588 oligonucleotides of the chip, 224 passed through the 2 filters and constituted the working list.
   2. Filtering to exclude genes with an intensity value close to the DMSO. This filter made it possible to work with genes with an intensity value minus average background noise greater than 550 arbitrary units (approximately 2 times the value of the DMSO) in more than 25% of the 30 samples (7 samples) which compose the Training group.
   3. Filtering to exclude genes with minimum intensity variation throughout the samples. Genes were worked with which had an interquartile range of normalized intensity throughout samples greater than 0.3. Two classification systems are used:

### 4.1.- Construction of a classification system with PAM.

To identify groups of genes which best characterize each type of sample and verify the classification rate of these groups of genes Prediction Analysis for Microarrays (PAM) was used, available as "pamr^{a}" package in R. It is a statistical technique which identifies a group of genes which best characterizes a predefined class and uses this group of genes to predict the class whereto new samples belong. PAM uses a modified version of the "nearest centroids" classification method (Tibshirani *et al.,* 2002) called "Nearest Shrunken Centroids". A validation called "10 fold cross validation" was performed, which consists of constructing the model with 90% of the samples and an attempt is made to predict the class of 10% of the samples which have not intervened in the construction of the model. This method is repeated 10 times and the classification error of 10% of the samples is added to calculate the overall error. This error reflects the number of badly classified samples (Bullinger *et al.,* 2005).

*4.1.1. Construction of the model.* From the filtered and normalized data of the 30 samples which compose the Training group, attributing in an arbitrary form the Healthy Group to group 0 and the CLL Group to group 1, performing the 10 cross-validations and with a threshold value of Delta 3.1. The model obtained was formed by the following oligonucleotides: SG459, SG428, SG507, SG508, SG117, SG237. The coefficients of the classifier corresponding to each one of these oligonucleotides are shown below in Table 8:

**Table 8.- Coefficients of the PAM classifier**

| id | Value 0 | Value 1 |
|---|---|---|
| [1,] SG459 | -0.4344 | 0.2172 |
| [2,] SG428 | -0.146 | 0.073 |
| [3,] SG507 | -0.1111 | 0.0555 |
| [4,] SG508 | -0.1044 | 0.0522 |
| [5,] SG117 | -0.1003 | 0.0502 |
| [6,] SG237 | -0.0539 | 0.027 |

### 4.1.2. Validation of the PAM classifier. The cross-validation of the samples which compose the Training group correctly classified 28 of the 30 samples.

From the filtered and normalized data of the 38 samples which compose the Test Group, probability values p were obtained belonging to group 0 (healthy group) or group 1 (CLL group). The greater the value of p, the greater the probability of belonging to that group. It has been considered that the values greater than 0.5 indicate belonging to that group. The values of p obtained for each sample are indicated in Table 9.

**Table 9.- Probability values obtained with the PAM classifier for the Test Group**

| **Sample** | **p (Group 0)** | **p (Group 1)** | |
|---|---|---|---|
| S229 | 0.8031905 | 0.1968095 | →Only badly classified sample |
| S231 | 0.7403173 | 0.2596827 | |
| S232 | 0.8810574 | 0.1189426 | |
| S233 | 0.7973159 | 0.2026841 | |
| S251 | 0.8714224 | 0.1285776 | |
| CLL166 | 0.3764637 | 0.6235363 | |
| CLL184 | 0.1278230 | 0.8721770 | |
| CLL132 | 0.2081423 | 0.7918577 | |
| CLL210 | 0.3248082 | 0.6751918 | |
| CLL213 | 0.3536033 | 0.6463967 | |
| CLL214 | 0.2705277 | 0.7294723 | |
| CLL221 | 0.3650277 | 0.6349723 | |
| CLL208 | 0.2323872 | 0.7676128 | |
| CLL225 | 0.4034316 | 0.5965684 | |
| CLL236 | 0.4893545 | 0.5106455 | |
| CLL240 | 0.3807527 | 0.6192473 | |
| CLL168 | 0.1616066 | 0.8383934 | |
| CLL172 | 0.2002317 | 0.7997683 | |
| CLL174 | 0.1601147 | 0.8398853 | |
| CLL175 | 0.6009558 | 0.3990442: | |
| CLL177 | 0.1634185 | 0.8365815 | |
| CLL179 | 0.2300440 | 0.7699560 | |
| CLL181 | 0.2177406 | 0.7822594 | |
| CLL182 | 0.3450880 | 0.6549120 | |
| CLL 164 | 0.2590083 | 0.7409917 | |
| CLL159 | 0.3688586 | 0.6311414 | |
| CLL142R | 0.2111712 | 0.7888288 | |
| CLL105 | 0.2962797 | 0.7037203 | |
| CLL107 | 0.3764637 | 0.6235363 | |
| CLL109 | 0.3525788 | 0.6474212 | |
| CLL112 | 0.2059187 | 0.7940813 | |
| CLL151 | 0.2951067 | 0.7048933 | |
| CLL158 | 0.1932882 | 0.8067118 | |
| CLL169 | 0.3525937 | 0.6474063 | |
| CLL171 | 0.1495153 | 0.8504847 | |
| CLL178 | 0.2260191 | 0.7739809 | |
| CLL111 | 0.2951168 | 0.7048832 | |
| CLL155 | 0.2832151 | 0.7167849 | |

With this model 37 of the 38 samples of the Test Group are correctly classified: all the samples corresponding to healthy individuals (those whose name is headed by the letter "S") have a probability greater than 0.5 of belonging to group 0, whilst all the samples corresponding to individuals suffering from CLL (which are the samples whose name starts with letters "CLL") minus one have a probability greater than 0.5 of belonging to group 1.

### 4.2.- Construction of a classification system with logistical regression.

*4.2.1.-* *Selection of genes* with statistically significant differences among *healthy and CLL (Training group).* From the filtered and normalized data as has been previously described, the "Step-down maxT multiple testing methods" method (maxT) was used for the selection of genes with significant differences, which is an application of the mt.maxT function of the multtest package of the software in R from Bioconductor, which applies a statistical test and carries out a strong control over the rate of false positives. The application of this statistical test, with a value of p<0.001, to the 224 oligonucleotides which passed through the filters, produced a list of 7 oligonucleotides: SG117, SG428, SG459, SG461, SG493, SG507, SG508.

The steps used to obtain the list of 7 significant genes among healthy and CLL were:
Method which makes permutations and adjusts the values of p resT<-mt.maxT(exprs(224 oligonucleotides which have passed through the filters and normalized of the training group, Types of samples in the training group, test="t",B=100000): mt.maxT function which through permutations adjusts the probability values (signification) which entails a strong control of the rate of false positives.

To this function the following should be provided:
1. Values on which one wants to apply the statistical tests, in this case, on the normalized values of the 83 oligonucleotides which passed through the filters
2. Groups of which one wants to seek differences, in this case the 5 samples of Jurkat cells against the 5 samples of cells U937
3. Number of permutations one wants to perform. In this case, 100,000 permutations are carried out.
4. By default, Welch's test was chosen as statistical test.

The statistically significant genes at a level of p<0.001 were selected by this test and a number of 7 was obtained.

*4.2.2.- Obtainment of the classification function with SPSS.* By logistical regression from the normalized values of the 7 statistically significant oligonucleotides obtained from the 30 samples which compose the Training group and assigning in arbitrary manner group 0 to the healthy samples and group 1 to the CLL samples, the values of the classification function were obtained. The coefficients corresponding to each oligonucleotide were those which are shown below in Table 10:

**Table 10.- Coefficients of the classification function Calculated by logistical regression**

| Oligonucleotide | Coefficients (Coeff) |
|---|---|
| SG117 | 2.44756372 |
| SG428 | 7.38657611 |
| SG459 | 23.1465464 |
| SG461 | 43.6287742 |
| SG493 | -19.3978182 |
| SG507 | -2.80282646 |
| SG508 | 49.5345672 |
| Constant | -719.241486 |

From these coefficients, for each sample i a value xᵢ is calculated as follows: ${x}_{i} = Constant + ( Coeff ohle ⁢ 0009 * {Imn}_{i} DG ⁢ 117 ) + ( Coeff SG ⁢ 428 * { Imn}_{i} SG ⁢ 428 ) + ( Coeff SG ⁢ 459 * { Imn}_{i} SG ⁢ 459 ) + ( Coeff SG ⁢ 461 * { Imn}_{i} SG ⁢ 461 ) + ( Coeff SG ⁢ 493 * { Imn}_{i} SG ⁢ 493 ) + ( Coeff SG ⁢ 507 * {Imn}_{i} SG ⁢ 507 ) + ( Coeff SG ⁢ 508 * { Imn}_{i} SG ⁢ 508 ) .$
where Imnᵢ is the average value of normalized intensity of the sample i.

From the value xᵢ a value of probability (pᵢ) is calculated. The closer the value of p is to 0, the greater the probability of belonging to the group of healthy subjects (assigned as group 0) and the closer the value of p is to 1, the greater the probability there is of the sample belonging to the group of CLL subjects (assigned as group 1). The formula used to determine the value of p is: ${p}_{i} = 1 / \left(1+{e}^{xi}\right) .$

As is shown in Table 11, the function obtained correctly classified the 30 samples belonging to the training group. The closer to 0, the greater the probability that it is healthy and the closer to 1 the greater probability of CLL.

**Table 11:**

| **Classification table ^{a}** | | | | | |
|---|---|---|---|---|---|
| Observed | | | Prognosis | | |
| | | | EVOL | | Correct percentage |
| | | | 0 | 1 | |
| Step 1 | EVOL | 0 | 10 | 0 | 100.0 |
| | | 1 | 0 | 20 | 100.0 |
| | Overall percentage | | | | 100.0 |

| | | | | | |
|---|---|---|---|---|---|
| a. The cut-off value is ,500 | | | | | |

*4.2.3. Validation of the system classifier.-* From the filtered and normalized filters as detailed above, the Imnᵢ values were obtained of the 7 oligonucleotides which compose the classifier of each one of the 38 samples which compose the Test Group.

Results of the validation of the system classifier. Below, tables are shown wherein the Imnᵢ value is obtained of each one of the 7 oligonucleotides included in the classifier and the values of xᵢ and pᵢ calculated according to the formulas previously described, obtained for each one of the 38 samples of the Test Group. The samples which begin with S correspond to healthy subjects and the samples which start with CLL are from CLL subjects. 37 out of 38 samples are correctly classified. Only sample CLL175, for which a value of pi =0 is obtained is incorrectly classified.

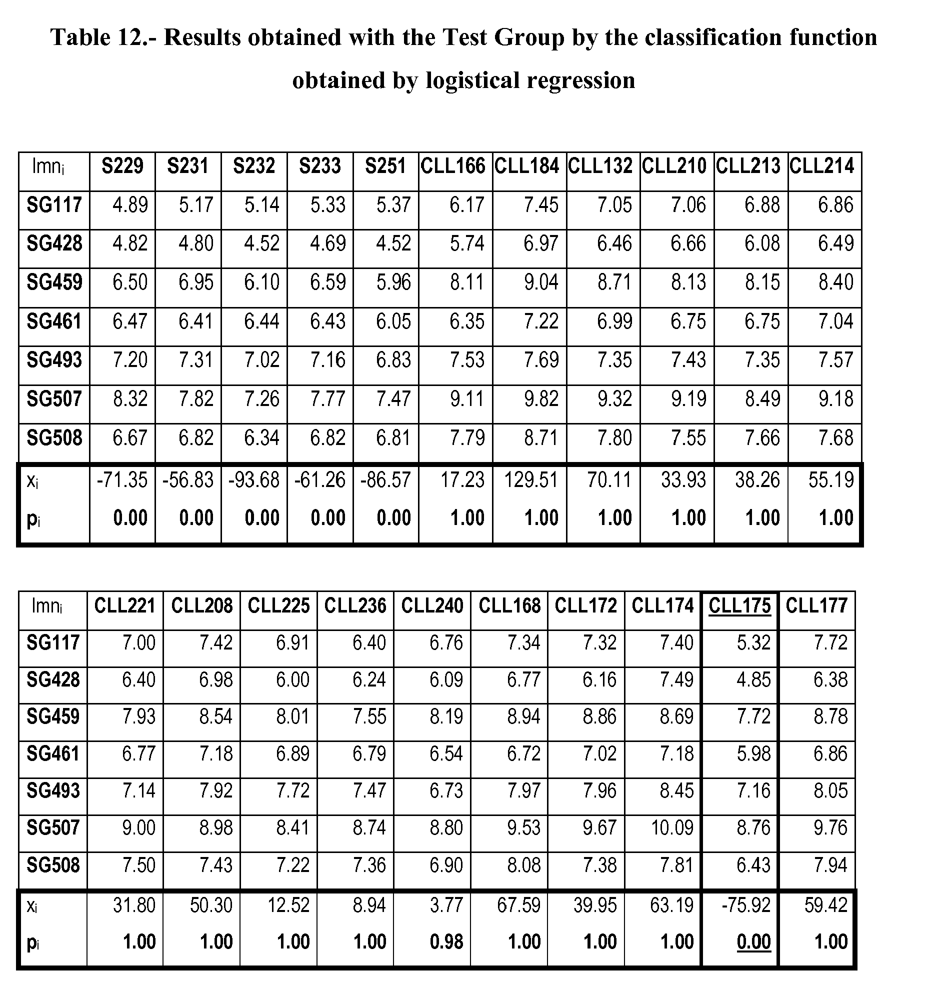

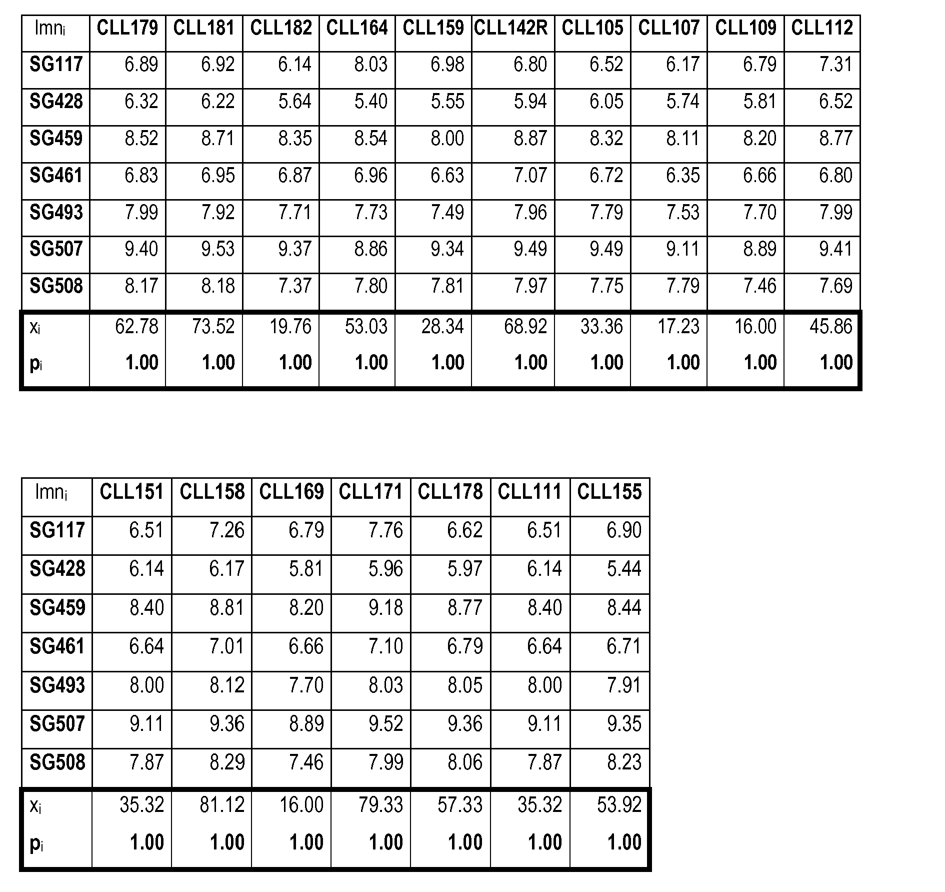

A third group of 40 samples was formed. To do this, replicas of hybridization or of labelling were used (the samples whose name begins with S and Strans are samples from people considered healthy and those which start with CLL are samples from patients with chronic lymphatic leukemia). This group of samples was used to validate the classification system. The data were normalized as has been previously described. The results of the classification are shown in the Table 13. 40 out of the 40 samples are correctly classified.

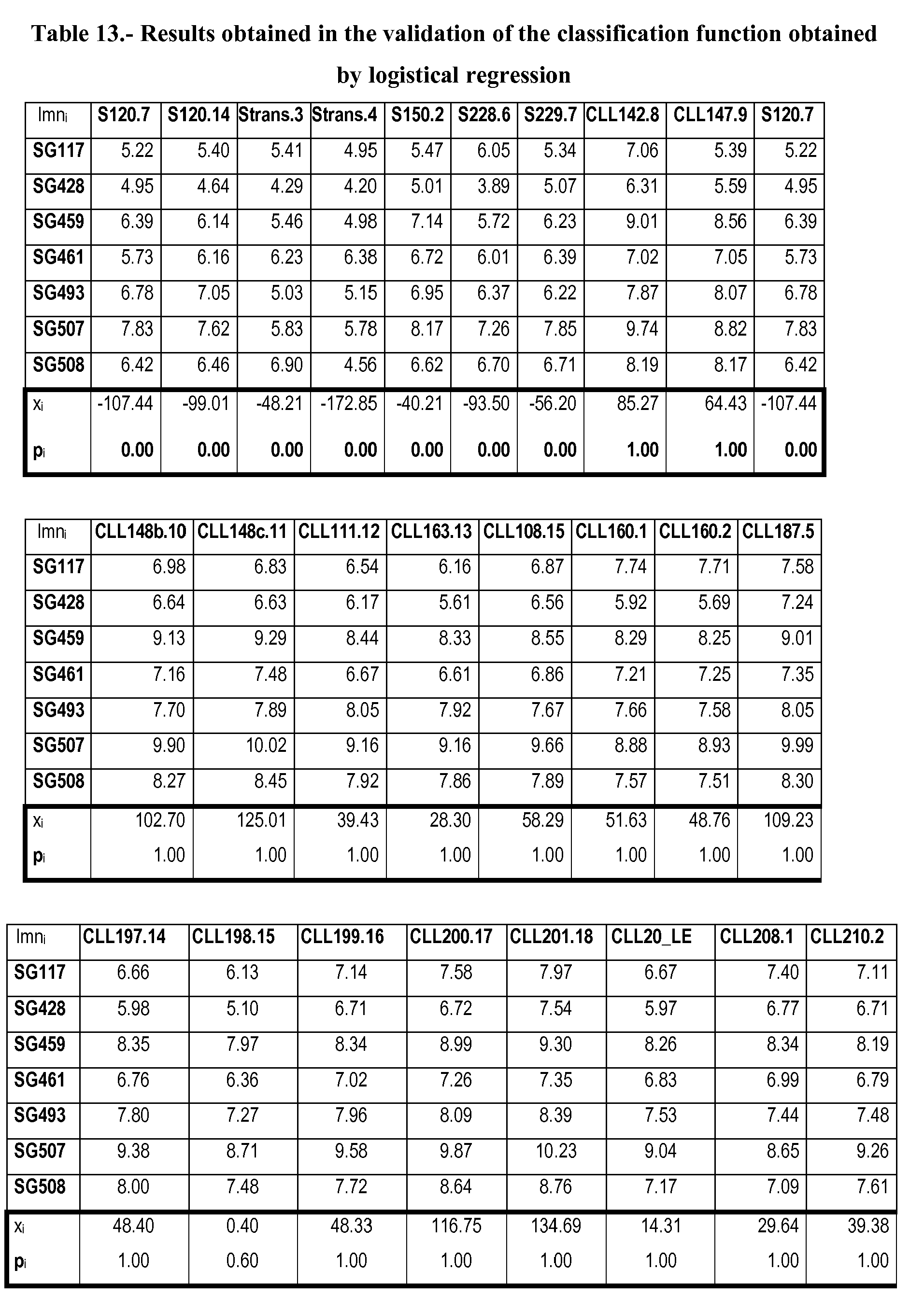

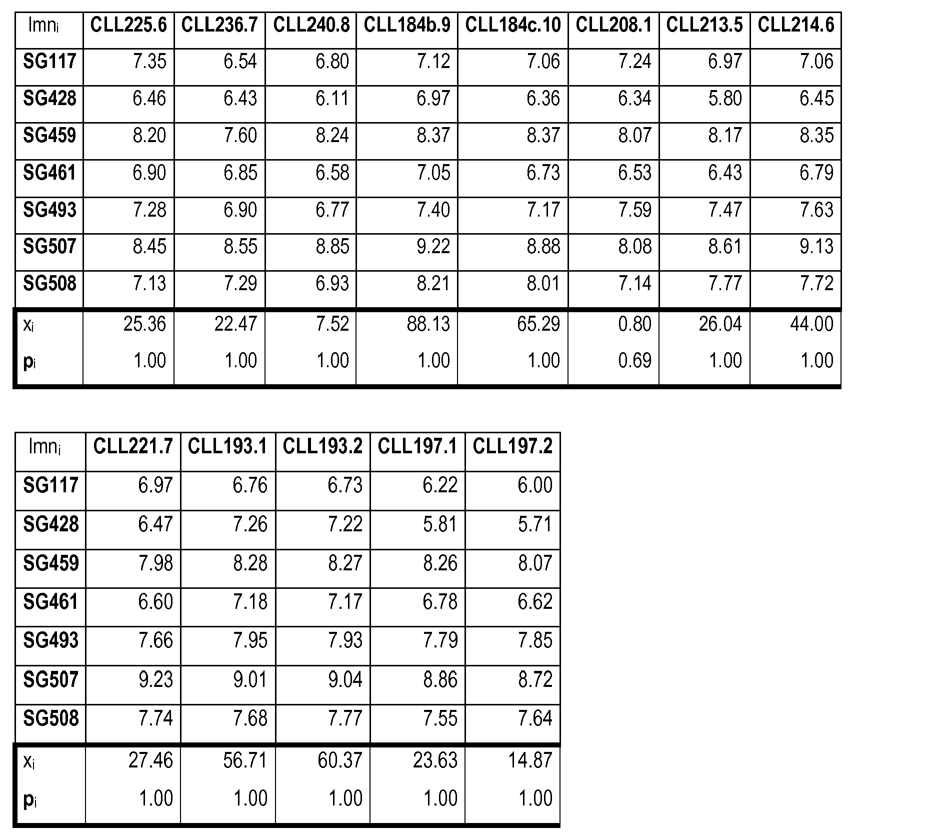

### EXAMPLE 5: RESULTS OBTAINED WITH "STABLE" CLL SAMPLES COMPARED WITH "PROGRESSIVE" CLL SAMPLES

"CLL-stable type" (S) samples are considered those of patients who have had stable CLL for over 5 years and "CLL-progressive type" (P) samples are considered the samples of patients classified as stable at the time of diagnosis and whose disease has progressed in less than one year.

In total 6 S samples and 6 P samples were analysed. The 12 samples were collected at the time of diagnosis, without clinical differences between them, but after one year, 6 of those patients had progressed. The 12 hybridizations have passed the aforementioned quality criteria.
Stable samples: E142R, E148, E156, E163, E164, E193
Progressive samples: P111, P105, P177, P158, P157 and P197.
All the data analysis was performed in R version 1.9.1.

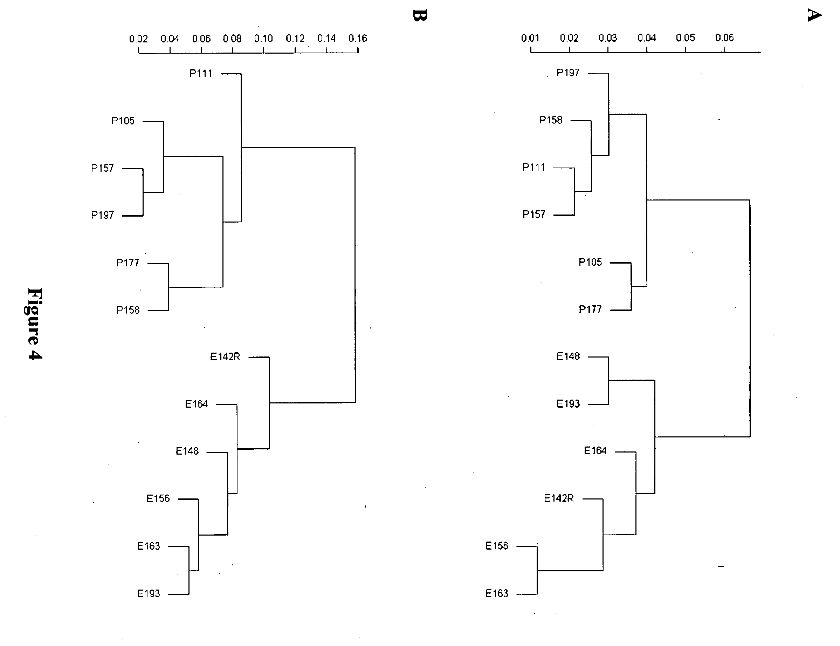

*Data normalization.* In this case, and to avoid the significant genes obtained are due to a real difference between samples and not to the effect of normalization, the data were normalized in two different forms ("variance stabilization normalization" (vsn) and by robust quantiles) and the same statistical analysis was performed with each one of the normalizations.
- Statistical analysis with normalized data by "variance stabilization normalization". The list of statistically significant genes was obtained from a Welch's test with the mt.maxT function of the multtest package in R, with a value of p<0.05 without adjusting, i.e. without performing any control on the false positives and produced a list of 29 genes with statistically significant differences between the CLL-stable type and CLL-progressive type groups.
   The statistically significant oligonucleotides obtained were:
   SG26, SG31, SG70, SG98, SG177, SG194, SG195, SG208, SG213, SG216, SG272, SG293, SG301, SG309, SG321, SG333, SG343, SG352, SG357, SG366, SG368, SG405, SG426, SG439, SG447, SG452, SG521, SG555, SG556.
   The samples were grouped, which was performed with the *hclust* function of the *stats* package in R applying Pearson correlations. The tree obtained is shown in part A of figure 4.
   The hierarchical grouping of the 12 samples in accordance with the expression of the 29 statistically significant genes obtained grouped the samples correctly: the tree contains two large branches, of which the right branch contains the 6 stable samples and the left branch contains the 6 progressive samples.
- Statistical analysis with normalized data by robust quantiles The list of statistically significant genes was obtained from a Welch's test with the mt.maxT function of the multtest package in R with the values of p without adjusting i.e. without exerting any control over the rate of false positives, with a value of p<0.05, and produced a list of 19 genes with statistically significant differences between the CLL-stable type and CLL-progressive type groups:
   SG26, SG31, SG177, SG194, SG195, SG197, SG213, SG216, SG293, SG301, SG309, SG333, SG343, SG357, SG366, SG439, SG452, SG555, SG556.

The supervised grouping of the 12 samples in accordance with the expression of the 19 statistically significant genes obtained gave rise to the tree which appears in part B of figure 4, wherein the samples also appear correctly grouped.

18 oligonucleotides common to both lists of statistically significant genes were selected and the average intensity of each one of them in the group of stable samples and in the group of progressive samples was calculated, as well as the variation in average intensity between the stable and progressive groups. The values obtained are shown in Table 14.

**Table 14.- Values corresponding to the intensity of 18 significant oligonucleotides to distinguish between CLL-stable and CLL-progressive**

| Probe | Significance (p data vsn) | Stable CLL group | | Progressive CLL group | | Change stable/ progressive |
|---|---|---|---|---|---|---|
| | | Average Intensity | SD | Average Intensity | SD | |
| SG177 | 0.001 | 14 | 1.71 | 21 | 4.84 | 0.7 |
| **SG366** | **0.001** | **18** | **2.33** | **14** | **1.80** | **1.3** |
| SG309 | 0.004 | 20 | 2.76 | 15 | 3.58 | 1.4 |
| **SG26** | **0.005** | **97** | **19.20** | **70,** | **13.24** | **1.4** |
| SG452 | 0.010 | 16 | 2.19 | 12 | 3.08 | 1.3 |
| **SG216** | **0.012** | **46** | **14.64** | **31** | **7.13** | **1.5** |
| **SG333** | **0.013** | **36** | **7.28** | **53** | **16.25** | **0.7** |
| **SG357** | **0.014** | **134** | **6.50** | **175** | **38.67** | **0.8** |
| SG213 | 0.014 | 26 | 5.51 | 41 | 17.20 | 0.6 |
| SG31 | 0.014 | 69 | 32.50 | 30 | 10.57 | 1.8 |
| SG301 | 0.014 | 21 | 5.02 | 16 | 3.10 | 1.4 |
| SG194 | 0.019 | 37 | 9.52 | 50 | 9.95 | 0.7 |
| SG456 | 0.022 | 11 | 2.06 | 14 | 2.08 | 0.8 |
| SG293 | 0.029 | 17 | 1.88 | 21 | 3.72 | 0.8 |
| SG343 | 0.033 | 27 | 7.43 | 21 | 1.61 | 1.3 |
| SG439 | 0.038 | 18 | 2.00 | 20 | 1.74 | 0.9 |
| SG195 | 0.041 | 21 | 3.56 | 25, | 4.60 | 0.8 |
| SG555 | 0.049 | 163 | 23.55 | 137 | 20.69 | 1.2 |

To validate the results obtained with the microarray, 5 of the common statistically significant probes were selected obtained on comparing expression data from stable CLL subjects compared to progressive CLL subjects and the expression was studied with RT-PCR of the genes represented by those probes. The criteria used to select the 5 probes were: hybridization intensity, change of intensity between groups of stable and progressive and value of statistical significance. In this way, 5 probes were selected which represent genes PSMB4, CD23A, LCP1, ABCC5 and POU2F2. The expression of these 5 genes was determined in11 of the 12 CLL type samples, as there was no total RNA of sample 105. With the expression value of the genes in each sample, the rate of change was determined between the group of stable and progressive and the value of significance of that variation and it was compared with the results obtained with the microarrays.

The technique used for the validation was RT-PCR or PCR in real time using a LightCycler. This technique is the technique of choice to validate data chips and as with the microarrays, measures mRNA level.

Primers were designed for each one of the 5 genes whose representative oligonucleotide was selected. The details thereof are shown below in Table 15.

**Table 15.- Primers and amplification products of the genes selected for their validation by RT-PCR**

| **Gene** | **Primer sequences (5'-3')** | **SEQ ID NO**: | **Amplified product size** | **Tₘ** |
|---|---|---|---|---|
| PSMB4 | Direct: PSMB4_F TTCTGGGAGATGGACACAGCTATA | SEQ ID NO: 598 | 95pb | 81°C |
| | Inverse: PSMB4_R CCACAAAGGGTTCATCTTCGA | SEQ ID NO: 599 | | |
| CD23A | Direct: CD23A_F TGCCCTGAAAAGTGGATCAAT | SEQ ID NO: 600 | 97pb | 82°C |
| | Reverse: CD23A_R CCATGTCGTCACAGGCATACC | SEQ ID NO: 601 | | |
| LCP1 | Direct: LCP1_F CCAGGTACCCTTCTCGCTTTT | SEQ ID NO: 602 | 126pb | 77°C |
| | Reverse: LCP1_R CTCCTGGCCCTCATCTTGAA | SEQ ID NO: 603 | | |
| ABCC5 | Direct: ABCC5_F CCCTCAAAGTCTGCAACTTTAAGC | SEQ ID NO: 604 | 119pb | 82°C |
| | Reverse: ABCC5_R ACACACCAAACCACACAGCAA | SEQ ID NO: 605 | | |
| POU2F2 | Direct: POU2F2_F GAGGACCAGCATCGAGACAAA | SEQ ID NO: 606 | 136pb | 82°C |
| | Reverse: POU2F2_R AACCAGACGCGGATCACTTC | SEQ ID NO: 607 | | |

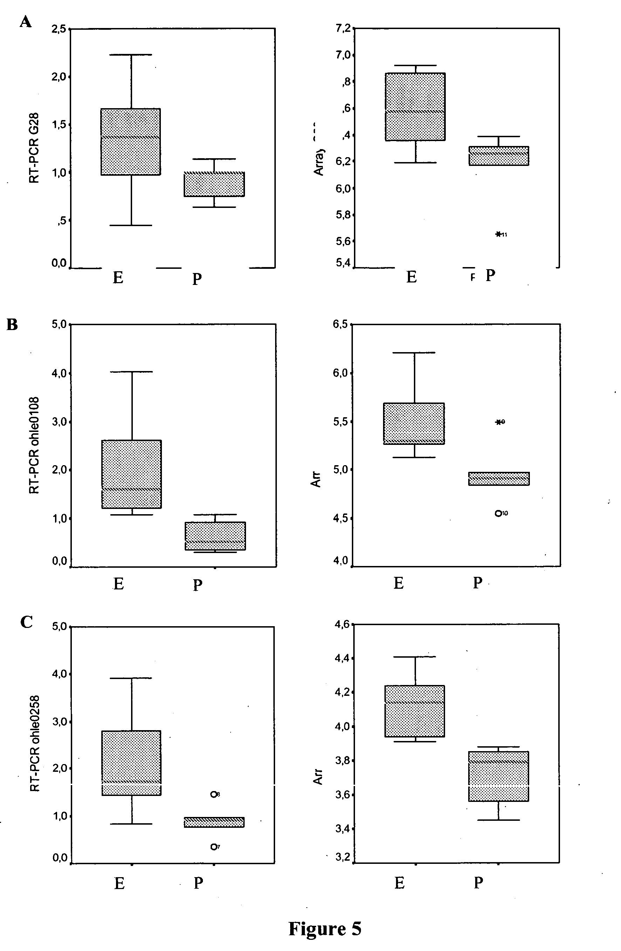

Figure 5 shows the distribution of the expression data obtained by RT-PCR (left graphic) and by the microarray (right graphic). Part A corresponds to gene PSMB4, part B to gene CD23A and part C to gene POU2F2.

Below, in Table 16, the results obtained with the microarray and with RT-PCR are obtained of the change values of the 5 genes selected in thr group of stable samples compared with the group of progressive samples obtained as significance of the change. In 3 of the 5 genes selected (PSMB4, CD23A and POU2F2) the values of change, the direction of the change and the significance values obtained with RT-PCR agree with those obtained with the microarray, for which reason those 3 genes are considered valid, i.e. the results obtained for those 3 genes with the microarray coincide with the results obtained by another techniques which also measures mRNA level.

**Table 16.- Values of change and significance of the change obtained with the microarray and by RT-PCR**

| | | Stable/progressive change | | Significance of the change | |
|---|---|---|---|---|---|
| Probes | Genes | Array | RT-PCR | Array | RT-PCR |
| **SG26** | **PSBM4** | **1.3** | **1.5** | **0.04** | **0.15** |
| **SG216** | **CD23A** | **1.5** | **3.2** | **0.04** | **0.03** |
| SG333 | LCP1 | 0.7 | 1 | 0.05 | 0.97 |
| SG357 | ABCC5 | 0.8 | 1.3 | 0.10 | 0.28 |
| **SG366** | **POU2F2** | **1.3** | **2.3** | **0.01** | **0.05** |

### BIBLIOGRAPHIC REFERENCES

Alizadeh A, Eisen M, Davis RE, et al The lymphochip: a specialized cDNA microarray for the genomic-scale analysis of gene expression in normal and malignant lymphocytes. Cold Spring Harb Symp Quant Biol. 1999;64:71-8.

Alizadeh AA, Eisen MB, Davis RE, et al Distinct types of diffuse large B-cell lymphoma identified by gene expression profiling. Nature. 2000;403:503-11.

Arico M, Valsecchi MG, Camitta B, Schrappe M, Chessells J, Baruchel A, Gaynon P, Silverman L, Janka-Schaub G, Kamps W, Pui CH, Masera G. Outcome of treatment in children with Philadelphia chromosome-positive acute lymphoblastic leukemia N Engl J Med. 2000;342:998-1006.).

Bea S, Zettl A, Wright G,et al. Diffuse Large B-Cell Lymphoma Subgroups Have Distinct Genetic Profiles that Influence Tumor Biology and Improve Gene Expression-Based Survival Prediction. Blood. 2005 04:-1399

Bene MC, Bernier M, Casasnovas RO, et al: Acute myeloid leukaemia M0: Haematological, immunophenotypic and cytogenetic characteristics and their prognostic significance: An analysis in 241 patients. Br J Haemat 113:737, 2001.

Benes V, Muckenthaler M. Standardization of protocols in cDNA microarray analysis. Trends Biochem Sci. 2003;28:244-9

Bennett JM, Catovsky D, Daniel MT, et al. Myelodysplastic syndromes: is another classification necessary? Br J Haematol. 1984;56:515-7.

Bennett JM, Catovsky D, Daniel MT, et al. Proposals for the classification of the acute leukaemias. French-American-British (FAB) co-operative group. Br J Haematol. 1976 ;33:451-8.

Bergh G, Ehinger M, Olsson I, Jacobsen SE, Gullberg U. Involvement of the retinoblastoma protein in monocytic and neutrophilic lineage commitment of human bone marrow progenitor cells Blood. 1999;94:1971-8

Bernard J, Levy JP, Varet B. Hematologie. Collection Medico Chirurgicale. Paris: Flammarion, 1976; 5-22

Binet JL, Auquier A, Dighiero G, et al.. A new prognostic classification of chronic lymphocytic leukemia derived from a multivariate survival analysis. Cancer. 1981;48:198-206

Boultwood J, Lewis S, Wainscoat JS The 5q-syndrome. Blood. 1994 ;84:3253-60

Braziel RM, Shipp MA, Feldman AL,et al. Molecular diagnostics. Hematology (Am Soc Hematol Educ Program). 2003;279-93

Bullinger, Döhner et al. Use of gene-expression profiling to identify prognostic subclasses in adult acute myeloid leukemia N Engl Med 2005;350:1605-16

Chan WC.Gene expression profiling in lymphoma diagnosis and research. Croat Med J. 2005; 46:349-59

Chen CC, Andrich MP, Metcalfe DD: A retrospective analysis of bone scan abnormalities in mastocytosis: Correlation with disease category and prognosis. J Nucl Med 35:1471, 1994

Cossman J: Gene expression analysis of single neoplastic cells and the pathogenesis of Hodgkin's lymphoma. J Histochem Cytochem 2001; 49:799-800

Crespo M, Bosch F, Villamor N, et al. ZAP-70 expression as a surrogate for immunoglobulin-variable-region mutations in chronic lymphocytic leukemia. N Engl J Med. 2003;348:1764-75

Datta SR, Brunet A, Greenberg ME. Cellular survival: A play in three acts. Genes Dev 1999; 13: 2905-2927

Devilard E, Bertucci F, Tremat P, et al: Gene expression profiling defines molecular subtypes of classical Hodgkin's lymphoma. Oncogene 2002; 21:3095-3102

Dohner K, Tobis K, Ulrich R, Frohling S, Benner A, Schlenk RF, Dohner H. Prognostic significance of partial tandem duplications of the MLL gene in adult patients 16 to 60 years old with acute myeloid leukemia and normal cytogenetics: a study of the Acute Myeloid Leukemia Study Group Ulm. J Clin Oncol. 2002;20:3254-61

Domen J, Weissman IL. Self-renewal, differentiation or death: regulation and manipulation of hematopoietic stem cell fate. Mol Med Today. 1999;5:201-8

Durig J, Naschar M, Schmucker U, Renzing-Kohler K, Holter T, Huttmann A, Duhrsen U. CD38 expression is an important prognostic marker in chronic lymphocytic leukaemia. Leukemia. 2002 ;16:30-5

Ferrando AA, Look AT Pathobiology of acute lymphoblastic leukemia. In Hoffman R Hematology Basic Principles and practice. 4 ed. Churchill Livingstone New York 2005; 1135-1147

Galton DA. The pathogenesis of chronic lymphocytic leukemia Can Med Assoc J. 1966 ;94:1005-10

Giles FJ, Keating A, Goldstone AH, Avivi I, Willman CL, Kantarjian HM Acute myeloid leukemia. Hematology (Am Soc Hematol Educ Program). 2002;73-110.).

Golub TR, Slonim DK, Tamayo P, et al. Molecular classification of cancer: class discovery and class prediction by gene expression monitoring. Science. 1999;286:531-7

Gong JZ, Lagoo AS, Peters D, Horvatinovich J, Benz P, Buckley PJ. Value of CD23 determination by flow cytometry in differentiating mantle cell lymphoma from chronic lymphocytic leukemia/small lymphocytic lymphoma Am J Clin Pathol. 2001;116:893-7

Greiner TC.mRNA microarray analysis in lymphoma and leukemia. Cancer Treat Res. 2004;121:1-12

Guttmacher AE, Collins FS. Welcome to the genomic era N Engl J Med. 2003;349:996-8

Hamblin TJ, Davis Z, Gardiner A, Oscier DG, Stevenson FK Unmutated Ig V(H) genes are associated with a more aggressive form of chronic lymphocytic leukemia. Blood. 1999;94:1848-54

Hanahan D, Weinberg RA: The hallmarks of cancer. Cell 100:57-70, 2000

Harris NL, Jaffe ES, Diebold J, et al. The World Health Organization classification of neoplastic diseases of the hematopoietic and lymphoid tissues. Report of the Clinical Advisory Committee meeting, Airlie House, Virginia, November, 1997 Ann Oncol. 1999;10:1419-32

Harris NL, Jaffe ES, Diebold J, et al. World Health Organization classification of neoplastic diseases of the hematopoietic and lymphoid tissues: report of the Clinical Advisory Committee meeting, Airlie House, Virginia, November 1997 J Clin Oncol. 1999;17:3835-49

Harris NL, Jaffe ES, Stein H, et al A revised European-American classification of lymphoid neoplasms: a proposal from the International Lymphoma Study Group Blood. 1994;84:1361-92

Harris NL, Stein H, Coupland SE, Hummel M, Favera RD, Pasqualucci L, Chan WC New approaches to lymphoma diagnosis. Hematology (Am Soc Hematol Educ Program). 2001;194-220.

Heaney ML, Golde DW Myelodysplasia. N Engl J Med. 1999;340:1649-60

Jaffe ES, Harris NL, Stein H, Vardiman JW. WHO Classification Tumours of Heamotopoietic and lymphoid tissues. En Pathology and Genetics of tumours of Haematopoietic and lymphoid tissues. IARC Press. Lyon, 2001.

Jemal A, Murray T, Samuels A, et al: Cancer statistics, 2003. CA Cancer J Clin 53:5, 2003

Kottaridis PD, Gale RE, Frew ME, et al. The presence of a FLT3 internal tandem duplication in patients with acute myeloid leukemia (AML) adds important prognostic information to cytogenetic risk group and response to the first cycle of chemotherapy: analysis of 854 patients from the United Kingdom Medical Research Council AML 10 and 12 trials. Blood. 2001;98:1752-9

Kuppers R, Klein U, Hansmann ML, Rajewsky K. Cellular origin of human B-cell lymphomas. N Engl J Med. 1999;341:1520-9

Küppers R: Molecular biology of Hodgkin's lymphoma. Adv Cancer Res 2002; 84:277-312

Kyle RA: Monoclonal gammopathy of undetermined significance and solitary plasmacytoma: Implications for progression to overt multiple myeloma. Hematol Oncol Clin North Am 11:71, 1997

Lee MF, Dang CV Control of cell division. In Hoffman R Hematology Basic Principles and practice. 4 ed. Churchill Livingstone New York 2005; 69-81

Leung AYH, Verfaillie CM Stem cell model of hematopoyesis. In Hoffman R Hematology Basic Principles and practice. 4 ed. Churchill Livingstone New York 2005; 200-214

Magli MC, Largman C, Lawrence HJ Effects of HOX homeobox genes in blood cell differentiation J Cell Physiol. 1997; 173:168-77

Mitelman F, Mertens F, Johansson B A breakpoint map of recurrent chromosomal rearrangements in human neoplasia. Nat Genet. 1997;15 Spec No:417-74

Montoto S, Lopez-Guillermo A, Colomer D, et al. Incidence and clinical significance ofbcl-2/IgH rearrangements in follicular lymphoma. Leuk Lymphoma. 2003;44:71-6.

Nichogiannopoulou A, Trevisan M, Friedrich C, Georgopoulos K. Ikaros in hemopoietic lineage determination and homeostasis Semin Immunol. 1998;10:119-25.

Nutt SL, Heavey B, Rolink AG, Busslinger M. Commitment to the B-lymphoid lineage depends on the transcription factor Pax5. Nature. 1999;401:556-62

O'Gorman DM, Cotter TG Molecular signals in anti-apoptotic survival pathways. Leukemia. 2001;15:21-34

Pane F, Intrieri M, Quintarelli C, Izzo B, Muccioli GC, Salvatore F BCR/ABL genes and leukemic phenotype: from molecular mechanisms to clinical correlations. Oncogene. 2002; 21:8652-67.

R Development Core Team. R: A language and environment for statistical computing. R Foundation for Statistical Computing, Vienna, Austria, 2004

Rai KR, Sawitsky A, Cronkite EP, Chanana AD, Levy RN, Pasternack BS. Clinical staging of chronic lymphocytic leukemia. Blood. 1975;46:219-34

Roumier C, Eclache V, Imbert M, et al. M0 AML, clinical and biologic features of the disease, including AML1 gene mutations: A report of 59 cases by the Groupe Francais d'Hematologie Cellulaire (GFHC) and the Groupe Francais de Bytogenetique Hematologique (GFCH). Blood 101:1277, 2003

Shaffer AL, Rosenwald A, Staudt LM Lymphoid malignancies: the dark side of B-cell differentiation Nat Rev Immunol. 2002;2:920-32

Tenen DG, Hromas R, Licht JD, Zhang DE Transcription factors, normal myeloid development, and leucemia. Blood. 1997;90:489-519

Tibshirani R, Hastie T, Narasimhan B, Chu G. Diagnosis of multiple cancer types by shrunken centroids of gene expression. Proc. Natl. Acad. Sci. 2002;99:6567-72

Vardiman JW, Harris NL, Brunning RD The World Health Organization (WHO) classification of the myeloid neoplasms. Blood. 2002;100:2292-302

Venables, Smith et al. An Introduction to R, Notes on R: A programming Environment for Data Analysis. 2004

Weissman IL Stem cells: Units of regeneration and units in evolution. Cell, 2000; 100:157-168.

Westbrook CA. The molecular basis of neoplasia. In Hoffman R Hematology Basic Principles and practice. 4 ed. Churchill Livingstone New York 2005; 941-945

Zhan F, Hardin J, Kordsmeier B, et al: Global gene expression profiling of multiple myeloma, monoclonal gammopathy of undetermined significance, and normal bone marrow plasma cells. Blood 99:1745, 2002

### Brief description of the figures

Figure 1 shows the grouping of samples of cells U937 compared with Jurkat cells in accordance with differences in the gene expression between the samples. Part A corresponds to the non-supervised grouping; part B corresponds to the supervised grouping.
Figure 2 shows the grouping of samples of healthy subjects compared with U937 and Jurkat cells in accordance with differences in the gene expression between the samples. Part A corresponds to the non-supervised grouping; part B corresponds to the supervised grouping.
Figure 3 shows the grouping of samples of patients with chronic lymphatic leukemia compared with U937 and Jurkat cells in accordance with differences in the gene expression between the samples. Part A corresponds to the non-supervised grouping; part B corresponds to the supervised grouping.
Figure 4 shows the grouping of samples of patients with "stable" chronic lymphatic leukemia compared with samples of patients with "progressive" chronic lymphatic leukemia in accordance with differences in gene expression. Part A corresponds to the grouping in accordance with the genes identified as significant after normalization with "vsn" and use of the mt.maxT function in R; part B corresponds to the grouping in accordance with the genes identified as significant after normalization by robust quartiles and use of the mt.maxT function in R.
Figure 5 shows the distribution of the expression data obtained by RT-PCR (lefthand graphic) and from the intensity values obtained from the microarray (right-hand graphic) for the PSMB4 genes (part A: upper graphic), CD23A (part B: intermediate graphic) and POU2F2 (part C: lower graphics) in samples of patients with "stable" chronic lymphatic leukemia (bars marked with "E") and in samples of patients with "progressive" chronic lymphatic leukemia (bars marked with "P").

## Claims

1. A composition which comprises at least one oligonucleotide from the group composed of:
SG1, SG2, SG3, SG4, SG5, SG6, SG7, SG8, SG9, SG10, SG11, SG12, SG13, SG14, SG15, SG16, SG17, SG18, SG19, SG20, SG21, SG22, SG23, SG24, SG25, SG26, SG27, SG28, SG29, SG30, SG31, SG32, SG33, SG34, SG35, SG36, SG37, SG38, SG39, SG40, SG41, SG42, SG43, SG44, SG45, SG46, SG47, SG48, SG49, SG50, SG51, SG52, SG53, SG54, SG55, SG56, SG57, SG58, SG59, SG60, SG61, SG62, SG63, SG64, SG65, SG66, SG67, SG68, SG69, SG70, SG71, SG72, SG73, SG74, SG75, SG76, SG77, SG78, SG79, SG80, SG81, SG82, SG83, SG84, SG85, SG86, SG87, SG88, SG89, SG90, SG91, SG92, SG93, SG94, SG95, SG96, SG97, SG98, SG99, SG100, SG101, SG102, SG103, SG104, SG105, SG106, SG107, SG108, SG109, SG110, SG111, SG112, SG113, SG114, SG115, SG116, SG117, SG118, SG119, SG120, SG121, SG122, SG123, SG124, SG125, SG126, SG127, SG128, SG129, SG130, SG131, SG132, SG133, SG134, SG135, SG136, SG137, SG138, SG139, SG140, SG141, SG142, SG143, SG144, SG145, SG146, SG147, SG148, SG149, SG150, SG151, SG152, SG153, SG154, SG155, SG156, SG157, SG158, SG159, SG160, SG161, SG162, SG163, SG164, SG165, SG166, SG167, SG168, SG169, SG170, SG171, SG172, SG173, SG174, SG175, SG176, SG177, SG178, SG179, SG180, SG181, SG182, SG183, SG184, SG185, SG186, SG187, SG188, SG189, SG190, SG191, SG192, SG193, SG194, SG195, SG196, SG197, SG198, SG199, SG200, SG201, SG202, SG203, SG204, SG205, SG206, SG207, SG208, SG209, SG210, SG211, SG212, SG213, SG214, SG215, SG216, SG217, SG218, SG219, SG220, SG221, SG222, SG223, SG224, SG225, SG226, SG227, SG228, SG229, SG230, SG231, SG232, SG233, SG234, SG235, SG236, SG237, SG238, SG239, SG240, SG241, SG242, SG243, SG244, SG245, SG246, SG247, SG248, SG249, SG250, SG251, SG252, SG253, SG254, SG255, SG256, SG257, SG258, SG259, SG260, SG261, SG262, SG263, SG264, SG265, SG266, SG267, SG268, SG269, SG270, SG271, SG272, SG273, SG274, SG275, SG276, SG277, SG278, SG279, SG280, SG281, SG282, SG283, SG284, SG285, SG286, SG287, SG288, SG289, SG290, SG291, SG292, SG293, SG294, SG295, SG296, SG297, SG298, SG299, SG300, SG301, SG302, SG303, SG304, SG305, SG306, SG307, SG308, SG309, SG310, SG311, SG312, SG313, SG314, SG315, SG316, SG317, SG318, SG319, SG320, SG321, SG322, SG323, SG324, SG325, SG326, SG327, SG328, SG329, SG330, SG331, SG332, SG333, SG334, SG335, SG336, SG337, SG338, SG339, SG340, SG341, SG342, SG343, SG344, SG345, SG346, SG347, SG348, SG349, SG350, SG351, SG352, SG353, SG354, SG355, SG356, SG357, SG358, SG359, SG360, SG361, SG362, SG363, SG364, SG365, SG366, SG367, SG368, SG369, SG370, SG371, SG372, SG373, SG374, SG375, SG376, SG377, SG378, SG379, SG380, SG381, SG382, SG383, SG384, SG385, SG386, SG387, SG388, SG389, SG390, SG391, SG392, SG393, SG394, SG395, SG396, SG397, SG398, SG399, SG400, SG401, SG402, SG403, SG404, SG405, SG406, SG407, SG408, SG409, SG410, SG411, SG412, SG413, SG414, SG415, SG416, SG417, SG418, SG419, SG420, SG421, SG422, SG423, SG424, SG425, SG426, SG427, SG428, SG429, SG430, SG431, SG432, SG433, SG434, SG435, SG436, SG437, SG438, SG439, SG440, SG441, SG442, SG443, SG444, SG445, SG446, SG447, SG448, SG449, SG450, SG451, SG452, SG453, SG454, SG455, SG456, SG457, SG458, SG459, SG460, SG461, SG462, SG465, SG468, SG469, SG470, SG471, SG472, SG473, SG474, SG475, SG476, SG477, SG478, SG479, SG480, SG481, SG482, SG483, SG484, SG485, SG486, SG487, SG488, SG489, SG490, SG491, SG492, SG493, SG494, SG495, SG496, SG497, SG498, SG499, SG500, SG501, SG502, SG503, SG504, SG505, SG506, SG507, SG508, SG509, SG510, SG511, SG512, SG513, SG514, SG515, SG516, SG517, SG518, SG519, SG520, SG521, SG522, SG523, SG524, SG525, SG526, SG527, SG428, SG529, SG530, SG531, SG532, SG533, SG534, SG535, SG536, SG537, SG538, SG539, SG540, SG541, SG542, SG543, SG544, SG545, SG546, SG547, SG548, SG549, SG550, SG551, SG552, SG553, SG554, SG555, SG556, SG557, SG558, SG559, SG560, SG561, SG562, SG563,
or combinations thereof, to be used as probe in the determination of the expression level of a gene which possesses a sequence complementary to said oligonucleotide by the evaluation of the mRNA level corresponding to that gene, of application in *the in vitro* diagnosis of neoplasias originating from hematopoietic cells and/or in *the in vitro* prognosis of the evolution of said disease.

2. A composition according to claim 1, which comprises at least the oligonucleotides SG117, SG428, SG459, SG507, SG508.

3. A composition according to claim 2, which additionally comprises at least oligonucleotides SG461 and SG493.

4. A composition according to claim 2, which additionally comprises at least the oligonucleotide SG237.

5. A composition according to claim 4, which additionally comprises at least one oligonucleotide selected from the group of SG2, SG4, SG8, SG10, SG13, SG15, SG16, SG19, SG20, SG23, SG26, SG28, SG31, SG34, SG36, SG39, SG48, SG58, SG60, SG65, SG76, SG77, SG84, SG89, SG94, SG9, SG97, SG99, SG102, SG106, SG107, SG463, SG115, SG116, SG120, SG129, SG134, SG135, SG138, SG139, SG141, SG145, SG158, SG161, SG163, SG176, SG178, SG185, SG207, SG208, SG210, SG217, SG227, SG231, SG237, SG264, SG272, SG277, SG281, SG283, SG286, SG294, SG298, SG299, SG307, SG308, SG319, SG328, SG330, SG333, SG336, SG342, SG344, SG345, SG347, SG361, SG384, SG389, SG395, SG404, SG407, SG416, SG423, SG430, SG432, SG434, SG444, SG446, SG453, SG458, SG464, SG465, SG466, SG467, SG471, SG473, SG474, SG475, SG481, SG485, SG487, SG491, SG498, SG511, SG517, SG518, SG522, SG526, SG530, SG533, SG538, SG541, SG554, SG558, SG561 or combinations thereof.

6. A composition according to any of claims 2 to 5, to be used in *the in vitro* diagnosis of chronic lymphatic leukemia.

7. A composition according to claim 1, which comprises at least oligonucleotides SG26, SG216, SG366.

8. A composition according to claim 7, which additionally comprises at least one oligonucleotide selected from the group of SG31, SG177, SG194, SG195, SG197, SG213, SG293, SG301, SG309, SG333, SG343, SG357, SG439, SG452, SG555, SG556.

9. A composition according to any of claims 7 or 8, to be used in *the in vitro* prognosis of the future evolution of the disease in a patient suffering from chronic lymphatic leukemia.

10. A composition according to claim 1, which comprises the totality of the nucleotides of the group composed of:
SG1, SG2, SG3, SG4, SG5, SG6, SG7, SG8, SG9, SG10, SG11, SG12, SG13, SG14, SG15, SG16, SG17, SG18, SG19, SG20, SG21, SG22, SG23, SG24, SG25, SG26, SG27, SG28, SG29, SG30, SG31, SG32, SG33, SG34, SG35, SG36, SG37, SG38, SG39, SG40, SG41, SG42, SG43, SG44, SG45, SG46, SG47, SG48, SG49, SG50, SG51, SG52, SG53, SG54, SG55, SG56, SG57, SG58, SG59, SG60, SG61, SG62, SG63, SG64, SG65, SG66, SG67, SG68, SG69, SG70, SG71, SG72, SG73, SG74, SG75, SG76, SG77, SG78, SG79, SG80, SG81, SG82, SG83, SG84, SG85, SG86, SG87, SG88, SG89, SG90, SG91, SG92, SG93, SG94, SG95, SG96, SG97, SG98, SG99, SG100, SG101, SG102, SG103, SG104, SG105, SG106, SG107, SG108, SG109, SG110, SG111, SG112, SG113, SG114, SG115, SG116, SG117, SG118, SG119, SG120, SG121, SG122, SG123, SG124, SG125, SG126, SG127, SG128, SG129, SG130, SG131, SG132, SG133, SG134, SG135, SG136, SG137, SG138, SG139, SG140, SG141, SG142, SG143, SG144, SG145, SG146, SG147, SG148, SG149, SG150, SG151, SG152, SG153, SG154, SG155, SG156, SG157, SG158, SG159, SG160, SG161, SG162, SG163, SG164, SG165, SG166, SG167, SG168, SG169, SG170, SG171, SG172, SG173, SG174, SG175, SG176, SG177, SG178, SG179, SG180, SG181, SG182, SG183, SG184, SG185, SG186, SG187, SG188, SG189, SG190, SG191, SG192, SG193, SG194, SG195, SG196, SG197, SG198, SG199, SG200, SG201, SG202, SG203, SG204, SG205, SG206, SG207, SG208, SG209, SG210, SG211, SG212, SG213, SG214, SG215, SG216, SG217, SG218, SG219, SG220, SG221, SG222, SG223, SG224, SG225, SG226, SG227, SG228, SG229, SG230, SG231, SG232, SG233, SG234, SG235, SG236, SG237, SG238, SG239, SG240, SG241, SG242, SG243, SG244, SG245, SG246, SG247, SG248, SG249, SG250, SG251, SG252, SG253, SG254, SG255, SG256, SG257, SG258, SG259, SG260, SG261, SG262, SG263, SG264, SG265, SG266, SG267, SG268, SG269, SG270, SG271, SG272, SG273, SG274, SG275, SG276, SG277, SG278, SG279, SG280, SG281, SG282, SG283, SG284, SG285, SG286, SG287, SG288, SG289, SG290, SG291, SG292, SG293, SG294, SG295, SG296, SG297, SG298, SG299, SG300, SG301, SG302, SG303, SG304, SG305, SG306, SG307, SG308, SG309, SG310, SG311, SG312, SG313, SG314, SG315, SG316, SG317, SG318, SG319, SG320, SG321, SG322, SG323, SG324, SG325, SG326, SG327, SG328, SG329, SG330, SG331, SG332, SG333, SG334, SG335, SG336, SG337, SG338, SG339, SG340, SG341, SG342, SG343, SG344, SG345, SG346, SG347, SG348, SG349, SG350, SG351, SG352, SG353, SG354, SG355, SG356, SG357, SG358, SG359, SG360, SG361, SG362, SG363, SG364, SG365, SG366, SG367, SG368, SG369, SG370, SG371, SG372, SG373, SG374, SG375, SG376, SG377, SG378, SG379, SG380, SG381, SG382, SG383, SG384, SG385, SG386, SG387, SG388, SG389, SG390, SG391, SG392, SG393, SG394, SG395, SG396, SG397, SG398, SG399, SG400, SG401, SG402, SG403, SG404, SG405, SG406, SG407, SG408, SG409, SG410, SG411, SG412, SG413, SG414, SG415, SG416, SG417, SG418, SG419, SG420, SG421, SG422, SG423, SG424, SG425, SG426, SG427, SG428, SG429, SG430, SG431, SG432, SG433, SG434, SG435, SG436, SG437, SG438, SG439, SG440, SG441, SG442, SG443, SG444, SG445, SG446, SG447, SG448, SG449, SG450, SG451, SG452, SG453, SG454, SG455, SG456, SG457, SG458, SG459, SG460, SG461, SG462, SG465, SG468, SG469, SG470, SG471, SG472, SG473, SG474, SG475, SG476, SG477, SG478, SG479, SG480, SG481, SG482, SG483, SG484, SG485, SG486, SG487, SG488, SG489, SG490, SG491, SG492, SG493, SG494, SG495, SG496, SG497, SG498, SG499, SG500, SG501, SG502, SG503, SG504, SG505, SG506, SG507, SG508, SG509, SG510, SG511, SG512, SG513, SG514, SG515, SG516, SG517, SG518, SG519, SG520, SG521, SG522, SG523, SG524, SG525, SG526, SG527, SG428, SG529, SG530, SG531, SG532, SG533, SG534, SG535, SG536, SG537, SG538, SG539, SG540, SG541, SG542, SG543, SG544, SG545, SG546, SG547, SG548, SG549, SG550, SG551, SG552, SG553, SG554, SG555, SG556, SG557, SG558, SG559, SG560, SG561, SG562, SG563.

11. A composition according to any of claims 1 to 10, **characterized in that** it additionally comprises at least one oligonucleotide selected from the group composed of SG463, SG464, SG466, SG467, SSPC1, SSPC2, SSPC3, SSPC4, SSPC5, SSPC6, SSPC7, SCN1, SCN2, SCN3, SCN5, SCN6, SCN7, SCN8, SCN10, SCN11, SCN12, SCN13, SC1, SC2, SC3, SC4, SC5, SC6 and SC7.

12. A composition according to claim 11, which comprises all the oligonucleotides from the group composed of SG463, SG464, SG466, SG467, SSPC1, SSPC2, SSPC3, SSPC4, SSPC5, SSPC6, SSPC7, SCN1, SCN2, SCN3, SCN5, SCN6, SCN7, SCN8, SCN10, SCN11, SCN12, SCN13, SC1, SC2, SC3, SC4, SC5, SC6 and SC7.

13. A composition according to any of claims 1 to 10, wherein the oligonucleotides are disposed on a solid support.

14. A composition according to claim 13, wherein the oligonucleotides are disposed in an ordered fashion on a solid support which is glass similar to a slide whereto the oligonucleotides are bound by covalent bonds, forming a microarray.

15. A composition in the form of microarray according to claim 14, which comprises the totality of the oligonucleotides from the group composed of
SG1, SG2, SG3, SG4, SG5, SG6, SG7, SG8, SG9, SG10, SG11, SG12, SG13, SG14, SG15, SG16, SG17, SG18, SG19, SG20, SG21, SG22, SG23, SG24, SG25, SG26, SG27, SG28, SG29, SG30, SG31, SG32, SG33, SG34, SG35, SG36, SG37, SG38, SG39, SG40, SG41, SG42, SG43, SG44, SG45, SG46, SG47, SG48, SG49, SG50, SG51, SG52, SG53, SG54, SG55, SG56, SG57, SG58, SG59, SG60, SG61, SG62, SG63, SG64, SG65, SG66, SG67, SG68, SG69, SG70, SG71, SG72, SG73, SG74, SG75, SG76, SG77, SG78, SG79, SG80, SG81, SG82, SG83, SG84, SG85, SG86, SG87, SG88, SG89, SG90, SG91, SG92, SG93, SG94, SG95, SG96, SG97, SG98, SG99, SG100, SG101, SG102, SG103, SG104, SG105, SG106, SG107, SG108, SG109, SG110, SG111, SG112, SG113, SG114, SG115, SG116, SG117, SG118, SG119, SG120, SG121, SG122, SG123, SG124, SG125, SG126, SG127, SG128, SG129, SG130, SG131, SG132, SG133, SG134, SG135, SG136, SG137, SG138, SG139, SG140, SG141, SG142, SG143, SG144, SG145, SG146, SG147, SG148, SG149, SG150, SG151, SG152, SG153, SG154, SG155, SG156, SG157, SG158, SG159, SG160, SG161, SG162, SG163, SG164, SG165, SG166, SG167, SG168, SG169, SG170, SG171, SG172, SG173, SG174, SG175, SG176, SG177, SG178, SG179, SG180, SG181, SG182, SG183, SG184, SG185, SG186, SG187, SG188, SG189, SG190, SG191, SG192, SG193, SG194, SG195, SG196, SG197, SG198, SG199, SG200, SG201, SG202, SG203, SG204, SG205, SG206, SG207, SG208, SG209, SG210, SG211, SG212, SG213, SG214, SG215, SG216, SG217, SG218, SG219, SG220, SG221, SG222, SG223, SG224, SG225, SG226, SG227, SG228, SG229, SG230, SG231, SG232, SG233, SG234, SG235, SG236, SG237, SG238, SG239, SG240, SG241, SG242, SG243, SG244, SG245, SG246, SG247, SG248, SG249, SG250, SG251, SG252, SG253, SG254, SG255, SG256, SG257, SG258, SG259, SG260, SG261, SG262, SG263, SG264, SG265, SG266, SG267, SG268, SG269, SG270, SG271, SG272, SG273, SG274, SG275, SG276, SG277, SG278, SG279, SG280, SG281, SG282, SG283, SG284, SG285, SG286, SG287, SG288, SG289, SG290, SG291, SG292, SG293, SG294, SG295, SG296, SG297, SG298, SG299, SG300, SG301, SG302, SG303, SG304, SG305, SG306, SG307, SG308, SG309, SG310, SG311, SG312, SG313, SG314, SG315, SG316, SG317, SG318, SG319, SG320, SG321, SG322, SG323, SG324, SG325, SG326, SG327, SG328, SG329, SG330, SG331, SG332, SG333, SG334, SG335, SG336, SG337, SG338, SG339, SG340, SG341, SG342, SG343, SG344, SG345, SG346, SG347, SG348, SG349, SG350, SG351, SG352, SG353, SG354, SG355, SG356, SG357, SG358, SG359, SG360, SG361, SG362, SG363, SG364, SG365, SG366, SG367, SG368, SG369, SG370, SG371, SG372, SG373, SG374, SG375, SG376, SG377, SG378, SG379, SG380, SG381, SG382, SG383, SG384, SG385, SG386, SG387, SG388, SG389, SG390, SG391, SG392, SG393, SG394, SG395, SG396, SG397, SG398, SG399, SG400, SG401, SG402, SG403, SG404, SG405, SG406, SG407, SG408, SG409, SG410, SG411, SG412, SG413, SG414, SG415, SG416, SG417, SG418, SG419, SG420, SG421, SG422, SG423, SG424, SG425, SG426, SG427, SG428, SG429, SG430, SG431, SG432, SG433, SG434, SG435, SG436, SG437, SG438, SG439, SG440, SG441, SG442, SG443, SG444, SG445, SG446, SG447, SG448, SG449, SG450, SG451, SG452, SG453, SG454, SG455, SG456, SG457, SG458, SG459, SG460, SG461, SG462, SG465, SG468, SG469, SG470, SG471, SG472, SG473, SG474, SG475, SG476, SG477, SG478, SG479, SG480, SG481, SG482, SG483, SG484, SG485, SG486, SG487, SG488, SG489, SG490, SG491, SG492, SG493, SG494, SG495, SG496, SG497, SG498, SG499, SG500, SG501, SG502, SG503, SG504, SG505, SG506, SG507, SG508, SG509, SG510, SG511, SG512, SG513, SG514, SG515, SG516, SG517, SG518, SG519, SG520, SG521, SG522, SG523, SG524, SG525, SG526, SG527, SG428, SG529, SG530, SG531, SG532, SG533, SG534, SG535, SG536, SG537, SG538, SG539, SG540, SG541, SG542, SG543, SG544, SG545, SG546, SG547, SG548, SG549, SG550, SG551, SG552, SG553, SG554, SG555, SG556, SG557, SG558, SG559, SG560, SG561, SG562, SG563.

16. A composition in the form of microarray according to claim 15, which additionally comprises at least one pair of oligonucleotides selected from that composed of the oligonucleotides SG463 and SG464 and that composed of the oligonucleotides SG466 and SG467, at least one oligonucleotide from the group composed of SSPC1, SSPC2, SSPC3, SSPC4, SSPC5, SSPC6 and SSPC7, at least one oligonucleotide from the group composed of SCN2, SCN3, SCN6, SCN8, SCN11, SCN12 and SCN13 and at least one oligonucleotide from the group composed of SC1, SC2, SC3, SC4, SC5, SC6, SC7, SCN1, SCN5, SCN7 and SCN10.

17. A composition in the form of microarray according to claim 16, which comprises the totality of the oligonucleotides from the group composed of SG463, SG464, SG466, SG467, SSPC1, SSPC2, SSPC3, SSPC4, SSPC5, SSPC6, SSPC7, SCN2, SCN3, SCN6, SCN8, SCN11, SCN12, SCN13, SC1, SC2, SC3, SC4, SC5, SC6, SC7, SCN1, SCN5, SCN7 and SCN10.

18. A composition in the form of microarray according to claim 17, which additionally comprises points lacking oligonucleotides wherein the solvent wherein the oligonucleotides are found on being deposited on said glass is bound to the glass.

19. A composition in the form of microarray according to claim 18, which comprises at least twelve copies of each one of the different oligonucleotides present therein, as well as at least twelve points lacking oligonucleotides wherein the solvent wherein the oligonucleotides are found on being deposited on said glass is bound to the glass.

20. A composition in the form of microarray according to any one of claims 18 to 19, wherein in the points lacking oligonucleotides the DMSO solvent is bound to the glass.

21. A composition in the form of microarray according to any of claims 15 to 20 to be used in *the in vitro* diagnosis of chronic lymphatic leukemia and/or for the in vitro prognosis of the evolution of said disease.

22. A device for *the in vitro* diagnosis of a neoplasia originating from hematopoietic cells and/or for *the in vitro* prognosis of the evolution thereof, which comprises a composition according to any one of claims 1 to 20.

23. A device for the diagnosis of a neoplasia originating from hematopoietic cells and/or for *the in vitro* prognosis of the evolution thereof according to claim 22, which comprises a composition in the form of microarray of any of claims 14 to 20.

24. A device for *the in vitro* diagnosis of a neoplasia originating from hematopoietic cells and/or for *the in vitro* prognosis of the evolution thereof according to claim 22, which comprises a composition in the form of microarray of any of claims 19 or 20.

25. A device for *the in vitro* diagnosis of a neoplasia originating from hematopoietic cells and/or for *the in vitro* prognosis of the evolution thereof according to any of claims 23 or 24, wherein the neoplasia which is diagnosed or a whose evolution a prognosis is made of is chronic lymphatic leukemia.

26. A method for *the in vitro* diagnosis of a neoplasia originating from hematopoietic cells *and*/*or in vitro* prognosis of the evolution thereof which comprises *the in vitro* detection from a biological sample and the statistical analysis of the expression level of at least one significant gene for classifying the sample associated or not to said neoplasia, a gene which is selected from the group composed of GABARAP, NPM3, ABCB1, ABCB4, ABCC3, ABCC5, ABCC6, ABHD1, ABL1, ACTN1, AF1q, AKR1A1, ALDH1A1, ALK, ANK2, ANPEP, ANXA6, ANXA7, APAF1, APEX, ARHGEF2, ARS2, ASNS, ATIC, ATM, ATP50, BAX, BCL10, BCL2, BCL2A1, BCL2L1, BCL2LAA, BCL3, BCL6, BCL7A, BCL7b, BCR, BECN1, BIK, BIRC3, BIRC5, BLMH, BLR1, BLVRB, BMI1, BMP6, BRMS1, BST2, BTG1, BUB1, C21 orf33, C5orf13, CA12, CALD1, CANP2, CASC3, CASP1, CASP3, CASP4, CASP5, CASP6, CASP7, CASP8, CASP9, CAST, CATSD, CBFA2T1, CBFB, CCNA1, CCNB1, CCND1, CCND2, CCND3, CCNE1, CCR6, CCR7, CCT6A, CD14, CD19, CD2, CD22, CD24, CD28, CD33, CD34, CD36, CD38, CD3E, CD4, CD44, CD47, CD48, CD5, CD58, CD59, CD6, CD7, CD79A, CD79B, CD8, CD81, CD83, CD86, CD9, CDA, CDC25A, CDC25B, CDK2, CDK4, CDK5R1, CDKN1A, CDKN1B, CDKN1C, CDKN2A, CDKN2B, CDKN2C, CDKN3, CDW52, CEBPA, CEBPB, CEBPD, CFL1, CKMT1, CKS2, CML66, COL3A1, COL4A6, CR2, CREB1, CREBBP, CRYAB, CSF2, CSF3, CSRP2, CTGF, CTSB, CUZD1, CXADR, CXCL9, CXCR3, CXCR4, CYC1, CYP1A1, CYP2A6, DAD-1, DAPK1, DCK, DDX6, DEK, DHFR, DLAD, DNAJA1, DNMT3B, DNTT, DOK1, DPF2, DPP4, DRG1, DRP2, E2F1, EB-1, EBI2, EDF1, EEF1A1, EEF1B2, EEF1D, EEF1G, EFNB1, EGFR, EGR1, EIF2B2, EIF3S2, EIF4B, EIF4E, EIF5A, ELF1, ELF4, ENPP1, EphA3, EPOR, ERBB2, ERBB4, ERCC1, ERCC2, ERCC3, ERCC5, ERCC6, ETS1, ETS2, ETV6, ETV7, EZH2, FABP5, FADD, FAIM3, FAM38A, FARP1, FAT, FCER2, FCGR3A, FCGR3B, FGFR1, FGFR3, FGR, FHIT, FKBP9, FLI1, FLJ22169, FLT3, FN1, FNTB, FOS, FUS, G1P2, GABPB2, GATA1, GATA2, GATA3, GCET2, GDI2, GGA3, GJA1, GLUD1, GNL3, GOT1, GRB2, GRIA3, GRK4, GSTP1, GSTT1, GUSB, GZMA, H2AFX, H3F3A, HCK, HELLS, HIF1A, HIST1H2BN, HLA-A, HLA-DPA1, HLA-DQA1, HLA-DRA, HLA-DRB3, HLF, HMMR, HNRPH3, HNRPL, HOXA10, HOXA9, HOXD8, HOXD9, HRAS, HSD17B1, HSPB1, IBSP, ICAM1, ICAM3, ID2, IER3, IFRD1, IGFBP2, IGFBP3, IGFIR, IGLV6-57, IL10, IL15, IL1B, IL2, IL2RA, IL3, IL32, IL3RA, IL4R, IL6, IL6R, IL8, ILF2, IRF1, IRF2, IRF4, IRF8, ITGA2, ITGA3, ITGA4, ITGA5, ITGA6, ITGAL, ITGAM, ITGAX, ITGB1, ITGB2, JAK1, JAK2, JUNB, KAI1, KIAA0247, KIAA0864, KIT, KLF1, KLF13, KRAS2, KRT18, LADH, LAG3, LASP1, LCK, LCP1, LEPR, LGALS3, LGALS7, LIF, LIMS1, LM02, LOC285148, LRP, LSP1, LYL1, LYN, LYZ, MAFB, MAFK, MAGEA1, MAL, MAP3K12, MAP4K1, MAPK10, MAZ, MBP1, MCL1, MCM3, MCM7, MDM2, MEIS1, MEN1, MERTK, MKI67, MLF1, MLF2, MLL, MLLT10, MME, MMP2, MMP7, MMP8, MMP9, MNDA, MPL, MPO, MRPL37, MS4A1, MTCP1, MUC-1, MX1, MYB, MYBL1, MYC, MYOD1, NCALD, NCAM1, NCL, NDP52, NDRG1, NDUFA1, NDUFB, NF1, NFATC1, NFIC, NFKB1, NFKB1A, NINJ1, NPM1, NR3C1, NUMA1, NXF1, ODC1, OGGI, OLIG2, OPRD1, p14ARF, P55CDC, PABPC1, PAX5, PAX6, PAX8, PBX1, PBX3, PCA1, PCD, PCNA, PDCD1, PDGFA, PDGFRB, PDHA1, PGF, PGRMC1, PICALM, PLA2G6, PLAU, PLK1, PLP, PLS3, PLZF, PML, PMM1, POLR2C, POU2F2, PPP1CC, PRAME, PRKCI, PRKCQ, PRKDC, PRL, PRTN3, PSMA5, PSMB4, PSMC5, PSMD7, PTEN, PTGS1, PTHLH, PTK2, PTK2B, PTN, PTPRCCD, PYGB, RAD51, RAF1, RAG1, RARA, RARB, RB1, RBBP4, RBBP6, RBBP8, RBP4, RET, RGS1, RGS1, RIS1, RORA, RPL17, RPL23A, RPL24, RPL36A, RPL37A, RPL41, RPS3, RPS5, RPS9, RUNX1, RXRA, S100A2, S100A8, SDC1, SDHD, SELE, SELL, SEPW1, SERPINA9, SERPINB5, SERPNINA9, SFTPB, SIAT4A, SLC7A5, SNRPB, SOSTDC1, SP1, SPI1, SPN, SPRR1A, SREBF1, SSBP1, STAT1, STAT3, STAT5B, SUMO1, TACSTD2, TAGLN2, TAL1, TBP, TCEB1, TCF1, TCF3, TCF7, TCL1A, TCRbeta, TEGT, TERF1, TERT, TFCP2, TFRC, THBS1, THPO, TIA-2, TIAM1, TK1, TLX1, TMEM4, TNF, TNFRSF10C, TNFRSF1A, TNFRSF25, TNFRSF5, TNFRSF6, TNFRSF8, TNFSF10, TNFSF5, TNFSF6, TOP2A, TOPORS, TP73, TRA@, TRADD, TRAF3, TRAP1,TRIB2, TXNRD1, UBE2C, UHRF1, UVRAG, VCAM1, VEGF, VPREB1, WBSCR20C, WNT16, WT1, XBP1, XP06, XRCC3, XRCC5, ZAP70, ZFPL1, ZNF42, ZNFN1A1, ZYX, 18S rRNA, 28S rRNA and whose expression level is determined by the evaluation of the concentration of its corresponding mRNA by the use of at least one probe which has a sequence complementary to a fragment of a strand of said gene, a probe which is selected from the group of oligonucleotides composed of:
SG1, SG2, SG3, SG4, SG5, SG6, SG7, SG8, SG9, SG10, SG11, SG12, SG13, SG14, SG15, SG16, SG17, SG18, SG19, SG20, SG21, SG22, SG23, SG24, SG25, SG26, SG27, SG28, SG29, SG30, SG31, SG32, SG33, SG34, SG35, SG36, SG37, SG38, SG39, SG40, SG41, SG42, SG43, SG44, SG45, SG46, SG47, SG48, SG49, SG50, SG51, SG52, SG53, SG54, SG55, SG56, SG57, SG58, SG59, SG60, SG61, SG62, SG63, SG64, SG65, SG66, SG67, SG68, SG69, SG70, SG71, SG72, SG73, SG74, SG75, SG76, SG77, SG78, SG79, SG80, SG81, SG82, SG83, SG84, SG85, SG86, SG87, SG88, SG89, SG90, SG91, SG92, SG93, SG94, SG95, SG96, SG97, SG98, SG99, SG100, SG101, SG102, SG103, SG104, SG105, SG106, SG107, SG108, SG109, SG110, SG111, SG112, SG113, SG114, SG115, SG116, SG117, SG118, SG119, SG120, SG121, SG122, SG123, SG124, SG125, SG126, SG127, SG128, SG129, SG130, SG131, SG132, SG133, SG134, SG135, SG136, SG137, SG138, SG139, SG140, SG141, SG142, SG143, SG144, SG145, SG146, SG147, SG148, SG149, SG150, SG151, SG152, SG153, SG154, SG155, SG156, SG157, SG158, SG159, SG160, SG161, SG162, SG163, SG164, SG165, SG166, SG167, SG168, SG169, SG170, SG171, SG172, SG173, SG174, SG175, SG176, SG177, SG178, SG179, SG180, SG181, SG182, SG183, SG184, SG185, SG186, SG187, SG188, SG189, SG190, SG191, SG192, SG193, SG194, SG195, SG196, SG197, SG198, SG199, SG200, SG201, SG202, SG203, SG204, SG205, SG206, SG207, SG208, SG209, SG210, SG211, SG212, SG213, SG214, SG215, SG216, SG217, SG218, SG219, SG220, SG221, SG222, SG223, SG224, SG225, SG226, SG227, SG228, SG229, SG230, SG231, SG232, SG233, SG234, SG235, SG236, SG237, SG238, SG239, SG240, SG241, SG242, SG243, SG244, SG245, SG246, SG247, SG248, SG249, SG250, SG251, SG252, SG253, SG254, SG255, SG256, SG257, SG258, SG259, SG260, SG261, SG262, SG263, SG264, SG265, SG266, SG267, SG268, SG269, SG270, SG271, SG272, SG273, SG274, SG275, SG276, SG277, SG278, SG279, SG280, SG281, SG282, SG283, SG284, SG285, SG286, SG287, SG288, SG289, SG290, SG291, SG292, SG293, SG294, SG295, SG296, SG297, SG298, SG299, SG300, SG301, SG302, SG303, SG304, SG305, SG306, SG307, SG308, SG309, SG310, SG311, SG312, SG313, SG314, SG315, SG316, SG317, SG318, SG319, SG320, SG321, SG322, SG323, SG324, SG325, SG326, SG327, SG328, SG329, SG330, SG331, SG332, SG333, SG334, SG335, SG336, SG337, SG338, SG339, SG340, SG341, SG342, SG343, SG344, SG345, SG346, SG347, SG348, SG349, SG350, SG351, SG352, SG353, SG354, SG355, SG356, SG357, SG358, SG359, SG360, SG361, SG362, SG363, SG364, SG365, SG366, SG367, SG368, SG369, SG370, SG371, SG372, SG373, SG374, SG375, SG376, SG377, SG378, SG379, SG380, SG381, SG382, SG383, SG384, SG385, SG386, SG387, SG388, SG389, SG390, SG391, SG392, SG393, SG394, SG395, SG396, SG397, SG398, SG399, SG400, SG401, SG402, SG403, SG404, SG405, SG406, SG407, SG408, SG409, SG410, SG411, SG412, SG413, SG414, SG415, SG416, SG417, SG418, SG419, SG420, SG421, SG422, SG423, SG424, SG425, SG426, SG427, SG428, SG429, SG430, SG431, SG432, SG433, SG434, SG435, SG436, SG437, SG438, SG439, SG440, SG441, SG442, SG443, SG444, SG445, SG446, SG447, SG448, SG449, SG450, SG451, SG452, SG453, SG454, SG455, SG456, SG457, SG458, SG459, SG460, SG461, SG462, SG465, SG468, SG469, SG470, SG471, SG472, SG473, SG474, SG475, SG476, SG477, SG478, SG479, SG480, SG481, SG482, SG483, SG484, SG485, SG486, SG487, SG488, SG489, SG490, SG491, SG492, SG493, SG494, SG495, SG496, SG497, SG498, SG499, SG500, SG501, SG502, SG503, SG504, SG505, SG506, SG507, SG508, SG509, SG510, SG511, SG512, SG513, SG514, SG515, SG516, SG517, SG518, SG519, SG520, SG521, SG522, SG523, SG524, SG525, SG526, SG527, SG428, SG529, SG530, SG531, SG532, SG533, SG534, SG535, SG536, SG537, SG538, SG539, SG540, SG541, SG542, SG543, SG544, SG545, SG546, SG547, SG548, SG549, SG550, SG551, SG552, SG553, SG554, SG555, SG556, SG557, SG558, SG559, SG560, SG561, SG562, SG563, or combinations thereof.

27. Method for *the in vitro* diagnosis of a neoplasia originating from hematopoietic cells and/or *the in vitro* prognosis of the evolution thereof according to claim 26, which additionally comprises a previous optional step of identification of genes significant for the classification of a sample as associated or not to a specific type of neoplasia originating from hematopoietic cells, a previous step which comprises the substeps of:
a) deciding the possible categories wherein the sample can be classified;
b) obtaining biological samples from individuals which have previously been assigned by a method different to that claimed to any of the possible classification categories, so that there are samples of each one of the possible categories;
c) obtaining the total mRNA of each one of the samples;
d) obtaining the corresponding total cRNA, labelled by a method which allows its subsequent detection, of at least one aliquot of each one of the samples of mRNA, an aliquot whereto is added before the obtainment of the cRNA at least one sequence of polyadenylated nucleotides of low homology with human genes for which it acts as internal positive control of the process;
e) adding to one of the aliquots of cRNA which are going to be used in step f) at least one oligonucleotide of low homology with human genes different from and not complementary to any possible sequence of nucleotides which have been added in step d), for which it acts as positive hybridization control;
f) hybridizing, in strict conditions, at least one aliquot of total cRNA of each one of the samples with at least one microarray which comprises at least two copies of each one of the oligonucleotides from the group composed of:
SG1, SG2, SG3, SG4, SG5, SG6, SG7, SG8, SG9, SG10, SG11, SG12, SG13, SG14, SG15, SG16, SG17, SG18, SG19, SG20, SG21, SG22, SG23, SG24, SG25, SG26, SG27, SG28, SG29, SG30, SG31, SG32, SG33, SG34, SG35, SG36, SG37, SG38, SG39, SG40, SG41, SG42, SG43, SG44, SG45, SG46, SG47, SG48, SG49, SG50, SG51, SG52, SG53, SG54, SG55, SG56, SG57, SG58, SG59, SG60, SG61, SG62, SG63, SG64, SG65, SG66, SG67, SG68, SG69, SG70, SG71, SG72, SG73, SG74, SG75, SG76, SG77, SG78, SG79, SG80, SG81, SG82, SG83, SG84, SG85, SG86, SG87, SG88, SG89, SG90, SG91, SG92, SG93, SG94, SG95, SG96, SG97, SG98, SG99, SG100, SG101, SG102, SG103, SG104, SG105, SG106, SG107, SG108, SG109, SG110, SG111, SG112, SG113, SG114, SG115, SG116, SG117, SG118, SG119, SG120, SG121, SG122, SG123, SG124, SG125, SG126, SG127, SG128, SG129, SG130, SG131, SG132, SG133, SG134, SG135, SG136, SG137, SG138, SG139, SG140, SG141, SG142, SG143, SG144, SG145, SG146, SG147, SG148, SG149, SG150, SG151, SG152, SG153, SG154, SG155, SG156, SG157, SG158, SG159, SG160, SG161, SG162, SG163, SG164, SG165, SG166, SG167, SG168, SG169, SG170, SG171, SG172, SG173, SG174, SG175, SG176, SG177, SG178, SG179, SG180, SG181, SG182, SG183, SG184, SG185, SG186, SG187, SG188, SG189, SG190, SG191, SG192, SG193, SG194, SG195, SG196, SG197, SG198, SG199, SG200, SG201, SG202, SG203, SG204, SG205, SG206, SG207, SG208, SG209, SG210, SG211, SG212, SG213, SG214, SG215, SG216, SG217, SG218, SG219, SG220, SG221, SG222, SG223, SG224, SG225, SG226, SG227, SG228, SG229, SG230, SG231, SG232, SG233, SG234, SG235, SG236, SG237, SG238, SG239, SG240, SG241, SG242, SG243, SG244, SG245, SG246, SG247, SG248, SG249, SG250, SG251, SG252, SG253, SG254, SG255, SG256, SG257, SG258, SG259, SG260, SG261, SG262, SG263, SG264, SG265, SG266, SG267, SG268, SG269, SG270, SG271, SG272, SG273, SG274, SG275, SG276, SG277, SG278, SG279, SG280, SG281, SG282, SG283, SG284, SG285, SG286, SG287, SG288, SG289, SG290, SG291, SG292, SG293, SG294, SG295, SG296, SG297, SG298, SG299, SG300, SG301, SG302, SG303, SG304, SG305, SG306, SG307, SG308, SG309, SG310, SG311, SG312, SG313, SG314, SG315, SG316, SG317, SG318, SG319, SG320, SG321, SG322, SG323, SG324, SG325, SG326, SG327, SG328, SG329, SG330, SG331, SG332, SG333, SG334, SG335, SG336, SG337, SG338, SG339, SG340, SG341, SG342, SG343, SG344, SG345, SG346, SG347, SG348, SG349, SG350, SG351, SG352, SG353, SG354, SG355, SG356, SG357, SG358, SG359, SG360, SG361, SG362, SG363, SG364, SG365, SG366, SG367, SG368, SG369, SG370, SG371, SG372, SG373, SG374, SG375, SG376, SG377, SG378, SG379, SG380, SG381, SG382, SG383, SG384, SG385, SG386, SG387, SG388, SG389, SG390, SG391, SG392, SG393, SG394, SG395, SG396, SG397, SG398, SG399, SG400, SG401, SG402, SG403, SG404, SG405, SG406, SG407, SG408, SG409, SG410, SG411, SG412, SG413, SG414, SG415, SG416, SG417, SG418, SG419, SG420, SG421, SG422, SG423, SG424, SG425, SG426, SG427, SG428, SG429, SG430, SG431, SG432, SG433, SG434, SG435, SG436, SG437, SG438, SG439, SG440, SG441, SG442, SG443, SG444, SG445, SG446, SG447, SG448, SG449, SG450, SG451, SG452, SG453, SG454, SG455, SG456, SG457, SG458, SG459, SG460, SG461, SG462, SG465, SG468, SG469, SG470, SG471, SG472, SG473, SG474, SG475, SG476, SG477, SG478, SG479, SG480, SG481, SG482, SG483, SG484, SG485, SG486, SG487, SG488, SG489, SG490, SG491, SG492, SG493, SG494, SG495, SG496, SG497, SG498, SG499, SG500, SG501, SG502, SG503, SG504, SG505, SG506, SG507, SG508, SG509, SG510, SG511, SG512, SG513, SG514, SG515, SG516, SG517, SG518, SG519, SG520, SG521, SG522, SG523, SG524, SG525, SG526, SG527, SG428, SG529, SG530, SG531, SG532, SG533, SG534, SG535, SG536, SG537, SG538, SG539, SG540, SG541, SG542, SG543, SG544, SG545, SG546, SG547, SG548, SG549, SG550, SG551, SG552, SG553, SG554, SG555, SG556, SG557, SG558, SG559, SG560, SG561, SG562, SG563, a microarray which additionally comprises:
a. at least two points which correspond to different aliquots of the solvent wherein nucleotides are found at the time of their deposit on the surface of the microarray, for which they serve as blank,
b. at least two copies of at least one oligonucleotide for each one of the polyadenylated sequences added in step d), an oligonucleotide whose sequence will correspond to a fragment, different from the polyadenylation zone, of the sequence of polyadenylated nucleotides whose evolution in the process has to be controlled;
c. for each one of the oligonucleotides added in step e), at least two copies of an oligonucleotide complementary thereto;
d. at least two copies of each member of at least one pair of oligonucleotides wherein the sequence of one of the members corresponds to a sequence of zone 5' and the sequence of the other corresponds to a sequence of zone 3' of the mRNA of a gene which is expressed in constitutive form in any cell of hematopoietic origin;
e. at least two copies of at least one oligonucleotide of low homology with human genes different from any of the oligonucleotides defined in section b. and different from any of the synthetic oligonucleotides added optionally in step e);
g) detecting and quantifying the signal of cRNA hybridized with each one of the copies of each one of the oligonucleotides present in the microarray, as well as the signal corresponding to the points of the solvent;
h) calculating the average level of intensity of hybridization of each one of the oligonucleotides of the microarray calculating the average of the intensities of the copies of each one of the oligonucleotides;
i) taking the hybridization as valid if the following conditions are complied with:
a. the ratio between the average intensity and the average background of all the oligonucleotides of the microarray is greater than 10;
b. the value of the average coefficient of variation of all the replicas of oligonucleotides should be less than 0.3;
c. the average value of negative control should be less than 2.5 times the average value of the points corresponding to the solvent;
d. there is a signal both in the hybridization controls and in the internal positive controls used as process control;
j) normalizing the data;
k) eliminating the oligonucleotides with values of average intensity minus average background noise less than approximately 2 times the average value obtained with the points corresponding to the solvent, as well as the oligonucleotides with an interquartile range of normalized intensity throughout the samples less than 0.3;
l) performing the statistical analysis to find the statistically significant oligonucleotides to differentiate between the different categories and be able to classify a sample which has not been previously assigned to any category, choosing said oligonucleotides among those which have not been eliminated in the previous steps, until obtaining "n" oligonucleotides which either have a value of p less than a limit which is chosen from the open range of 0 to 0.05, preferably using for it a method with capacity to reduce false positives, or that which best defines the category established;
m) checking that the grouping of the samples according to the differences in intensities between the different samples detected for the statistically significant oligonucleotides gives rise to the samples being classified in the same categories as those which had previously been assigned by a different method.

28. Method according to claim 27, wherein the microarray comprises at least four copies of each one of the oligonucleotides present in it and the average of the intensities of the copies of each one of the oligonucleotides which is calculated in h) is a trimmed mean.

29. Method according to claim 28, wherein the normalization is carried out using the "variance stabilization normalization" method available in the "vsn" package in R.

30. Method according to any of claims 27 to 29, wherein the statistical analysis to find the statistically significant oligonucleotides to differentiate between the different categories is carried out using the mt.maxT function of the multtest package in R.

31. Method according to any of claims 27 to 30, wherein a diagnostic device is used according to claim 24.

32. Method according to the claims 27 to 31, which comprises an optional step of obtainment of a classification function for each sample by the arbitrary assignment of the value of 0 to one of the possible categories "a" and of the value 1 to the other possible category "b" wherein it is possible to classify the sample and the obtainment by logistical regression of a coefficient for each one of the oligonucleotides which make it possible to calculate a value xᵢ for each sample by a function of the type: ${x}_{i} = constant + {\sum }_{m = 1}^{n} \left(coeff_{oliga}_{m}*{Imn}_{i_}⁢{olig}_{m}\right)$
where
coeff_oligₘ represents the coefficient calculated for a specific oligonucleotide "m"
Imni_oligₘ represents the average value of normalized intensity obtained in the hybridization of the sample i calculated for the oligonucleotide "m"
"m" varies from 1 to "n"
n is the total number of oligonucleotides considered significant value "xᵢ" wherefrom the probability "pᵢ" that a sample "i" belongs to one or another category is calculated using the formula pᵢ = 1/(1+e^{-xi}) and classifying the sample as belonging to category "a" or "b" according to its corresponding value pᵢ is closer to 0 or 1, respectively.

33. Method according to any of claims 27 to 29, wherein the statistical analysis to find the significant oligonucleotides to differentiate between the different categories is carried out using the "Nearest Shrunken Centroids" method.

34. Method according to any of claims 27 to 33, wherein the biological samples analysed *in vitro* are samples of peripheral blood.

35. Method according to claim 34, wherein a leukemia is diagnosed *in vitro* or a prognosis is made of the evolution thereof.

36. Method according to claim 35, wherein it is diagnosed *in vitro* if an individual suffers from chronic lymphatic leukemia.

37. Method according to claim 35, wherein *an in vitro* prognosis is made of the evolution of the chronic lymphatic leukemia in a subject classifying a sample of blood extracted therefrom as "associated to stable chronic lymphatic leukemia" or as "associated to progressive chronic lymphatic leukemia".

38. Method to make *an in vitro* diagnosis of a neoplasia originating from hematopoietic cells and/or *an in vitro* prognosis of the evolution thereof which comprises *the in vitro* detection and the statistical analysis of the expression level of at least one significant gene for classifying the sample as belonging to a healthy individual or associating it to a type of neoplasia originating from hematopoietic cells according to claim 26, wherein the neoplasia which is diagnosed and/or whose evolution a prognosis is made of is a leukemia.

39. Method according to claim 38, wherein a diagnosis/or prognosis is made of the evolution of the chronic lymphatic leukemia.

40. Method to make *an in vitro* diagnosis of chronic lymphatic leukemia and/or make *an in vitro* prognosis of its evolution according to claim 39, wherein the *in vitro* detection of the expression level of at least one significant gene is carried out from samples of peripheral blood.

41. Method to make *an in vitro* diagnosis of chronic lymphatic leukemia according to claim 40, wherein the subjects wherefrom the corresponding blood samples have been taken are classified in the category of subject not suffering from CLL or in the category of subject suffering from CLL.

42. Method to make *an in vitro* diagnosis of chronic lymphatic leukemia according to claim 41, wherein the classification of the subjects is carried out after *the in vitro* detection and the statistical analysis of the expression level in the corresponding blood samples of at least genes CD79A, FAIM3, HLA-DRA, HLA-DRB3, HLA-DQA1.

43. Method to make *an in vitro* diagnosis of chronic lymphatic leukemia according to claim 42, wherein the classification of the subjects is carried out after *the in vitro* detection and the statistical analysis of the expression level in the corresponding blood samples additionally of genes IRF8 and COL3A1.

44. Method to make *an in vitro* diagnosis of chronic lymphatic leukemia according to claim 43, wherein *the in vitro* detection and the statistical analysis of the expression level of genes CD79A, FAIM3, HLA-DRA, HLA-DRB3, HLA-DQA1, IRF8 and COL3A1 is carried out by the evaluation of the corresponding mRNA by hybridization of its corresponding cRNA using as probes the oligonucleotides SG117, SG428, SG459, SG507, SG508, SG461 and SG493.

45. Method to make *an in vitro* diagnosis of chronic lymphatic leukemia according to claim 44, wherein the oligonucleotides form part of a composition in the form of microarray.

46. Method to make *an in vitro* diagnosis of chronic lymphatic leukemia according to claim 45, wherein the evaluation of the hybridized cRNA is carried out thanks to the prior labelling of cRNA with biotin, the staining of the hybridized microarray with streptavidin conjugated with a fluorophore and the detection of the signal emitted by said fluorophore.

47. Method to make *an in vitro* diagnosis of chronic lymphatic leukemia according to claim 46, wherein the fluorophore is Cy3.

48. Method to make *an in vitro* diagnosis of chronic lymphatic leukemia according to claim 47, wherein the classification of a subject from which the sample I has been taken analysed in the category of subject not suffering from CLL or in the category of subject suffering from CLL is carried out by calculating for said subject a value of probability pᵢ = 1/(1+e-^{xi}) after obtaining its corresponding value of xᵢ by the formula ${x}_{i} = - 719.241486 + ( 2.44756372 * {Imn}_{i} _CD ⁢ 79 ⁢ A ) + ( 7.38657611 * { Imn}_{i} _FAIM ⁢ 3 ) + ( 23.1465464 * { Imn}_{i} _HLA - DRA ) + ( 43.6287742 * { Imn}_{i} _IRF ⁢ 8 ) - ( 19.3978182 * { Imn}_{i} _COL ⁢ 3 ⁢ A ⁢ 1 ) - ( 2.8028646 * {Imn}_{i} _HLA - DRB ⁢ 3 ) + ( 49.5345672 * { Imn}_{i} _HLA - DQA ⁢ 1 ) .$ formula wherein each one of the values called abbreviation "Imnᵢ" followed by the abbreviation of a gene makes reference to the average value of normalized intensity obtained after detecting the hybridization signal corresponding to the oligonucleotide which is being used as probe to evaluate the expression of the said gene
and classifying the subject as subject not suffering from CLL if the value of pᵢ is less than 0.5 and as subject suffering from CLL if the value of pᵢ is greater than 0.5.

49. Method to make *an in vitro* prognosis of the evolution of the disease in a subject suffering from chronic lymphatic leukemia according to claim 40, wherein the subjects from which the corresponding blood samples have been taken are classified in the category of subject with stable CLL or in the category of subject with progressive CLL.

50. Method to make *an in vitro* prognosis of the evolution of the disease in a subject suffering from chronic lymphatic leukemia according to claim 49, wherein the classification of the subjects is carried out after *the in vitro* detection and the statistical analysis of the expression level in the corresponding blood samples of at least genes PSMB4, FCER2 and POU2F2.

51. Method to make *an in vitro* prognosis of the evolution of the disease in a subject suffering from chronic lymphatic leukemia according to claim 50, wherein the classification of the subjects is carried out after *the in vitro* detection and the statistical analysis of the expression level in the corresponding blood samples additionally of at least one gene selected from the group composed of ODC1, CD79A, CD2, CD3E, CD5, MS4A1, EIF4E, FHIT, NR3C1, LCP1, MAPK10, ABCC5, XRCC3, CML66, PLZF, RBP4.

52. Method to make *an in vitro* prognosis of the evolution of the disease in a subject suffering from chronic lymphatic leukemia according to claim 51, wherein the classification of the subjects is carried out after *the in vitro* detection and the statistical analysis of the expression level in the corresponding blood samples of at least the genes of the group composed of PSMB4, FCER2, POU2F2, ODC1, CD79A, CD2, CD3E, CDS, MS4A1, EIF4E, FHIT, NR3C1, LCP1, MAPK10, ABCC5, XRCC3, CML66, PLZF, RBP4.

53. Method to make an in vitro prognosis of the evolution of the disease in a subject suffering from chronic lymphatic leukemia according to either of claims 51 or 52, wherein *the in vitro* detection and the statistical analysis of the expression level of the genes examined is carried out by the evaluation of the corresponding mRNA by hybridization of its corresponding cRNA using as probes the corresponding oligonucleotides selected from the group composed of SG26, SG216, SG366, SG31, SG177, SG194, SG195, SG197, SG213, SG293, SG301, SG309, SG33, SG343, SG357, SG439, SG452, SG555, SG556.

54. Method to make *an in vitro* prognosis of the evolution of the disease in a subject suffering from chronic lymphatic leukemia according to claim 53, wherein the oligonucleotides form part of a composition in the form of microarray.

55. Method to make *an in vitro* prognosis of the evolution of the disease in a subject suffering from chronic lymphatic leukemia according to claim 54, wherein the evaluation of the corresponding mRNA of the sample analysed by the detection of the corresponding hybridized cRNA to the corresponding oligonucleotide is carried out thanks to the previous labelling of the cRNA with biotin, the staining of the microarray hybridized with streptavidin conjugated with a fluorophore and the detection of the signal emitted by said fluorophore.

56. Method to make *an in vitro* prognosis of the evolution of the disease in an individual suffering from chronic lymphatic leukemia according to claim 55, wherein the fluorophore is Cy3.

57. Use of a device for evaluation of the expression level of at least one gene of the group composed of PSMB4, FCER2, POU2F2, ODC1, CD79A, CD2, CD3E, CD5, MS4A1, EIF4E, FHIT, NR3C1, LCP1, MAPK10, ABCC5, XRCC3, CML66, PLZF, RBP4, CD79A, FAIM3, HLA-DRA, HLA-DRB3, HLA-DQA1, IRF8 and COL3A1 for *the in vitro* diagnosis of the existence of chronic lymphatic leukemia in a subject and/or for *the in vitro* prognosis of the evolution of the chronic lymphatic leukemia in a subject.

58. Use of a device for evaluation of the expression level of genes according to claim 57, wherein the expression level of at least one gene of the group composed of CD79A, FAIM3, HLA-DRA, HLA-DRB3, HLA-DQA1, IRF8 and COL3A1 is evaluated for *the in vitro* diagnosis of the existence of chronic lymphatic leukemia in a subject.

59. Use of a device for evaluation of the expression level of genes according to any of claims 57 and 58, wherein the expression level of at least genes CD79A, FAIM3, HLA-DRA, HLA-DRB3, HLA-DQA1 is evaluated for *the in vitro* diagnosis of the existence of chronic lymphatic leukemia in a subject.

60. Use of a device for evaluation of the expression level of genes according to claim 59, wherein additionally the expression level of at least genes IRF8 and COL3A1 are evaluated for *the in vitro* diagnosis of the existence of chronic lymphatic leukemia in a subject.

61. Use of a device for evaluation of the expression level of genes according to claim 57, wherein the expression level of at least one gene of the group composed of PSMB4, FCER2, POU2F2, ODC1, CD79A, CD2, CD3E, CD5, MS4A1, EIF4E, FHIT, NR3C1, LCP1, MAPK10, ABCC5, XRCC3, CML66, PLZF, RBP4 is evaluated, to make *an in vitro* prognosis of the evolution of the disease in a subject suffering from chronic lymphatic leukemia.
